# EUROPEAN PATENT SPECIFICATION

(11) **EP 2 791 137 B1**
(45) Date of publication and mention of the grant of the patent: **23.01.2019**
(21) Application number: 12808328.4
(22) Date of filing: 13.12.2012
(51) Int. Cl.: C07D 471/04, A61K 31/437, A61P 35/00, A61P 35/04

(54) **PYRAZOLOPYRIDINE DERIVATIVES, PREPARATION PROCESS THEREFOR AND THERAPEUTIC USE THEREOF**
PYRAZOLOPYRIDINDERIVATE, HERSTELLUNGSVERFAHREN DAFÜR UND THERAPEUTISCHE VERWENDUNG DAVON
DÉRIVÉS DE PYRAZOLOPYRIDINE, PROCÉDÉ D'ÉLABORATION DE CES DÉRIVÉS, ET UTILISATION THÉRAPEUTIQUE DE CEUX-CI

(30) Priority: 14.12.2011 FR 1161589
(43) Date of publication of application: 22.10.2014
(73) Proprietor: SANOFI, 75008 Paris (FR)
(72) Inventor: ALCOUFFE, Chantal, F-75008 Paris (FR); BJEGARDE, Kirsten, Bridgewater, NJ 08807 (US); MAUGER, Jacques, Bridgewater, NJ 08807 (US)
(74) Representative: Nony
(86) International application number: PCT/EP2012/075328
(87) International publication number: WO 2013/087744

(56) References cited:
- WO-A1-2006/050076
- WO-A1-2006/130673
- WO-A1-2008/028617
- WO-A1-2010/078427
- WO-A1-2011/045344
- WO-A2-2006/124863
- WO-A2-2009/038385
- WO-A2-2009/069032
- US-A1- 2008 182 844
- US-A1- 2009 030 010
- CHEBANOV V. A. ET AL.: "Cyclocondensation reactions of 5-aminopyrazoles, pyruvic acids and aldehydes. Multicomponent approaches to pyrazolopyridines and related products", TETRAHEDRON, vol. 63, 11 December 2006 (2006-12-11), pages 1229-1242,

## Description

The present invention relates to pyrazolopyrimidine derivatives that inhibit the FGF (Fibroblast Growth Factor) receptors, to a process for preparing them and to the therapeutic use thereof.

FGFs are a family of polypeptides synthesized by a large number of cells during embryonic development and by adult tissue cells under various pathological conditions.

The present invention discloses compounds corresponding to formula (I): 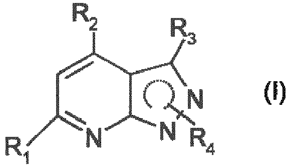 in which:
the representation of the pyrazole ring indicates that the substituent R₄ may be borne either by the nitrogen alpha to the pyridine ring (I') or by the nitrogen alpha to the carbon bearing a substituent R₃ (I") such that: 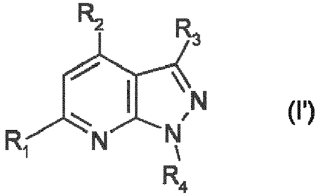 Either 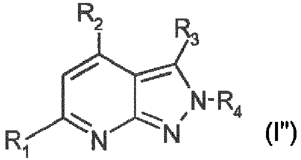
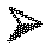 R₁ represents an aryl, pyridyl or pyrazolyl group optionally substituted with one or more substituents chosen from:
   ∘ a halogen atom,
   ∘ a -CF3 group,
   ∘ a cyano group,
   ∘ a group -NReRe' in which R₆ and R₆'are as defined below,
   ∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom so as advantageously to form a pyrazole, morpholine, pyrrolidine or piperidine, optionally substituted with one or more substituents chosen from a halogen atom and a linear or branched alkyl group,
   ∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom so as advantageously to form a morpholinyl group,
   ∘ a group -COR₁₂ in which R₁₂ represents a hydroxyl group or a group -NR₆R₆', in which R₆ and R₆' are as defined below,
   ∘ a group -CONR₇R₇' such that R₇ and R₇' form, together with the nitrogen atom to which they are attached, a heterocycloalkyl comprising one or more heteroatoms chosen from a nitrogen atom and an oxygen atom so as advantageously to form a pyrrolidinyl group,
   ∘ a group -(CH₂)ₚNHSO₂CH₃ in which p represents 0 or 1,
   ∘ a group -OR₁₃ in which R₁₃ represents a linear group (C₁-C₃)alkyl,
   ∘ a group (C₁-C₃)alkyl,
   Or R₁ represents a bicyclic group of formula A below: 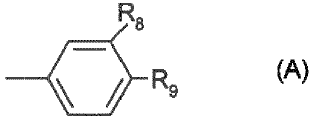 in which R₈ and R₉ form, together with the carbon atoms to which they are attached, a saturated or unsaturated heterocycle comprising one or more heteroatoms chosen from a nitrogen atom, an oxygen atom and a sulfur atom, such that the group (A) advantageously forms a dihydrobenzimidazolonyl, indolyl, dihydrobenzoxazinyl, benzothiazolyl or benzimidazolyl group, optionally substituted with one or more linear alkyl groups,
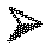 R2 represents:
   ∘ a -CF₃ group,
   ∘ a -CHF₂ group,
   ∘ a -COOH group,
   or
   ∘ a group -CONHR₅, in which R₅ is as defined below,
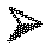 R₃ represents:
   ∘ a hydrogen atom,
   ∘ an aryl group, optionally substituted with an alkoxymethyl group,
   ∘ a cycloalkyl group,
   or
   ∘ a heteroaryl group chosen from thienyl and pyridyl groups,
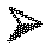 R₄ represents:
   ∘ a hydrogen atom,
   ∘ a linear group (C₁-C₃)alkyl, optionally substituted with a group -NR₆R'₆ in which Re and R'₆ are as defined below or a group - NR₇R₇' such that R₇ and R_{7'} form, together with the nitrogen atom to which they are attached, a heterocycloalkyl comprising one or more heteroatoms chosen from a nitrogen atom and an oxygen atom,
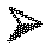 R₅ represents:
   ∘ a hydrogen atom,
   ∘ a linear group (C₁-C₃)alkyl, optionally substituted with a pyridyl group,
   or
   ∘ an aromatic group chosen from aryl and pyridyl,
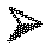 R₆ and R'₆, which may be identical or different, represent a hydrogen atom or a linear alkyl group,
in the form of the base or of an acid-addition or base-addition salt.

In particular, the present invention relates to compound corresponding to formula (I): 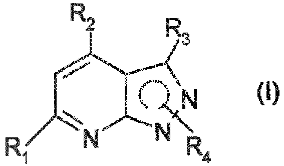 in which:
the representation of the pyrazole ring indicates that the substituent R₄ may be borne either by the nitrogen alpha to the pyridine ring (I') or by the nitrogen alpha to the carbon bearing a substituent R₃ (I") such that: 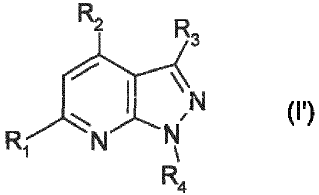 Either 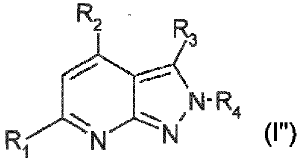
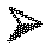 R₁ represents an aryl, pyridyl or pyrazolyl group optionally substituted with one or more substituents chosen from:
   ∘ a fluorine atom,
   ∘ a group -CF₃,
   ∘ a cyano group,
   ∘ a group -NR₆R₆' in which R₆ and R₆' are as defined below,
   ∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
   ∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
   ∘ a group -COR₁₂ in which R₁₂ represents a hydroxyl group or a group -NR₆R₆', in which Re and R₆' are as defined below,
   ∘ a group -CONR₇R₇' such that R₇ and R₇' form, together with the nitrogen atom to which they are attached, a heterocycloalkyl comprising one or more heteroatoms chosen from a nitrogen atom and an oxygen atom,
   ∘ a group -(CH₂)ₚNHSO₂CH₃ in which p represents 0 or 1,
   ∘ a group -OR₁₃ in which R₁₃ represents a linear group (C₁-C₃)alkyl,
   ∘ a group ethyl, propyl or isopropyl,
      Or R₁ represents a bicyclic group of formula A below: 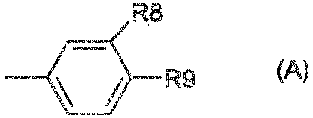 in which R₈ and R₉ form, together with the carbon atoms to which they are attached, a saturated or unsaturated heterocycle comprising one or more heteroatoms chosen from a nitrogen atom, an oxygen atom and a sulfur atom, such that the group (A) forms a dihydrobenzimidazolonyl, indolyl, dihydrobenzoxazinyl, benzothiazolyl or benzimidazolyl group, optionally substituted with one or more linear alkyl groups,
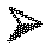 R₂ represents a group:
   ∘ -CF₃,
   ∘ -CHF₂,
   or
   ∘ -CONHR₅, in which R₅ is as defined below,
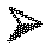 R₃ represents:
   ∘ a hydrogen atom,
   ∘ an aryl group, optionally substituted with an alkoxymethyl group,
   or
   ∘ a heteroaryl group chosen from thienyl and pyridyl groups,
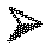 R₄ represents:
   ∘ a hydrogen atom,
   ∘ a (C₁)alkyl, linear (C₃)alkyl, or linear (C₁-C₃)alkyl substituted with a group - NR₆R_{6'} in which R₆ and R'₆ are as defined below or a group -NR₇R_{7'} such that R₇ and R₇' form, together with the nitrogen atom to which they are attached, a heterocycloalkyl comprising one or more heteroatoms chosen from a nitrogen atom and an oxygen atom,
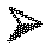 R₅ represents:
   ∘ a hydrogen atom,
   ∘ a linear group (C₁-C₃)alkyl, optionally substituted with a pyridyl group,
      or
   ∘ an aromatic group chosen from aryl and pyridyl,
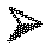 R₆ and R'₆, which may be identical or different, represent a hydrogen atom or a linear alkyl group,
in the form of the base or of an acid-addition or base-addition salt, with the exception of the following compounds:
2-Fluoro-5-(3-phenyl-4-trifluoromethyl-1H-pyrazolo[3,4-b]pyridin-6-yl)benzoic acid;
3-(1-Methyl-3-phenyl-4-trifluoromethyl-1H-pyrazolo[3,4-b]pyridin-6-yl)benzamide;
N-[4-(1-Methyl-3-phenyl-4-trifluoromethyl-1H-pyrazolo[3,4-b]pyridin-6-yl)benzyl]methanesulfonamide;
1-Methyl-6-(1-methyl-1H-indol-6-yl)-3-phenyl-4-trifluoromethyl-1H-pyrazolo[3.4-b]pyridine;
N-[3-(1-Methyl-3-phenyl-4-trifluoromethyl-1H-pyrazolo[3,4-b]pyridin-6-yl)phenyl]methanesulfonamide;
4-Methyl-7-(1-methyl-3-phenyl-4-trifluoromethyl-1H-pyrazolo[3,4-b]pyridin-6-yl)-3,4-dihydro-2H-benzo[1,4]oxazine;
N-[3-(1-Methyl-3-phenyl-4-trifluoromethyl-1H-pyrazolo[3,4-b]pyridin-6-yl)benzyl]methanesulfonamide;
6-(4-Methoxyphenyl)-1-methyl-3-phenyl-4-trifluoromethyl-1H-pyrazolo[3,4-b]pyridine;
2-Fluoro-N-methyl-5-(1-methyl-3-phenyl-4-trifluoromethyl-1H-pyrazolo[3,4-b]pyridin-6-yl)benzamide;
Dimethyl[3-(1-methyl-3-phenyl-4-trifluoromethyl-1H-pyrazolo[3,4-b]pyridin-6-yl)phenyl]amine;
6-[4-(3,5-Dimethylpyrazol-1-yl)phenyl]-1-methyl-3-phenyl-4-trifluoromethyl-1H-pyrazolo[3,4-b]pyridine;
1-Methyl-6-(3-morpholin-4-ylmethylphenyl)-3-phenyl-4-trifluoromethyl-1H-pyrazolo[3,4-b]pyridine;
5-(1-Methyl-3-phenyl-4-trifluoromethyl-1H-pyrazolo[3,4-b]pyridin-6-yl)nicotinonitrile;
4-(1-Methyl-3-phenyl-4-trifluoromethyl-1H-pyrazolo[3,4-b]pyridin-6-yl)benzoic acid;
N,N-Dimethyl-4-(1-methyl-3-phenyl-4-trifluoromethyl-1H-pyrazolo[3,4-b]pyridin-6-yl)benzamide;
N,N-Dimethyl-3-(1-methyl-3-phenyl-4-trifluoromethyl-1H-pyrazolo[3,4-b]pyridin-6-yl)benzamide;
1-Methyl-6-(6-morpholin-4-ylpyridin-3-yl)-3-phenyl-4-trifluoromethyl-1H-pyrazolo[3,4-b]pyridine;
6-(6-Methoxypyridin-3-yl)-1-methyl-3-phenyl-4-trifluoromethyl-1H-pyrazolo[3,4-b]pyridine; 1-Methyl-3-phenyl-6-(6-pyrrolidin-1-ylpyridin-3-yl)-4-trifluoromethyl-1H-pyrazolo[3,4-b]pyridine;
1-Methyl-6-(2-methyl-5-trifluoromethyl-2H-pyrazol-3-yl)-3-phenyl-4-trifluoromethyl-1H-pyrazolo[3,4-b]pyridine;
6-Benzothiazol-5-yl-1-methyl-3-phenyl-4-trifluoromethyl-1H-pyrazolo[3,4-b]pyridine;
N,N-Dimethyl-4-(3-phenyl-4-trifluoromethyl-1H-pyrazolo[3,4-b]pyridin-6-yl)benzamide;
6-(4-Morpholin-4-ylphenyl)-3-phenyl-4-trifluoromethyl-1H-pyrazolo[3,4-b]pyridine;
6-(6-Morpholin-4-ylpyridin-3-yl)-3-phenyl-4-trifluoromethyl-1H-pyrazolo[3,4-b]pyridine;
6-(6-Methoxypyridin-3-yl)-3-phenyl-4-trifluoromethyl-1H-pyrazolo[3,4-b]pyridine;
6-(3-Morpholin-4-ylphenyl)-3-phenyl-4-trifluoromethyl-1H-pyrazolo[3,4-b]pyridine;
N-Methyl-3-(3-phenyl-4-trifluoromethyl-1H-pyrazolo[3,4-b]pyridin-6-yl)benzamide;
N-[3-(3-Phenyl-4-trifluoromethyl-1H-pyrazolo[3,4-b]pyridin-6-yl)phenyl]methanesulfonamide;
3-Phenyl-6-(3-piperidin-1-ylphenyl)-4-trifluoromethyl-1H-pyrazolo[3,4-b]pyridine;
2-Fluoro-N-methyl-5-(3-phenyl-4-trifluoromethyl-1H-pyrazolo[3,4-b]pyridin-6-yl)benzamide;
5-(3-Phenyl-4-trifluoromethyl-1H-pyrazolo[3,4-b]pyridin-6-yl)nicotinonitrile;
2-Fluoro-5-(1-methyl-3-phenyl-4-trifluoromethyl-1H-pyrazolo[3,4-b]pyridin-6-yl)benzoic acid;
2-Amino-5-(4-difluoromethyl-2-methyl-2H-pyrazolo[3,4-b]pyridin-6-yl)benzonitrile;
Dimethyl[4-(3-phenyl-4-trifluoromethyl-1H-pyrazolo[3,4-b]pyridin-6-yl)phenyl]amine;
4-(3-Phenyl-4-trifluoromethyl-1H-pyrazolo[3,4-b]pyridin-6-yl)phenylamine;
6-(4-Methoxyphenyl)-3-phenyl-4-trifluoromethyl-1H-pyrazolo[3,4-b]pyridine; and
2-Fluoro-5-(3-phenyl-4-trifluoromethyl-1H-pyrazolo[3,4-b]pyridin-6-yl)benzonitrile;
6-(1-ethyl-1H-pyrazol-4-yl)-1-methyl-4(trifluoromethyl)-1H-pyrazolo[3,4-b]pyridine;
4-(difluoromethyl)-6-(1-ethyl-1H-pyrazol-4-yl)-1-methyl-1H-pyrazolo[3,4-b]pyridine;
6-(4-ethylphenyl)-1-methyl-4-(trifluoromethyl)-1H-pyrazolo[3,4-b]pyridine;
6-(4-methoxyphenyl)-1-methyl-4-(trifluoromethyl)-1H-pyrazolo[3,4-b]pyridine;
6-(4-fluorophenyl)-1-metyl-4-(trifluoromethyl)-1H-pyrazolo[3,4-b]pyridine;
4-(difluoromethyl)-6-(2-methoxyphenyl)-1-methyl-1H-pyrazolo[3,4-b)pyridine;
6-(3-methoxyphenyl)-1-methyl-4-(trifluoromethyl)-1H-pyrazolo[3,4-b]pyridine;
6-(2,5-dimethoxyphenyl)-1-methyl-4-(trifluoromethyl)-1H-pyrazolo[3,4-b]pyridine;
4-(difluotomethyl)-6-(4-ethylphenyl)-1-methyl-1H-pyrazolo[3,4-b]pyridine;
4-methyl-1H-pyrazolo[3,4-b]pyridine;
4-(difluoromethyl)-6-(3-methoxyphenyl)-1-methyl-1H-pyrazolo[3,4-b]pyridine;
4-(difluoromethyl)-6-(4-fluorophenyl)-1-methyl-1H-pyrazolo[3,4-b]pyridine;
4-(difluoromethyl)-6-(3,4-dimethoxyphenyl)-1-methyl-1H-pyrazolo[3,4-b]pyridine;
4-(difluoromethyl)-1-methyl-6-phenyl-1H-pyrazolo[3,4-b]pyridine;
1-methyl-6-phenyl-4-(trifluoromethyl)-1H-pyrazolo[3,4-b]pyridine.

The compounds of formula (I) may exist in the form of bases or salified with acids or bases, especially pharmaceutically acceptable acids or bases. Such addition salts form part of the invention.

These salts are advantageously prepared with pharmaceutically acceptable acids, but salts of other acids that are of use, for example, for purifying or isolating the compounds of formula (I) are also described in the invention.

In the context of the present invention, and unless otherwise mentioned in the text, the following definitions apply:
- a halogen atom: a fluorine, chlorine, bromine or iodine atom;
- an alkyl group: a linear or branched saturated hydrocarbon-based aliphatic group, comprising from 1 to 6 carbon atoms. Examples that may be mentioned include methyl, ethyl, propyl, isopropyl, butyl, isobutyl, pentylgroups, etc;
- a cycloalkyl group: a 3- to 8-membered cyclic alkyl group, comprising between 3 and 6 carbon atoms, the said cycloalkyl group being optionally substituted with one or more halogen atoms and/or alkyl groups. Examples that may be mentioned include cyclopropyl and cyclopentyl groups;
- an alkoxy group: a radical -O-alkyl in which the alkyl group is as defined previously;
- an alkoxyalkyl group: a radical of formula alkyl-O-alkyl, in which the identical or different alkyl groups are as defined previously;
   an aryl group: a cyclic aromatic group comprising between 5 and 10 carbon atoms, for example a phenyl group;
- a heteroaryl group: a cyclic aromatic group comprising between 3 and 10 atoms including one or more heteroatoms, for example between 1 and 4 heteroatoms, such as nitrogen, oxygen or sulfur, this group comprising one or more rings, preferably one or two rings. The heteroaryls may comprise several fused rings. The heteroaryls are optionally substituted with one or more alkyl groups or an oxygen atom. Examples that may be mentioned include thienyl, pyridyl, pyrazolyl, imidazolyl, thiazolyl and triazolyl groups;
- a heterocycloalkyl: a cyclic alkyl group comprising from 4 to 9 atoms forming this ring and of which one or two are heteroatoms, such as oxygen, nitrogen or sulfur. Mention may be made especially of piperidyl, pyrrolidinyl, piperazinyl, tetrahydropyranyl, morpholinyl and homopiperazinyl groups;
- a heterocyclic group: a heteroaryl group or a heterocycloalkyl group as defined previously.
A subject of the present invention is particularly compounds of formula (I) as defined above in which R₂ represents a group:
∘ -CHF₂, except when R₄ located on the nitrogen alpha to R₃ represents a methyl group and R3 represents a hydrogen atom,
or
∘ -CONHR₅, in which R₅ represents a hydrogen atom or a linear group (C₁-C₃)alkyl, optionally substituted with a pyridyl group or an aromatic group chosen from aryl and pyridyl,
in the form of the base or of an acid-addition or base-addition salt.
A subject of the present invention is particularly compounds of formula (I) as defined above in which R₁ represents an aryl, pyridyl or pyrazolyl group, advantageously a phenyl group, optionally substituted with one or more substituents chosen from:
∘ a fluorine atom;
and
∘ a group -COR₁₂, in which R₁₂ represents a hydroxyl group,
in the form of the base or of an acid-addition or base-addition salt.

The last two subgroups defined above taken separately or in combination also form part of the invention.

A subject of the present invention is particularly compounds of formula (I) as defined above in which R₁ represents an aryl group, in the form of the base or of an acid-addition or base-addition salt.

Another subject of the present invention is particularly compounds of formula (I) as defined above in which R₂ represents a group:
∘ -CF₃,
∘ -CHF₂,
or
∘ -CONHR₅, in which R₅ represents a hydrogen atom or a linear group (C₁-C₃)alkyl, optionally substituted with a pyridyl group or an aromatic group chosen from aryl and pyridyl,
in the form of the base or of an acid-addition or base-addition salt.

Among the compounds of formula (I) that are subjects of the invention, mention may be made especially of the following compounds:
[4-(4-Difluoromethyl-3-phenyl-1H-pyrazolo[3,4-b]pyridin-6-yl)phenyl]pyrrolidin-1-ylmethanone;
4-Difluoromethyl-6-(1-methyl-1H-pyrazol-4-yl)-3-phenyl-1H-pyrazolo[3,4-b]pyridine;
4-Difluoromethyl-6-(3,5-dimethyl-1H-pyrazol-4-yl)-3-phenyl-1H-pyrazo)o[3,4-b]pyridine:
4-Difluoromethyl-3-phenyl-6-(1H-pyrazol-4-yl)-1H-pyrazolo[3,4-b]pyridine;
4-Difluoromethyl-6-(6-methoxypyridin-3-yl)-3-phenyl-1H-pyrazolo[3,4-b]pyridine;
[3-(4-Difluoromethyl-3-phenyl-1H-pyrazolo[3,4-b]pyridin-6-yi)phenyl]pyrrolidin-1-ylmethanone;
4-Difluoromethyl-3-phenyl-6-(3-piperidin-1-ylphenyl)-1H-pyrazolo[3,4-b]pyridine;
6-(4-Amino-3-methoxyphenyl)-3-phenyl-1H-pyrazolo[3,4-b]pyridine-4-carboxylic acid methylamide;
6-(4-Amino-3-methoxyphenyl)-3-phenyl-1H-pyrazolo[3,4-b]pyridine-4-carboxylic acid amide; compound with trifluoroacetic acid;
6-(4-Amino-3-methoxyphenyl)-1H-pyrazolo[3,4-b]pyridine-4-carboxylic acid methylamide;
4-(4-Amino-3-methoxyphenyl)-1H-pyrazolo[3,4-b]pyridine-6-carboxylic acid methylamide;
6-(4-Amino-3-methoxyphenyl)-1H-pyrazolo[3,4-b]pyridine-4-carboxylic acid amide;
6-(4-Amino-3-methoxyphenyl)-2-methyl-2H-pyrazolo[3,4-b]pyridine-4-carboxylic acid amide;
5-(4-Carbamoyl-3-phenyl-1H-pyrazolo[3,4-b]pyridin-6-yl)-2-fluorobenzoic acid;
2-Amino-5-(4-carbamoyl-3-phenyl-1H-pyrazolo[3,4-b]pyridin-6-yl)benzoicacid;
6-(4-Amino-3-cyanophenyl)-3-phenyl-1H-pyrazolo[3,4-b]pyridine-4-carboxylic acid amide;
6-(4-Amino-3-cyanophenyl)-3-thiophen-2-yl-1H-pyrazolo[3,4-b]pyridine-4-carboxylic acid amide;
5-(4-Carbamoyl-1H-pyrazolo[3,4-b]pyridin-6-yl)-2-fluorobenzoic acid;
6-(3-Cyano-4-fluorophenyl)-3-phenyl-1H-pyrazolo[3,4-b]pyridine-4-carboxylic acid amide;
5-(4-Difluoromethyl-3-phenyl-1H-pyrazolo[3,4-b]pyridin-6-yl)-2-fluorobenzoic acid;
;2-Amino-5-(4-diffuoromethyl-3-phenyl-1H-pyrazolo[3,4-b]pyridin-6-yl)benzonitrile;
2-Amino-5-(1-methyl-3-phenyl-4-trifluoromethyl-1H-pyrazolo[3,4-b]pyridin-6-yl)benzonitrile;
6-(3-Carbamoyl-4-fluorophenyl)-3-pyridin-3-yl-1H-pyrazolo[3,4-b]pyridine-4-carboxylic acid amide;
5-(4-Difluoromethyl-2-methyl-2H-pyrazolo[3,4-b]pyridiri-6-yl)-2-fluorobenzonitrile;
6-(1H-Indol-6-yl)-3-phenyl-4-trifluoromethyl-1H-pyrazolo[3,4-b]pyridine;
5-(3-Phenyl-4-trifluoromethyl-1H-pyrazolo[3,4-b]pyridin-6-yl)-1,3-dihydrobenzimidazol-2-one;
6-(4-Amino-3-cyanophenyl)-1H-pyrazolo[3,4-b]pyridine-4-carboxylic acid phenylamide;
6-(4-Amino-3-cyanophenyl)-1H-pyrazolo[3,4-b]pyridine-4-carboxylic acid (pyridin-2-ylmethyl)amide;
6-(4-Amino-3-cyanophenyl)-1H-pyrazolo[3,4-b]pyridine-4-carboxylic acid pyridin-2-ylamide;
6-(4-Amino-3-cyanophenyl)-1H-pyrazolo[3,4-b]pyridine-4-carboxylic acid pyridin-3-ylamide;
6-(4-Amino-3-cyanophenyl)-1H-pyrazolo[3,4-b]pyridine-4-carboxylic acid pyridin-4-ylamide;
5-(4-Difluoromethyl-2-methyl-2H-pyrazolo[3,4-b]pyridin-6-yl)-2-fluorobenzamide;
5-(4-Difluoromethyl-1-methyl-3-pyridin-4-yl-1H-pyrazolo[3,4-b]pyridin-6-yl)-2-fluorobenzonitrile;
2-Amino-5-(2-methyl-3-phenyl-4-trifluoromethyl-2H-pyrazolo[3,4-b]pyridin-6-yl)benzonitrile;
6-(1H-Benzimidazol-5-yl)-1H-pyrazolo[3,4-b]pyridine-4-carboxylic acid phenylamide;
6-(1H-Benzimidazol-5-yl)-1H-pyrazolo[3,4-b]pyridine-4-carboxylic acid pyridin-2-ylamide;
6-(1H-Benzimidazol-5-yl)-1H-pyrazolo[3,4-b]pyridine-4-carboxylic acid (pyridin-3-ylmethyl)amide;
6-(2-Oxo-2,3-dihydro-1H-benzimidazol-5-yl)-1H-pyrazolo[3,4-b]pyridine-4-carboxylic acid phenylamide;
2-Amino-5-(4-difluoromethyl-1-methyl-1H-pyrazolo[3,4-b]pyridin-6-yl)benzonitrile;
3-(4-Difluoromethyl-1-methyl-1H-pyrazolo[3,4-b]pyridin-6-yl)benzonitrile;
4-(4-Difluoromethyl-1-methyl-1H-pyrazolo[3,4-b]pyridin-6-yl)phenylamine;
2-Amino-5-[2-(2-dimethylaminoethyl)-3-phenyl-4-trifluoromethyl-2H-pyrazolo[3,4-b]pyridin-6-yl]benzonitrile;
2-Amino-5-(4-difluoromethyl-1H-pyrazolo[3,4-b]pyridin-6-yl)benzonitrile;
2-Amino-5-[1-(2-dimethylaminoethyl)-3-phenyl-4-trifluoromethyl-1H-pyrazolo[3,4-b]pyridin-6-yl]benzonitrile;
2-Amino-5-[2-(2-morpholin-4-ylethyl)-3-phenyl-4-trifluoromethyl-2H-pyrazolo[3,4-b]pyridin-6-yl]benzonitrile;
2-Methoxy-5-(1-methyl-3-phenyl-4-trifluoromethyl-1H-pyrazolo[3,4-b]pyridin-6-yl)nicotinonitrile;
2-Amino-5-(4-difluoromethyl-1-methyl-3-phenyl-1H-pyrazolo[3,4-b]pyridin-6-yl)benzonitrile;
4-(4-Difluoromethyl-1-methyl-3-phenyl-1H-pyrazolo[3,4-b]pyridin-6-yl)phenylamine;
[3-(4-Difluoromethyl-1-methyl-3-phenyl-1H-pyrazolo[3,4-b]pyridin-6-yl)phenyl]dimethylamine;
2-Amino-5-[3-phenyl-1-(2-piperidin-1-ylethyl)-4-trifluoromethyl-1H-pyrazolo[3,4-b]pyridin-6-yl]benzonitrile;
Dimethyl{3-[3-phenyl-1-(2-piperidin-1-ylethyl)-4-trifluoromethyl-1H-pyrazolo[3,4-b]pyridin-6-yl]phenyl}amine;
2-Amino-5-[4-difluoromethyl-2-(2-dimethylaminoethyl)-2H-pyrazolo[3,4-b]pyridin-6-yl]benzonitrile;
2-Amino-5-[4-difluoromethyl-2-(2-morpholin-4-ylethyl)-2H-pyrazolo[3,4-b]pyridin-6-yl]benzonitrile;
2-Amino-5-[4-difluoromethyl-1-(2-dimethylaminoethyl)-3-phenyl-1H-pyrazolo[3,4-b]pyridin-6-yl]benzonitrile;
2-(2-Morpholin-4-ylethyl)-6-(3-morpholin-4-ylmethylphenyl)-4-trifluoromethyl-2H-pyrazolo[3,4-b]pyridine;
Dimethyl{3-[1-(2-morpholin-4-ylethyl)-4-trifluoromethyl-1H-pyrazolo[3,4-b]pyridin-6-yl]phenyl}amine;
5-[1-(2-Morpholin-4-ylethyl)-4-trifluoromethyl-1H-pyrazolo[3,4-b]pyridin-6-yl]nicotinonitrile;
5-[1-(2-Morpholin-4-ylethyl)-4-trifluoromethyl-1H-pyrazolo[3,4-b]pyridin-6-yl]nicotinamide;
2-Amino-5-(2-methyl-4-trifluoromethyl-2H-pyrazolo[3,4-b]pyridin-6-yl)benzonitrile;
1-Methyl-6-(3-morpholin-4-ylmethylphenyl)-4-trifluoromethyl-1H-pyrazolo[3,4-b]pyridine;
2-Amino-5-(1-methyl-4-trifluoromethyl-1H-pyrazolo[3,4-b]pyridin-6-yl)benzonitrile;
Dimethyl[3-(1-methyl-4-trifluoromethyl-1H-pyrazolo[3,4-b]pyridin-6-yl)phenyl]amine;
Dimethyl[3-(3-phenyl-2-propyl-4-trifluoromethyl-2H-pyrazolo[3,4-b]pyridin-6-yl)phenyl]amine;
2Amino-5-[4-difluoromethyl-2-(2-piperidin-1-ylethyl)-2H-pyrazolo(3,4-b)pyridin-6-yl]benzonitrile;
2-Amino-5-(4-difluoromethyl-3-pyridin-3-yl-1H-pyrazolo[3,4-b]pyridin-6-yl)benzonitrile;
2-Amino-5-(4-difluoromethyl-2-propyl-2H-pyrazolo[3,4-b]pyridin-6-yl)benzonitrile;
2-Amino-5-(4-difluoromethyl-3-pyridin-4-yl-1H-pyrazolo[3,4-b]pyridin-6-yl)benzonitrile;
1-Methyl-6-(3-morpholin-4-ylmethylphenyl)-3-pyridin-3-yl-4-trifluoromethyl-1H-pyrazolo[3,4-b]pyridine;
2-Amino-5-[4-difluoromethyl-3-(3-methoxyphenyl)-1H-pyrazolo[3,4-b]pyridin-6-yl]benzonitrile;
2-Amino-5-(2-propyl-4-trifluoromethyl-2H-pyrazolo[3,4-b]pyridin-6-yl)benzonitrile;
6-(3-Morpholin-4-ylmethylphenyl)-2-propyl-4-trifluoromethyl-2H-pyrazolo[3,4-b]pyridine;
Dimethyl[3-(2-propyl-4-trifluoromethyl-2H-pyrazolo[3,4-b]pyridin-6-yl)phenyl]amine;
6-(3-Morpholin-4-ylmethylphenyl)-1-propyl-trifluoromethyl-1H-pyrazolo[3,4-b]pyridine;
6-(4-Methoxyphenyl)-1-propyl-4-trifluoromethyl-1H-pyrazolo[3,4-b]pyridine;
5-[3-(3-Methoxyphenyl)-1-methyl-4-trifluoromethyl-1H-pyrazolo[3,4-b]pyridin-6-yl]nicotinonitrile;
3-(3-Methoxyphenyl)-1-methyl-6-(3-morpholin-4-ylmethylphenyl)-4-trifluoromethyl-1H-pyrazolo[3,4-b]pyridine;
{3-[3-(3-Methoxyphenyl)-1-methyl-4-trifluoromethyl-1H-pyrazolo[3,4-b]pyridin-6-yi]phenyl}dimethylamine;
3-(3-Methoxyphenyl)-6-(4-methoxyphenyl)-1-methyl-4-trifluoromethyl-1H-pyrazolo[3,4-b]pyridine.
It should be noted that the above compounds have been named according to the IUPAC nomenclature, by means of the ACDLABS 10.0 ACD/name software (Advanced Chemistry development) or the AutoNom software (Beilstein Informations system).

In the following text, the term "protecting group (P)" means a group that can, firstly, protect a reactive function such as a hydroxyl or an amine during a synthesis and, secondly, regenerate the intact reactive function at the end of the synthesis. Examples of protecting groups and also of protection and deprotection methods are given in Protective Groups in Organic Synthesis, Greene et al., 3rd Edition (John Wiley & Sons, Inc., New York).

The compounds of general formula (I) may be prepared according to the process that follows.
The compound of formula (IV) when R₂ represents a -CF₃ group is obtained via methods known in the literature from the 2-aminopyrazole (III) and the 4,4,4-trifluoroacetoacetate (II), according to the following reaction scheme described in the Polish Journal of Chemistry, 1983, 57, 789*.* 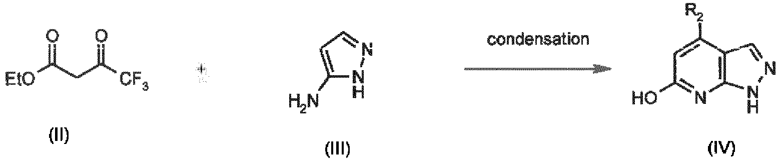 The compound of formula (IV) when R₂ represents a -CHF₂ group is obtained via a method similar to that described previously by condensation of the 2-aminopyrazole (III) and 4,4-difluoroacetoacetate. 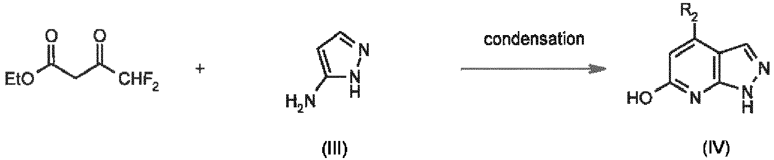 The compound of formula (XII) in which R₂ represents a group -CHF₂ or -CF₃ is obtained by chlorination in the presence of POCl₃ of the compound of formula (IV) in which R₂ represents a group -CHF₂ or -CF₃. 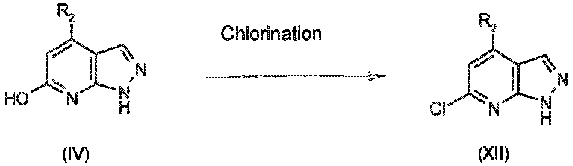 The compound of formula (VI) in which R₂ represents a group -CF₃ and R₃ is a phenyl is obtained via methods known in the literature from 3-phenyl-1H-pyrazol-5-amine (V) and ethyl 4,4,4-trifluoro-3-oxobutanoate, according to the following reaction scheme described in the Polish Journal of Chemistry, 1983, 57, 789*.* 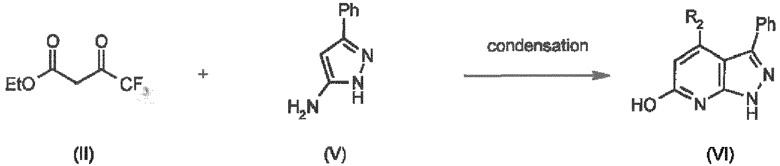 The compound of formula (VI) in which R₂ represents a -CHF₂ group is obtained via a method similar to that described previously from 3-phenyl-1H-pyrazol-5-amine (V) and ethyl 4,4-difluoro-3-oxobutanoate.
Scheme 1 presents a route for obtaining compounds of formula (I) in which R₁ is as defined previously, and R₂ represents a group -CF₃ or -CHF₂.

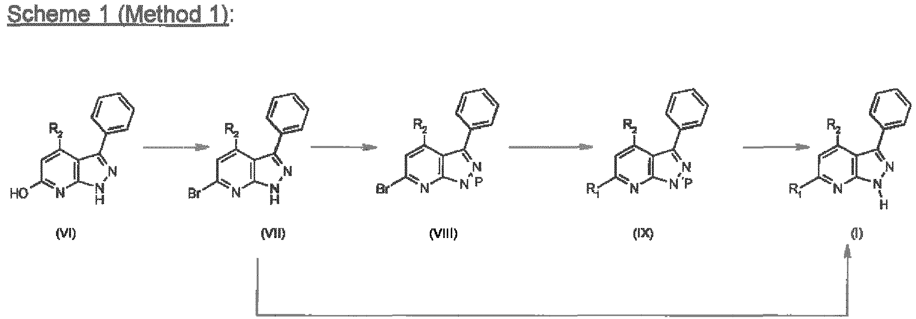

The compound of formula (VI) is subjected to a bromination reaction in the presence of POBr₃ in order to obtain the compound of formula (VII). The compound of formula (VII) is subjected to an alkylation reaction in the presence of a protecting group P in order to obtain the compound of formula (VIII). The compound of formula (VIII) is subjected, in the presence of a palladium catalyst, a ligand and a base, to a reaction with phenylboronic or heteroarylboronic derivatives or phenylboronate esters or heteroarylboronate esters according to a Suzuki coupling, in order to obtain the compound of formula (IX). The compound of formula (IX) is subjected to a deprotection reaction in order to obtain the compounds of formula (I) in which R₁ is as defined previously, and R₂ represents a group -CF₃ or -CHF₂.
The compound of formula (VII) may optionally be subjected, in the presence of a palladium catalyst, a ligand and a base, to a reaction with phenylboronic or heteroarylboronic derivatives or phenylboronate esters or heteroarylboronate esters according to a Suzuki coupling, in order to obtain the compound of formula (I) in which R₁ is as defined previously, and R2 represents a group -CF₃ or -CHF₂.

Scheme 2 presents a route for obtaining compounds of formula (I) in which R₁ and R₄ are as defined previously with the exception of a hydrogen atom.

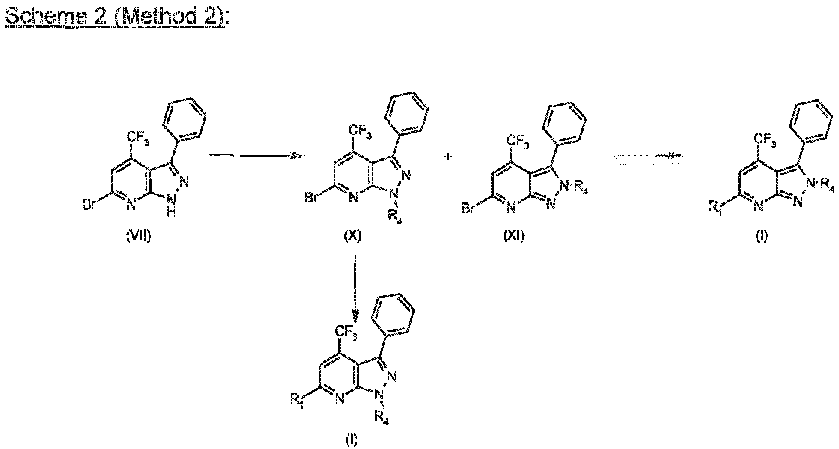

The compound of formula (VII) is subjected to an alkylation reaction in the presence of a base and a halogenated derivative of formula R₄-X in order to obtain the compounds of formulae (X) and (XI). The compounds of formulae (X) and (XI) are subjected separately, in the presence of a palladium catalyst, a ligand and a base, to a reaction with phenylboronic or heteroarylboronic derivatives or phenylboronate esters or heteroarylboronate esters according to a Suzuki coupling, in order to obtain the compounds of formula (I) in which R₁ and R₄ are as defined previously.

Scheme 3 presents a route for obtaining compounds of formula (I) in which R₂ represents a group -CHF₂ or -CF₃; R₁ and R₄ are as defined previously, with the exception that R₄ represents a hydrogen atom.

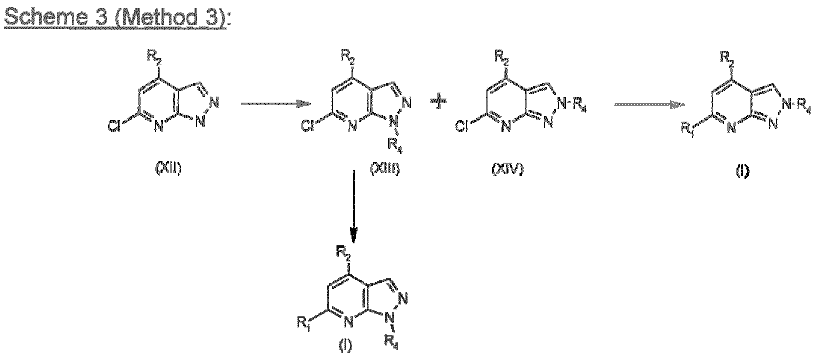

The compound of formula (XII) is subjected to an alkylation reaction in the presence of a halogenated derivative of formula R₄-X in order to obtain the compounds of formulae (XIII) and (XIV). The compounds of formulae (XIII) and (XIV) are separately subjected, in the presence of a palladium catalyst, a ligand and a base such as caesium carbonate, to a reaction with phenylboronic or heteroarylboronic derivatives or phenylboronate esters or heteroarylboronate esters according to a Suzuki coupling, in order to obtain the compound of formula (I) in which R₂ represents a group -CHF₂ or -CF₃; R₁ and R₄ are as defined previously.

Scheme 4 presents a route for obtaining compounds of formula (I) in which R₂ represents a group -CHF₂ or -CF₃, R₃ and R₄ represent a hydrogen atom and R₁ is as defined previously.

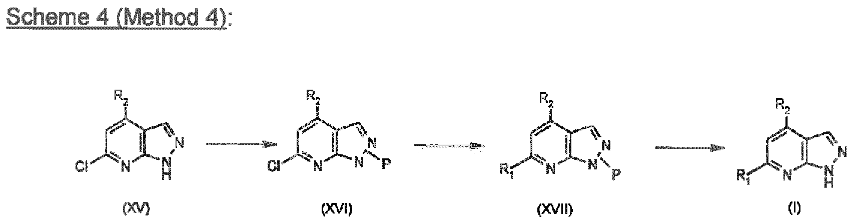

The compound of formula (XV) is subjected to an alkylation reaction in the presence of a protecting group P in order to obtain the compound of formula (XVI). The compound of formula (XVI) is subjected, in the presence of a palladium catalyst, a ligand and a base, to a reaction with phenylboronic or heteroarylboronic derivatives or phenylboronate esters or heteroarylboronate esters according to a Suzuki coupling, in order to obtain the compound of formula (XVII). The compound of formula (XVII) is then subjected to a deprotection reaction in order to obtain the compound of formula (I) in which R₂ represents a group -CHF₂ or -CF₃ and R₁ is as defined previously.

Scheme 5 presents a first process according to the present invention for preparing compounds of formula (I) as claimed, in which R₂ represents a group -CHF₂ or -CF₃; R₁, R₃ and R₄ are as defined previously, with the exception that R₃ and R₄ represent a hydrogen atom.

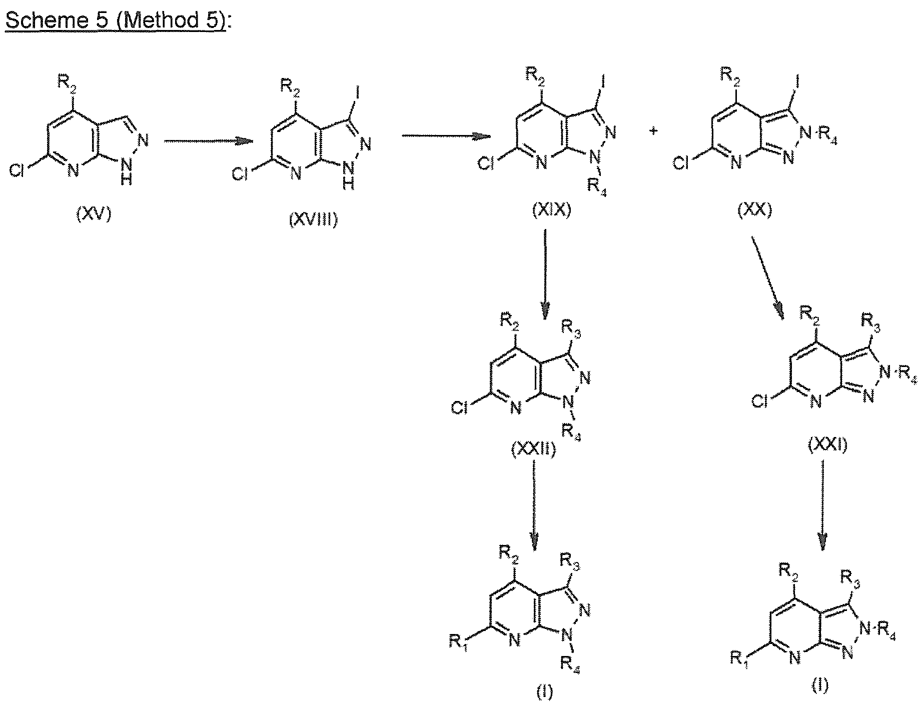

The compound of formula (XV) is subjected to an iodination reaction in the presence of N-iodosuccinimide in order to obtain the compound of formula (XVIII). The compound of formula (XVIII) is then subjected to an alkylation reaction in the presence of a halogenated derivative of formula R₄-X in order to obtain the compounds of formulae (XIX) and (XX). The compounds of formulae (XIX) and (XX) are separately subjected, in the presence of a palladium catalyst, a ligand and a base, to a reaction with phenylboronic or heteroarylboronic derivatives or phenylboronate esters or heteroarylboronate esters according to a Suzuki coupling, in order to obtain the compounds of formulae (XXI) and (XXII). The compounds of formulae (XXI) and (XXII) are subjected separately, in the presence of a palladium catalyst, a ligand and a base, to a reaction with phenylboronic or heteroarylboronic derivatives or phenylboronate esters or heteroarylboronate esters according to a Suzuki coupling, in order to obtain the compound of formula (I) in which R₂ represents a group -CHF₂ or -CF₃ and R₁, R₃ and R₄ are as defined previously, with the exception of a hydrogen atom.

Scheme 6 presents a second process according to the invention for preparing compounds of formula (I) as claimed, in which R₂ represents a group -CHF₂ or -CF₃ and R₁ and R₃ are as defined previously, with the exception of R₃ representing a hydrogen atom.

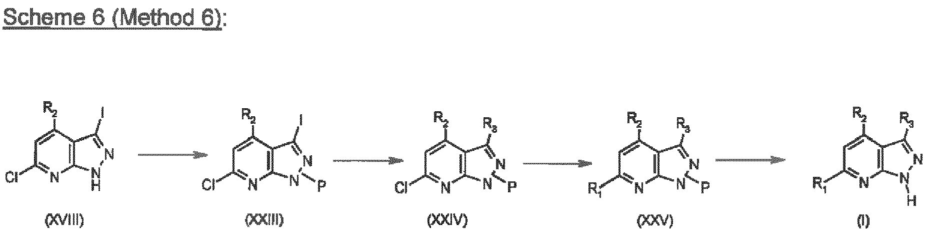

The compound of formula (XVIII) is subjected to an alkylation reaction in the presence of a protecting group P in order to obtain the compound of formula (XXIII). The compound of formula (XXIII) is subjected, in the presence of a palladium catalyst, a ligand and a base, to a reaction with phenylboronic or heteroarylboronic derivatives or phenylboronate esters or heteroarylboronate esters according to a Suzuki coupling, in order to obtain the compound of formula (XXIV). The compound of formula (XXIV) is subjected, in the presence of a palladium catalyst, a ligand and a base, to a reaction with phenylboronic or heteroarylboronic derivatives or phenylboronate esters or heteroarylboronate esters according to a Suzuki coupling, in order to obtain the compound of formula (XXV). The compound of formula (XXV) is then subjected to a protection reaction in order to obtain the compound of formula (I) in which R₂ represents a group -CHF₂ or -CF₃ and R₁ and R₃ are as defined previously, with the exception that R₃ and R₄ represent a hydrogen atom.

Scheme 7 presents a route for obtaining compounds of formula (I) in which R₂ is as defined previously, with the exception of a group -CHF₂ or -CF₃; R₁, R₃ and R₄ are as defined previously.

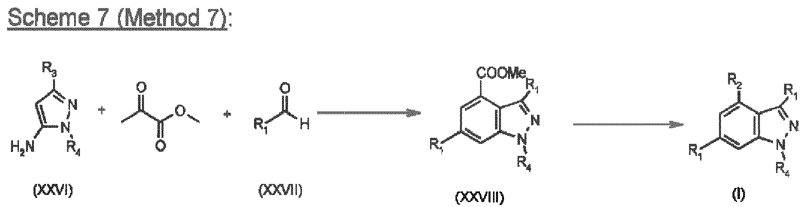

The compound of formula (XXVI) is subjected to a condensation reaction with the compound of formula (XXVII) and methyl 2-oxopropanoate in order to obtain the compound of formula (XXVIII). The compound of formula (XXVIII) is subjected to a saponification reaction or to substitution with an amine in order to obtain the compound of formula (I) in which R₂ is as defined previously, except for a group -CHF₂ or -CF₃; R₁, R₃ and R₄ are as defined previously.

In the preceding schemes, the starting compounds, the reagents and the intermediates, when their preparation method is not described, are commercially available or described in the literature, or alternatively may be prepared according to methods that are described therein or that are known to those skilled in the art.

The invention also describes the compounds of formulae (II) to (XXVIII) defined above. These compounds are useful as intermediates for synthesizing the compounds of formula (I).

The examples that follow describe the preparation of certain compounds in accordance with the invention and outside the invention. These examples are not limiting and serve merely to illustrate the present invention. The numbers of the exemplified compounds refer to those given in the table below, which illustrates the chemical structures and physical properties of a number of compounds according to the invention and outside the invention.

The present invention is also illustrated below in two figures such that:
Figure 1: *in vitro* angiogenesis (length of pseudotubules) of HUVEC cells stimulated with FGF-2 in the absence or presence of FGF-R antagonists.
Figure 2: Effect of FGF-R antagonists in a model of inflammatory angiogenesis on the dry weight of skin (weight of the granuloma) or on their content of carmine red dye (dye).

The following abbreviations and empirical formulae are used:
AcOH: acetic acid
PTSA: para-toluenesulfonic acid
DME: ethylene glycol dimethyl ether
DMF: N,N-dimethylformamide
DMSO: dimethyl sulfoxide
g: gram
(M)Hz: (mega)Hertz
mL: millilitre
POBr₃: dibromophosphanyl hypobromite
TBAF: tetrabutylammonium fluoride
TFA: trifluoroacetic acid
THF: tetrahydrofuran

In the examples that follow:
- the NMR analyses were performed on Brüker Avance 250 MHz, 300 MHz, 400 MHz and 600 MHz machines. The proton magnetic resonance spectrum (¹H NMR), as described below, are recorded at 400 MHz or 600 MHz in DMSO-d₆, using the DMSO-d₆ peak as reference. The chemical shifts δ are expressed in parts per million (ppm). The signals observed are expressed as follows: s = singlet; d = doublet; t = triplet; m = mass or broad singlet; H = proton (for the rotamers, H*_{M}* and H*ₘ* are noted with reference to the major or minor isomers *M* and *m*, respectively).
- the melting points were measured on a BUCHI B-545 machine.
- the mass spectrometry analyses were performed on an Alliance 2695 machine (UV: PDA 996, MS: ZQ (simple Quad) ZQ2), Waters UPLC Acquity (UV: Acquity PDA, MS: SQD (simple Quad) SQW)

### Example 1: (compound 46 according to the invention)

### 5-(4-Carbamoyl-3-phenyl-1H-pyrazolo[3,4-b]pyridin-6-yl)-2-fluorobenzoic acid

To 5 ml of a 0.3 M solution in ethanol of 3-phenyl-1H-pyrazol-5-amine in a sealed tube are added 5 ml of a 0.3 M solution in ethanol of 2-fluoro-5-formylbenzoic acid and 1.5 mmol of ethyl 2-oxopropanoate at room temperature under an inert atmosphere of nitrogen. The tube is sealed and maintained at a temperature of 75°C for 18 hours. The capsule is removed and heating is continued for 4 hours at 60°C. The reaction medium is then concentrated under reduced pressure. The residue is taken up in a sealed tube with a 7 N solution of ammonia in methanol. The medium is then heated for 3 days at 80°C and then concentrated under reduced pressure. After purification by column chromatography on C-18 reverse-phase silica gel, eluting with an acetonitrile/H₂O/0.1% TFA mixture, 23.7 mg of a lyophilizate are obtained.
MH⁺: 377
¹H NMR (600 MHz, DMSO-de): δ□□14.10 (s, 1 H), 13.43 (s (broad), 1 H), 8.79 (dd, *J_{A}* = 7.2 Hz, *J_{B}* = 2.3 Hz, 1 H), 8.50 (m, 1 H), 8.14 (s, 1 H), 7.87 (s, 1 H), 7.80 (s, 1 H), 7.70 (dd, *J_{A}* = 7.8 Hz, *J_{B}=* 1.6 Hz, 1 H), 7.51 (m, 1 H), 7.46 (m, 2 H), 7.41 (m, 1 H)

### Example 2: (compound 38 outside the invention)

### 6-(4-Amino-3-methoxyphenyl)-1H-pyrazolo[3,4-b]pyridine-4-carboxylic acid

### 6-(4-Amino-3-methoxyphenyl)-1H-pyrazolo[3,4-b]pyridine-4-carboxylic acid

To 20 g (0.12 mol) of 3-hydroxy-4-nitrobenzaldehyde in 200 ml of anhydrous DMF are added 42 g (0.13 mol) of caesium carbonate. The solution obtained is ultrasonicated for 5 minutes, and 9.4 ml (0.29 mol) of methyl iodide are then added. The reaction medium is heated at 80°C for 18 hours and then diluted with ethyl acetate. The organic phase is washed with saturated aqueous sodium chloride solution, dried over magnesium sulfate and then concentrated under reduced pressure. The residue is recrystallized from 300 ml of a hot 1/2 DMF/ethanol mixture. The crystals formed are filtered off, rinsed with cold ethanol and with hexane, and then dried under reduced pressure. 12.1 g of a solid are obtained.

To 2 g (11 mmol) of 3-methoxy-4-nitrobenzaldehyde in 150 ml of anhydrous ethanol in a sealed tube are added 1.17 g (13.3 mmol) of pyruvic acid and 1.1 g (15.5 mmol) of 1H-pyrazol-5-amine. The reaction medium is heated at 80°C for 18 hours and then concentrated under reduced pressure. The residue is dissolved in 160 ml of a 3/1 DMSO/methanol mixture, to which are added 80 g of Dowex 1×8-400 resin. The reaction medium is stirred at room temperature for 1 hour and then filtered. The resin is rinsed several times with DMSO and then with methanol, and finally treated for 30 minutes in a 10% solution of TFA in methanol. After filtration, the organic phase is concentrated under reduced pressure. The residue obtained is taken up in 100 ml of ethanol and 40 ml of acetic acid. 300 mg of zinc powder are added. The reaction medium is stirred at room temperature. 1 g of zinc powder are added after 15 minutes. The reaction medium is filtered and then concentrated under reduced pressure. After purification by column chromatography on C-18 reverse-phase silica gel, eluting with an acetonitrile/H₂O/0.1% TFA mixture, 23.7 mg of a lyophilizate are obtained.
MH⁺: 285
¹H NMR (600 MHz, DMSO-d₆): δ 8.28 (s, 1 H) 8.08 (s, 1 H), 7.67 (d, *J* = 2.1 Hz, 1 H), 7.59 (dd, *J_{A}* = 8.4 Hz, *J_{B}* = 1.9 Hz, 1 H), 6.77 (d, *J* = 8.2 Hz, 1 H), 3.91 (s, 3 H)

### Example 3: (compound 53 according to the invention)

### 5-[4-(Difluoromethyl)-3-phenyl-1H-pyrazolo[3,4-b]pyridin-6-yl)-2-fluorobenzoic acid

### 4-(difluoromethyl)-3-phenyl-1H-indazol-6-ol

To 2.1 g (12.7 mmol) of ethyl 4,4-difluoro-3-oxobutanoate in 16 ml of a 1/1 AcOH/H2O mixture are added 2 g (12.5 mmol) of 3-phenyl-1H-pyrazol-5-amine. The reaction medium is heated at 90°C for 18 hours. The medium is cooled and the precipitate obtained is filtered off, washed with aqueous 20% acetic acid solution and then dried under reduced pressure. 2.5 g of a solid are obtained.
MH⁺: 262

### 6-bromo-4-(difluoromethyl)-3-phenyl-1H-pyrazolo[3,4-b]pyridine

To 1 g (3.8 mmol) of 4-(difluoromethyl)-3-phenyl-1H-indazol-6-ol in 20 ml of toluene are added 3.1 g (10.8 mmol) of POBr₃. The reaction medium is heated at 90°C for 18 hours. The reaction medium is concentrated under reduced pressure. The residue obtained is purified by column chromatography on silica gel, eluting with a 4/1 hexane/ethyl acetate mixture. 620 mg of a solid are obtained.
MH⁺: 324

### 5-[4-(Difluoromethyl)-3-phenyl-1H-pyrazolo[3,4-b]pyridin-6-yl)-2-fluorobenzoic acid

To 78 mg (0.24 mmol) of 6-bromo-4-(difluoromethyl)-3-phenyl-1H-pyrazolo[3,4-b]pyridine in 4 ml of a 4/1 THF/water mixture are added 92 mg (0.43 mmol) of [3-(ethoxycarbonyl)-4-fluorophenyl]boronic acid, 35 mg (0.03 mmol) of tetrakis(triphenylphosphine)palladium and 261 mg (0.8 mmol) of caesium carbonate, under an inert atmosphere of argon. The reaction medium is heated at 150°C for 60 minutes by microwave. The organic phase is separated out by settling of the phases, diluted with THF, washed with saturated aqueous sodium chloride solution and concentrated under reduced pressure. After purification by column chromatography on C-18 reverse-phase silica gel, eluting with an acetonitrile/H₂O/0.1% TFA mixture, 12.3 mg of a lyophilizate are obtained.
MH⁺: 384
¹H NMR (600 MHz, DMSO-d₆): δ 14.31 (s, 1 H), 8.76 (dd, *J_{A}* = 7.2 Hz, *J_{B} =* 2.3 Hz, 1 H), 8.47 (m, 1 H), 8.04 (s, 1 H), 7.67 (d, *J_{A}* = 7.9 Hz, 2 H), 7.51 (m, 4 H), 7.33 (t, *J_{A}* = 54.6 Hz, 1 H)

### Example 4: (compound 56 according to the invention)

### 2-Fluoro-5-[3-phenyl-4-(trifluoromethyl)-1H-pyrazolo[3,4-b]pyridin-6-yl]benzoic acid

### 3-phenyl-4-(trifluoromethyl)-1H-pyrazolo[3,4-b]pyridin-6-ol

To 2.1 g (11.4 mmol) of ethyl 4,4,4-trifluoro-3-oxobutanoate in 16 ml of a 1/1 AcOH/H₂O mixture are added 2 g (12.5 mmol) of 3-phenyl-1H-pyrazol-5-amine. The reaction medium is heated at 90°C for 18 hours. The medium is cooled and the precipitate obtained is filtered off, washed with aqueous 20% acetic acid solution and then dried under reduced pressure. 2.5 g of a solid are obtained.
MH⁺: 280

### 6-bromo-3-phenyl-4-(trifluoromethyl)-1H-pyrazolo[3,4-b]pyridine

To 1 g (3.8 mmol) of 3-phenyl-4-(trifluoromethyl)-1H-pyrazolo[3,4-b]pyridin-6-ol in 20 ml of toluene are added 3.1 g (10.8 mmol) of POBr₃. The reaction medium is heated at 90°C for 18 hours. The reaction medium is concentrated under reduced pressure and then purified by column chromatography on silica gel, eluting with a 4/1 hexane/ethyl acetate mixture. 338 mg of a solid are obtained.
MH⁺: 306

### 2-Fluoro-5-[3-phenyl-4-(trifluoromethyl)-1H-pyrazolo[3,4-b]pyridin-6-yl]benzoic acid

To 103 mg (0.33 mmol) of 6-bromo-3-phenyl-4-(trifluoromethyl)-1H-pyrazolo[3,4-b]pyridine in 4 ml of a 4/1 THF/water mixture are added 187 mg (0.88 mmol) of [3-(ethoxycarbonyl)-4-fluorophenyl]boronic acid, 41 mg (0.035 mmol) of tetrakis(triphenylphosphine)palladium and 293 mg (0.9 mmol) of caesium carbonate, under an inert atmosphere of argon. The reaction medium is heated at 150°C for 60 minutes by microwave. The organic phase is separated out by settling of the phases, diluted with THF, washed with saturated aqueous sodium chloride solution and concentrated under reduced pressure. The residue obtained is purified by column chromatography on C-18 reverse-phase silica gel, eluting with an acetonitrile/H₂O/0.1% TFA mixture. The solid obtained is taken up in a 1/1 DMF/NaOH (1N) mixture and stirred for 1 hour at room temperature. After purification by column chromatography on C-18 reverse-phase silica gel, eluting with an acetonitrile/H₂O/0.1% TFA mixture, 34 mg of a lyophilizate are obtained.
MH⁺: 402
¹H NMR (600 MHz, DMSO-d₆): δ 14.51 (s, 1 H), 13.51 (s (broad), 1 H), 8.80 (dd, *J_{A}* = 7.1 Hz, *J_{B}* = 2.4 Hz, 1 H), 8.51 (m, 1 H), 8.17 (s, 1 H), 7.51 (m, 6 H)

### Example 5: (compound 61 according to the invention)

### N,N-Dimethyl-4-[3-phenyl-4-(trifluoromethyl)-1H-pyrazolo[3,4-b]pyridin-6-yl]aniline

### 6-bromo-3-phenyl-4-(trifluoromethyl)-1-{[2-(trimethylsilyl)ethoxy]methyl}-1H-pyrazolo[3,4-b]pyridine

To 10 g (29 mmol) of 6-bromo-3-phenyl-4-(trifluoromethyl)-1H-pyrazolo[3,4-b]pyridine in 100 ml of anhydrous DMF, under an inert atmosphere of nitrogen, are added 7.3 g (43.8 mmol) of [2-(chloromethoxy)ethyl](trimethyl)silane and 6.11 ml (43.8 mmol) of triethylamine, at room temperature. The reaction medium is stirred for 2 hours and then hydrolysed with water. The aqueous phase is extracted with ethyl acetate. The organic phase obtained is washed with water, dried over sodium sulfate and then concentrated under reduced pressure. The colourless oil obtained is purified by column chromatography on silica gel, eluting with a heptane/ethyl acetate mixture. 13.3 g of a colourless oil are obtained.
MH⁺: 472

### N,N-dimethyl-4-[3-phenyl4-(trifluoromethyl)-1-{[2-(trimethylsilyl)ethoxy]methyl}-1H-pyrazolo[3,4-b]pyridin-6-yl]aniline

To 0.4 g (0.85 mmol) of 6-bromo-3-phenyl-4-(trifluoromethyl)-1-{[2-(trimethylsilyl)ethoxy]methyl}-1H-pyrazolo[3,4-b]pyridine in 4 ml of a 1/1 DME/H₂O mixture under an inert atmosphere of argon are added 0.168 g (1.02 mmol) of [4-(dimethylamino)phenyl]boronic acid, 0.63 g (2.54 mmol) of potassium phosphate dihydrate and 19.6 mg (0.02 mmol) of tetrakis(triphenylphosphine)palladium. The reaction medium is heated at 150°C for 15 minutes by microwave. The reaction medium is hydrolysed with water and then extracted with ethyl acetate. The organic phase is dried over sodium sulfate and then concentrated under reduced pressure. The residue obtained is purified by column chromatography on silica gel, eluting with a heptane/dichloromethane mixture. 380 mg of a yellow solid are obtained.
MH⁺: 513
Melting point: 98°C

### N,N-dimethyl-4-[3-phenyl-4(trifluoromethyl)-1H-pyrazolo[3,4-b]pyridin-6-yl]aniline

To 0.38 g (0.74 mmol) of N,N-dimethyl-4-[3-phenyl-4-(trifluoromethyl)-1-{[2-(trimethylsilyl)ethoxy]methyl}-1H-pyrazolo[3,4-b]pyridin-6-yl]aniline are added 3.56 ml (3.56 mmol) of a 1N solution of TBAF in THF at room temperature under an inert atmosphere. The reaction medium is refluxed for 8 hours, a further 1 ml of the 1N solution of TBAF in THF is added, and heating is continued for 8 hours. This step is repeated three times and the reaction medium is then hydrolysed with water and concentrated under reduced pressure. The residue is taken up in an H₂O/methanol mixture. The precipitate obtained is filtered off, rinsed with water and dried at 50°C under reduced pressure for 18 hours. 260 mg of a yellow solid are obtained.
MH⁺: 383
Melting point: 227°C
¹H NMR (400 MHz, DMSO-de): δ 14.16 (br. s., 1 H) 8.14 (d, J=9.1 Hz, 2 H) 7.96 (s, 1 H) 7.44 - 7.54 (m, 5 H) 6.85 (d, J=9.1 Hz, 2 H) 3.03 (s, 6 H)

### Example 6: (compound 57 according to the invention)

### 2-Amino-5-[1-methyl-3-phenyl-4-(trifluoromethyl)-1H-pyrazolo[3,4-b]pyridin-6-yl]benzonitrile

### 6-bromo-1-methyl-3-phenyl-4-(trifluoromethyl)-1H-pyrazolo[3,4-b]pyridine

To 10 g (29 mmol) of 6-bromo-3-phenyl-4-(trifluoromethyl)-1H-pyrazolo[3,4-b]pyridine in 200 ml of anhydrous DMF, under an inert atmosphere of nitrogen, are added 2.18 ml (35 mmol) of methyl iodide and 4.8 g (35.08 mmol) of potassium carbonate, at room temperature. The reaction medium is stirred for 2 hours and then hydrolysed with water. The aqueous phase is extracted with ethyl acetate. The organic phase obtained is washed with water, dried over sodium sulfate and then concentrated under reduced pressure. The colourless oil obtained is purified by column chromatography on silica gel, eluting with a heptane/dichloromethane mixture. 7.03 g of a colourless oil are obtained.
MH⁺: 356

### 2-amino-5-(4,4,5,5-tetramethyl-1,3,2-dioxaborolan-2-yl)benzonitrile

To 3 g (19.7 mmol) of 2-amino-5-chlorobenzonitrile in 95 ml of dioxane under an inert atmosphere of argon are added 6 g (23.6 mmol) of 4,4,4',4'5,5,5',5'-octamethyl-2,2'-bi-1,3,2-dioxaborolane, 2.4 g (29.5 mmol) of sodium acetate, 540 mg (0.59 mmol) of tris(dibenzylideneacetone)dipalladium and 386 mg (1.38 mmol) of tricyclohexylphosphine. The reaction medium is heated at 90°C for 30 hours and is then hydrolysed with water and extracted with ethyl acetate. The organic phase is dried over sodium sulfate and then concentrated under reduced pressure. The residue obtained is taken up in petroleum ether. The precipitate obtained is filtered off and then dried under reduced pressure at 50°C for 18 hours. 2.81 g of a white solid are obtained.
MH⁺: 245

### 2-amino-5-[1-methyl-3-phenyl-4-(trifluoromethyl)-1H-pyrazolo[3,4-b]pyridin-6-yl]benzonitrile

To 250 mg (0.7 mmol) of 6-bromo-1-methyl-3-phenyl-4-(trifluoromethyl)-1H-pyrazolo[3,4-b]pyridine in 4 ml of DMF under an inert atmosphere of argon are added 0.205 g (0.84 mmol) of 2-amino-5-(4,4,5,5-tetramethyl-1,3,2-dioxaborolan-2-yl)benzonitrile, 0.556 g (2.11 mmol) of potassium phosphate dihydrate and 16 mg (0.01 mmol) of tetrakis(triphenylphosphine)palladium. The reaction medium is heated at 150°C for 15 minutes by microwave. The reaction medium is hydrolysed with water and then extracted with ethyl acetate. The organic phase is dried over sodium sulfate and then concentrated under reduced pressure. The residue obtained is purified by column chromatography on silica gel, eluting with a heptane/dichloromethane mixture. 170 mg of a white solid are obtained.
MH⁺: 394
Melting point: 269°C
¹H NMR (400 MHz, DMSO-d₆): δ 8.50 (d, J=2.2 Hz, 1 H) 8.36 (dd, J=8.9, 2.2 Hz, 1 H) 8.10 (s, 1 H) 7.45 - 7.53 (m, 5 H) 6.96 (d, J=8.9 Hz, 1 H) 6.65 (s, 2 H) 4.20 (s, 3 H)

### Example 7: (compound 108 according to the invention)

### 2-Amino-5-[2-methyl-3-phenyl-4-(trifluoromethyl)-2H-pyrazolo[3,4-b]pyridin-6-yl]benzonitrile

### 6-bromo-2-methyl-3-phenyl-4-(trifluoromethyl)-2H-pyrazolo[3,4-b]pyridine

To 10 g (29 mmol) of 6-bromo-3-phenyl-4-(trifluoromethyl)-1H-pyrazolo[3,4-b]pyridine in 200 ml of anhydrous DMF, under an inert atmosphere of nitrogen, are added 2.18 ml (35 mmol) of methyl iodide and 4.8 g (35.08 mmol) of potassium carbonate, at room temperature. The reaction medium is stirred for 2 hours and then hydrolysed with water. The aqueous phase is extracted with ethyl acetate. The organic phase obtained is washed with water, dried over sodium sulfate and then concentrated under reduced pressure. The colourless oil obtained is purified by column chromatography on silica gel, eluting with a heptane/dichloromethane mixture. 2.11 g of a colourless oil are obtained.
MH⁺: 356

### 2-amino-5-[2-methyl-3-phenyl-4-(trifluoromethyl)-2H-pyrazolo[3,4-b]pyridin-6-yl]benzonitrile

To 200 mg (0.56 mmol) of 6-bromo-2-methyl-3-phenyl-4-(trifluoromethyl)-2H-pyrazolo[3,4-b]pyridine in 3 ml of a 1/1 DME/H₂O mixture under an inert atmosphere of argon are added 0.164 g (0.67 mmol) of 2-amino-5-(4,4,5,5-tetramethyl-1,3,2-dioxaborolan-2-yl)benzonitrile, 0.418 g (1.68 mmol) of potassium phosphate dihydrate and 13 mg (0.01 mmol) of tetrakis(triphenylphosphine)palladium. The reaction medium is heated at 150°C for 15 minutes by microwave. The reaction medium is hydrolysed with water and then extracted with ethyl acetate. The organic phase is dried over sodium sulfate and then stirred for 2 hours in the presence of mercaptopropyl silica gel. After filtration, the organic phase is concentrated under reduced pressure. The residue obtained is taken up in methanol. The precipitate obtained is filtered off and then dried under reduced pressure at 50°C for 18 hours. 196 mg of a yellow solid are obtained.
MH⁺: 394
Melting point: 295°C
¹H NMR (400 MHz, DMSO-d₆):□□ δ □□08.39 (d, J=2.2 Hz, 1 H) 8.29 (dd, J=8.9, 2.2 Hz, 1 H) 8.01 (s, 1 H) 7.46 - 7.65 (m, 5 H) 6.94 (d, J=8.9 Hz, 1 H) 6.59 (s, 2 H) 3.92 (s, 3 H)

### Example 8: (compound 88 according to the invention)

### 2-Amino-5-[1-methyl-4-(trifluoromethyl)-1H-pyrazolo[3,4-b]pyridin-6-yl]benzonitrile

### 6-chloro-4-(trifluoromethyl)-1H-pyrazolo[3,4-b]pyridine

5 g (24.6 mmol) of 4-(trifluoromethyl)-1*H*-pyrazolo[3,4-b]pyridin-6-ol are dissolved in 50 ml of POCl₃ under an inert atmosphere of nitrogen. The reaction medium is heated at 80°C for 5 hours and then concentrated under reduced pressure. The residue is taken up in ethyl acetate and then hydrolysed with saturated aqueous sodium hydrogen carbonate solution. The reaction medium is extracted with ethyl acetate. The organic phase is washed with water, dried over sodium sulfate and then concentrated under reduced pressure. 5 g of a beige-coloured solid are obtained.
MH⁺: 222
Melting point: 112°C

### 6-chloro-1-methyl-4-(trifluoromethyl)-1H-pyrazolo[3,4-b]pyridine

To 2 g (9 mmol) of 6-chloro-4-(trifluoromethyl)-1H-pyrazolo[3,4-b]pyridine in 30 ml of anhydrous DMF, under an inert atmosphere of nitrogen, are added 0.67 ml (10.8 mmol) of methyl iodide and 3.5 g (10.83 mmol) of caesium carbonate, at room temperature. The reaction medium is stirred for 20 hours and then hydrolysed with water. The aqueous phase is extracted with ethyl acetate. The organic phase obtained is washed with water, dried over sodium sulfate and then concentrated under reduced pressure. The colourless oil obtained is purified by column chromatography on silica gel, eluting with a heptane/dichloromethane mixture. 1.42 g of a white solid are obtained.
MH⁺: 236
Melting point: 123°C

### 2-amino-5-[1-methyl-4-(trifluoromethyl)-1H-pyrazolo[3,4-b]pyridin-6-yl]benzonitrile

To 200 mg (0.85 mmol) of 6-chloro-1-methyl-4-(trifluoromethyl)-1H-pyrazolo[3,4-b]pyridine in 3 ml of a 1/1 DME/H₂O mixture under an inert atmosphere of argon are added 0.248 g (1.02 mmol) of 2-amino-5-(4,4,5,5-tetramethyl-1,3,2-dioxaborolan-2-yl)benzonitrile, 0.632 g (2.55 mmol) of potassium phosphate dihydrate and 19.6 mg (0.02 mmol) of tetrakis(triphenylphosphine)palladium. The reaction medium is heated at 150°C for 15 minutes by microwave. The reaction medium is hydrolysed with water and then extracted with dichloromethane. The organic phase is dried over sodium sulfate and then concentrated under reduced pressure. The residue obtained is taken up in THF. The solution obtained is stirred for 2 hours in the presence of mercaptopropyl silica gel (Sigma-Aldrich). After filtration, the medium is concentrated under reduced pressure. The residue obtained is taken up in methanol. The precipitate obtained is filtered off and then dried under reduced pressure at 50°C for 18 hours. 216 mg of a yellow solid are obtained.
MH⁺: 318
Melting point: 276°C
¹H NMR (400 MHz, DMSO-d₆) δ □□8.49 (d, J=2.2 Hz, 1 H) 8.34 (dd, J=8.9, 2.2 Hz, 1 H) 8.21 - 8.23 (m, 1 H) 8.14 (s, 1 H) 6.94 (d, J=9.0 Hz, 1 H) 6.64 (s, 2 H) 4.16 (s, 3 H)

### Example 9: (compound 114 according to the invention)

### 2-Amino-5-[2-methyl-4-(trifluoromethyl)-2H-pyrazolo[3,4-b]pyridin-6-yl]benzonitrile

### 6-chloro-2-methyl-4-(trifluoromethyl)-2H-pyrazolo[3,4-b]pyridine

To 2 g (9 mmol) of 6-chloro-4-(trifluoromethyl)-1 H-pyrazolo[3,4-b]pyridine in 30 ml of anhydrous DMF, under an inert atmosphere of nitrogen, are added 0.67 ml (10.8 mmol) of methyl iodide and 3.5 g (10.83 mmol) of caesium carbonate, at room temperature. The reaction medium is stirred for 20 hours and then hydrolysed with water. The aqueous phase is extracted with ethyl acetate. The organic phase obtained is washed with water, dried over sodium sulfate and then concentrated under reduced pressure. The colourless oil obtained is purified by column chromatography on silica gel, eluting with a heptane/dichloromethane mixture. 0.425 g of a yellow solid is obtained.
MH⁺: 236
Melting point: 124°C

### 2-amino-5-[2-methyl-4-(trifluoromethyl)-2H-pyrazolo[3,4-b]pyridin-6-yl]benzonitrile

To 425 mg (1.8 mmol) of 6-chloro-2-methyl-4-(trifluoromethyl)-2H-pyrazolo[3,4-b]pyridine in 10 ml of a 1/1 DME/H₂O mixture under an inert atmosphere of argon are added 0.528 g (2.16 mmol) of 2-amino-5-(4,4,5,5-tetramethyl-1,3,2-dioxaborolan-2-yl)benzonitrile, 1.34 g (5.41 mmol) of potassium phosphate dihydrate and 42 mg (0.04 mmol) of tetrakis(triphenylphosphine)palladium. The reaction medium is heated at 150°C for 15 minutes by microwave. The reaction medium is hydrolysed with water and then extracted with dichloromethane. The organic phase is dried over sodium sulfate and then concentrated under reduced pressure. The residue obtained is purified by column chromatography on silica gel, eluting with a heptane/dichloromethane mixture. The solid obtained is taken up in a dichloromethane/pentane mixture. The precipitate obtained is filtered off and then dried under reduced pressure at 50°C for 18 hours. 267 mg of a yellow solid are obtained.
MH⁺: 318
Melting point: 249°C
¹H NMR (400 MHz, DMSO-de) δ □□8.62 (s, 1 H) 8.39 (d, J=2.2 Hz, 1 H) 8.27 (dd, J=8.9, 2.2 Hz, 1 H) 8.08 (s, 1 H) 6.93 (d, J=8.9 Hz, 1 H) 6.58 (s, 2 H) 4.24 (s, 3 H)

### Example 10: (compound 72 according to the invention)

### 2-Amino-5-[4-(difluoromethyl)-1-methyl-1H-pyrazolo[3,4-b]pyridin-6-yl]benzonitrile

### 6-chloro-4-(difluoromethyl)-1H-pyrazolo[3,4-b]pyridine

To 5 g (60.2 mmol) of 3-aminopyrazole in an acetic acid/H₂O mixture are added 10 g (60.2 mmol) of ethyl 4,4-difluoro-3-oxobutanoate. The reaction medium is heated at 85°C for 8 hours. After cooling to room temperature, the precipitate obtained is filtered off, washed with water and then dried under reduced pressure. 7.2 g of a solid are obtained, and are taken up in 28.7 g (187.1 mmol) of POCl₃. The reaction medium is heated at 85°C for 4 hours and then concentrated under reduced pressure. After purification by chromatography on silica gel, eluting with an ethyl acetate/cyclohexane mixture, 2.56 g of a white solid are obtained.
MH⁺: 204

### 6-chloro-4-(difluoromethyl)-1-methyl-1H-pyrazolo[3,4-b]pyridine

To 1 g (4.91 mmol) of 6-chloro-4-(difluoromethyl)-1H-pyrazolo[3,4-b]pyridine in 20 ml of anhydrous DMF, under an inert atmosphere of nitrogen, are added 0.37 ml (5.89 mmol) of methyl iodide and 0.814 g (5.89 mmol) of potassium carbonate, at room temperature. The reaction medium is stirred for 20 hours and then hydrolysed with water. The aqueous phase is extracted with ethyl acetate. The organic phase obtained is washed with water, dried over sodium sulfate and then concentrated under reduced pressure. The colourless oil obtained is purified by column chromatography on silica gel, eluting with a heptane/dichloromethane mixture. 0.715 g of a white solid are obtained.
MH⁺: 218
Melting point: 105°C

### 2-amino-5-[4-(difluoromethyl)-1-methyl-1H-pyrazolo[3,4-b]pyridin-6-yl]benzonitrile

To 200 mg (0.92 mmol) of 6-chloro-4-(difluoromethyl)-1-methyl-1H-pyrazolo[3,4-b]pyridine in 4 ml of a 1/1 DME/H₂O mixture under an inert atmosphere of argon are added 0.269 g (1.10 mmol) of 2-amino-5-(4,4,5,5-tetramethyl-1,3,2-dioxaborolan-2-yl)benzonitrile, 0684 g (2.76 mmol) of potassium phosphate dihydrate and 21 mg (0.02 mmol) of tetrakis(triphenylphosphine)palladium. The reaction medium is heated at 150°C for 15 minutes by microwave. The reaction medium is hydrolysed with water and then extracted with dichloromethane. The organic phase is dried over sodium sulfate and then concentrated under reduced pressure. The residue obtained is taken up in THF. The solution obtained is stirred for 2 hours in the presence of mercaptopropyl silica gel (Sigma-Aldrich). After filtration, the medium is concentrated under reduced pressure. The residue obtained is taken up in methanol. The precipitate obtained is filtered off and then dried under reduced pressure at 50°C for 18 hours. 134 mg of a beige-coloured solid are obtained.
MH⁺: 300
Melting point: 251°C
¹H NMR (400 MHz, DMSO-de): δ □□8.36 (d, J=2.2 Hz, 1 H), 8.28 (dd, J=8.9, 2.2 Hz, 1 H), 8.16 (t, J=1.1 Hz, 1 H), 7.97 (t, J=1.3 Hz, 1 H), 7.38 (t, J=54.6 Hz, 1 H), 6.94 (d, J=8.9 Hz, 1 H), 6.60 (s, 2 H), 4.13 (s, 3 H)

### Example 11: (compound 106 according to the invention)

### 2-Amino-5-[4-difluoromethyl)-2-methyl-2H-pyrazolo[3,4-b]pyridin-6-yl]benzonitrile

### 6-chloro-4-(difluoromethyl)-2-methyl-2H-pyrazolo[3,4-b]pyridine

To 1 g (4.91 mmol) of 6-chloro-4-(difluoromethyl)-1H-pyrazolo[3,4-b]pyridine in 20 ml of anhydrous DMF, under an inert atmosphere of nitrogen, are added 0.37 ml (5.89 mmol) of methyl iodide and 0.814 g (5.89 mmol) of potassium carbonate, at room temperature. The reaction medium is stirred for 20 hours and then hydrolysed with water. The aqueous phase is extracted with ethyl acetate. The organic phase obtained is washed with water, dried over sodium sulfate and then concentrated under reduced pressure. The colourless oil obtained is purified by column chromatography on silica gel, eluting with a heptane/dichloromethane mixture. 0.145 g of a white solid are obtained.
MH⁺: 218
Melting point: 152°C

### 2-amino-5-[4-(difluoromethyl)-2-methyl-2H-pyrazolo[3,4-b]pyridin-6-yl]benzonitrile

To 145 mg (0.67 mmol) of 6-chloro-4-(difluoromethyl)-2-methyl-2H-pyrazolo[3,4-b]pyridine in 3 ml of a 1/1 DME/H₂O mixture under an inert atmosphere of argon are added 0.195 g (0.8 mmol) of 2-amino-5-(4,4,5,5-tetramethyl-1,3,2-dioxaborolan-2-yl)benzonitrile, 0.469 g (2 mmol) of potassium phosphate dihydrate and 15 mg (0.01 mmol) of tetrakis(triphenylphosphine)palladium. The reaction medium is heated at 150°C for 15 minutes by microwave. The reaction medium is hydrolysed with water and then extracted with dichloromethane. The organic phase is dried over sodium sulfate and then concentrated under reduced pressure. The residue obtained is taken up in THF. The solution obtained is stirred for 2 hours in the presence of mercaptopropyl silica gel. After filtration, the medium is concentrated under reduced pressure. The residue obtained is taken up in dichloromethane. The precipitate obtained is filtered off and then dried under reduced pressure at 50°C for 18 hours. The solid obtained is purified by column chromatography on silica gel, eluting with a toluene/acetone mixture. 0.015g of a yellow solid is obtained.
MH⁺: 300
¹H NMR (400 MHz, DMSO-de) δ 8.51 (s, 1 H), 8.26 (d, J=2.2 Hz, 1 H), 8.22 (dd, J=8.9, 2.2 Hz, 1 H), 7.91 (s, 1 H), 7.28 (t, J=54.9 Hz, 1 H), 6.93 (d, J=8.9 Hz, 1 H), 6.55 (s, 2 H), 4.22 (s, 3 H)

### Example 12: (compound 75 according to the invention)

### 2-Amino-5-[4-(difluoromethyl)-1H-pyrazolo[3,4-b]pyridin-6-yl]benzonitrile

### 6-chloro-4-(difluoromethyl)-1-(tetrahydro-2H-pyran-2-yl)-1H-pyrazolo[3,4-b]pyridine

To 10 g (49.12 mmol) of 6-chloro-4-(difluoromethyl)-1H-pyrazolo[3,4-b]pyridine in 200 ml of anhydrous dichloromethane, under an inert atmosphere of nitrogen, are added 5.38 ml (58.95 mmol) of 3,4-dihydro-2H-pyran and 0.934 g (4.91 mmol) of PTSA, at 0°C. The reaction medium is stirred for 3 hours at room temperature and then hydrolysed with water. The aqueous phase is extracted with dichloromethane. The organic phase obtained is washed with water, dried over sodium sulfate and then concentrated under reduced pressure. The residue obtained is taken up in a dichloromethane/pentane mixture. The precipitate obtained is filtered off, rinsed with pentane and then dried under reduced pressure at 50°C for 18 hours. 3.3 g of a beige-coloured powder are obtained after recrystallization from dichloromethane.
MH⁺: 288
Melting point: 93°C

### 2-amino-5-[4-(difluoromethyl)-1-(tetrahydro-2H-pyran-2-yl)-1H-pyrazolo[3,4-b]pyridin-6-yl]benzonitrile

To 700 mg (2.43 mmol) of 6-chloro-4-(difluoromethyl)-1(tetrahydro-2H-pyran-2-yl)-1H-pyrazolo[3,4-b]pyridine in 12 ml of a 1/1 DME/H₂O mixture under an inert atmosphere of argon are added 0.831 g (3.41 mmol) of 2-amino-5-(4,4,5,5-tetramethyl-1,3,2-dioxaborolan-2-yl)benzonitrile, 1.81 g (7.30 mmol) of potassium phosphate dihydrate and 53 mg (0.05 mmol) of tetrakis(triphenylphosphine)palladium. The reaction medium is heated at 150°C for 15 minutes by microwave. The reaction medium is hydrolysed with water and then extracted with dichloromethane. The organic phase is dried over sodium sulfate and then concentrated under reduced pressure. The residue obtained is purified by column chromatography on silica gel, eluting with a toluene/acetone mixture. The residue obtained is taken up in a dichloromethane/heptane mixture. The precipitate obtained is filtered off and then dried under reduced pressure at 50°C for 18 hours. The solid obtained is purified by column chromatography on silica gel, eluting with a toluene/acetone mixture. 0.6 g of a white solid is obtained.
MH⁺: 370
Melting point: 192°C

### 2-amino-5-[4-(difluoromethyl)-1H-pyrazolo[3,4-b]pyridin-6-yl]benzonitrile

To 339 mg (0.92 mmol) of 2-amino-5-[4-(difluoromethyl)-1-(tetrahydro-2H-pyran-2-yl)-1H-pyrazolo[3,4-b]pyridin-6-yl]benzonitrile in 4 ml of methanol is added 0.34 ml of a 4N solution of hydrogen chloride in dioxane at room temperature, under an inert atmosphere of nitrogen. The reaction medium is stirred for 4 hours and then hydrolysed with saturated aqueous sodium hydrogen carbonate solution. The precipitate obtained is filtered off, rinsed with water and then dried under reduced pressure at 50°C for 18 hours. 196 mg of a yellow powder are obtained.
MH⁺: 286
Melting point: 263°C
¹H NMR (400 MHz, DMSO-d₆) δ 13.90 (br. s., 1H), 8.27 (d, J= 2.2 Hz, 1H), 8.16 - 8.22 (m, 2 H), 7.94 (s, 1 H), 7.37 (t, J= 54.7 Hz, 1 H), 6.93 (d, J= 9.0 Hz, 1 H), 6.58 (s, 2 H)

### Example 13: (compound 83 according to the invention)

### 2-Amino-5-(4-(difluoromethyl)-1-[2-(dimethylamino)ethyl]-1H-pyrazolo[3,4-b]pyridin-6-yl}benzonitrile

### 6-chloro-4-(difluoromethyl)-3-iodo-1H-pyrazolo[3,4-b]pyridine

To 10 g (49.12 mmol) of 6-chloro-4-(difluoromethyl)-1H-pyrazolo[3,4-b]pyridine in 200 ml of dichloroethane are added 12.1 g (54.03 mmol) of N-iodosuccinimide at room temperature under an inert atmosphere of nitrogen. The reaction medium is refluxed for 9 hours and then hydrolysed with saturated aqueous sodium hydrogen carbonate solution. The reaction medium is extracted with dichloromethane. The organic phase is washed with water, dried over sodium sulfate and then concentrated under reduced pressure. The solid obtained is taken up in a minimum amount of dichloromethane, filtered off and then dried under reduced pressure at 50°C for 18 hours.
12.63 g of a beige-coloured solid are obtained.
MH⁺: 330
Melting point: 175°C

### 2-[6-chloro-4-(difluoromethyl)-3-iodo-1H-pyrazolo[3,4-b]pyridin-1-yl]-N,N-dimethylethanamine

To 2 g (6.07 mmol) of 6-chloro-4-(difluoromethyl)-3-iodo-1H-pyrazolo[3,4-b]pyridine in 30 ml of anhydrous DMF, under an inert atmosphere of nitrogen, are added 1 g (7.28 mmol) of 2-chloro-N,N-dimethylethanamine hydrochloride and 4.74 g (14.57 mmol) of caesium carbonate, at room temperature. The reaction medium is stirred for 6 hours, followed by addition of 0.5 g of 2-chloro-N,N-dimethylethanamine hydrochloride and 2.4 g of caesium carbonate. The reaction medium is stirred for 18 hours at room temperature and then hydrolysed with water. The aqueous phase is extracted with ethyl acetate. The organic phase obtained is washed with water, dried over sodium sulfate and then concentrated under reduced pressure. The brown oil obtained is purified by column chromatography on silica gel, eluting with a dichloromethane/methanol mixture. 1.51 g of a beige-coloured solid are obtained.
MH⁺: 401

### 2-[6-chloro-4-(difluoromethyl)-3-phenyl-1H-pyrazolo[3,4-b]pyridin-1-yl]-N,N-dimethylethanamine

To 200 mg (0.5 mmol) of 2-[6-chloro-4-(difluoromethyl)-3-iodo-1H-pyrazolo[3,4-b]pyridin-1-yl]-N,N-dimethylethanamine in 3 ml of a 1/1 DME/H₂O mixture under an inert atmosphere of argon are added 0.06 g (0.5 mmol) of phenylboronic acid, 0.371 g (1.5 mmol) of potassium phosphate dihydrate and 11 mg (0.01 mmol) of tetrakis(triphenylphosphine)palladium. The reaction medium is heated at 90°C in a sealed tube for 24 hours. The reaction medium is hydrolysed with water and then extracted with dichloromethane. The organic phase is dried over sodium sulfate and then concentrated under reduced pressure. The residue obtained is purified by column chromatography on silica gel, eluting with a dichloromethane/methanol mixture. 0.07 g of a yellow oil is obtained.
MH⁺: 351

### 2-amino-5-{4-(difluoromethy))-1-[2-(dimethylamino)ethy)]-1H-pyrazolo[3,4-b]pyridin-6-yl}benzonitrile

To 213 mg (0.61 mmol) of 2-[6-chloro-4-(difluoromethyl)-3-phenyl-1H-pyrazolo[3,4-b]pyridin-1-yl]-N,N-dimethylethanamine in 3 ml of a 1/1 DME/H₂O mixture under an inert atmosphere of argon are added 0.178 g (0.73 mmol) of 2-amino-5-(4,4,5,5-tetramethyl-1,3,2-dioxaborolan-2-yl)benzonitrile, 0.452 g (1.82 mmol) of potassium phosphate dihydrate and 14 mg (0.01 mmol) of tetrakis(triphenylphosphine)palladium. The reaction medium is heated at 150°C for 15 minutes by microwave. The reaction medium is hydrolysed with water and then extracted with dichloromethane. The organic phase is dried over sodium sulfate and then concentrated under reduced pressure. The residue obtained is purified by column chromatography on silica gel, eluting with a dichloromethane/methanol mixture. The residue obtained is taken up in a dichloromethane/pentane mixture. The precipitate obtained is filtered off and then dried under reduced pressure at 50°C for 18 hours. 0.161 g of a white solid is obtained.
MH⁺: 433
Melting point: 163°C
¹H NMR (400 MHz, DMSO-de) δ 8.40 (d, J=2.2 Hz, 1 H), 8.30 (dd, J=9.0, 2.2 Hz, 1 H), 7.96 (s, 1 H), 7.65 (dd, J=7.7, 1.7 Hz, 2 H), 7.46 - 7.55 (m, 3 H), 7.28 (t, J=54.6 Hz, 1 H), 6.96 (d, J=9.0 Hz, 1 H), 6.62 (s, 2 H), 4.69 (t, J=6.3 Hz, 2 H), 2.85 (t, J=6.3 Hz, 2 H), 2.21 (s, 6 H)

### Example 14: (compound 93 according to the invention)

### 1-Methyl-6-[3-(morpholin-4-ylmethyl)phenyl]-3-(pyridin-3-yl)-4-(trifluoromethyl)-1H-pyrazolo[3,4-b]pyridine

### 6-chloro-3-iodo-4-(trifluoromethyl)-1H-pyrazolo[3,4-b]pyridine

To 3 g (13.54 mmol) of 6-chloro-4-(trifluoromethyl)-1H-pyrazolo[3,4-b]pyridine in 50 ml of dichloroethane are added 3.35 g (14.89 mmol) of N-iodosuccinimide at room temperature under an inert atmosphere of nitrogen. The reaction medium is refluxed for 9 hours, followed by addition of 600 mg of N-iodosuccinimide. The reaction medium is refluxed for 9 hours and then hydrolysed with saturated aqueous sodium hydrogen carbonate solution. The reaction medium is extracted with dichloromethane. The organic phase is washed with water, dried over sodium sulfate and then concentrated under reduced pressure. The solid obtained is taken up in a minimum amount of dichloromethane, filtered off and then dried under reduced pressure at 50°C for 18 hours. 3.8 g of a beige-coloured solid are obtained.
MH⁺: 347
Melting point: 204°C

### 6-chloro-3-iodo-1-methyl-4-(trifluoromethyl)-1H-pyrazolo[3,4-b]pyridine

To 3.8 g (10.94 mmol) of 6-chloro-3-iodo-4-(trifluoromethyl)-1H-pyrazolo[3,4-b]pyridine in 40 ml of anhydrous DMF, under an inert atmosphere of nitrogen, are added 0.82 ml (13.12 mmol) of methyl iodide and 4.27 g (13.12 mmol) of caesium carbonate, at room temperature. The reaction medium is stirred for 6 hours and then hydrolysed with water. The aqueous phase is extracted with ethyl acetate. The organic phase obtained is washed with water, dried over sodium sulfate and then concentrated under reduced pressure. The solid obtained is purified by column chromatography on silica gel, eluting with a heptane/dichloromethane mixture. 2.94 g of a beige-coloured solid are obtained.
MH⁺: 362

### 6-chloro-1-methyl-3-(pyridin-3-yl)-4-(trifluoromethyl)-1H-pyrazolo[3,4-b]pyridine

To 1.2 g (3.32 mmol) of 6-chloro-3-iodo-1-methyl-4-(trifluoromethyl)-1H-pyrazolo[3,4-b]pyridine in 16 ml of a 1/1 DME/H₂O mixture under an inert atmosphere of argon are added 0.490 g (3.98 mmol) of 3-pyridylboronic acid, 2.47 g (9.96 mmol) of potassium phosphate dihydrate and 77 mg (0.07 mmol) of tetrakis(triphenylphosphine)palladium. The reaction medium is heated at 90°C in a sealed tube for 24 hours. The reaction medium is hydrolysed with water and then extracted with dichloromethane. The organic phase is dried over sodium sulfate and then concentrated under reduced pressure. The residue obtained is purified by column chromatography on silica gel, eluting with a dichloromethane/methanol mixture. The residue obtained is taken up in a dichloromethane/pentane mixture. The precipitate obtained is filtered off and then dried under reduced pressure at 50°C for 18 hours. 0.298 g of a brown solid is obtained.
MH⁺: 313
Melting point: 147°C

### 1-methyl-6-[3-(morpholin-4-ylmethyl)phenyl]-3-(pyridin-3-yl)-4-(trifluoromethyl)-1H-pyrazolo[3,4-b]pyridine

To 149 mg (0.48 mmol) of 6-chloro-1-methyl-3-(3-pyridyl)-4-(trifluoromethyl)-1H-pyrazolo[3,4-b]pyridine in 4.8 ml of a 1/1 DME/H₂O mixture under an inert atmosphere of argon are added 0.173 g (0.57 mmol) of 4-[3-(4,4,5,5-tetramethyl-1,3,2-dioxaborolan-2-yl)benzyl]morpholine, 0.355 g (1.43 mmol) of potassium phosphate dihydrate and 11 mg (0.01 mmol) of tetrakis(triphenylphosphine)palladium. The reaction medium is heated at 150°C for 15 minutes by microwave. The reaction medium is hydrolysed with water and then extracted with dichloromethane. The organic phase is dried over sodium sulfate and then concentrated under reduced pressure. The residue obtained is purified by column chromatography on silica gel, eluting with a dichloromethane/methanol mixture. After recrystallization from diisopropyl ether, 0.106 g of a white solid is obtained.
MH⁺: 454
Melting point: 155°C
¹H NMR (400 MHz, DMSO-d₆) δ 8.68 - 8.74 (m, 2 H), 8.22 - 8.29 (m, 2 H), 8.19 (s, 1 H), 7.96 (dt, J=7.9 Hz, 1.7 Hz, 1 H), 7.50 - 7.61 (m, 3 H), 4.28 (s, 3 H), 3.55 - 3.69 (m, 6 H), 2.43 (m, 4 H)

### Example 15: (compound 91 according to the invention)

### 2-Amino-5-[4-(difluoromethyl)-3-(pyridin-3-yl)-1H-pyrazolo[3,4-b]pyridin-6-yl]benzonitrile

### 6-chloro-4-(difluoromethyl)-3-iodo-1-(tetrahydro-2H-pyran-2-yl)-1H-pyrazolo[3,4-b]pyridine

To 11.3 g (34.45 mmol) of 6-chloro-4-(difluoromethyl)-3-iodo-1H-pyrazolo[3,4-b]pyridine in 150 ml of anhydrous dichloromethane, under an inert atmosphere of nitrogen, are added 3.77 ml (41.34 mmol) of dihydropyran and 0.655 g (3.44 mmol) of PTSA, at 0°C. The reaction medium is stirred for 3 hours at room temperature and then hydrolysed with water. The aqueous phase is extracted with dichloromethane. The organic phase obtained is washed with water, dried over sodium sulfate and then concentrated under reduced pressure. The residue obtained is taken up in a dichloromethane/pentane mixture. The precipitate obtained is filtered off, rinsed with pentane and then dried under reduced pressure at 50°C for 18 hours. 11.93 g of a beige-coloured powder are obtained.
MH⁺: 413
Melting point: 157°C

### 6-chloro-4-(difluoromethyl)-3-(pyridin-3-yl)-1-(tetrahydro-2H-pyran-2-yl)-1H-pyrazolo[3,4-b]pyridine

To 0.8 g (1.93 mmol) of 6-chloro-4-(difluoromethyl)-3-iodo-1-(tetrahydro-2H-pyran-2-yl)-1H-pyrazolo[3,4-b]pyridine in 10 ml of a 1/1 DME/H₂O mixture under an inert atmosphere of argon are added 0.237 g (1.93 mmol) of 3-pyridylboronic acid, 1.44 g (9.96 mmol) of potassium phosphate dihydrate and 45 mg (0.04 mmol) of tetrakis(triphenylphosphine)palladium. The reaction medium is heated at 90°C in a sealed tube for 24 hours. The reaction medium is hydrolysed with water and then extracted with dichloromethane. The organic phase is dried over sodium sulfate and then concentrated under reduced pressure. The residue obtained is purified by column chromatography on silica gel, eluting with a dichloromethane/methanol mixture. The residue obtained is taken up in a dichloromethane/pentane mixture. The precipitate obtained is filtered off and then dried under reduced pressure at 50°C for 18 hours. 0.517 g of a yellow solid is obtained.
MH⁺: 365

### 2-amino-5-[4-(difluoromethyl)-3-(pyridin-3-yl)-1-(tetrahydro-2H-pyran-2-yl)-1H-pyrazolo[3,4-b]pyridin-6-yl]benzonitrile

To 200 mg (0.55 mmol) of 6-chloro-4-(difluoromethyl)-3-(pyridin-3-yl)-1-(tetrahydro-2H-pyran-2-yl)-1H-pyrazolo[3,4-b]pyridine in 4 ml of a 1/1 DME/H₂O mixture under an inert atmosphere of argon are added 0.160 g (0.66 mmol) of 2-amino-5-(4,4,5,5-tetramethyl-1,3,2-dioxaborolan-2-yl)benzonitrile, 0.408 g (1.64 mmol) of potassium phosphate dihydrate and 13 mg (0.01 mmol) of tetrakis(triphenylphosphine)palladium. The reaction medium is heated at 150°C for 15 minutes by microwave. The reaction medium is hydrolysed with water and then extracted with dichloromethane. The organic phase is dried over sodium sulfate and then concentrated under reduced pressure. The residue obtained is purified by column chromatography on silica gel, eluting with a dichloromethane/methanol mixture. The residue obtained is taken up in a dichloromethane/pentane mixture. The precipitate obtained is filtered off and then dried under reduced pressure at 50°C for 18 hours. 0.204 g of a yellow solid is obtained.
MH⁺: 447
Melting point: 140°C

### 2-amino-5-[4-(difluoromethyl)-3-(pyridin-3-yl)-1H-pyrazolo[3,4-b]pyridin-6-yl]benzonitrile

To 204 mg (0.46 mmol) of 2-amino-5-[4-(difluoromethyl)-3-(pyridin-3-yl)-1-(tetrahydro-2H-pyran-2-yl)-1H-pyrazolo[3,4-b]pyridin-6-yl]benzonitrile in 5 ml of an 8/2 dioxane/acetone mixture is added 0.57 ml of a 4N solution of hydrogen chloride in dioxane at room temperature, under an inert atmosphere of nitrogen. The reaction medium is stirred for 24 hours, followed by addition of methanol and 0.6 ml of a 4N solution of hydrogen chloride in dioxane. The reaction medium is stirred for 24 hours and then hydrolysed with saturated aqueous sodium hydrogen carbonate solution. The precipitate obtained is filtered off, rinsed with water and then dried under reduced pressure at 50°C for 18 hours. 131 mg of a yellow powder are obtained.
MH⁺: 363
Melting point: 296°C
¹H NMR (400 MHz, DMSO-d₆): δ 14.26 (br. s., 1 H), 8.82 (d, J=1.6 Hz, 1 H), 8.68 (dd, J=4.8, 1.6 Hz, 1 H), 8.33 (d, J=2.1 Hz, 1 H), 8.24 (dd, J=8.9, 2.1 Hz, 1 H), 8.05 (dt, J=7.9, 1.8 Hz, 1 H), 7.98 (s, 1 H), 7.54 (dd, J=7.9, 4.8 Hz, 1 H), 7.28 (t, J=54.7 Hz, 1 H), 6.95 (d, J=8.9 Hz, 1 H), 6.62 (s, 2 H)

The table that follows illustrates the chemical structures and physical properties of a number of examples of compounds according to the invention and outside the invention. In this table:
- Me and Et represent, respectively, methyl and ethyl groups;
- Ph represents a phenyl group;
- "m.p." represents the melting point of the compound, expressed in degrees Celsius;
- "M+H⁺" represents the mass of the compound, obtained by LC-MS (Liquid Chromatography - Mass Spectroscopy). The high-performance liquid chromatography analytical method used is detailed below:
   Column: Kromasil, 50x2.1 mm, 3.5 □m
   Solvent A: H₂O/ACN/TFA (1000/30/0.5); solvent B: ACN/TFA (1000/0.5); flow rate = 0.5 mL/min
      Gradient: 100/0 (0 min) to 0/100 (12 min) to 0/100 (15 min)
      Detection: 220 nM
   Ionization: ESI+
- in the "salt" column, "/" represents a compound in free base form, whereas "HCl" represents a compound in hydrochloride form and TFA represents a compound in the form of the trifluoroacetic acid salt.

**Table**

| | | | | | | | |
|---|---|---|---|---|---|---|---|
| 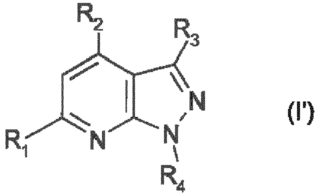 | | | | | | | |

| **No.** | **R₂** | **R₃** | **R₁** | **R₄** | **Salt** | **m.p. (°C)** | **M+H⁺** |
|---|---|---|---|---|---|---|---|
| **1 (Invention)** | CF₃ | Ph | 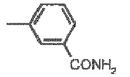 | Me | / | / | 397 |
| **2 (Invention)** | CF₃ | Ph | 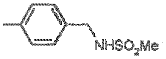 | Me | / | / | 461 |
| **3 (Invention)** | CF₃ | Ph | 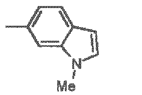 | Me | / | / | 407 |
| **4 (Invention)** | CF₃ | Ph | 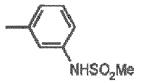 | Me | / | / | 447 |
| 5 **(Invention)** | CF₃ | Ph | 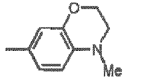 | Me | / | / | 426 |
| **6 (Invention)** | CF₃ | Ph | 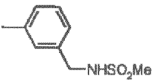 | Me | / | / | 461 |
| **7 (Invention)** | CF₃ | Ph | 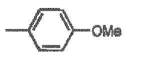 | Me | / | / | 384 |
| 8 **(Invention)** | CF₃ | Ph | 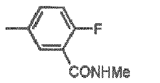 | Me | / | / | 429 |
| **9 (Invention)** | CF₃ | Ph | 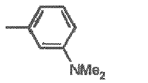 | Me | / | / | 397 |
| **10 (Invention)** | CF₃ | Ph | 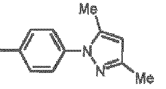 | Me | / | / | 448 |
| **11 (Invention)** | CF₃ | Ph | 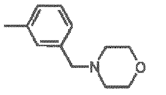 | Me | / | / | 453 |
| **12 (Invention)** | CF₃ | Ph | 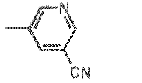 | Me | / | / | 380 |
| **13 (Invention)** | CF₃ | Ph | 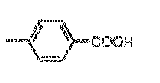 | Me | / | / | 398 |
| **14 (Invention)** | CF₃ | Ph | 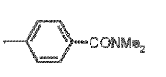 | Me | / | / | 425 |
| **15 (invention)** | CF₃ | Ph | 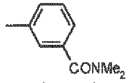 | Me | / | / | 425 |
| **16 (Invention)** | CF₃ | Ph | 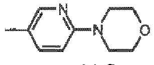 | Me | / | / | 440 |
| **17 (Invention)** | CF₃ | Ph | 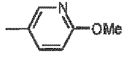 | Me | / | / | 385 |
| **18 (Invention)** | CF₃ | Ph | 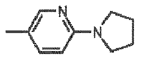 | Me | / | / | 424 |
| **19 (Outside invention)** | CF₃ | Ph | 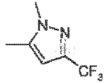 | Me | / | / | 426 |
| **20 (Invention)** | CF₃ | Ph | 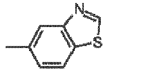 | Me | / | / | 411 |
| **21 (Invention)** | CF₃ | Ph | 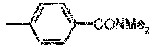 | H | HCl | / | 411 |
| **22 (Invention)** | CF₃ | Ph | 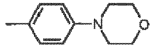 | H | HCl | / | 425 |
| **23 (Invention)** | CF₃ | Ph | 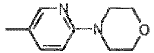 | H | HCl | / | 426 |
| **24 (Invention)** | CF₃ | Ph | 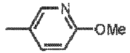 | H | HCl | / | 371 |
| **25 (Invention)** | CF₃ | Ph | 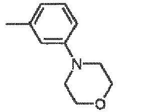 | H | HCl | / | 425 |
| **26 (Invention)** | CF₃ | Ph | 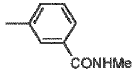 | H | HCl | / | 397 |
| **27 (Invention)** | CF₃ | Ph | 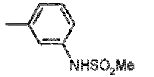 | H | HCl | / | 433 |
| **28 (Invention)** | CF₃ | Ph | 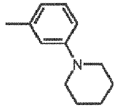 | H | HCl | / | 423 |
| **29 (Invention)** | CF₃ | Ph | 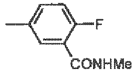 | H | HCl | / | 415 |
| **30 (Invention)** | CF₃ | Ph | 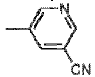 | H | HCl | / | 366 |
| **31 (Invention)** | CHF₂ | Ph | 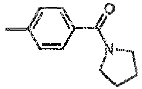 | H | HCl | 248 | 455 |
| **32 (Outside invention)** | CHF₂ | Ph | 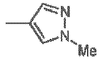 | H | HCl | / | 362 |
| **33 (Outside invention)** | CHF₂ | Ph | 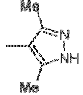 | H | HCl | / | 376 |
| **34 (Invention)** | CHF₂ | Ph | 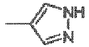 | H | HCl | / | 348 |
| **35 (Invention)** | CHF₂ | Ph | 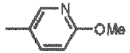 | H | HCl | / | 389 |
| **36 (Invention)** | CHF₂ | Ph | 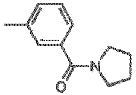 | H | HCl | / | 455 |
| **37 (Invention)** | CHF₂ | Ph | 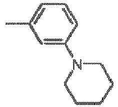 | H | HCl | / | 441 |
| **38 Ex.2 (Outside invention)** | COOH | H | 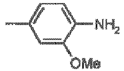 | H | / | / | 285 |
| **39 (Invention)** | CONHMe | Ph | 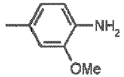 | H | TFA | / | 458 |
| **40 (Invention)** | CONH₂ | Ph | 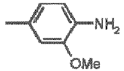 | H | TFA | / | 474 |
| **41 (Invention)** | CONHMe | H | 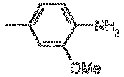 | H | TFA | / | 412 |
| **42 (Invention)** | CONH₂ | H | 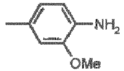 | H | TFA | / | 398 |
| **43 (Outside invention)** | COOH | Ph | 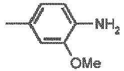 | H | / | / | 361 |
| **44 (Outside invention)** | COOH | Ph | 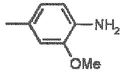 | Me | / | / | 375 |
| **45 (Outside invention)** | COOH | H | 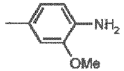 | Me | / | / | 299 |
| **46 Ex. 1 (Invention)** | CONH₂ | Ph | 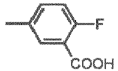 | H | TFA | / | 377 |
| 47 **(Invention)** | CONH₂ | Ph | 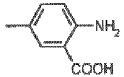 | H | / | / | 374 |
| **48 (Invention)** | CONH₂ | Ph | 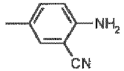 | H | TFA | / | 355 |
| **49 (Invention)** | CONH₂ | 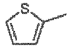 | 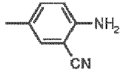 | H | / | / | 361 |
| **50 (Outside invention)** | COOH | cPr | 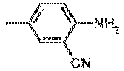 | H | / | / | 320 |
| **51 (Invention)** | CONH₂ | H | 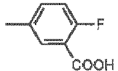 | H | / | / | 301 |
| **52 (Invention)** | CONH₂ | Ph | 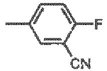 | H | / | / | 358 |
| **53 Ex. 3 (Invention)** | CHF₂ | Ph | 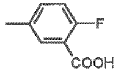 | H | / | / | 384 |
| **54 (Invention)** | CF₃ | Ph | 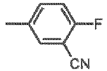 | H | / | / | 383 |
| **55 (Invention)** | CHF₂ | Ph | 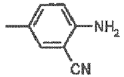 | H | HCl | 282 | 362 |
| **56 Ex. 4 (Invention)** | CF₃ | Ph | 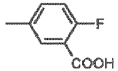 | H | / | / | 402 |
| **57 Ex. 6 (Invention)** | CF₃ | Ph | 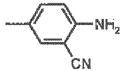 | Me | / | 269 | 394 |
| **58 (Invention)** | CF₃ | Ph | 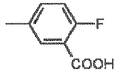 | Me | / | / | 416 |
| **59 (Invention)** | CF₃ | Ph | 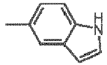 | H | / | / | 379 |
| **60 (Invention)** | CF₃ | Ph | 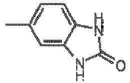 | H | / | 380 | 396 |
| **61 Ex. 5 (Invention)** | CF₃ | Ph | 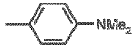 | H | / | 227 | 383 |
| **62 (Invention)** | CONHPh | H | 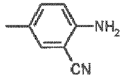 | H | HCl | / | 355 |
| **63 (Invention)** | 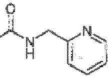 | H | 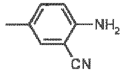 | H | HCl | / | 370 |
| **64 (Invention)** | 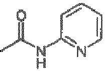 | H | 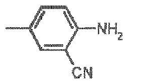 | H | HCl | / | 356 |
| **65 (Invention)** | 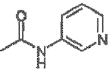 | H | 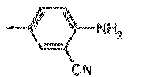 | H | HCl | / | 356 |
| **66 (Invention)** | 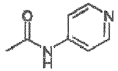 | H | 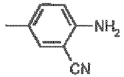 | H | HCl | / | 356 |
| **67 (Invention)** | CHF₂ | 4-Py | 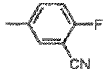 | Me | / | / | 380 |
| **68 (Invention)** | CONHPh | H | 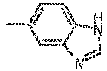 | H | HCl | / | 355 |
| **69 (Invention)** | 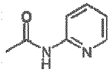 | H | 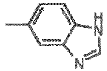 | H | HCl | / | 356 |
| **70 (Invention)** | 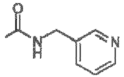 | H | 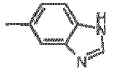 | H | HCl | / | 370 |
| **71 (Invention)** | CONHPh | H | 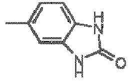 | H | / | / | 371 |
| **72 Ex. 10 (Invention)** | CHF₂ | H | 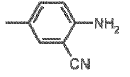 | Me | / | 251 | 300 |
| **73 (Invention)** | CHF₂ | H | 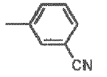 | Me | / | 162 | 285 |
| **74 (Invention)** | CHF₂ | H | 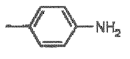 | Me | / | 149 | 275 |
| **75 Ex. 12 (Invention)** | CHF₂ | H | 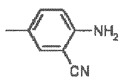 | H | / | 263 | 286 |
| **76 (Invention)** | CF₃ | Ph | 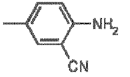 | 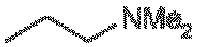 | / | 183 | 451 |
| **77 (Invention)** | CF₃ | Ph | 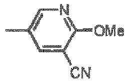 | Me | / | 250 | 410 |
| **78 (Invention)** | CHF₂ | Ph | 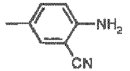 | Me | / | 246 | 376 |
| **79 (Invention)** | CHF₂ | Ph | 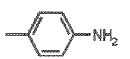 | Me | / | 176 | 351 |
| **80 (Invention)** | CHF₂ | Ph | 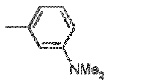 | Me | / | 154 | 379 |
| **81 (Invention)** | CF₃ | Ph | 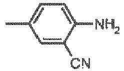 | 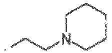 | / | 192 | 491 |
| **82 (Invention)** | CF₃ | Ph | 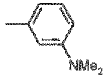 | 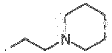 | HCl | 227 | 494 |
| **83 Ex. 13 (Invention)** | CHF₂ | Ph | 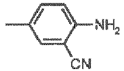 | 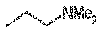 | / | 163 | 433 |
| **84 (Invention)** | CF₃ | H | 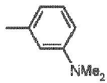 | 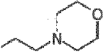 | / | 110 | 420 |
| **85 (Invention)** | CF₃ | H | 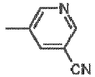 | 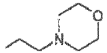 | / | / | 403 |
| **86 (Invention)** | CF₃ | H | 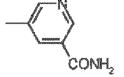 | 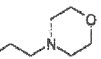 | / | 238 | 421 |
| **87 (Invention)** | CF₃ | H | 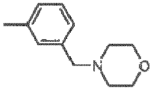 | Me | / | 105 | 377 |
| **88 Ex. 8 (Invention)** | CF₃ | H | 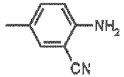 | Me | / | 276 | 318 |
| **89 (Invention)** | CF₃ | Ph | 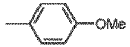 | Me | / | 181 | 384 |
| **90 (Invention)** | CF₃ | H | 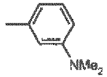 | Me | / | 91 | 321 |
| **91 Ex. 15 (Invention)** | CHF₂ | 3-Py | 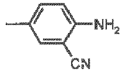 | H | / | 296 | 363 |
| **92 (Invention)** | CHF₂ | 4-Py | 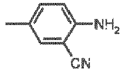 | H | / | 325 | 363 |
| **93 Ex. 14 (Invention)** | CF₃ | 3-Py | 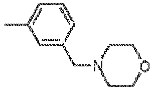 | Me | / | 155 | 454 |
| **94 (Invention)** | CHF₂ | MeO-Ph | 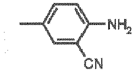 | Me | / | 233 | 392 |
| **95 (Invention)** | CF₃ | H | 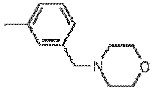 | Pr | HCl | 271 | 405 |
| **96 (Invention)** | CF₃ | H | 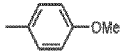 | Pr | / | 72 | 336 |
| **97 (Invention)** | CF₃ | MeO-Ph | 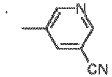 | Me | / | 194 | 410 |
| **98 (Invention)** | CF₃ | MeO-Ph | 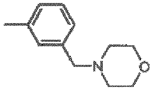 | Me | / | 114 | 483 |
| **99 (Invention)** | CF₃ | MeO-Ph | 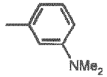 | Me | / | 138 | 427 |
| **100 (Invention)** | CF₃ | MeO-Ph | 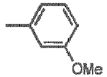 | Me | / | 133 | 414 |
| **101 (Invention)** | CONH₂ | 3-Py | 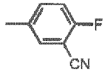 | H | / | / | 377 |

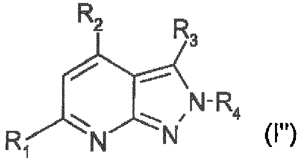

| **No.** | **R₂** | **R₃** | **Ri** | **R₄** | **Salt** | **m.p.(°C)** | **M+H⁺** |
|---|---|---|---|---|---|---|---|
| **102 (Outside invention)** | COOH | H | 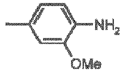 | Me | / | / | 299 |
| **103 (Invention)** | CONH₂ | H | 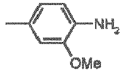 | Me | TFA | / | 412 |
| **104 (Outside invention)** | COOH | H | 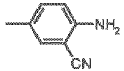 | Me | / | / | 294 |
| **105 (Invention)** | CHF₂ | H | 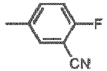 | Me | / | / | 303 |
| **106 Ex. 11 (Invention)** | CHF₂ | H | 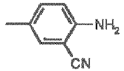 | Me | / | / | 300 |
| **107 (Invention)** | CHF₂ | H | 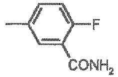 | Me | / | / | 321 |
| **108 Ex. 7 (Invention)** | CF₃ | Ph | 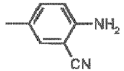 | Me | / | 295 | 394 |
| **109 (Invention)** | CF₃ | Ph | 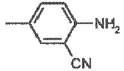 | 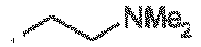 | / | 237 | 451 |
| **110 (Invention)** | CF₃ | Ph | 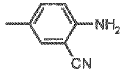 | 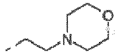 | / | 249 | 493 |
| **111 (Invention)** | CHF₂ | H | 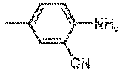 | 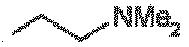 | / | 182 | 357 |
| **112 (Invention)** | CHF₂ | H | 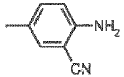 | 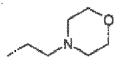 | / | 242 | 399 |
| **113 (Invention)** | CF₃ | H | 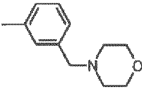 | 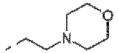 | / | 101 | 476 |
| **114 Ex. 9 (Invention)** | CF₃ | H | 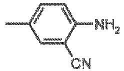 | Me | / | 249 | 318 |
| **115 (Invention)** | CF₃ | H | 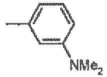 | Pr | HCl | 181 | 425 |
| **116 (Invention)** | CHF₂ | H | 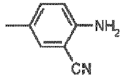 | 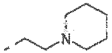 | / | 230 | 397 |
| **117 (Invention)** | CHF₂ | H | 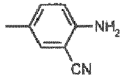 | Pr | / | 214 | 328 |
| **118 (Invention)** | CF₃ | H | 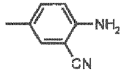 | Pr | / | 239 | 346 |
| **(Invention)** | CF₃ | H | 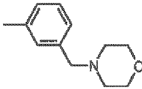 | Pr | / | 288 | 405 |
| **120 (Invention)** | CF₃ | H | 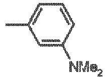 | Pr | / | 89 | 349 |

The compounds according to the invention and outside the invention underwent pharmacological trials to determine their inhibitory effect on the FGF receptors.

### Example 16: in vitro angiogenesis of HUVEC cells induced with FGF-2

In order to demonstrate the capacity of the FGF-R antagonists of the present invention to inhibit FGF-induced angiogenesis, *in vitro* angiogenesis experiments were performed with human endothelial cells of HUVEC type, stimulated with FGF-2 or b-FGF.

To do this, matrices composed of matrigel (growth factor reduced matrigel, Becton Dickinson 356230) and of collagen (rat tail collagen type I, Becton Dickinson 354236) are deposited in an amount of 160 µl into each chamberslide well (Biocoat Cellware collagen, Type I, 8-well culturesides: Becton dickinson 354630), or 60 µl per well of 96-plate wells (Biocoat collagenl cellware, Becton Dickinson 354407). The matrix is prepared by mixing 1/3 of matrigel, 1 mg/ml final of collagen, 0.1 N NaOH (0.026× the volume of collagen in µl), PBS 1x, and the volume is then adjusted with water. The gels are kept for 1 hour at 37°C to allow their polymerization. Next, the human venous endothelial cells (HUVEC ref.: C-12200 - Promocell) were seeded at 15×10³ or 6×10³ cells/well in 400 or 120 µl (for the 8-well or 96-well plates, respectively) of EBM medium (Clonetics C3121) + 2% FBS + hEGF 10 µg/ml. They are stimulated with 1 or 3 ng/ml of FGF-2 (R&D system, 133-FB-025; Invitrogen, PHG0026) for 24 hours at 37°C in the presence of 5% CO₂. After 24 hours, the length of the network of microtubules formed is measured by means of the computer-assisted image analysis system (Imagenia Biocom, Courtaboeuf, France) and the total length of the pseudotubules in each well is determined. The mean total length of the microcapillary network is calculated in µm for each condition corresponding to the mean on 6 replicates

Stimulation with FGF-2 allows induction of the formation of new tubules. An FGF-R antagonist is considered as being active in this test if it is capable of partially inhibiting this angiogenesis at a dose of less than or equal to 300 nM.

### Example of screening of FGF-R antagonists

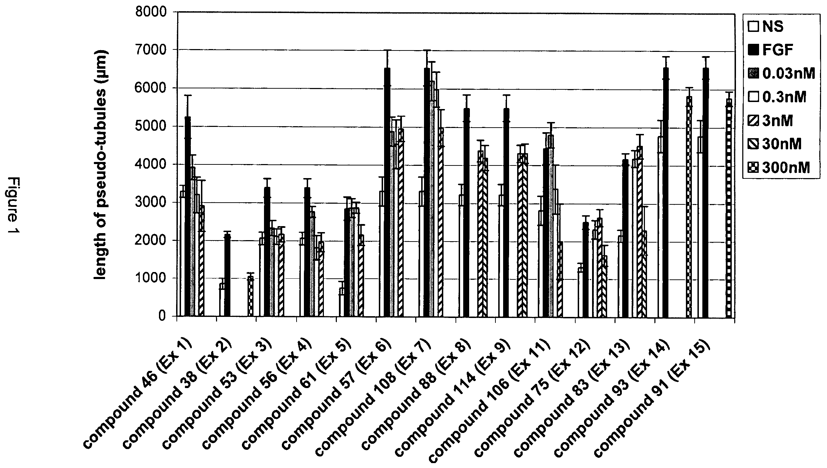

In this experiment, the molecules are evaluated from 0.03 nM to 300 nM depending on the molecule with regard to induction of the angiogenesis of human HUVEC cells with FGF-2. Compounds 38 outside the invention (Example 2), 46 according to the invention (Example 1), 53 according to the invention (Example 3), 56 according to the invention (Example 4), 57 according to the invention (Example 6), 61 according to the invention (Example 5), 75 according to the invention (Example 12), 83 according to the invention (Example 13), 88 according to the invention (Example 8), 91 according to the invention (Example 15), 93 according to the invention (Example 14), 106 according to the invention (Example 11), 108 according to the invention (Example 7) and 114 according to the invention (Example 9) are active since they have inhibitory activity on the formation of pseudotubules of greater than or equal to 20% at a dose of less than or equal to 300 nM (figure 1).

### Example 17: Model of inflammatory angiogenesis in mice

Angiogenesis is required for the development of chronic inflammatory diseases such as rheumatoid arthritis. The formation of new blood vessels allows not only the perfusion of the pathological tissues but also the transportation of cytokines which are responsible for establishing the chronic state of the disease.

The model described by Colville-NH et al. in 1995 makes it possible to study pharmacological agents that are capable of modulating the appearance of angiogenesis in an inflammatory context. The model is developed on female OF1 mice (Charles River laboratories) weighing about 25 g, and per group of 12. The animals are anaesthetized intraperitoneally with pentobarbital sodium (60 mg/kg; Sanofi Nutrition Santé Animale). An air pocket is created on the mouse's back by injecting 3 ml of air subcutaneously. After waking up, the animals receive a treatment in general by gavage and receive an injection of 0.5 ml of Freud's adjuvant (Sigma) with 0.1% croton oil (Sigma) into the pocket. Seven days later, the mice are again anaesthetized and placed on a hotplate at 40°C. 1 ml of carmine red (Aldrich Chemicals, 5% in 10% of gelatin) is injected into the caudal vein. The animals are then placed at 4°C for 2-3 hours. The skins are then removed and dried for 24 hours in an oven at 56°C. The dry tissues are weighed and placed in 1.8 ml of digestion solution (dithiothreitol 2 mM, Na₂HPO₄ 20 mM, EDTA 1 mM, papain 12 U/ml) for 24 hours. The dye is then dissolved in 0.2 ml of 5M NaOH. The skins are centrifuged at 2000 rpm for 10 minutes at room temperature. The supernatants are filtered through 0.2 µm cellulose acetate membranes. The filtrates are read in a spectrophotometer at 492 nm against a calibration range of carmine red. Two parameters are studied: the dry weight of the granuloma and the amount of dye after digestion of the tissues. The results are expressed as mean values (± sem). The differences between the groups are tested with an ANOVA followed by a Dunnett test, in which the reference group is the "solvent control" group.

The FGF-R antagonists are evaluated between 1 and 50 mg/kg using methylcellulose/Tween (0.6% v/v) as vehicle or any other vehicle that enables dissolution of the active principle. The molecules are daily administered orally (once or twice a day) by gavage. The antagonists of the present invention are considered as active if they enable either a significant reduction in the mass of the granuloma by measuring the mass of the dried skin, or a significant reduction in the angiogenic parameter by measuring the amount of carmine red dye in the skins of the treated animals.

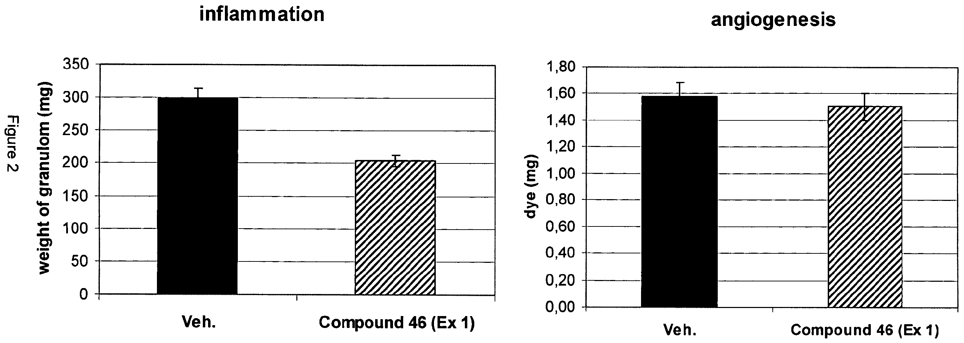

Example of evaluation of FGF-R antagonists in the model of inflammatory angiogenesis in mice. Compound 46 according to the invention (Example 1) at 30 mg/kg, after one week of the treatment, significantly reduces the weight of granuloma (dry weight of the skin; figure 2).

In general, the FGFs and their receptors are significantly involved, via autocrine, paracrine or juxtacrine secretions, in the phenomena of deregulation of stimulation of the growth of cancer cells. Furthermore, FGFs and the receptors thereof affect tumour angiogenesis, which plays a predominant role both on the growth of tumour and also on the metastatic phenomena.

Angiogenesis is a process of generation of new capillaries from pre-existing blood vessels or by mobilization and differentiation of bone marrow cells. Thus, both uncontrolled proliferation of endothelial cells and mobilization of angioblasts from bone marrow are observed in the processes of tumour neovascularization. It has been shown *in vitro* and *in vivo* that several growth factors stimulate endothelial proliferation, and especially FGF-1 or a-FGF and FGF-2 or b-FGF. These two factors induce proliferation, migration and the production of proteases by the endothelial cells in culture and neovascularization *in vivo.* a-FGF and b-FGF interact with the epithelial cells via two classes of receptor, the high-affinity receptors with tyrosine kinase activity (FGF-R) and the low-affinity receptors of heparan sulfate proteoglycan (HSPG) type located at the surface of the cells and in the extracellular matrices. While the paracrine role of these two factors on endothelial cells is widely described, these FGFs might also intervene on these cells by means of an autocrine process. Thus, FGFs and their receptors represent very pertinent targets for therapies directed towards inhibiting angiogenesis processes (Keshet E., Ben-Sasson S.A., J. Clin. Invest, (1999), vol. 501, pp. 104-1497; Presta M., Rusnati M., Dell'Era P., Tanghetti E., Urbinati C., Giuliani R. *et a*/*., New York: Plenum Publishers*, (2000), pp. 7-34, Billottet C., Janji B., Thiery J.P., Jouanneau J., Oncogene, (2002) vol. 21, pp. 8128-8139).

Moreover, systematic studies aimed at determining the expression due to FGFs and their receptors (FGF-R) on various tumour cell types reveal that a cellular response to these factors is functional in a large majority of studied human tumour lines. These results support the hypothesis that an FGF receptor antagonist might also inhibit the proliferation of tumour cells (Chandler L.A., Sosnowski B.A., Greenlees L., Aukerman S.L., Baird A., Pierce G.F., Int. J. Cancer, (1999), vol. 58, pp. 81-451).

FGFs play an important role in the growth and maintenance of prostate cells. It has been shown both in animal models and in man that an impairment of the cellular response to these factors plays a fundamental role in the progress of prostate cancer. Specifically, in these pathologies, an increase in the production of a-FGF, b-FGF, FGF-6, FGF-8, etc. by the fibroblasts, stromal cells, residual basal cells and endothelial cells present in the tumour and an increase in the expression of the FGF receptors and of the ligands by the tumour cells are recorded. Thus, paracrine stimulation of the cancer cells of the prostate operates, and this process is considered to be a major component of this pathology. A compound with FGF receptor antagonist activity such as the compounds of the present invention might represent a therapy of choice in these pathologies (Giri D., Ropiquet F., Clin. Cancer Res., (1999), vol. 71, pp. 5-1063; Doll J.A., Reiher F.K., Crawford S.E., Pins M.R., Campbell S.C., Bouck N.P., Prostate, (2001), vol. 305, pp. 49-293) (Sahadevan *et al.,* 2007) (Kwabi-Addo *et al.,* 2004).

Several studies show the presence of FGFs and of their FGF-R receptors both in human mammary tumour lines (especially MCF7) and in tumour biopsies. These factors are thought to be responsible in this pathology for the appearance of a very aggressive phenotype that induces strong metastasization. Thus, a compound with FGF-R receptor antagonist activity, such as the compounds of formula I, may represent a therapy of choice in these pathologies (Vercoutter-Edouart A-S, Czeszak X, Crépin M, Lemoine J, Boilly B, Le Bourhis X et al., Exp.Cell Res., (2001), vol. 262, pp. 59-68) (Schwertfeger, 2009).

Cancerous melanomas are tumours that induce metastases in high frequency and that are highly resistant to the various chemotherapy treatments. Angiogenesis processes play a predominant role in the progress of a cancerous melanoma. Furthermore, it has been shown that the probability of appearance of metastases increases very greatly as the vascularization of the primary tumour increases. Melanoma cells produce and secrete various angiogenic factors including a-FGF and b-FGF. Moreover, it has been shown that inhibition of the cellular effect of these two factors by the soluble FGF-R1 receptor blocks the proliferation and survival of tumoral melanoma cells *in vitro* and blocks the tumour progress *in vivo.* Thus, a compound with FGF receptor antagonist activity, such as the compounds of the present invention, may represent a therapy of choice in these pathologies (Rofstad E.K., Halsor E.F., Cancer Res., (2000); Yayon A., Ma Y-S, Safran M., Klagsbrun M., Halaban R., Oncogene, (1997), vol. 14, pp. 2999-3009).

Glioma cells produce a-FGF and b-FGF *in vitro* and *in vivo* and have various FGF receptors at their surface. This therefore suggests that these two factors via an autocrine and paracrine effect play a pivotal role in the progress of this type of tumour. Furthermore, as for the majority of solid tumours, the progress of gliomas and their capacity to induce metastases is very much dependent on the angiogenesis processes in the primary tumour. It has also been shown that FGF-R1 receptor antisense factors block the proliferation of human astrocytomas. Furthermore, naphthalenesulfonates are described for inhibiting the cellular effects of a-FGF and b-FGF *in vitro* and the angiogenesis induced by these growth factors *in vivo.* Intracerebral injection of these compounds induces a very significant increase in apoptosis and a substantial decrease in angiogenesis, reflected by considerable regression of gliomas in rats. Thus, a compound with a-FGF and/or b-FGF and/or FGF receptor antagonist activity, such as the compounds of the present invention, may represent a therapy of choice in these pathologies (Yamada S.M., Yamaguchi F., Brown R., Berger M.S., Morrison R.S., Glia, (1999), vol. 76, pp. 28-66; Auguste P., Gursel D.B., Lemière S., Reimers D., Cuevas P., Carceller F. et al., Cancer Res., (2001), vol. 26, pp. 61-1717) (Loilome *et al*., 2008).

Active angiogenesis is also described for hepatocarcinomas or hepatocellular carcinoma (HCC). *in vivo,* the tumour progress of HCC necessitates a substantial supply of oxygen and nutrients. Hepatocarcinomas are typically angiogenic tumours, since drastic impairment is observed in the arterial vascularization, and that this leads to the acquisition of an invasive and metastatic potential (Tanaka et *al*., 2006). FGFs actively participate in the development of tumoral angiogenesis in HCCs and are frequently associated with the inflammatory process. They are also overexpressed in the case of chronic hepatitis and cirrhosis of the liver (Uematsu et *al*., 2005), and the level of FGF in the serum has been correlated with the clinico-pathological progress of HCCs. Furthermore, the FGF-R4 and FGF-R1 receptors have been described as actively participating in the tumour genesis of HCCs (Huang *et al*., 2006) (Nicholes *et al*., 2002). The antagonists of the present invention may thus be a treatment of choice for hepatocellular carcinomas or hepatocarcinomas.

In lung cancers of NSCLC type (non-small-cell lung cancer), recent studies show that b-FGF, FGF-9, FGF-R1 and FGF-R2 are regularly co-expressed in the NSCLC cancer lines and especially in those resistant to the anti-EGFR treatment such as gefitinib. These expressions are in relation with the capacity for proliferation by autocrine cellular signalling and for independent growth of an anchoring of tumours of NSCLC type and mainly that which is insensitive to treatment with gefitinib (Marek *et al*., 2008). Furthermore, b-FGF has been suggested as playing an important role in the survival of NSCLC cells during chemotherapy treatment by inducing the overexpression of the anti-apoptosis proteins BCL-2, BCL-X, XIAP or BIRC3 (PArdo *et al*., 2002, 2003 and 2006). Thus, an FGF receptor antagonist such as those of the present invention may represent a therapy of choice for lung cancers of NSCLC type, alone or in combination with EGF receptor inhibitors or chemotherapies.

In about 10% of stomach cancers, a gene amplification of FGF-R2 is observed. This amplification is associated with a poor vital prognosis for cancers of diffuse type. The proliferation of the tumour cells may be independent of the ligand or dependent on paracrine activation with FGF-7 (Turner *et al*., 2010). The antagonists of the present invention may thus be a treatment of choice for stomach cancers.

More recently, the potential role of pro-angiogenic agents in leukaemias and lymphomas has been documented. Specifically, in general, it has been reported that cellular clones in these pathologies either may be naturally destroyed by the immune system or may transform into an angiogenic phenotype that favours their survival and then their proliferation. This change of phenotype is induced by an overexpression of angiogenic factors especially by the macrophages and/or mobilization of these factors from the extracellular matrix (Thomas D.A., Giles F.J., Cortes J., Albitar M., Kantarjian H.M., Acta Haematol., (2001), vol. 207, pp. 106-190). Among the angiogenic factors, b-FGF has been detected in numerous lymphoblastic and haematopoietic tumoral cell lines. FGF receptors are also present on the majority of these lines, suggesting a possible autocrine cellular effect of a-FGF and b-FGF inducing the proliferation of these cells. Moreover, it has been reported that the angiogenesis of bone marrow via paracrine effects was correlated to the progress of some of these pathologies.

More particularly, it has been shown in CLL (chronic lymphocytic leukaemia) cells that b-FGF induces an increase in the expression of anti-apoptotic protein (Bcl2) leading to an increase in the survival of these cells, and thus participates substantially in their cancerization. Furthermore, the levels of b-FGF measured in these cells are highly correlated with the degree of clinical advancement of the disease and the resistance to chemotherapy applied in this pathology (fludarabine). Thus, a compound with FGF receptor antagonist activity, such as the compounds of the present invention, may represent a therapy of choice alone or in combination with fludarabine or other products that are active in this pathology (Thomas D.A., Giles F.J., Cortes J., Albitar M., Kantarjian H.M., Acta Haematol., (2001), vol. 207, pp. 106-190; Gabrilove J.L., Oncologist, (2001), vol. 6, pp. 4-7).

Furthermore, it has been shown in numerous recent studies that FGFs and FGF-Rs participate actively in the resistance of tumoral and/or endothelial cells to chemotherapy or radiotherapy treatments or alternatively to anti-VEGF therapies. These resistances involve different cell mechanisms such as protection against apoptosis by a positive regulation of the protein Bcl-xl by FGF-R4 in the case of resistance of breast cancer to doxorubicin (Roidl *et a*/*.,* 2009) or the production of FGF-2 in the case of a resistance to cisplatin of bladder tumours (Miyake *et al*., 1998), by activation of the Pi3K/AKT pathway by the FGF2/FGF-R1 couple in the case of the resistance to cytarabine of acute myeloid leukaemia cells (Karajannis *et al*., 2006), by stimulation of the RAS/MAP-K, PI3-K and mTOR pathway by FGF-1 for certain mammary tumours resistant to anti-oestrogen treatments (Manuvakhova *et al*., 2006). The FGFs/FGF-Rs couple is also involved in resistance to anti-VEGF treatments in the context of pancreatic carcinomas (Casanovas *et al*., 2005) or glioblastomas (Batchelor *et al*., 2007) or alternatively in radiotherapy resistance phenomena (Gu *et al*., 2004; Moyal *et al*., 2009). Thus, the compounds of the present invention could be combined with the existing therapies to limit the appearance of resistance phenomena.

Furthermore, tumour innovation, which is one of the hallmarks of malignancy, consists of the translocation of tumour cells from the initial neoplastic focus to the surrounding host tissues, enabling the tumour to penetrate into the vascular endothelial in order to circulate and to form metastatic foci remote from the primary tumour. An increasing number of recent articles suggest that changes in tissue architecture at the periphery of the tumour are the cause of the epithelial-mesenchymal transition (EMT). EMT is a cell process via which epithelial cells modulate their phenotype and acquire properties of mesenchymal cells by disrupting intercellular adhesion and increasing cell motility, thus playing a crucial role in tumour progress by imparting an invasive and metastatic phenotype to carcinomas. Growth factors such as FGFs participate in this cell process by means of their stimulatory activity on cell migration and invasion, but also, for the FGF receptors, via their capacity to interact with cadherins, thus facilitating the migration of tumour cells (Cowin *et al*., 2005). The FGF-R antagonists described here may be used to prevent these metastatic phases of a large number of cancers.

There is a correlation between the angiogenesis process of bone marrow and "extramedullar disease" in CML (chronic myelomonocytic leukaemia). Various studies demonstrate that the inhibition of angiogenesis, in particular by a compound with FGF receptor antagonist activity, might represent a therapy of choice in this pathology.

The proliferation and migration of vascular smooth muscle cells contributes towards intimal hypertrophy of the arteries and thus plays a predominant role in atherosclerosis and in restenosis after angioplasty and endoarterectomy.
*in vivo* studies show after lesion of the carotid artery by "balloon injury", a local production of a-FGF and b-FGF. In this same model, an anti-FGF2 neutralizing antibody inhibits the proliferation of the vascular smooth muscle cells and thus decreases the intimal hypertrophy.
An FGF2 chimeric protein linked to a molecule such as saporin inhibits the proliferation of vascular smooth muscle cells *in vitro* and intimal hypertrophy *in vivo* (Epstein C.E., Siegall C.B., Biro S, Fu Y.M., FitzGerald D., Circulation, (1991), vol. 87, pp. 84-778; Waltenberger J., Circulation, (1997), pp. 96-4083).
Thus, FGF receptor antagonists, such as the compounds of the present invention, represent a therapy of choice, either alone or in combination with antagonist compounds of other growth factors involved in these pathologies such as PDGF, in the treatment of pathologies associated with the proliferation of vascular smooth muscle cells, such as atherosclerosis, post-angioplasty restenosis or restenosis following the insertion of endovascular prostheses (stents) or during aorto-coronary bypasses.

Cardiac hypertrophic arises in response to stress on the ventricular wall induced by an overload in terms of pressure or volume. This overload may be the consequence of numerous physiopathological conditions such as hypertension, AC (aortic coarctation), myocardial infarction and various vascular disorders. The consequences of this pathology are morphological, molecular and functional changes such as hypertrophy of the cardiac myocytes, the accumulation of matrix proteins and the re-expression of foetal genes. b-FGF is involved in this pathology. Specifically, the addition of b-FGF to newborn rat cardiomyocyte cultures modifies the profile of the genes corresponding to the contractile proteins, leading to a gene profile of foetal type. In a complementary manner, adult rat myocytes show a hypertrophic response under the effect of b-FGF, this response being blocked by anti-b-FGF neutralizing antibodies. Experiments performed *in vivo* on b-FGF "knockout" transgenic mice show that b-FGF is a major stimulating factor of the hypertrophy of cardiac myocytes in this pathology (Schultz JeJ, Witt S.A., Nieman M.L., Reiser P.J., Engle S.J., Zhou M. et al., J.Clin. Invest., (1999), vol. 19, pp. 104-709). Thus, a compound with FGF receptor antagonist activity, such as the compounds of the present invention, represents a therapy of choice in the treatment of cardiac insufficiency and any other pathology associated with degeneration of cardiac tissue. This treatment could be performed alone or in combination with common treatments (beta-blockers, diuretics, angiotensin antagonists, antiarrhythmics, anti-calcium, antithrombotic etc. agents).
Diabetes-related vascular disorders are characterized by an impairment of vascular reactivity and of the blood flow, hyperpermeability, an exacerbated proliferative response and an increase in matrix protein deposits. More precisely, a-FGF and b-FGF are present in the preretinal membranes of patients with diabetic retinopathy, in membranes of the subjacent capillaries and in the vitreous humour of patients suffering from proliferative retinopathy. A soluble FGF receptor that is capable of binding both to a-FGF and b-FGF is developed in diabetes-related vascular disorders (Tilton R.G., Dixon R.A.F., Brock T.A., Exp. Opin. Invest. Drugs, (1997), vol. 84, pp. 6-1671). Thus, a compound with FGF receptor antagonist activity, such as the compounds of formula I, represents a therapy of choice either alone or in combination with compounds that are antagonists of other growth factors involved in these pathologies, for instance VEGF, such as the anti-VEGF therapy mentioned above.

Fibrosis is the abnormal formation of scar tissue following a tissue lesion, and leads to chronic and progressive impairment of the affected organ, which may result in serious dysfunction of the affected organ. It may arise in any tissue, but is mainly prevalent in organs exposed to chemical or biological attack, such as the lungs, the skin, the kidneys, the digestive tube, the liver, etc. FGFs participate in this cell process and promote the production and accumulation of extracellular matrices by the fibroblasts, and their proliferation and infiltration into numerous organs such as the kidneys or the lungs (Khalil *et al*., 2005) (Strutz *et al*., 2003). Antagonists of the activity of these FGFs, such as the molecules of the present invention, may be used alone or in combination in the treatment of fibrosis.

Rheumatoid arthritis (RA) is a chronic disease of unknown aetiology. Although it affects numerous organs, the most severe form of RA is gradual synovial inflammation of the joints, leading to their destruction. Angiogenesis appears to substantially affect the progress of this pathology. Thus, a-FGF and b-FGF have been detected in synovial tissue and in the articular fluid of patients suffering from RA, indicating that this growth factor intervenes in the initiation and/or progress of this pathology. In models of AIA (adjuvant-induced model of arthritis) in rats, it has been shown that the overexpression of b-FGF increases the severity of the disease, whereas an anti-b-FGF neutralizing antibody blocks the progress of RA (Malemud, 2007) (Yamashita A, Yonemitsu Y, Okano S, Nakagawa K, Nakashima Y, Irisa T et a/., J. Immunol., (2002), vol. 57, pp. 168-450; Manabe N, Oda H, Nakamura K, Kuga Y, Uchida S, Kawaguchi H, Rheumatol, (1999), vol. 20, pp. 38-714). Thus, the compounds according to the invention represent a therapy of choice in this pathology.

Recent scientific articles document the involvement of b-FGF in neuropathic pain. Specifically, an increase in the production of astroglial b-FGF is observed in astrocytes following lesion of the spinal cord (Madiai *et al*., 2003). This b-FGF contributes towards the neuropathic contact pain or allodynia. Treatment using an anti-FGF2 neutralizing antibody reduces this mechanical allodynia (Madiai *et al*., 2005). The antagonists of the present invention are treatments of choice for pain by inhibiting the effect of FGF-2 on these receptors.

It has also been described that the levels of growth factors with pro-angiogenic activity such as FGF-1 and -2 were greatly increased in the synovial fluid of patients suffering from osteoarthritis. In this type of pathology, a substantial modification is recorded in the balance between the pro- and anti-angiogenic factors inducing the formation of new blood vessels, and consequently the vascularization of non-vascularized structures such as articular cartilage or intervertebral discs. Thus, angiogenesis represents a key factor in bone formation (osteophytes), thus contributing towards the progress of the disease. In a complementary manner, the innervation of new blood vessels may also contribute towards the chronic pain associated with this pathology (Walsh D.A., Curr. Opin. Rheumatol. 2004 Sep;16(5):609-15) Thus, the compounds according to the invention represents a therapy of choice in this pathology.

IBD (inflammatory bowel disease) comprises two forms of chronic inflammatory disease of the intestine: UC (ulcerative colitis) and Crohn's disease (CD). IBD is characterized by an immune dysfunction that is reflected by an inappropriate production of inflammatory cytokines, inducing the establishment of a local microvascular system. A consequence of this angiogenesis of inflammatory origin is a vasoconstriction-induced intestinal ischaemia. Substantial circulating and local levels of b-FGF have been measured in the case of patients suffering from these pathologies (Kanazawa S, Tsunoda T, Onuma E, Majima T, Kagiyama M, Kkuchi K., American Journal of Gastroenterology, (2001), vol. 28, pp. 96-822; Thorn M, Raab Y, Larsson A, Gerdin B, Hallgren R., Scandinavian Journal of Gastroenterology, (2000), vol. 12, pp. 35-408). The compounds of the invention with substantial anti-angiogenic activity in a model of inflammatory angiogenesis represent a therapy of choice in these pathologies.

Another disease with a substantial inflammatory component and for which a strong involvement of the FGFs and FGF-Rs is described is benign prostate hyperplasia (BPH). BPH is an age-related disease which is characterized by hyperplasia of the glandular tissues and of stroma around the urethra up to the point of its obstruction. At the cellular level, this pathology involves hyperplasia of the basal cells, an increase in the stromal mass, an amplified deposition of matrix or a reduction in the elasticity of the tissues (Untergasser *et al.,* 2005). FGFs participate in the development of this disease by stimulating the proliferation of the prostate stromal and epithelial cells and especially FGF-7 or KGF, but also FGF-2 or FGF-17 (Wang 2008, Boget 2001, Giri 2001). Furthermore, the FGFs promote the transdifferentiation step by modifying the epithelial cell/stromal cell interactions, in combination with TGF-β (Untergasser 2005). Finally, certain receptors such as FGF-R1 are overexpressed in BPH, promoting induction of the pathology and potentiating the paracrine effects of FGF-2 (Boget 2001). An antagonist of the effect of these FGFs is thus a treatment of choice for benign prostate hyperplasia.

Psoriasis is a chronic skin disease caused by hyperproliferation of the epidermal keratinocytes, while clear cell acanthoma (CCA) is a benign epidermal neoplasm also involving abnormal keratinocyte proliferation. These two skin diseases have similar histological characteristics despite having different underlying causes: thickening of the epidermis, inflammatory infiltrations of lymphocytes and neutrophils, dilation and tortuosity of the papillary capillaries. In both cases, KGF or FGF-7 plays an a predominant role in the development of the pathology (Kovacs *et al*., 2006) (Finch *et al*., 1997). The use of the antagonists of the present invention may make it possible to slow down the development of such skin diseases.

FGF-R1, -R2 and -R3 are involved in chronogenesis and osteogenesis processes. Mutations leading to the expression of FGF-Rs that are always activated have been linked to a large number of human genetic diseases reflected by skeletal malformations, such as the Pfeiffer, Crouzon, Apert, Jackson-Weiss and Bear-Stevenson cutis gyrata syndromes. Some of these mutations more particularly affecting the FGF-R3 receptor lead especially to achondroplasias (ACH), hypochondroplasias (HCH) and TD (thanatophoric dysplasia); ACH being the most common form of dwarfism. From a biochemical viewpoint, the sustained activation of these receptors takes place via dimerization of the receptor in the absence of ligand (Chen L., Adar R., Yang X. Monsonego E.O., LI C., Hauschka P.V., Yagon A. and Deng C.X., (1999), The Journ. of Clin. Invest., vol. 104, No. 11, pp. 1517-1525). Thus, the compounds of the invention with antagonist activity towards the FGFs or the FGF receptors and which inhibit FGF-R-dependent intracellular signalling represent a therapy of choice in these pathologies.

Moreover, it is known that adipose tissue is one of the rare tissues that can develop or regress in adults. This tissue is highly vascularized and a very dense network of microvessels surrounds each adipocyte. These observations led to testing of the effect of anti-angiogenic agents on the development of adipose tissue in adults. Thus, it appears that in pharmacological models in ob/ob mice, the inhibition of angiogenesis is reflected by a significant loss of weight of the mice (Rupnick M.A. et al, (2002), PNAS, vol. 99, No. 16, pp. 10730-10735). Furthermore, FGFs appear as key regulators of adipogenesis in man (Hutley *et al.,* 2004). Thus, an FGF receptor antagonist compound with powerful anti-angiogenic activity may represent a therapy of choice in obesity-related pathologies.

By virtue of their low toxicity and their pharmacological and biological properties, the compounds of the present invention find their use in the treatment and prevention of any carcinoma having a substantial degree of vascularization, such as lung, breast, prostate, oesophageal, pancreatic, liver, bowel or kidney carcinomas or which induce metastases, such as bowel, breast, liver and stomach carcinomas, melanomas, or which are sensitive to a-FGF or to b-FGF in an autocrine manner, or alternatively in pathologies such as glioma, lymphoma and leukaemia, or finally in any therapy-resistance phenomenon. These compounds represent a therapy of choice either alone or in combination with chemotherapy, radiotherapy or any other suitable treatment. The compounds according to the invention also find their use in the treatment and prevention of cardiovascular diseases such as atherosclerosis, post-angioplasty restenosis, in the treatment of diseases associated with complications arising after the insertion of endovascular prostheses and/or aorto-coronary bypasses or other vascular grafts and cardiac hypertrophy or vascular complications of diabetes such as diabetic retinopathy. The compounds according to the invention also find their use in the treatment and prevention of chronic inflammatory diseases such as rheumatoid arthritis, IBD or benign prostate hyperplasia. Finally, the compounds according to the invention may be used in the treatment and prevention of achondroplasias (ACH), hypochondroplasias (HCH) and TD (thanatophoric dysplasia), and also in the treatment of obesity.

A subject of the present invention relates to compound of formula (I) 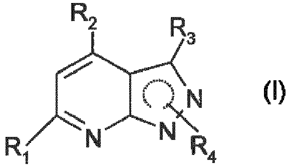 in which:
the representation of the pyrazole ring indicates that the substituent R₄ may be borne either by the nitrogen alpha to the pyridine ring (I') or by the nitrogen alpha to the carbon bearing a substituent R₃ (I") such that: 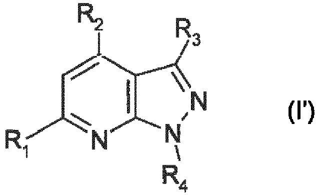 Either 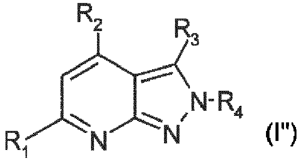
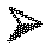 **R₁** represents an aryl, pyridyl or pyrazolyl group optionally substituted with one or more substituents chosen from:
   ∘ a halogen atom,
   ∘ a group -CF3,
   ∘ a cyano group,
   ∘ a group -NR₆R₆' in which Re and R₆' are as defined below,
   ∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
   ∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
   ∘ a group -COR₁₂ in which R₁₂ represents a hydroxyl group or a group -NR₆R₆', in which R₆ and R₆' are as defined below,
   ∘ a group -CONR₇R₇' such that R₇ and R₇' form, together with the nitrogen atom to which they are attached, a heterocycloalkyl comprising one or more heteroatoms chosen from a nitrogen atom and an oxygen atom,
   ∘ a group -(CH₂)ₚNHSO₂CH₃ in which p represents 0 or 1,
   ∘ a group -OR₁₃ in which R₁₃ represents a linear group (C₁₋C₃)alkyl,
   ∘ a group (C₁-C₃)alkyl,
   Or R₁ represents a bicyclic group of formula A below: 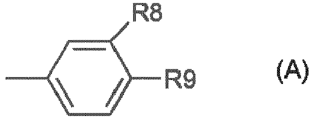 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
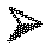 **R₂** represents a group:
   ∘ -CF₃,
   ∘ -CHF₂,
   ∘ -COOH,
   or
   ∘ -CONHR₅, in which R₅ is as defined below,
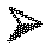 **R₃** represents:
   ∘ a hydrogen atom,
   ∘ an aryl group, optionally substituted with an alkoxymethyl group,
   ∘ a cycloalkyl group,
   or
   ∘ a heteroaryl group chosen from thienyl and pyridyl groups,
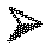 **R₄** represents:
   ∘ a hydrogen atom,
   ∘ a linear group (C₁-C₃)alkyl, optionally substituted with a group-NR₆R₆' in which R₆ and R'₆ are as defined below or a group -NR₇R_{7'}' such that R₇ and R_{7'} form, together with the nitrogen atom to which they are attached, a heterocycloalkyl comprising one or more heteroatoms chosen from a nitrogen atom and an oxygen atom,
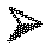 **R₅** represents:
   ∘ a hydrogen atom,
   ∘ a linear group (C₁-C₃)alkyl, optionally substituted with a pyridyl group,
      or
   c an aromatic group chosen from aryl and pyridyl,
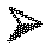 **R₆** and **R'₆**, which may be identical or different, represent a hydrogen atom or a linear alkyl group,
in the form of the base or of an acid-addition or base-addition salt, for use as a medicament.

In particular, the present invention relates to compound selected from the following compounds:

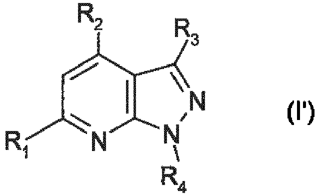

| **No.** | **R₂** | **R₃** | **R₁** | **R₄** | **Salt** | **m.p. (°C)** | **M+H⁺** |
|---|---|---|---|---|---|---|---|
| **1** | CF₃ | Ph | 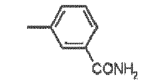 | Me | / | / | 397 |
| **2** | CF₃ | Ph | 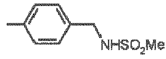 | Me | / | / | 461 |
| **3** | CF₃ | Ph | 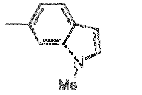 | Me | / | / | 407 |
| **4** | CF₃ | Ph | 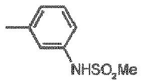 | Me | / | / | 447 |
| **5** | CF₃ | Ph | 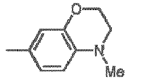 | Me | / | / | 426 |
| **6** | CF₃ | Ph | 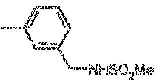 | Me | / | / | 461 |
| **7** | CF₃ | Ph | 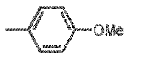 | Me | / | / | 384 |
| **8** | CF₃ | Ph | 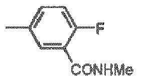 | Me | / | / | 429 |
| **9** | CF₃ | Ph | 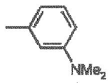 | Me | / | / | 397 |
| **10** | CF₃ | Ph | 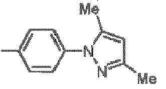 | Me | / | / | 448 |
| **11** | CF₃ | Ph | 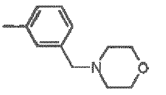 | Me | / | / | 453 |
| **12** | CF₃ | Ph | 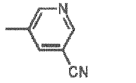 | Me | / | / | 380 |
| **13** | CF₃ | Ph | 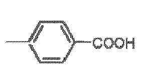 | Me | / | / | 398 |
| **14** | CF₃ | Ph | 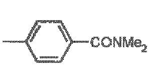 | Me | / | / | 425 |
| **15** | CF₃ | Ph | 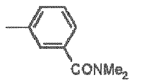 | Me | / | / | 425 |
| **16** | CF₃ | Ph | 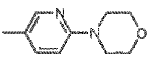 | Me | / | / | 440 |
| **17** | CF₃ | Ph | 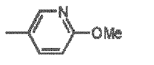 | Me | / | / | 385 |
| **18** | CF₃ | Ph | 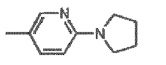 | Me | / | / | 424 |
| **19** | CF₃ | Ph | 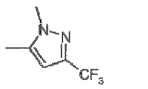 | Me | / | / | 426 |
| **20** | CF₃ | Ph | 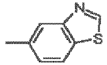 | Me | / | / | 411 |
| **21** | CF₃ | Ph | 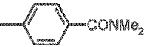 | H | HCl | / | 411 |
| **22** | CF₃ | Ph | 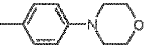 | H | HCl | / | 425 |
| **23** | CF₃ | Ph | 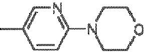 | H | HCl | / | 426 |
| **24** | CF₃ | Ph | 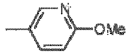 | H | HCl | / | 371 |
| **25** | CF₃ | Ph | 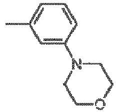 | H | HCl | / | 425 |
| **26** | CF₃ | Ph | 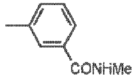 | H | HCl | / | 397 |
| **27** | CF₃ | Ph | 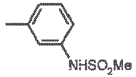 | H | HCl | / | 433 |
| **28** | CF₃ | Ph | 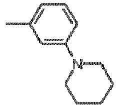 | H | HCl | / | 423 |
| **29** | CF₃ | Ph | 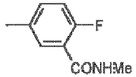 | H | HCl | / | 415 |
| **30** | CF₃ | Ph | 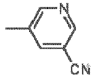 | H | HCl | / | 366 |
| **31** | CHF₂ | Ph | 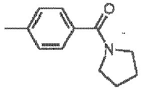 | H | HCl | 248 | 455 |
| **32** | CHF₂ | Ph | 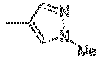 | H | HCl | / | 362 |
| **33** | CHF₂ | Ph | 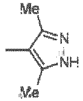 | H | HCl | / | 376 |
| **34** | CHF₂ | Ph | 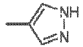 | H | HCl | / | 348 |
| **35** | CHF₂ | Ph | 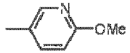 | H | HCl | / | 389 |
| **36** | CHF₂ | Ph | 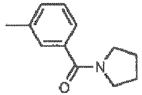 | H | HCl | / | 455 |
| **37** | CHF₂ | Ph | 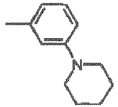 | H | HCl | / | 441 |
| **38 Ex. 2** | COOH | H | 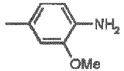 | H | / | / | 285 |
| **39** | CONHMe | Ph | 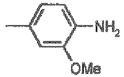 | H | TFA | / | 458 |
| **40** | CONH₂ | Ph | 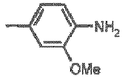 | H | TFA | / | 474 |
| **41** | CONHMe | H | 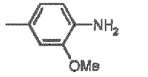 | H | TFA | / | 412 |
| **42** | CONH₂ | H | 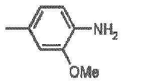 | H | TFA | / | 398 |
| **43** | COOH | Ph | 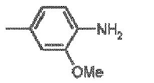 | H | / | / | 361 |
| **44** | COOH | Ph | 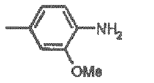 | Me | / | / | 375 |
| **45** | COOH | H | 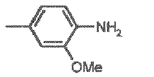 | Me | / | / | 299 |
| **46 Ex. 1** | CONH₂ | Ph | 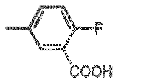 | H | TFA | / | 377 |
| **47** | CONH₂ | Ph | 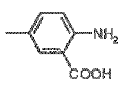 | H | / | / | 374 |
| **48** | CONH₂ | Ph | 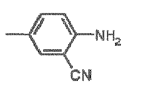 | H | TFA | / | 355 |
| **49** | CONH₂ | 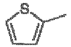 | 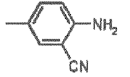 | H | / | / | 361 |
| **50** | COOH | **cPr** | 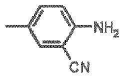 | H | / | / | 320 |
| **51** | CONH₂ | H | 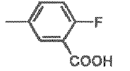 | H | / | / | 301 |
| **52** | CONH₂ | Ph | 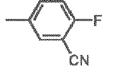 | H | / | / | 358 |
| **53 Ex. 3** | CHF₂ | Ph | 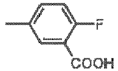 | H | / | / | 384 |
| **54** | CF₃ | Ph | 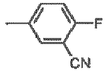 | H | / | / | 383 |
| **55** | CHF₂ | Ph | 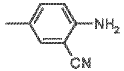 | H | HCl | 282 | 362 |
| **56 Ex. 4** | CF₃ | Ph | 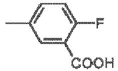 | H | / | / | 402 |
| **57 Ex. 6** | CF₃ | Ph | 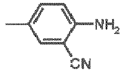 | Me | / | 269 | 394 |
| **58** | CF₃ | Ph | 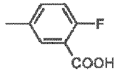 | Me | / | / | 416 |
| **59** | CF₃ | Ph | 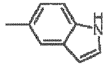 | H | / | / | 379 |
| **60** | CF₃ | Ph | 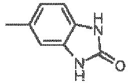 | H | / | 380 | 396 |
| **61 Ex. 5** | CF₃ | Ph | 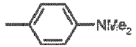 | H | / | 227 | 383 |
| **62** | CONHPh | H | 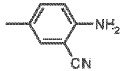 | H | HCl | / | 355 |
| **63** | 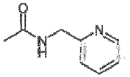 | H | 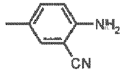 | H | HCl | I | 370 |
| **64** | 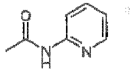 | H | 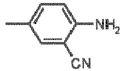 | H i | HCl | / | 356 |
| **65** | 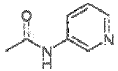 | H | 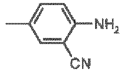 | H | HCl | / | 356 |
| **66** | 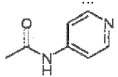 | H | 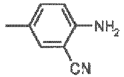 | H | HCl | / | 356 |
| **67** | CHF₂ | 4-Py | 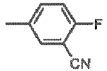 | Me | / | / | 380 |
| **68** | CONHPh | H | 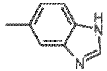 | H | HCl | / | 355 |
| **69** | 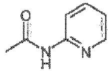 | H | 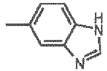 | H | HCl | / | 356 |
| **70** | 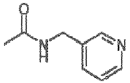 | H | 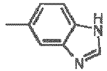 | H | HCl | / | 370 |
| **71** | CONHPh | H | 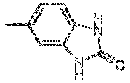 | H | / | / | 371 |
| **72 Ex. 10** | CHF₂ | H | 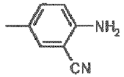 | Me | / | 251 | 300 |
| **73** | CHF₂ | H | 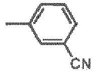 | Me | / | 162 | 285 |
| **74** | CHF₂ | H | 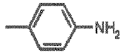 | Me | / | 149 | 275 |
| **75 Ex. 12** | CHF₂ | H | 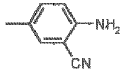 | H | / | 263 | 286 |
| **76** | CF₃ | Ph | 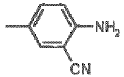 | 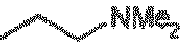 | / | 183 | 451 |
| **77** | CF₃ | Ph | 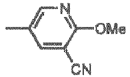 | Me | / | 250 | 410 |
| **78** | CHF₂ | Ph | 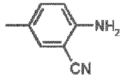 | Me | / | 246 | 376 |
| **79** | CHF₂ | Ph | 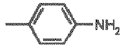 | Me | / | 176 | 351 |
| **80** | CHF₂ | Ph | 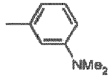 | Me | / | 154 | 379 |
| **81** | CF₃ | Ph | 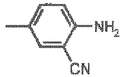 | 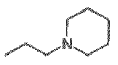 | / | 192 | 491 |
| **82** | CF₃ | Ph | 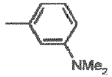 | 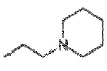 | HCl | 227 | 494 |
| **83 Ex. 13** | CHF₂ | Ph | 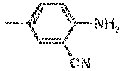 | 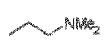 | / | 163 | 433 |
| **84** | CF₃ | H | 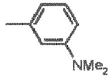 | 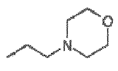 | / | 110 | 420 |
| **85** | CF₃ | H | 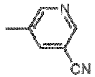 | 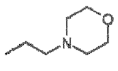 | / | / | 403 |
| **86** | CF₃ | H | 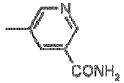 | 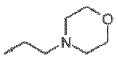 | / | 238 | 421 |
| **87** | CF₃ | H | 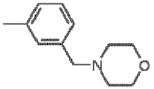 | Me | / | 105 | 377 |
| **88 Ex. 8** | CF₃ | H | 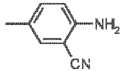 | Me | / | 276 | 318 |
| **89** | CF₃ | Ph | 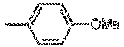 | Me | / | 181 | 384 |
| **90** | CF₃ | H | 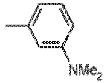 | Me | / | 91 | 321 |
| **91 Ex. 15** | CHF₂ | 3-Py | 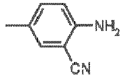 | H | / | 296 | 363 |
| **92** | CHF₂ | 4-Py | 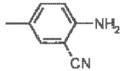 | H | / | 325 | 363 |
| **93 Ex. 14** | CF₃ | 3-Py | 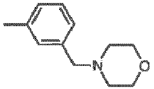 | Me | / | 155 | 454 |
| **94** | CHF₂ | 3MeO-Ph | 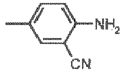 | Me | / | 233 | 392 |
| **95** | CF₃ | H | 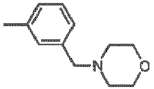 | Pr | HCl | 271 | 405 |
| **96** | CF₃ | H | 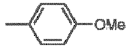 | Pr | / | 72 | 336 |
| **97** | CF₃ | 3MeO-Ph | 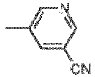 | Me | / | 194 | 410 |
| **98** | CF₃ | 3MeO-Ph | 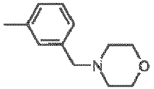 | Me | / | 114 | 483 |
| **99** | CF₃ | 3MeO-Ph | 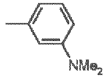 | Me | / | 138 | 427 |
| **100** | CF₃ | 3MeO-Ph | 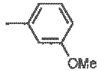 | Me | / | 133 | 414 |
| **101** | CONH₂ | 3-Py | 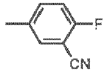 | H | / | / | 377 |

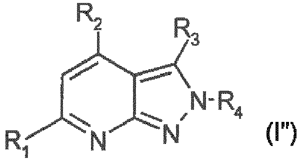

| **No.** | **R₂** | **R₃** | **R₁** | **R₄** | **Salt** | **m.p. (°C)** | **M+H⁺** |
|---|---|---|---|---|---|---|---|
| **102** | COOH | H | 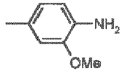 | Me | / | / | 299 |

| **No.** | **R₂** | **R₃** | **R₁** | **R₄** | **Salt** | **m.p.(°C)** | **M+H⁺** |
|---|---|---|---|---|---|---|---|
| **103** | CONH₂ | H | 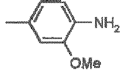 | Me | TFA | / | 412 |
| **104** | COOH | H | 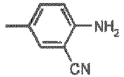 | Me | / | / | 294 |
| **105** | CHF₂ | H | 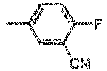 | Me | / | / | 303 |
| **106 Ex. 11** | CHF₂ | H | 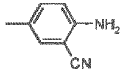 | Me | / | / | 300 |
| **107** | CHF₂ | H | 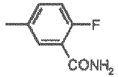 | Me | / | / | 321 |
| **108 Ex. 7** | CF₃ | Ph | 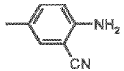 | Me | / | 295 | 394 |
| **109** | CF₃ | Ph | 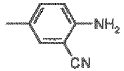 | 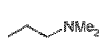 | / | 237 | 451 |
| **110** | CF₃ | Ph | 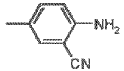 | 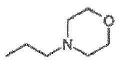 | / | 249 | 493 |
| **111** | CHF₂ | H | 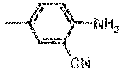 | 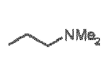 | / | 182 | 357 |
| **112** | CHF₂ | H | 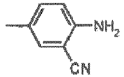 | 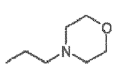 | / | 242 | 399 |
| **113** | CF₃ | H | 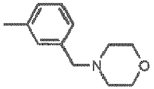 | 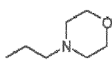 | / | 101 | 476 |
| **114 Ex. 9** | CF₃ | H | 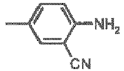 | Me | / | 249 | 318 |
| **115** | CF₃ | H | 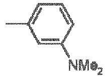 | Pr | HCl | 181 | 425 |
| **116** | CHF₂ | H | 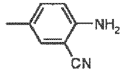 | 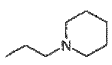 | / | 230 | 397 |
| **117** | CHF₂ | H | 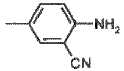 | Pr | / | 214 | 328 |
| **118** | CF₃ | H | 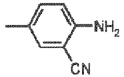 | Pr | / | 239 | 346 |
| **119** | CF₃ | H | 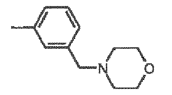 | Pr | / | 288 | 405 |
| **120** | CF₃ | H | 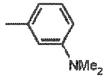 | Pr | / | 89 | 349 |

for use as a medicament.

The products according to the invention also find their use in the treatment and prevention of macular degeneration, especially age-related macular degeneration (AMD). A major feature of the loss of vision in adults is the consecutive neovascularization and haemorrhaging, which cause major functional disorders in the eye and which are reflected by early-onset blindness. Recently, study of the mechanisms involved in ocular neovascularization phenomena have revealed the involvement of pro-angiogenic factors in these pathologies. By using a model of laser-induced choroid neoangiogenesis, it has been possible to confirm that the products according to the invention also make it possible to modulate the neovascularization of the choroid.

Moreover, the products of the invention may be used in the treatment or prevention of thrombopenia caused especially by anticancer chemotherapy. Specifically, it has been demonstrated that the products of the invention can improve the levels of circulating platelets during chemotherapy.

Finally, the products according to the invention find a use in the treatment and prevention of skin diseases such as psoriasis or clear-cell acanthoma, in combating the progress of hepatic, renal or pulmonary fibrosis, and also in the treatment of neuropathic pain.

The invention also describes medicaments comprising a compound of formula (I), or a pharmaceutically acceptable acid-addition or base-addition salt thereof.

These medicaments find their use for example in the treatment and prevention of any carcinoma having a substantial degree of vascularization, such as lung, breast, prostate, oesophageal, pancreatic, liver, bowel or kidney carcinomas or which induce metastases, such as bowel, breast, liver and stomach carcinomas, melanomas, or which are sensitive to a-FGF or to b-FGF in an autocrine manner, or alternatively in pathologies such as glioma, lymphoma and leukaemia, or finally in any therapy-resistance phenomenon. These medicaments also find their use in the treatment and prevention of cardiovascular diseases such as atherosclerosis, post-angioplasty restenosis, in the treatment of diseases associated with complications arising after the insertion of endovascular prostheses and/or aorto-coronary bypasses or other vascular grafts and cardiac hypertrophy or vascular complications of diabetes such as diabetic retinopathy. They also find their use in the treatment and prevention of chronic inflammatory diseases such as rheumatoid arthritis, IBD or benign prostate hyperplasia. They may be used in the treatment and prevention of achondroplasias (ACH), hypochondroplasias (HCH) and TD (thanatophoric dysplasia), and also in the treatment of obesity.

The medicaments disclosed in the invention also find their use in the treatment and prevention of macular degeneration, especially age-related macular degeneration (AMD). They also make it possible to modulate neovascularization of the choroid.

Moreover, the medicaments described in the invention may be used in the treatment or prevention of thrombopenia caused especially by anticancer chemotherapy.

A subject of the present invention is also the use of a compound of formula (I) 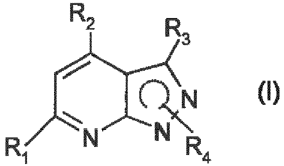 in which:
the representation of the pyrazole ring indicates that the substituent R₄ may be borne either by the nitrogen alpha to the pyridine ring (I') or by the nitrogen alpha to the carbon bearing a substituent R₃ (I") such that: 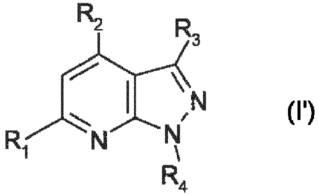 Either 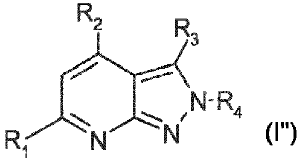
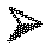 **R₁** represents an aryl, pyridyl or pyrazolyl group optionally substituted with one or more substituents chosen from:
   ∘ a halogen atom,
   ∘ a group -CF3,
   ∘ a cyano group,
   ∘ a group -NR₆R₆' in which R₆ and R₆' are as defined below,
   ∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
   ∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
   ∘ a group -COR₁₂ in which R₁₂ represents a hydroxyl group or a group -NR₆R₆', in which R₆ and Re' are as defined below,
   ∘ a group -CONR₇R₇' such that R₇ and R₇' form, together with the nitrogen atom to which they are attached, a heterocycloalkyl comprising one or more heteroatoms chosen from a nitrogen atom and an oxygen atom,
   ∘ a group -(CH₂)ₚNHSO₂CH₃ in which p represents 0 or 1,
   ∘ a group -OR₁₃ in which R₁₃ represents a linear group (C₁-C3)alkyl,
   ∘ a group (C₁-C₃)alkyl,
   Or **R₁** represents a bicyclic group of formula A below: 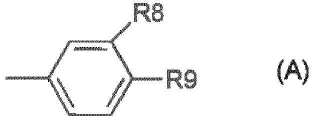 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
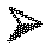 **R₂** represents a group:
   ∘ -CF₃,
   ∘ -CHF₂,
   ∘ -COOH,
   or
   ∘ -CONHR₅, in which R₅ is as defined below,
> **R₃** represents:
   ∘ a hydrogen atom,
   ∘ an aryl group, optionally substituted with an alkoxymethyl group,
   ∘ a cycloalkyl group,
   or
   ∘ a heteroaryl group chosen from thienyl and pyridyl groups,
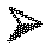 **R₄** represents:
   ∘ a hydrogen atom,
   ∘ a linear group (C₁-C₃)alkyl, optionally substituted with a group - NR₆R₆' in which R₆ and R'₆ are as defined below or a group -NR₇R_{7'}' such that R₇ and R_{7'} form, together with the nitrogen atom to which they are attached, a heterocycloalkyl comprising one or more heteroatoms chosen from a nitrogen atom and an oxygen atom,
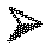 **R₅** represents:
   ∘ a hydrogen atom,
   ∘ a linear group (C₁-C₃)alkyl, optionally substituted with a pyridyl group,
      or
   ∘ an aromatic group chosen from aryl and pyridyl,
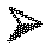 **R₆** and **R'₆**, which may be identical or different, represent a hydrogen atom or a linear alkyl group,
   in the form of the base or of an acid-addition or base-addition salt
   for the preparation of a medicament for treating and preventing diseases necessitating a modulation of the b-FGFs.

The present invention also describes a compound of formula (I) as defined above, for its use in the treatment and prevention of diseases necessitating a modulation of the FGFs.

A subject of the present invention is also a compound 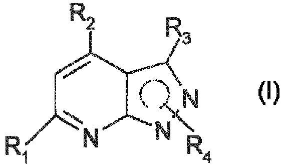 in which:
the representation of the pyrazole ring indicates that the substituent R₄ may be borne either by the nitrogen alpha to the pyridine ring (I') or by the nitrogen alpha to the carbon bearing a substituent R₃ (I") such that: 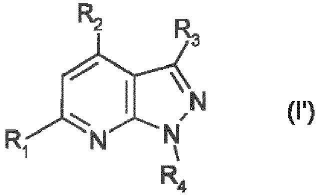 Either 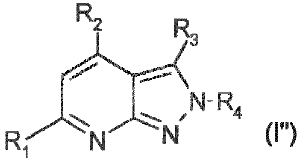
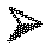 **R₁** represents an aryl, pyridyl or pyrazolyl group optionally substituted with one or more substituents chosen from:
   ∘ a halogen atom,
   ∘ a group -CF3,
   ∘ a cyano group,
   ∘ a group -NR₆R₆' in which R₆ and R₆' are as defined below,
   ∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
   ∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
   ∘ a group -COR₁₂ in which R₁₂ represents a hydroxyl group or a group -NR₆R₆', in which R₆ and R₆' are as defined below,
   ∘ a group -CONR₇R₇' such that R₇ and R₇' form, together with the nitrogen atom to which they are attached, a heterocycloalkyl comprising one or more heteroatoms chosen from a nitrogen atom and an oxygen atom,
   ∘ a group -(CH₂)ₚNHSO₂CH₃ in which p represents 0 or 1,
   ∘ a group -OR₁₃ in which R₁₃ represents a linear group (C₁-C3)alkyl,
   ∘ a group (C₁-C₃)alkyl,
   Or R₁ represents a bicyclic group of formula A below: 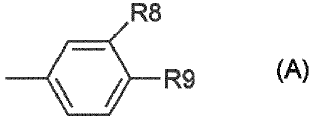 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
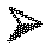 **R₂** represents a group:
   ∘ -CF₃,
   ∘ -CHF₂,
   ∘ -COOH,
   or
   ∘ -CONHR₅, in which R₅ is as defined below,
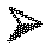 **R₃** represents:
   ∘ a hydrogen atom,
   ∘ an aryl group, optionally substituted with an alkoxymethyl group,
   ∘ a cycloalkyl group,
   or
   ∘ a heteroaryl group chosen from thienyl and pyridyl groups,
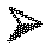 **R₄** represents:
   ∘ a hydrogen atom,
   ∘ a linear group (C₁-C₃)alkyl, optionally substituted with a group - NR₆R₆' in which R₆ and R'₆ are as defined below or a group -NR₇R_{7'}' such that R₇ and R_{7'} form, together with the nitrogen atom to which they are attached, a heterocycloalkyl comprising one or more heteroatoms chosen from a nitrogen atom and an oxygen atom,
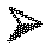 **R₅** represents:
   ∘ a hydrogen atom,
   ∘ a linear group (C₁-C₃)alkyl, optionally substituted with a pyridyl group,
      or
   ∘ an aromatic group chosen from aryl and pyridyl,
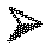 **R₆** and **R'₆**, which may be identical or different, represent a hydrogen atom or a linear alkyl group,
   in the form of the base or of an acid-addition or base-addition salt, for its use in the treatment and prevention of cancers, especially carcinomas with a substantial degree of vascularization such as lung, breast, prostate, pancreatic, bowel, kidney and oesophageal carcinomas, cancers that induce metastases, such as bowel cancer, liver cancer and stomach cancer, melanomas, gliomas, lymphomas and leukaemias, and also thrombopenias.

A compound of formula (I) according to the present invention may be administered alone or in combination with one or more compounds with anti-angiogenic activity or with one or more cytotoxic compounds (chemotherapy), or alternatively in combination with a radiotherapy. Thus, a subject of the present invention is also a compound of formula (I) as defined above administered in combination with one or more anticancer active principles and/or with any anti-VEGF and/or administered in combination with a radiotherapy.

A subject of the present invention is also a compound 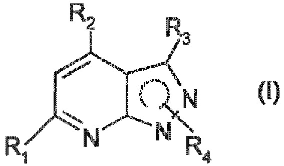 in which:
the representation of the pyrazole ring indicates that the substituent R₄ may be borne either by the nitrogen alpha to the pyridine ring (I') or by the nitrogen alpha to the carbon bearing a substituent R₃ (I") such that: 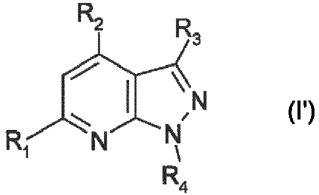 Either 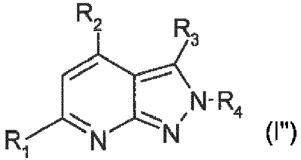
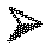 **R₁** represents an aryl, pyridyl or pyrazolyl group optionally substituted with one or more substituents chosen from:
   c a halogen atom,
   ∘ a group -CF3,
   c a cyano group,
   ∘ a group -NR₆R₆' in which R₆ and R₆' are as defined below,
   ∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
   ∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
   ∘ a group -COR₁₂ in which R₁₂ represents a hydroxyl group or a group -NR₆R₆', in which R₆ and R₆' are as defined below,
   ∘ a group -CONR₇R₇' such that R₇ and R₇' form, together with the nitrogen atom to which they are attached, a heterocycloalkyl comprising one or more heteroatoms chosen from a nitrogen atom and an oxygen atom,
   ∘ a group -(CH₂)ₚNHSO₂CH₃ in which p represents 0 or 1,
   ∘ a group -OR₁₃ in which R₁₃ represents a linear group (C₁-C3)alkyl,
   ∘ a group (C₁-C₃)alkyl,
   Or R₁ represents a bicyclic group of formula A below: 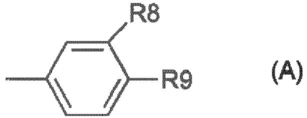 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
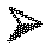 **R₂** represents a group:
   ∘ -CF₃,
   ∘ -CHF₂,
   ∘ -COOH,
   or
   ∘ -CONHR₅, in which R₅ is as defined below,
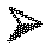 **R₃** represents:
   ∘ a hydrogen atom,
   ∘ an aryl group, optionally substituted with an alkoxymethyl group,
   ∘ a cycloalkyl group,
   or
   ∘ a heteroaryl group chosen from thienyl and pyridyl groups,
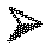 **R₄** represents:
   ∘ a hydrogen atom,
   ∘ a linear group (C₁-C₃)alkyl, optionally substituted with a group - NR₆R₆' in which R₆ and R'₆ are as defined below or a group -NR₇R_{7'}' such that R₇ and R_{7'} form, together with the nitrogen atom to which they are attached, a heterocycloalkyl comprising one or more heteroatoms chosen from a nitrogen atom and an oxygen atom,
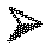 **R₅** represents:
   ∘ a hydrogen atom,
   ∘ a linear group (C₁-C₃)alkyl, optionally substituted with a pyridyl group, or
   ∘ an aromatic group chosen from aryl and pyridyl,
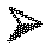 **R₆** and **R'₆**, which may be identical or different, represent a hydrogen atom or a linear alkyl group,
   in the form of the base or of an acid-addition or base-addition salt,
   for its use in the treatment and prevention of cardiovascular diseases such as atherosclerosis, post-angioplasty restenosis, in the treatment of diseases associated with complications arising after the insertion of endovascular prostheses and/or aorto-coronary bypasses or other vascular grafts, cardiac hypertrophy, vascular complications of diabetes such as diabetic retinopathy, hepatic, renal and pulmonary fibroses, neuropathic pain, chronic inflammatory diseases such as rheumatoid arthritis or IBD, prostate hyperplasia, psoriasis, clear-cell acanthoma, osteoarthritis, achondroplasias (ACH), hypochondroplasias (HCH), TD (thanatophoric dysplasia), obesity, and macular degeneration, such as age-related macular degeneration (AMD).

According to the three preceding aspects of the invention, the compound is more particularly selected from the following compounds:

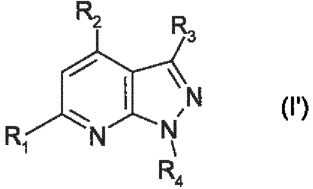

| **No.** | **R₂** | **R₃** | **R₁** | **R₄** | **Salt** | **m.p. (°C)** | **M+H⁺** |
|---|---|---|---|---|---|---|---|
| **1** | CF₃ | Ph | 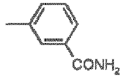 | Me | / | / | 397 |
| **2** | CF₃ | Ph | 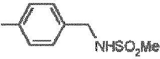 - | Me | / | / | 461 |
| **3** | CF₃ | Ph | 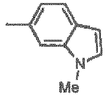 | Me | / | / | 407 |
| **4** | CF₃ | Ph | 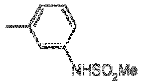 | Me | / | / | 447 |
| **5** | CF₃ | Ph | 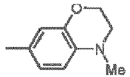 | Me | / | / | 426 |
| **6** | CF₃ | Ph | 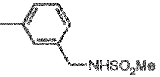 | Me | / | / | 461 |
| **7** | CF₃ | Ph | 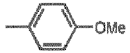 | Me | / | / | 384 |
| **8** | CF₃ | Ph | 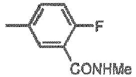 | Me | / | / | 429 |
| **9** | CF₃ | Ph | 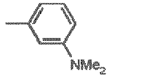 | Me | / | / | 397 |
| **10** | CF₃ | Ph | 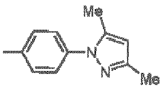 | Me | / | / | 448 |
| **11** | CF₃ | Ph | 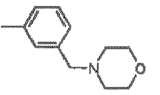 | Me | / | / | 453 |
| **12** | CF₃ | Ph | 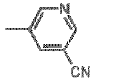 | Me | / | / | 380 |
| **13** | CF₃ | Ph | 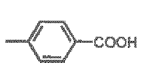 | Me | / | / | 398 |
| **14** | CF₃ | Ph | 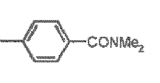 | Me | / | / | 425 |
| **15** | CF₃ | Ph | 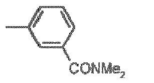 | Me | / | / | 425 |
| **16** | CF₃ | Ph | 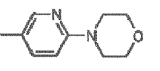 | Me | / | / | 440 |
| **17** | CF₃ | Ph | 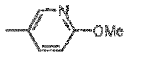 | Me | / | / | 385 |
| **18** | CF₃ | Ph | 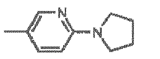 | Me | / | / | 424 |
| **19** | CF₃ | Ph | 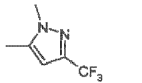 | Me | / | / | 426 |
| **20** | CF₃ | Ph | 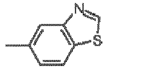 | Me | / | / | 411 |
| **21** | CF₃ | Ph | 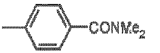 | H | HCl | / | 411 |
| **22** | CF₃ | Ph | 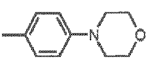 | H | HCl | / | 425 |
| **23** | CF₃ | Ph | 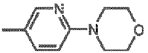 | H | HCl | / | 426 |
| **24** | CF₃ | Ph | 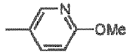 | H | HCl | / | 371 |
| **25** | CF₃ | Ph | 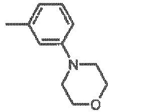 | H | HCl | / | 425 |
| **26** | CF₃ | Ph | 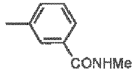 | H | HCl | / | 397 |
| **27** | CF₃ | Ph | 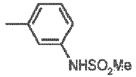 | H | HCl | / | 433 |
| **28** | CF₃ | Ph | 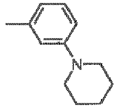 | H | HCl | / | 423 |
| **29** | CF₃ | Ph | 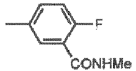 | H | HCl | / | 415 |
| **30** | CF₃ | Ph | 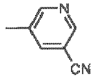 | H | HCl | / | 366 |
| **31** | CHF₂ | Ph | 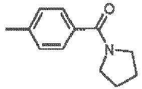 | H | HCl | 248 | 455 |
| **32** | CHF₂ | Ph | 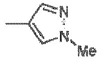 | H | HCl | / | 362 |
| **33** | CHF₂ | Ph | 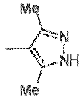 | H | HCl | / | 376 |
| **34** | CHF₂ | Ph | 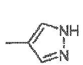 | H | HCl | / | 348 |
| **35** | CHF₂ | Ph | 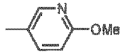 | H | HCl | / | 389 |
| **36** | CHF₂ | Ph | 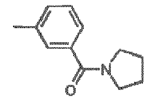 | H | HCl | / | 455 |
| **37** | CHF₂ | Ph | 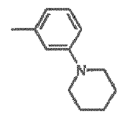 | H | HCl | / | 441 |
| **38 Ex. 2** | COOH | H | 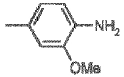 | H | / | / | 285 |
| **39** | CONHMe | Ph | 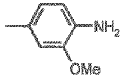 | H | TFA | / | 458 |
| **40** | CONH₂ | Ph | 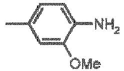 | H | TFA | / | 474 |
| **41** | CONHMe | H | 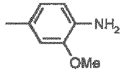 | H | TFA | / | 412 |
| **42** | CONH₂ | H | 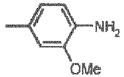 | H | TFA | / | 398 |
| **43** | COOH | Ph | 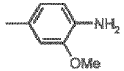 | H | / | / | 361 |
| **44** | COOH | Ph | 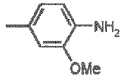 | Me | / | / | 375 |
| **45** | COOH | H | 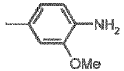 | Me | / | / | 299 |
| **46 Ex. 1** | CONH₂ | Ph | 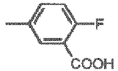 | H | TFA | / | 377 |
| **47** | CONH₂ | Ph | 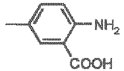 | H | / | / | 374 |
| **48** | CONH₂ | Ph | 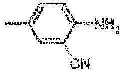 | H | TFA | / | 355 |
| **49** | CONH₂ | 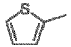 | 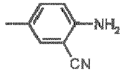 | H | / | / | 361 |
| **50** | COOH | cPr | 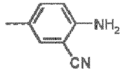 | H | / | / | 320 |
| **51** | CONH₂ | H | 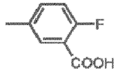 | H | / | / | 301 |
| **52** | CONH₂ | Ph | 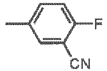 | H | / | / | 358 |
| **53 Ex. 3** | CHF₂ | Ph | 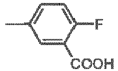 | H | / | / | 384 |
| **54** | CF₃ | Ph | 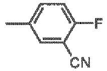 | H | / | / | 383 |
| **55** | CHF₂ | Ph | 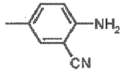 | H | HCl | 282 | 362 |
| **56 Ex. 4** | CF₃ | Ph | 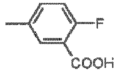 | H | / | / | 402 |
| **57 Ex. 6** | CF₃ | Ph | 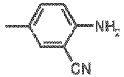 | Me | / | 269 | 394 |
| **58** | CF₃ | Ph | 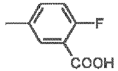 | Me | / | / | 416 |
| **59** | CF₃ | Ph | 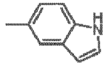 | H | / | / | 379 |
| **60** | CF₃ | Ph | 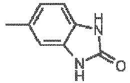 | H | / | 380 | 396 |
| **61 Ex. 5** | CF₃ | Ph | 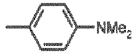 | H | / | 227 | 383 |
| **62** | CONHPh | H | 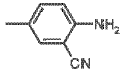 | H | HCl | / | 355 |
| **63** | 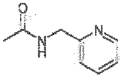 | H | 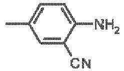 | H | HCl | / | 370 |
| **64** | 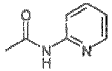 | H | 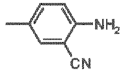 | H | HCl | / | 356 |
| **65** | 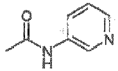 | H | 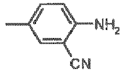 | H | HCl | / | 356 |
| **66** | 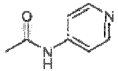 | H | 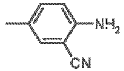 | H | HCl | / | 356 |
| **67** | CHF₂ | 4-Py | 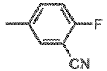 | Me | / | / | 380 |
| **68** | CONHPh | H | 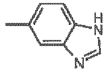 | H | HCl | / | 355 |
| **69** | 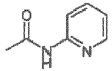 | H | 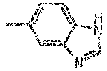 | H | HCl | / | 356 |
| **70** | 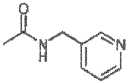 | H | 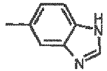 | H | HCl | / | 370 |
| **71** | CONHPh | H | 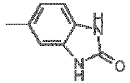 | H | / | / | 371 |
| **72 Ex. 10** | CHF₂ | H | 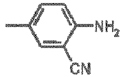 | Me | / | 251 | 300 |
| **73** | CHF₂ | H | 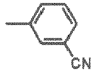 | Me | / | 162 | 285 |
| **74** | CHF₂ | H | 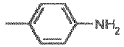 | Me | / | 149 | 275 |
| **75 Ex. 12** | CHF₂ | H | 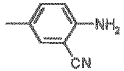 | H | / | 263 | 286 |
| **76** | CF₃ | Ph | 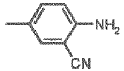 | 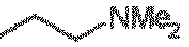 | / | 183 | 451 |
| **77** | CF₃ | Ph | 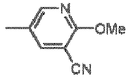 | Me | / | 250 | 410 |
| **78** | CHF₂ | Ph | 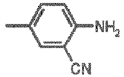 | Me | / | 246 | 376 |
| **79** | CHF₂ | Ph | 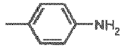 | Me | / | 176 | 351 |
| **80** | CHF₂ | Ph | 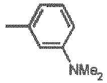 | Me | / | 154 | 379 |
| **81** | CF₃ | Ph | 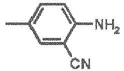 | 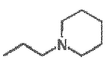 | / | 192 | 491 |
| **82** | CF₃ | Ph | 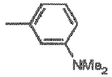 | 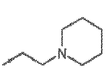 | HCl | 227 | 494 |
| **83 Ex. 13** | CHF₂ | Ph | 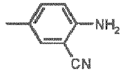 | 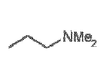 | / | 163 | 433 |
| **84** | CF₃ | H | 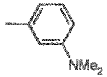 | 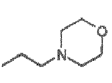 | / | 110 | 420 |
| **85** | CF₃ | H | 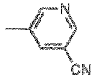 | 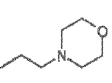 | / | / | 403 |
| **86** | CF₃ | H | 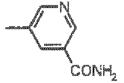 | 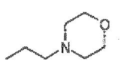 | / | 238 | 421 |
| **87** | CF₃ | H | 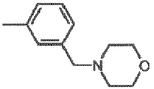 | Me | / | 105 | 377 |
| **88 Ex. 8** | CF₃ | H | 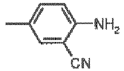 | Me | / | 276 | 318 |
| **89** | CF₃ | Ph | 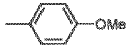 | Me | / | 181 | 384 |
| **90** | CF₃ | H | 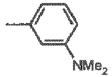 | Me | / | 91 | 321 |
| **91 Ex. 15** | CHF₂ | 3-Py | 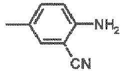 | H | / | 296 | 363 |
| **92** | CHF₂ | 4-Py | 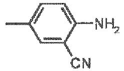 | H | / | 325 | 363 |
| **93 Ex. 14** | CF₃ | 3-Py | 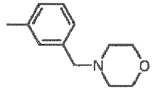 | Me | / | 155 | 454 |
| **94** | CHF₂ | 3MeO-Ph | 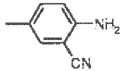 | Me | / | 233 | 392 |
| **95** | CF₃ | H | 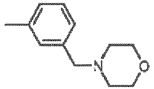 | Pr | HCl | 271 | 405 |
| **96** | CF₃ | H | 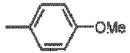 | Pr | / | 72 | 336 |
| **97** | CF₃ | 3MeO-Ph | 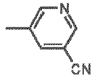 | Me | / | 194 | 410 |
| **98** | CF₃ | 3MeO-Ph | 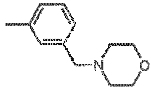 | Me | / | 114 | 483 |
| **99** | CF₃ | 3MeO-Ph | 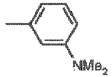 | Me | / | 138 | 427 |
| **100** | CF₃ | 3MeO-Ph | 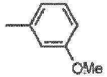 | Me | / | 133 | 414 |
| **101** | CONH₂ | 3-Py | 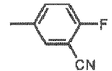 | H | / | / | 377 |

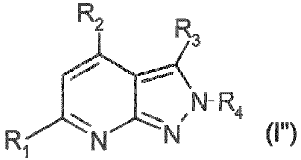

| **No.** | **R₂** | **R₃** | **R₁** | **R₄** | **Salt** | **m.p. (°C)** | **M+H⁺** |
|---|---|---|---|---|---|---|---|
| **102** | COOH | H | 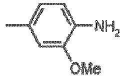 | Me | / | / | 299 |
| **103** | CONH₂ | H | 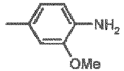 | Me | TFA | / | 412 |
| **104** | COOH | H | 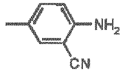 | Me | / | / | 294 |
| **105** | CHF₂ | H | 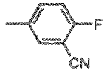 | Me | / | / | 303 |
| **106 Ex. 11** | CHF₂ | H | 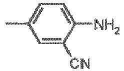 | Me | / | / | 300 |
| **107** | CHF₂ | H | 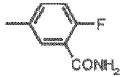 | Me | / | / | 321 |
| **108 Ex. 7** | CF₃ | Ph | 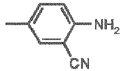 | Me | / | 295 | 394 |
| **109** | CF₃ | Ph | 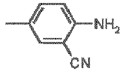 | 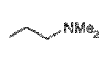 | / | 237 | 451 |
| **110** | CF₃ | Ph | 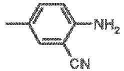 | 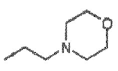 | / | 249 | 493 |
| **111** | CHF₂ | H | 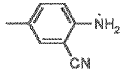 | 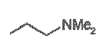 | / | 182 | 357 |
| **112** | CHF₂ | H | 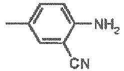 | 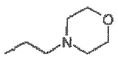 | / | 242 | 399 |
| **113** | CF₃ | H | 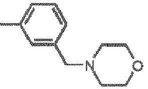 | 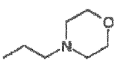 | / | 101 | 476 |
| **114 Ex. 9** | CF₃ | H | 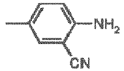 | Me | / | 249 | 318 |
| **115** | CF₃ | H | 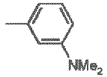 | Pr | HCl | 181 | 425 |
| **116** | CHF₂ | H | 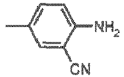 | 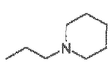 | / | 230 | 397 |
| **117** | CHF₂ | H | 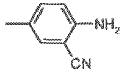 | Pr | / | 214 | 328 |
| **118** | CF₃ | H | 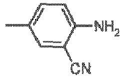 | Pr | / | 239 | 346 |
| **119** | CF₃ | H | 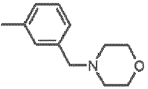 | Pr | / | 288 | 405 |
| **120** | CF₃ | H | 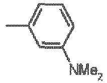 | Pr | / | 89 | 349 |

A further subject of the present invention is also the combination of a compound 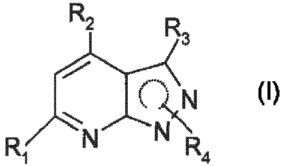 in which:
the representation of the pyrazole ring indicates that the substituent R₄ may be borne either by the nitrogen alpha to the pyridine ring (I') or by the nitrogen alpha to the carbon bearing a substituent R₃ (I") such that: 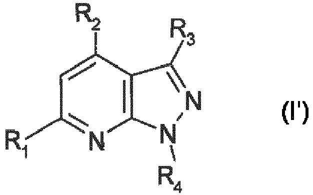 Either 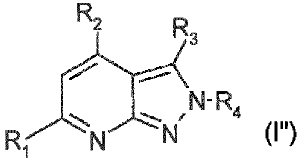
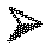 **R₁** represents an aryl, pyridyl or pyrazolyl group optionally substituted with one or more substituents chosen from:
   ∘ a halogen atom,
   ∘ a group -CF₃,
   ∘ a cyano group,
   ∘ a group -NR₆R₆' in which R₆ and R₆' are as defined below,
   ∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
   ∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
   ∘ a group -COR₁₂ in which R₁₂ represents a hydroxyl group or a group -NR₆R₆', in which Re and R₆' are as defined below,
   ∘ a group -CONR₇R₇' such that R₇ and R₇' form, together with the nitrogen atom to which they are attached, a heterocycloalkyl comprising one or more heteroatoms chosen from a nitrogen atom and an oxygen atom,
   ∘ a group -(CH₂)ₚNHSO₂CH₃ in which p represents 0 or 1,
   ∘ a group -OR₁₃ in which R₁₃ represents a linear group (C₁-C₃)alkyl,
   ∘ a group (C₁-C₃)alkyl,
   Or R₁ represents a bicyclic group of formula A below: 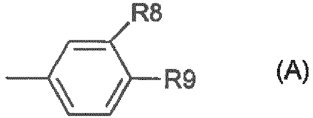 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
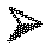 **R₂** represents a group:
   ∘ -CF₃,
   ∘ -CHF₂,
   ∘ -COOH,
   or
   ∘ -CONHR₅, in which R₅ is as defined below,
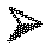 **R₃** represents:
   ∘ a hydrogen atom,
   ∘ an aryl group, optionally substituted with an alkoxymethyl group,
   ∘ a cycloalkyl group,
   or
   ∘ a heteroaryl group chosen from thienyl and pyridyl groups,
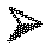 **R₄** represents:
   ∘ a hydrogen atom,
   ∘ a linear group (C₁-C₃)alkyl, optionally substituted with a group - NR₆R₆' in which R₆ and R'₆ are as defined below or a group -NR₇R₇' such that R₇ and R₇' form, together with the nitrogen atom to which they are attached, a heterocycloalkyl comprising one or more heteroatoms chosen from a nitrogen atom and an oxygen atom,
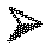 **R₅** represents:
   ∘ a hydrogen atom,
   ∘ a linear group (C₁-C₃)alkyl, optionally substituted with a pyridyl group,
      or
   ∘ an aromatic group chosen from aryl and pyridyl,
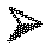 **R₆** and **R'₆,** which may be identical or different, represent a hydrogen atom or a linear alkyl group,
   in the form of the base or of an acid-addition or base-addition salt with one or more anticancer active principles and/or with any anti-VEGF

According to another of its aspects, the present invention relates to pharmaceutical compositions comprising a compound of formula (I). 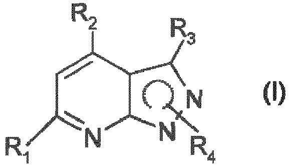 in which:
the representation of the pyrazole ring indicates that the substituent R₄ may be borne either by the nitrogen alpha to the pyridine ring (I') or by the nitrogen alpha to the carbon bearing a substituent R₃ (I") such that: 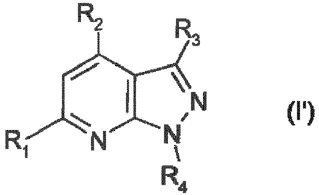 Either 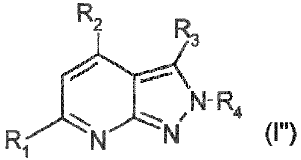
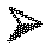 **R₁** represents an aryl, pyridyl or pyrazolyl group optionally substituted with one or more substituents chosen from:
   ∘ a halogen atom,
   ∘ a group -CF3,
   ∘ a cyano group,
   ∘ a group -NR₆R₆' in which Re and R₆' are as defined below,
   ∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
   ∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
   ∘ a group -COR₁₂ in which R₁₂ represents a hydroxyl group or a group -NR₆R₆', in which R₆ and R₆' are as defined below,
   ∘ a group -CONR₇R₇' such that R₇ and R₇' form, together with the nitrogen atom to which they are attached, a heterocycloalkyl comprising one or more heteroatoms chosen from a nitrogen atom and an oxygen atom,
   ∘ a group -(CH₂)ₚNHSO₂CH₃ in which p represents 0 or 1,
   ∘ a group -OR₁₃ in which R₁₃ represents a linear group (C₁-C₃)alkyl,
   ∘ a group (C₁-C₃)alkyl,
   Or R₁ represents a bicyclic group of formula A below: 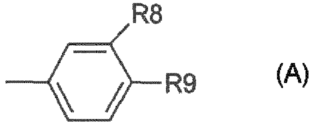 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
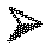 **R2** represents a group:
   ∘ -CF₃,
   ∘ -CHF₂,
   or
   ∘ -CONHR₅, in which R₅ is as defined below,
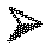 **R₃** represents:
   ∘ a hydrogen atom,
   ∘ an aryl group, optionally substituted with an alkoxymethyl group,
   ∘ a cycloalkyl group,
   or
   ∘ a heteroaryl group chosen from thienyl and pyridyl groups,
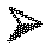 **R₄** represents:
   ∘ a hydrogen atom,
   ∘ a linear group (C₁-C₃)alkyl, optionally substituted with a group - NR₆R₆' in which R₆ and R'₆ are as defined below or a group -NR₇R₇' such that R₇ and R_{7'} form, together with the nitrogen atom to which they are attached, a heterocycloalkyl comprising one or more heteroatoms chosen from a nitrogen atom and an oxygen atom,
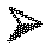 **R₅** represents:
   ∘ a hydrogen atom,
   ∘ a linear group (C₁-C₃)alkyl, optionally substituted with a pyridyl group, or
   ∘ an aromatic group chosen from aryl and pyridyl,
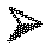 **R₆** and **R'₆,** which may be identical or different, represent a hydrogen atom or a linear alkyl group,
   in the form of the base or of an acid-addition or base-addition salt, and also at least one pharmaceutically acceptable excipient.

According to this aspect, the present invention particularly relates to pharmaceutical compositions comprising a compound selected from the following compounds

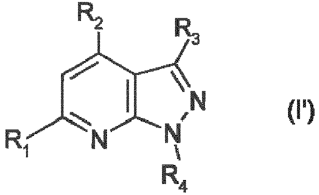

| **No.** | **R₂** | **R₃** | **R₁** | **R₄** | **Salt** | **m.p. (°C)** | **M+H⁺** |
|---|---|---|---|---|---|---|---|
| **1** | CF₃ | Ph | 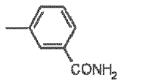 | Me | / | / | 397 |
| **2** | CF₃ | Ph | 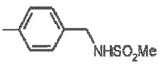 | Me | / | / | 461 |
| **3** | CF₃ | Ph | 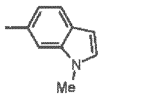 | Me | / | / | 407 |
| **4** | CF₃ | Ph | 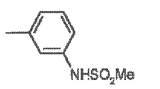 | Me | / | / | 447 |
| **5** | CF₃ | Ph | 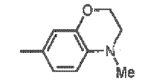 | Me | / | / | 426 |
| **6** | CF₃ | Ph | 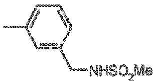 | Me | / | / | 461 |
| **7** | CF₃ | Ph | 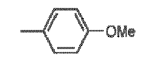 | Me | / | / | 384 |
| **8** | CF₃ | Ph | 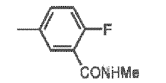 | Me | / | / | 429 |
| **9** | CF₃ | Ph | 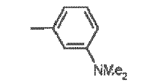 | Me | / | / | 397 |
| **10** | CF₃ | Ph | 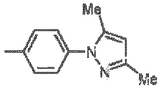 | Me | / | / | 448 |
| **11** | CF₃ | Ph | 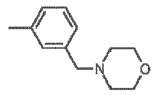 | Me | / | / | 453 |
| **12** | CF₃ | Ph | 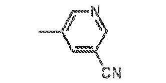 | Me | / | / | 380 |
| **13** | CF₃ | Ph | 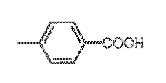 | Me | / | | 398 |
| **14** | CF₃ | Ph | 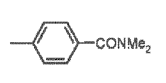 | Me | / | / | 425 |
| **15** | CF₃ | Ph | 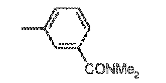 | Me | / | / | 425 |
| **16** | CF₃ | Ph | 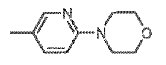 | Me | / | / | 440 |
| **17** | CF₃ | Ph | 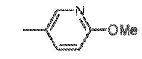 | Me | / | / | 385 |
| **18** | CF₃ | Ph | 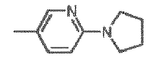 | Me | / | / | 424 |
| **19** | CF₃ | Ph | 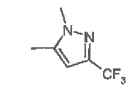 | Me | / | / | 426 |
| **20** | CF₃ | Ph | 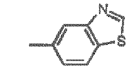 | Me | / | / | 411 |
| **21** | CF₃ | Ph | 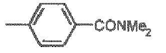 | H | HCl | / | 411 |
| **22** | CF₃ | Ph | 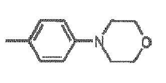 | H | HCl | / | 425 |
| **23** | CF₃ | Ph | 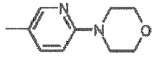 | H | HCl | / | 426 |
| **24** | CF₃ | Ph | 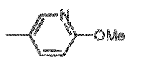 | H | HCl | / | 371 |
| **25** | CF₃ | Ph | 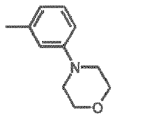 | H | HCl | / | 425 |
| **26** | CF₃ | Ph | 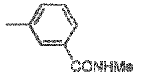 | H | HCl | / | 397 |
| **27** | CF₃ | Ph | 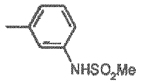 | H | HCl | / | 433 |
| **28** | CF₃ | Ph | 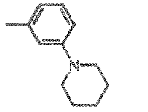 | H | HCl | / | 423 |
| **29** | CF₃ | Ph | 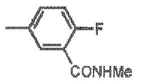 | H | HCl | / | 415 |
| **30** | CF₃ | Ph | 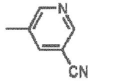 | H | HCl | / | 366 |
| **31** | CHF₂ | Ph | 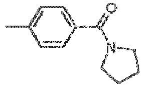 | H | HCl | 248 | 455 |
| **32** | CHF₂ | Ph | 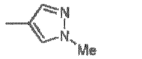 | H | HCl | / | 362 |
| **33** | CHF₂ | Ph | 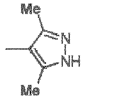 | H | HCl | / | 376 |
| **34** | CHF₂ | Ph | 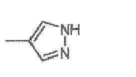 | H | HCl | / | 348 |
| **35** | CHF₂ | Ph | 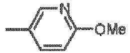 | H | HCl | / | 389 |
| **36** | CHF₂ | Ph | 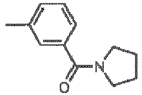 | H | HCl | / | 455 |
| **37** | CHF₂ | Ph | 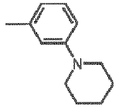 | H | HCl | / | 441 |
| **39** | CONHMe | Ph | 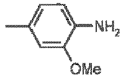 | H | TFA | / | 458 |
| **40** | CONH₂ | Ph | 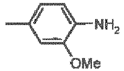 | H | TFA | / | 474 |
| **41** | CONHMe | H | 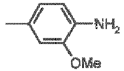 | H | TFA | / | 412 |
| **42** | CONH₂ | H | 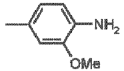 | H | TFA | / | 398 |
| **46 Ex. 1** | CONH₂ | Ph | 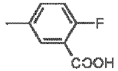 | H | TFA | / | 377 |
| **47** | CONH₂ | Ph | 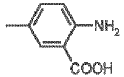 | H | / | / | 374 |
| **48** | CONH₂ | Ph | 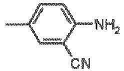 | H | TFA | / | 355 |
| **49** | CONH₂ | 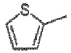 | 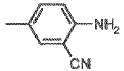 | H | / | / | 361 |
| **51** | CONH₂ | H | 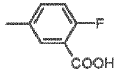 | H | / | / | 301 |
| **52** | CONH₂ | Ph | 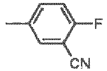 | H | / | / | 358 |
| **53 Ex. 3** | CHF₂ | Ph | 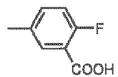 | H | / | / | 384 |
| **54** | CF₃ | Ph | 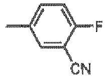 | H | / | / | 383 |
| **55** | CHF₂ | Ph | 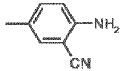 | H | HCl | 282 | 362 |
| **56 Ex. 4** | CF₃ | Ph | 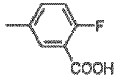 | H | / | / | 402 |
| **57 Ex. 6** | CF₃ | Ph | 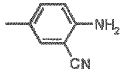 | Me | / | 269 | 394 |
| **58** | CF₃ | Ph | 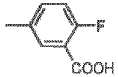 | Me | / | / | 416 |
| **59** | CF₃ | Ph | 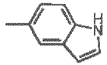 | H | / | / | 379 |
| **60** | CF₃ | Ph | 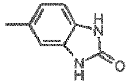 | H | / | 380 | 396 |
| **61 Ex.5** | CF₃ | Ph | 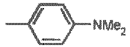 | H | / | 227 | 383 |
| **62** | CONHPh | H | 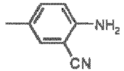 | H | HCl | / | 355 |
| **63** | 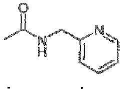 | H | 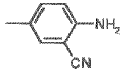 | H | HCl | / | 370 |
| **64** | 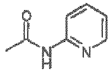 | H | 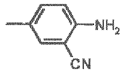 | H | HCl | / | 356 |
| **65** | 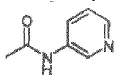 | H | 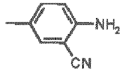 | H | HCl | / | 356 |
| **66** | 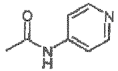 | H | 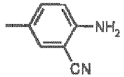 | H | HCl | / | 356 |
| **67** | CHF₂ | 4-Py | 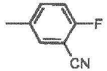 | Me | / | / | 380 |
| **68** | CONHPh | H | 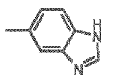 | H | HCl | / | 355 |
| **69** | 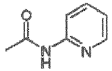 | H | 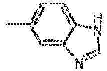 | H | HCl | / | 356 |
| **70** | 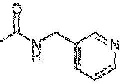 | H | 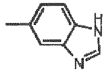 | H | HCl | / | 370 |
| **71** | CONHPh | H | 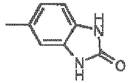 | H | / | / | 371 |
| **72 Ex. 10** | CHF₂ | H | 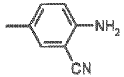 | Me | / | 251 | 300 |
| **73** | CHF₂ | H | 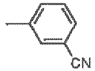 | Me | / | 162 | 285 |
| **74** | CHF₂ | H | 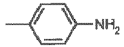 | Me | / | 149 | 275 |
| **75 Ex. 12** | CHF₂ | H | 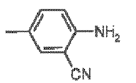 | H | / | 263 | 286 |
| **76** | CF₃ | Ph | 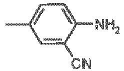 | 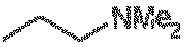 | / | 183 | 451 |
| **77** | CF₃ | Ph | 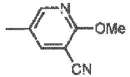 | Me | / | 250 | 410 |
| **78** | CHF₂ | Ph | 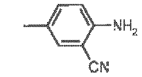 | Me | / | 246 | 376 |
| **79** | CHF₂ | Ph | 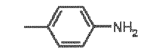 | Me | / | 176 | 351 |
| **80** | CHF₂ | Ph | 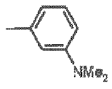 | Me | / | 154 | 379 |
| **81** | CF₃ | Ph | 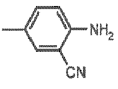 | 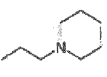 | / | 192 | 491 |
| **82** | CF₃ | Ph | 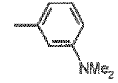 | 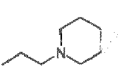 | HCl | 227 | 494 |
| **83 Ex. 13** | CHF₂ | Ph | 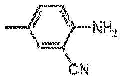 | 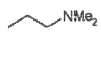 | / | 163 | 433 |
| **84** | CF₃ | H | 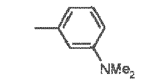 | 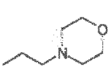 | / | 110 | 420 |
| **85** | CF₃ | H | 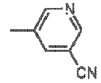 | 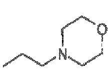 | / | / | 403 |
| **86** | CF₃ | H | 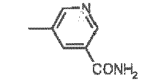 | 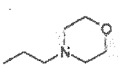 | / | 238 | 421 |
| **87** | CF₃ | H | 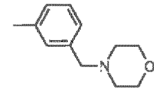 | Me | / | 105 | 377 |
| **88 Ex. 8** | CF₃ | H | 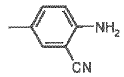 | Me | / | 276 | 318 |
| **89** | CF₃ | Ph | 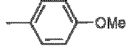 | Me | / | 181 | 384 |
| **90** | CF₃ | H | 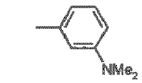 | Me | / | 91 | 321 |
| **91 Ex. 15** | CHF₂ | 3-Py | 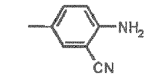 | H | / | 296 | 363 |
| **92** | CHF₂ | 4-Py | 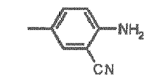 | H | / | 325 | 363 |
| **93 Ex. 14** | CF₃ | 3-Py | 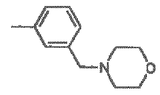 | Me | / | 155 | 454 |
| **94** | CHF₂ | 3MeO-Ph | 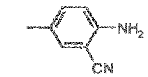 | Me | / | 233 | 392 |
| **95** | CF₃ | H | 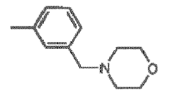 | Pr | HCl | 271 | 405 |
| **96** | CF₃ | H | 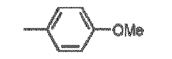 | Pr | / | 72 | 336 |
| **97** | CF₃ | 3MeO-Ph | 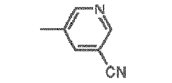 | Me | / | 194 | 410 |
| **98** | CF₃ | 3MeO-Ph | 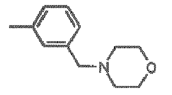 | Me | / | 114 | 483 |
| **99** | CF₃ | 3MeO-Ph | 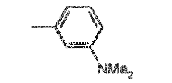 | Me | / | 138 | 427 |
| **100** | CF₃ | 3MeO-Ph | 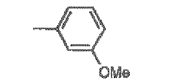 | Me | / | 133 | 414 |
| **101** | CONH₂ | 3-Py | 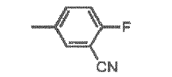 | H | / | / | 377 |

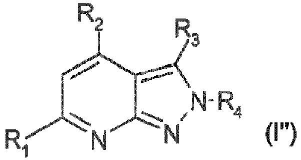

| **No.** | **R₂** | **R₃** | **R₁** | **R₄** | **Salt** | **m.p. (°C)** | **M+H⁺** |
|---|---|---|---|---|---|---|---|
| **103** | CONH₂ | H | 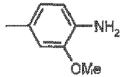 | Me | TFA | / | 412 |
| **105** | CHF₂ | H | 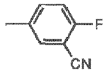 | Me | / | / | 303 |
| **106 Ex. 11** | CHF₂ | H | 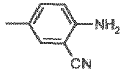 | Me | / | / | 300 |
| **107** | CHF₂ | H | 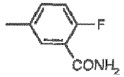 | Me | / | / | 321 |
| **108 Ex. 7** | CF₃ | Ph | 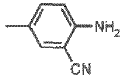 | Me | / | 295 | 394 |
| **109** | CF₃ | Ph | 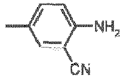 | 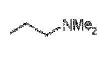 | / | 237 | 451 |
| **110** | CF₃ | Ph | 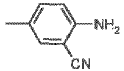 | 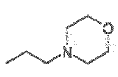 | / | 249 | 493 |
| **111** | CHF₂ | H | 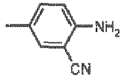 | 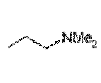 | / | 182 | 357 |
| **112** | CHF₂ | H | 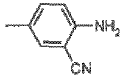 | 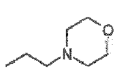 | / | 242 | 399 |
| **113** | CF₃ | H | 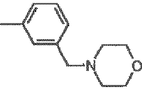 | 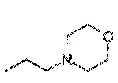 | / | 101 | 476 |
| **114 Ex.9** | CF₃ | H | 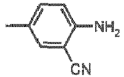 | Me | / | 249 | 318 |
| **115** | CF₃ | H | 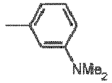 | Pr | HCl | 181 | 425 |
| **116** | CHF₂ | H | 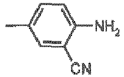 | 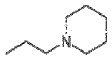 | / | 230 | 397 |
| **117** | CHF₂ | H | 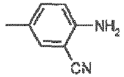 | Pr | / | 214 | 328 |
| **118** | CF₃ | H | 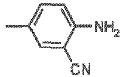 | Pr | / | 239 | 346 |
| **119** | CF₃ | H | 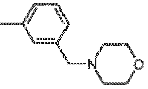 | Pr | / | 288 | 405 |
| **120** | CF₃ | H | 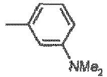 | Pr | / | 89 | 349 |

and also at least one pharmaceutically acceptable excipient.

These pharmaceutical compositions contain an effective dose of at least one compound as defined above, or a pharmaceutically acceptable salt, and also at least one pharmaceutically acceptable excipient. The said excipients are chosen, according to the pharmaceutical form and the mode of administration desired, from the usual excipients that are known to those skilled in the art.

In the pharmaceutical compositions for oral, sublingual, subcutaneous, intramuscular, intravenous, topical, local, intratracheal, intranasal, transdermal or rectal administration, the compound of formula (I) above, or the salt thereof, may be administered in unit administration form, as a mixture with standard pharmaceutical excipients, to man and animals for the treatment of the disorders or diseases mentioned previously.

The appropriate unit administration forms include oral-route forms such as tablets, soft or hard gel capsules, powders, granules and oral solutions or suspensions, sublingual, buccal, intratracheal, intraocular and intranasal administration forms, inhalation forms, topical, transdermal, subcutaneous, intramuscular or intravenous administration forms, rectal administration forms and implants. For topical administration, the disclosed compounds may be used in creams, gels, pomades or lotions.

The pharmaceutical compositions according to the present invention are preferably administered orally.

By way of example, a unit administration form of a compound according to the invention in tablet form may comprise the following components:

| | |
|---|---|
| Compound according to the invention | 50.0 mg |
| Mannitol | 223.75 mg |
| Croscaramellose sodium | 6.0 mg |
| Corn starch | 15.0 mg |
| Hydroxypropylmethylcellulose | 2.25 mg |
| Magnesium stearate | 3.0 mg |

The present invention also describes a pharmaceutical composition as defined above, as a medicament.

The compositions disclosed in the invention, for oral administration, contain recommended doses of 0.01 to 700 mg. There may be special cases in which higher or lower dosages are appropriate; such dosages are not outside the scope of the invention. According to the usual practice, the dosage that is appropriate to each patient is determined by the doctor according to the mode of administration, the age, weight and response of the patient, and also according to the degree of progress of the disease.

## Claims

1. Compound corresponding to formula (I): 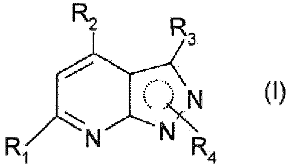 in which:
the representation of the pyrazole ring indicates that the substituent R₄ may be borne either by the nitrogen alpha to the pyridine ring (I') or by the nitrogen alpha to the carbon bearing a substituent R₃ (I") such that: 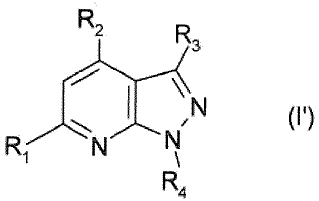 Either 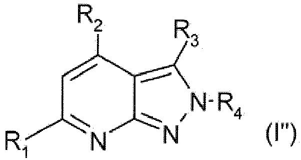
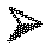 **R₁** represents an aryl, pyridyl or pyrazolyl group optionally substituted with one or more substituents chosen from:
∘ a fluorine atom,
∘ a group -CF3,
∘ a cyano group,
∘ a group -NR₃R₃' in which R₆ and R₆' are as defined below,
∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
∘ a group -COR₁₂ in which R₁₂ represents a hydroxyl group or a group -NR₆R₆', in which R₆ and R₆ are as defined below,
∘ a group -CONR₇R₇' such that R₇ and R₇' form, together with the nitrogen atom to which they are attached, a heterocycloalkyl comprising one or more heteroatoms chosen from a nitrogen atom and an oxygen atom,
∘ a group -(CH₂)ₚNHSO₂CH₃ in which p represents 0 or 1,
∘ a group -OR₁₃ in which R₁₃ represents a linear group (C₁-C₃)alkyl,
∘ a group ethyl, propyl or isopropyl,,
Or R₁ represents a bicyclic group of formula A below: 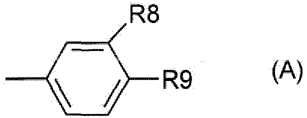 in which R₈ and R₉ form, together with the carbon atoms to which they are attached, a saturated or unsaturated heterocycle comprising one or more heteroatoms chosen from a nitrogen atom, an oxygen atom and a sulfur atom, such that the group (A) forms a dihydrobenzimidazolonyl, indolyl, dihydrobenzoxazinyl, benzothiazolyl or benzimidazolyl group, optionally substituted with one or more linear alkyl groups,
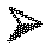 **R₂** represents a group:
∘ -CF₃,
∘ -CHF₂,
or
∘ -CONHR₅, in which R₅ is as defined below,
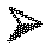 **R₃** represents:
∘ a hydrogen atom,
∘ an aryl group, optionally substituted with an alkoxymethyl group,
or
∘ a heteroaryl group chosen from thienyl and pyridyl groups,
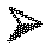 **R₄** represents:
∘ a hydrogen atom,
∘ a (C₁)alkyl, linear (C₃)alkyl, or linear (C₁-C₃)alkyl substituted with a group - NR₆R₆' in which R₆ and R'₆ are as defined below or a group -NR₇R₇' such that R₇ and R₇' form, together with the nitrogen atom to which they are attached, a heterocycloalkyl comprising one or more heteroatoms chosen from a nitrogen atom and an oxygen atom,
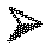 **R₅** represents:
∘ a hydrogen atom,
∘ a linear group (C₁-C₃)alkyl, optionally substituted with a pyridyl group,
or
∘ an aromatic group chosen from aryl and pyridyl,
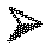 **R₆** and **R'₆,** which may be identical or different, represent a hydrogen atom or a linear alkyl group,
in the form of the base or of an acid-addition or base-addition salt,
with the exception of the following compounds :
2-Fluoro-5-(3-phenyl-4-trifluoromethyl-1H-pyrazolo[3,4-b]pyridin-6-yl)benzoic acid;
3-(1-Methyl-3-phenyl-4-trifluoromethyl-1H-pyrazolo[3,4-b]pyridin-6-yl)benzamide;
N-[4-(1-Methyl-3-phenyl-4-trifluoromethyl-1H-pyrazolo[3,4-b]pyridin-6-yl)benzyl]methanesulfonamide;
1-Methyl-6-(1-methyl-1H-indol-6-yl)-3-phenyl-4-trifluoromethyl-1H-pyrazolo[3,4-b]pyridine;
N-[3-(1-Methyl-3-phenyl-4-trifluoromethyl-1H-pyrazolo[3,4-b]pyridin-6-yl)phenyl]methanesulfonamide;
4-Methyl-7-(1-methyl-3-pheny)-4-trifluoromethyl-1H-pyrazolo[3,4-b]pyridin-6-yl)-3,4-dihydro-2H-benzo[1,4]oxazine;
N-[3-(1-Methyl-3-phenyl-4-trifluoromethyl-1H-pyrazolo[3,4-b]pyridin-6-yl)benzyl]methanesulfonamide;
6-(4-Methoxyphenyl)-1-methyl-3-phenyl-4-trifluoromethyl-1H-pyrazolo[3,4-b]pyridine;
2-Fluoro-N-methyl-5-(1-methyl-3-phenyl-4-trifluoromethyl-1H-pyrazolo[3,4-b]pyridin-6-yl)benzamide;
Dimethyl[3-(1-methyl-3-phenyl-4-trifluoromethyl-1H-pyrazolo[3,4-b]pyridin-6-yl)phenyl]amine;
6-[4-(3,5-Dimethylpyrazol-1-yl)phenyl]-1-methyl-3-phenyl-4-trifluoromethyl-1H-pyrazolo[3,4-b]pyridine;
1-Methyl-6-(3-morpholin-4-ylmethylphenyl)-3-phenyl-4-trifluoromethyl-1H-pyrazolo[3,4-b]pyridine;
5-(1-Methyl-3-phenyl-4-trifluoromethyl-1H-pyrazolo[3,4-b]pyridin-6-yl)nicotinonitrile;
4-(1-Methyl-3-phenyl-4-trifluoromethyl-1H-pyrazolo[3,4-b]pyridin-6-yl)benzoic acid;
N,N-Dimethyl-4-(1-methyl-3-phenyl-4-trifluoromethyl-1H-pyrazolo[3,4-b]pyridin-6-yl)benzamide;
N,N-Dimethyl-3-(1-methyl-3-phenyl-4-trifluoromethyl-1H-pyrazolo[3,4-b]pyridin-6-yl)benzamide;
1-Methyl-6-(6-morpholin-4-ylpyridin-3-yl)-3-phenyl-4-trifluoromethyl-1H-pyrazolo[3,4-b]pyridine;
6-(6-Methoxypyridin-3-yl)-1-methyl-3-phenyl-4-trifluoromethyl-1H-pyrazolo[3,4-b]pyridine;
1-Methyl-3-phenyl-6-(6-pyrrolidin-1-y)pyridin-3-yl)-4-trifluoromethyl-1H-pyrazolo[3,4-b]pyridine;
1-Methyl-6-(2-methyl-5-trifluoromethyl-2H-pyrazol-3-yl)-3-phenyl-4-trifluoromethyl-1H-pyrazolo[3,4-b]pyridine;
6-Benzothiazol-5-yl-1-methyl-3-phenyl-4-trifluoromethyl-1H-pyrazolo[3,4-b]pyridine;
N,N-Dimethyl-4-(3-phenyl-4-trifluoromethyl-1H-pyrazolo[3,4-b]pyridin-6-yl)benzamide;
6-(4-Morpholin-4-ylphenyl)-3-phenyl-4-trifluoromethyl-1H-pyrazolo[3,4-b]pyridine;
6-(6-Morpholin-4-ylpyridin-3-yl)-3-phenyl-4-trifluoromethyl-1H-pyrazolo[3,4-b]pyridine;
6-(6-Methoxypyridin-3-yl)-3-phenyl-4-trifluoromethyl-1H-pyrazolo[3,4-b]pyridine;
6-(3-Morpholin-4-ylphenyl)-3-phenyl-4-trifluoromethyl-1H-pyrazolo[3,4-b]pyridine;
N-Methyl-3-(3-phenyl-4-trifluoromethyl-1H-pyrazolo[3,4-b]pyridin-6-yl)benzamide;
N-[3-(3-Phenyl-4-trifluoromethyl-1H-pyrazolo[3,4-b]pyridin-6-yl)phenyl]methanesulfonamide;
3-Phenyl-6-(3-piperidin-1-ylphenyl)-4-trifluoromethyl-1H-pyrazolo[3,4-b]pyridine;
2-Fluoro-N-methyl-5-(3-phenyl-4-trifluoromethyl-1H-pyrazolo[3,4-b]pyridin-6-yl)benzamide;
5-(3-Phenyl-4-trifluoromethyl-1H-pyrazolo[3,4-b]pyridin-6-yl)nicotinonitrile;
2-Fluoro-5-(1-methyl-3-phenyl-4-trifluoromethyl-1H-pyrazolo[3,4-b]pyridin-6-yl)benzoic acid;
2-Amino-5-(4-difluoromethyl-2-methyl-2H-pyrazolo[3,4-b]pyridin-6-yl)benzonitrile;
Dimethyl[4-(3-phenyl-4-trifluoromethyl-1H-pyrazolo[3,4-b]pyridin-6-yl)phenyl]amine;
4-(3-Phenyl-4-trifluoromethyl-1H-pyrazolo[3,4-b]pyridin-6-yl)phenylamine;
6-(4-Methoxyphenyl)-3-phenyl-4-trifluoromethyl-1H-pyrazolo[3,4-b]pyridine;
2-Fluoro-5-(3-phenyl-4-trifluoromethyl-1H-pyrazolo[3,4-b]pyridin-6-yl)benzonitrile;
6-(1-ethyl-1H-pyrazol-4-yl)-1-methyl-4(trifluoromethyl)-1H-pyrazolo[3,4-b]pyridine;
4-(difluoromethyl)-6-(1-ethyl-1H-pyrazol-4-yl)-1-methyl-1H-pyrazolo[3,4-b]pyridine;
6-(4-ethylphenyl)-1-methyl-4-(trifluoromethyl)-1H-pyrazolo[3,4-b]pyridine;
6-(4-methoxypheny))-1-methyl-4-(trifluoromethyl)-1H-pyrazolo[3,4-b]pyridine;
6-(4-fluorophenyl)-1-metyl-4-(trifluoromethyl)-1H- pyrazolo[3,4-b]pyridine;
4-(difluoromethyl)-6-(2-methoxyphenyl)-1-methyl-1H-pyrazolo[3,4-b)pyridine;
6-(3-methoxyphenyl)-1-methyl-4-(trifluoromethyl)-1H-pyrazolo[3,4-b]pyridine;
6-(2,5-dimethoxyphenyl)-1-methyl-4-(trifluoromethyl)-1H-pyrazolo[3,4-b]pyridine;
4-(difluotomethyl)-6-(4-ethylphenyl)-1-methyl-1H-pyrazolo[3,4-b]pyridine;
4-(difluoromethyl)-6-(4-methoxyphenyl)-1-methyl-1H-pyrazolo[3,4-b]pyridine;
4-(difluoromethyl)-6-(3-methoxyphenyl)-1-methyl-1H-pyrazolo[3,4-b]pyridine;
4-(difluoromethyl)-6-(-methyl-1H-pyrazolo[3,4-b]pyridine;
4-(difluoromethyl)-6-(3,4-dimethoxyphenyl)-1-methyl-1H-pyrazolo[3,4-b]pyridine;
4-(difluoromethyl)-1-methyl-6-phenyl-1H-pyrazolo[3,4-b]pyridine;
1-methyl-6-phenyl-4-(trifluoromethyl)-1H-pyrazolo[3,4-b]pyridine.

2. Compounds of formula (I) according to Claim 1, **characterized in that** R₂ represents a group:
∘ -CHF₂, except when R₄ located on the nitrogen alpha to R₃ represents a methyl group and R₃ represents a hydrogen atom,
or
∘ -CONHR₅, in which R₅ represents a hydrogen atom or a linear group (C₁-C₃)alkyl, optionally substituted with a pyridyl group or an aromatic group chosen from aryl and pyridyl,
in the form of the base or of an acid-addition or base-addition salt.

3. Compounds of formula (I) according to any one of Claims 1 or 2, **characterized in that** R₁ represents an aryl, pyridyl or pyrazolyl group, optionally substituted with one or more substituents chosen from:
∘ a fluorine atom,
and
a group -COR₁₂, in which R₁₂ represents a hydroxyl group, in the form of the base or of an acid-addition or base-addition salt.

4. Compounds of formula (I) according to Claims 1 to 3, **characterized in that R₁** represents an aryl group, in the form of the base or of an acid-addition or base-addition salt.

5. Compounds of formula (I) according to any one of Claims 1, 3 and 4, **characterized in that R₂** represents a group:
∘ -CF₃,
∘ -CHF₂,
or
∘ -CONHR₅, in which R₅ represents a hydrogen atom or a linear group (C₁-C₃)alkyl, optionally substituted with a pyridyl group or an aromatic group chosen from aryl and pyridyl,
in the form of the base or of an acid-addition or base-addition salt.

6. Compound of formula (I) according to one of the preceding claims, **characterized in that** it is chosen from the following compounds:
[4-(4-Difluoromethyl-3-phenyl-1H-pyrazolo[3,4-b]pyridin-6-yl)phenyl]pyrrolidin-1-ylmethanone;
4-Difluoromethyl-6-(1-methyl-1H-pyrazol-4-yl)-3-phenyl-1H-pyrazolo[3,4-b]pyridine;
4-Difluoromethyl-6-(3,5-dimethyl-1H-pyrazol-4-yl)-3-phenyl-1H-pyrazolo[3,4-b]pyridine;
4-Difluoromethyl-3-phenyl-6-(H-pyrazol-4-yl)-1H-pyrazolo[3,4-b]pyridine;
4-Difluoromethyl-6-(6-methoxypyridin-3-yl)-3-phenyl-1H-pyrazolo[3,4-b]pyridine;
[3-(4-Difluoromethyl-3-phenyl-1H-pyrazolo[3,4-b]pyridin-6-yl)phenyl]pyrrolidin-1-ylmethanone;
4-Difluoromethyl-3-phenyl-6-(3-piperidin-1-ylphenyl)-1H-pyrazolo[3,4-b]pyridine;
6-(4-Amino-3-methoxyphenyl)-3-phenyl-1H-pyrazolo[3,4-b]pyridine-4-carboxylic acid methylamide;
6-(4-Amino-3-methoxyphenyl)-3-phenyl-1H-pyrazolo[3,4-b]pyridine-4-carboxylic acid amide ;
6-(4-Amino-3-methoxyphenyl)-1H-pyrazolo[3,4-b]pyridine-4-carboxylic acid methylamide;
4-(4-Amino-3-methoxyphenyl)-1H-pyrazolo[3,4-b]pyridine-6-carboxylic acid methylamide;
6-(4-Amino-3-methoxyphenyl)-1H-pyrazolo[3,4-b]pyridine-4-carboxylic acid amide;
6-(4-Amino-3-methoxyphenyl)-2-methyl-2H-pyrazolo[3,4-b]pyridine-4-carboxylic acid amide;
5-(4-Carbamoyl-3-phenyl-1H-pyrazolo[3,4-b]pyridin-6-yl)-2-fluorobenzoic acid;
2-Amino-5-(4-carbamoyl-3-phenyl-1H-pyrazolo[3,4-b]pyridin-6-yl)benzoic acid;
6-(4-Amino-3-cyanophenyl)-3-phenyl-1H-pyrazolo[3,4-b]pyridine-4-carboxylic acid amide;
6-(4-Amino-3-cyanophenyl)-3-thiophen-2-yl-1H-pyrazolo[3,4-b]pyridine-4-carboxylic acid amide;
5-(4-Carbamoyl-1H-pyrazolo[3,4-b]pyridin-6-yl)-2-fluorobenzoic acid;
6-(3-Cyano-4-fluorophenyl)-3-phenyl-1H-pyrazolo[3,4-b]pyridine-4-carboxylic acid amide;
5-(4-Difluoromethyl-3-phenyl-1H-pyrazolo[3,4-b]pyridin-6-yl)-2-fluorobenzoic acid;
2-Amino-5-(4-difluoromethyl-3-phenyl-1H-pyrazolo[3,4-b]pyridin-6-yl)benzonitrile;
2-Amino-5-(1-methyl-3-phenyl-4-trifluoromethyl-1H-pyrazolo[3,4-b]pyridin-6-yl)benzonitrile;
6-(3-Carbamoyl-4-fluorophenyl)-3-pyridin-3-yl-1H-pyrazolo[3,4-b]pyridine-4-carboxylic acid amide;
5-(4-Difluoromethyl-2-methyl-2H-pyrazolo[3,4-b]pyridin-6-yl)-2-fluorobenzonitrile;
6-(1H-lndol-6-yl)-3-phenyl-4-trifluoromethyl-1H-pyrazolo[3,4-b]pyridine;
5-(3-Phenyl-4-trifluoromethyl-1H-pyrazolo[3,4-b]pyridin-6-yl)-1,3-dihydrobenzimidazol-2-one;
6-(4-Amino-3-cyanophenyl)-1H-pyrazolo[3,4-b]pyridine-4-carboxylic acid phenylamide;
6-(4-Amino-3-cyanophenyl)-1H-pyrazolo[3,4-b]pyridine-4-carboxylic acid (pyridin-2-ylmethyl)amide;
6-(4-Amino-3-cyanophenyl)-1H-pyrazolo[3,4-b]pyridine-4-carboxylic acid pyridin-2-ylamide;
6-(4-Amino-3-cyanophenyl)-1H-pyrazolo[3,4-b]pyridine-4-carboxylic acid pyridin-3-ylamide;
6-(4-Amino-3-cyanophenyl)-1H-pyrazolo[3,4-b]pyridine-4-carboxylic acid pyridin-4-ylamide;
5-(4-Difluoromethyl-2-methyl-2H-pyrazolo[3,4-b]pyridin-6-yl)-2-fluorobenzamide;
5-(4-Difluoromethyl-1-methyl-3-pyridin-4-yl-1H-pyrazolo[3,4-b]pyridin-6-yl)-2-fluorobenzonitrile;
2-Amino-5-(2-methyl-3-phenyl-4-trifluoromethyl-2H-pyrazolo[3,4-b]pyridin-6-yl)benzonitrile;
6-(1H-Benzimidazol-5-yl)-1H-pyrazolo[3,4-b]pyridine-4-carboxylic acid phenylamide;
6-(1H-Benzimidazol-5-yl)-1H-pyrazolo[3,4-b]pyridine-4-carboxylic acid pyridin-2-ylamide;
6-(1H-Benzimidazol-5-yl)-1H-pyrazolo[3,4-b]pyridine-4-carboxylic acid (pyridin-3-ylmethyl)amide;
6-(2-Oxo-2,3-dihydro-1H-benzimidazol-5-yl)-1H-pyrazolo[3,4-b]pyridine-4-carboxylic acid phenylamide;
2-Amino-5-(4-difluoromethyl-1-methyl-1H-pyrazolo[3,4-b]pyridin-6-yl)benzonitrile;
3-(4-Difluoromethyl-1-methyl-1H-pyrazolo[3,4-b]pyridin-6-yl)benzonitrile;
4-(4-Difluoromethyl-1-methyl-1H-pyrazolo[3,4-b]pyridin-6-yl)phenylamine;
2-Amino-5-[2-(2-dimethylaminoethyl)-3-phenyl-4-trifluoromethyl-2H-pyrazolo[3,4-b]pyridin-6-yl]benzonitrile;
2-Amino-5-(4-difluoromethyl-1H-pyrazolo[3,4-b]pyridin-6-yl)benzonitrile;
2-Amino-5-[1-(2-dimethylaminoethyl)-3-phenyl-4-trifluoromethyl-1H-pyrazolo[3,4-b]pyridin-6-yl]benzonitrile;
2-Amino-5-[2-(2-morpholin-4-ylethyl)-3-phenyl-4-trifluoromethyl-2H-pyrazolo[3,4-b]pyridin-6-yl]benzonitrile;
2-Methoxy-5-(1-methyl-3-phenyl-4-trifluoromethyl-1H-pyrazolo[3,4-b]pyridin-6-yl)nicotinonitrile;
2-Amino-5-(4-difluoromethyl-1-methyl-3-phenyl-1H-pyrazolo[3,4-b]pyridin-6-yl)benzonitrile;
4-(4-Difluoromethyl-1-methyl-3-phenyl-1H-pyrazolo[3,4-b]pyridin-6-yl)phenylamine;
[3-(4-Difluoromethyl-1-methyl-3-phenyl-1H-pyrazolo[3,4-b]pyridin-6-yl)phenyl]dimethylamine;
2-Amino-5-[3-phenyl-1-(2-piperidin-1-ylethyl)-4-trifluoromethyl-1H-pyrazolo[3,4-b]pyridin-6-yl]benzonitrile;
Dimethyl{3-[3-phenyl-1-(2-piperidin-1-ylethyl)-4-trifluoromethyl-1H-pyrazolo[3,4-b]pyridin-6-yl]phenyl}amine;
2-Amino-5-[4-difluoromethyl-2-(2-dimethylaminoethyl)-2H-pyrazolo[3,4-b]pyridin-6-yl]benzonitrile;
2-Amino-5-[4-difluoromethyl-2-(2-morpholin-4-ylethyl)-2H-pyrazolo[3,4-b]pyridin-6-yl]benzonitrile;
2-Amino-5-[4-difluoromethyl-1-(2-dimethylaminoethyl)-3-phenyl-1H-pyrazolo[3,4-b]pyridin-6-yl]benzonitrile;
2-(2-Morpholin-4-ylethyl)-6-(3-morpholin-4-ylmethylphenyl)-4-trifluoromethyl-2H-pyrazolo[3,4-b]pyridine;
Dimethyl{3-[1-(2-morpholin-4-ylethyl)-4-trifluoromethyl-1H-pyrazolo[3,4-b]pyridin-6-yl]phenyl}amine;
5-[1-(2-Morpholin-4-ylethyl)-4-trifluoromethyl-1H-pyrazolo[3,4-b]pyridin-6-yl]nicotinonitrile;
5-[1-(2-Morpholin-4-ylethyl)-4-trifluoromethyl-1H-pyrazolo[3,4-b]pyridin-6-yl]nicotinamide;
2-Amino-5-(2-methyl-4-trifluoromethyl-2H-pyrazolo[3,4-b]pyridin-6-yl)benzonitrile;
1-Methyl-6-(3-morpholin-4-ylmethylphenyl)-4-trifluoromethyl-1H-pyrazolo[3,4-b]pyridine;
2-Amino-5-(1-methyl-4-trifluoromethyl-1H-pyrazolo[3,4-b]pyridin-6-yl)benzonitrife;
Dimethyl[3-(1-methyl-4-trifluoromethyl-1H-pyrazolo[3,4-b]pyridin-6-yl)phenyl]amine;
Dimethyl[3-(3-phenyl-2-propyl-4-trifluoromethyl-2H-pyrazolo[3,4-b]pyridin-6-yl)phenyl]amine;
2-Amino-5-[4-difluoromethyl-2-(2-piperidin-1-ylethyl)-2H-pyrazolo[3,4-b]pyridin-6-yl]benzonitrile;
2-Amino-5-(4-difluoromethyl-3-pyridin-3-yl-1H-pyrazolo[3,4-b]pyridin-6-yl)benzonitrile;
2-Amino-5-(4-difluoromethyl-2-propyl-2H-pyrazolo[3,4-b]pyridin-6-yl)benzonitrile;
2-Anrtino-5-(4-difluoromethyl-3-pyridin-4-yl-1H-pyrazolo[3,4-b]pyridin-6-yl)benzonitrile;
1-Methyl-6-(3-morpholin-4-ylmethylphenyl)-3-pyridin-3-yl-4-trifluoromethyl-1H-pyrazolo[3,4-b]pyridine;
2-Amino-5-[4-difluoromethyl-3-(3-methoxyphenyl)-1H-pyrazolo[3,4-b]pyridin-6-yl]benzonitrile;
2-Amino-5-(2-propyl-4-trifluoromethyl-2H-pyrazolo[3,4-b]pyridin-6-yl)benzonitrile;
6-(3-Morpholin-4-ylmethylphenyl)-2-propyl-4-trifluoromethyl-2H-pyrazolo[3,4-b]pyridine;
Dimethyl[3-(2-propyl-4-trifluoromethyl-2H-pyrazolo[3,4-blpyridin-6-yl)phenyl]amine;
6-(3-Morpholin-4-ylmethylphenyl)-1-propyl-4-trifluoromethyl-1H-pyrazolo[3,4-b]pyridine;
6-(4-Methoxyphenyl)-1-propyl-4-trifluoromethyl-1H-pyrazolo[3,4-b]pyridine;
5-[3-(3-Methoxyphenyl)-1-methyl-4-trifluoromethyl-1H-pyrazolo[3,4-b]pyridin-6-yl]nicotinonitrile;
3-(3-Methoxyphenyl)-1-methyl-6-(3-morpholin-4-ylmethylphenyl)-4-trifluoromethyl-1H-pyrazolo[3,4-b]pyridine;
{3-{3-(3-Methoxyphenyl)-1-methyl-4-triftuoromethyl-1H-pyrazolo[3,4-b]pyridin-6-yl]phenyl}dimethylamine;
3-(3-Methoxyphenyl)-6-(4-methoxyphenyl)-1-methyl-4-trifluoromethyl-1H-pyrazolo[3,4-b]pyridine.

7. Process for preparing the compounds of formula (I) according to Claim 1, according to scheme 5 as following 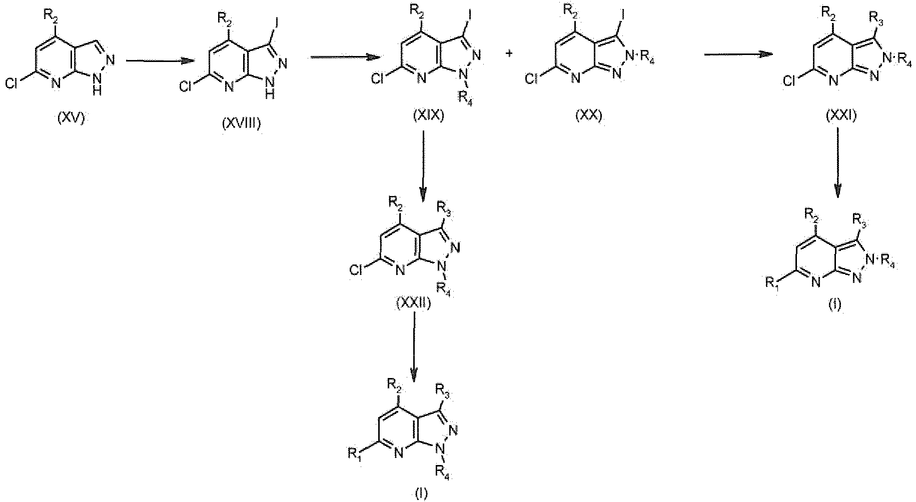 in which R₂ represents a group -CHF₂ or -CF₃, and R₁, R₃ and R₄ are as defined previously, with the exception of R₃ and R₄ representing a hydrogen atom, **characterized in that**:
- the compound of formula (XV) is subjected to an iodination reaction in the presence of N-iodosuccinimide in order to obtain the compound of formula (XVIII),
- the compound of formula (XVIII) is then subjected to an alkylation reaction in the presence of a halogenated derivative of formula R₄-X in order to obtain the compounds of formulae (XIX) and (XX),
- the compounds of formulae (XIX) and (XX) are separately subjected, in the presence of a palladium catalyst, a ligand and a base, to a reaction with phenylboronic or heteroarylboronic derivatives or phenylboronate esters or heteroarylboronate esters according to a Suzuki coupling, in order to obtain the compounds of formulae (XXI) and (XXII),
- the compounds of formulae (XXI) and (XXII) are separately subjected, in the presence of a palladium catalyst, a ligand and a base, to a reaction with phenylboronic or heteroarylboronic derivatives or phenylboronate esters or heteroarylboronate esters according to a Suzuki coupling, in order to obtain the compound of formula (I) in which R₂ represents a group -CHF2 or -CF₃ and R₁, R₃ and R₄ are as defined previously.

8. Process for preparing the compounds of formula (I) according to Claim 1, according to scheme 6 as following 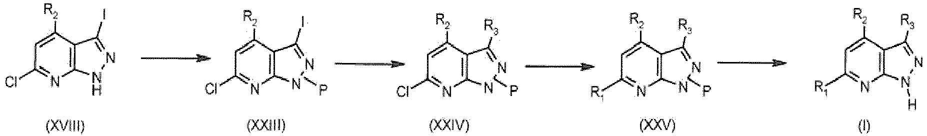 in which R₂ represents a group -CHF₂ or -CF₃, and R₁ and R₃ are as defined previously, with the exception of R₃ representing a hydrogen atom, **characterized in that**:
- the compound of formula (XVIII) is subjected to an alkylation reaction in the presence of a protecting group P in order to obtain the compound of formula (XXIII),
- the compound of formula (XXIII) is subjected to a reaction with phenylboronic or heteroarylboronic derivatives or phenylboronate esters or heteroarylboronate esters according to a Suzuki coupling, in order to obtain the compound of formula (XXIV),
- the compound of formula (XXIV) is subjected to a reaction with phenylboronic or heteroarylboronic derivatives or phenylboronate esters or heteroarylboronate esters according to a Suzuki coupling, in order to obtain the compound of formula (XXV),
- the compound of formula (XXV) is subjected to a deprotection reaction in order to obtain the compound of formula (I) in which R₂ represents a group -CHF₂ or -CF₃.

9. Compound of formula (I) 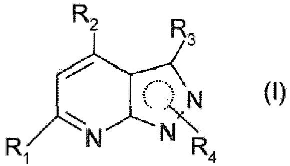 in which:
the representation of the pyrazole ring indicates that the substituent R₄ may be borne either by the nitrogen alpha to the pyridine ring (I') or by the nitrogen alpha to the carbon bearing a substituent R₃ (I") such that: 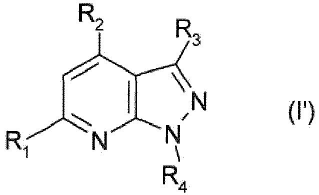 Either 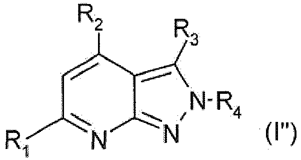
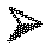 R₁ represents an aryl, pyridyl or pyrazolyl group optionally substituted with one or more substituents chosen from:
∘ a halogen atom,
∘ a group -CF3,
∘ a cyano group,
∘ a group -NR₆R₆' in which R₆ and R₆' are as defined below,
∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
∘ a group -COR₁₂ in which R₁₂ represents a hydroxyl group or a group -NR₆R₆', in which R₆ and R₆' are as defined below,
∘ a group -CONR₇R₇' such that R₇ and R₇' form, together with the nitrogen atom to which they are attached, a heterocycloalkyl comprising one or more heteroatoms chosen from a nitrogen atom and an oxygen atom,
∘ a group -(CH₂)ₚNHSO₂CH₃ in which p represents 0 or 1,
∘ a group -OR₁₃ in which R₁₃ represents a linear group (C₁-C₃)alkyl,
∘ a group (C₁-C₃)alkyl,
Or R₁ represents a bicyclic group of formula A below: 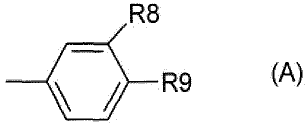 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
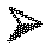 **R₂** represents a group:
∘ -CF₃,
∘ -CHF₂,
∘ -COOH,
or
∘ -CONHR₅, in which R₅ is as defined below,
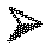 **R₃** represents:
∘ a hydrogen atom,
∘ an aryl group, optionally substituted with an alkoxymethyl group,
∘ a cycloalkyl group,
or
∘ a heteroaryl group chosen from thienyl and pyridyl groups,
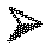 **R₄** represents:
∘ a hydrogen atom,
∘ a linear group (C₁-C₃)alkyl, optionally substituted with a group -NR₆R₆' in which R₆ and R'₆ are as defined below or a group -NR₇R_{7'}' such that R₇ and R_{7'} form, together with the nitrogen atom to which they are attached, a heterocycloalkyl comprising one or more heteroatoms chosen from a nitrogen atom and an oxygen atom,
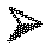 **R₅** represents:
∘ a hydrogen atom,
∘ a linear group (C₁-C₃)alkyl, optionally substituted with a pyridyl group,
or
∘ an aromatic group chosen from aryl and pyridyl,
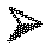 **R₆** and **R'₆**, which may be identical or different, represent a hydrogen atom or a linear alkyl group,
in the form of the base or of an acid-addition or base-addition salt, for use as a medicament.

10. Compound according to the preceding claim wherein said compound is selected form the following compounds : 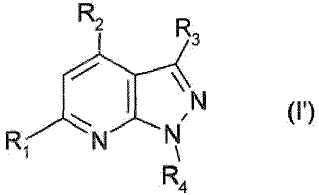
| **No.** | **R₂** | **R₃** | **R₁** | **R₄** | **Salt** | **m.p. (°C)** | **M+H⁺** |
|---|---|---|---|---|---|---|---|
| **1** | CF₃ | Ph | 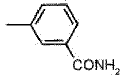 | Me | / | / | 397 |
| **2** | CF₃ | Ph | 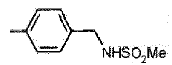 | Me | / | / | 461 |
| **3** | CF₃ | Ph | 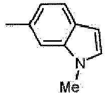 | Me | / | / | 407 |
| **4** | CF₃ | Ph | 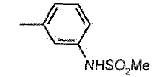 | Me | / | / | 447 |
| **5** | CF₃ | Ph | 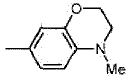 | Me | / | / | 426 |
| **6** | CF₃ | Ph | 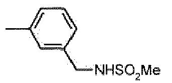 | Me | / | / | 461 |
| **7** | CF₃ | Ph | 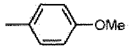 | Me | / | / | 384 |
| **8** | CF₃ | Ph | 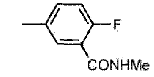 | Me | / | / | 429 |
| **9** | CF₃ | Ph | 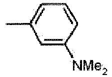 | Me | / | / | 397 |
| **10** | CF₃ | Ph | 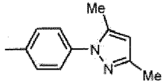 | Me | / | / | 448 |
| **11** | CF₃ | Ph | 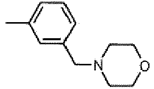 | Me | / | / | 453 |
| **12** | CF₃ | Ph | 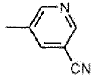 | Me | / | / | 380 |
| **13** | CF₃ | Ph | 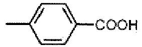 | Me | / | / | 398 |
| **14** | CF₃ | Ph | 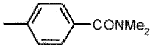 | Me | / | / | 425 |
| **15** | CF₃ | Ph | 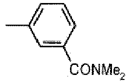 | Me | / | / | 425 |
| **16** | CF₃ | Ph | 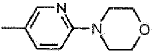 | Me | / | / | 440 |
| **17** | CF₃ | Ph | 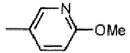 | Me | / | / | 385 |
| **18** | CF₃ | Ph | 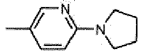 | Me | / | / | 424 |
| **19** | CF₃ | Ph | 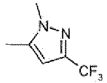 | Me | / | / | 426 |
| **20** | CF₃ | Ph | 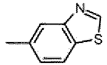 | Me | / | / | 411 |
| **21** | CF₃ | Ph | 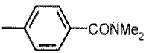 | H | HCl | / | 411 |
| **22** | CF₃ | Ph | 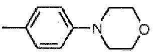 | H | HCl | / | 425 |
| **23** | CF₃ | Ph | 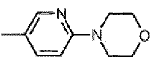 | H | HCl | / | 426 |
| **24** | CF₃ | Ph | 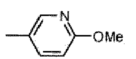 | H | HCl | / | 371 |
| **25** | CF₃ | Ph | 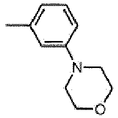 | H | HCl | / | 425 |
| **26** | CF₃ | Ph | 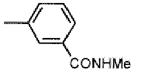 | H | HCl | / | 397 |
| **27** | CF₃ | Ph | 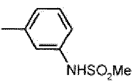 | H | HCl | / | 433 |
| **28** | CF₃ | Ph | 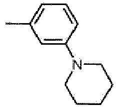 | H | HCl | / | 423 |
| **29** | CF₃ | Ph | 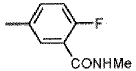 | H | HCl | / | 415 |
| **30** | CF₃ | Ph | 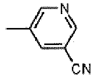 | H | HCl | / | 366 |
| **31** | CHF₂ | Ph | 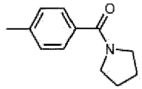 | H | HCl | 248 | 455 |
| **32** | CHF₂ | Ph | 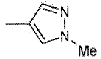 | H | HCl | / | 362 |
| **33** | CHF₂ | Ph | 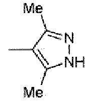 | H | HCl | / | 376 |
| **34** | CHF₂ | Ph | 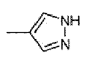 | H | HCl | / | 348 |
| **35** | CHF₂ | Ph | 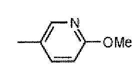 | H | HCl | / | 389 |
| **36** | CHF₂ | Ph | 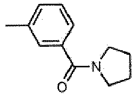 | H | HCl | / | 455 |
| **37** | CHF₂ | Ph | 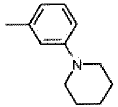 | H | HCl | / | 441 |
| **38 Ex. 2** | COOH | H | 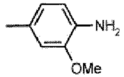 | H | / | / | 285 |
| **39** | CONHMe | Ph | 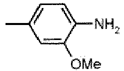 | H | TFA | / | 458 |
| **40** | CONH₂ | Ph | 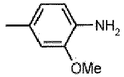 | H | TFA | / | 474 |
| **41** | CONHMe | H | 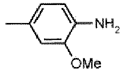 | H | TFA | / | 412 |
| **42** | CONH₂ | H | 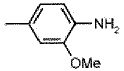 | H | TFA | / | 398 |
| **43** | COOH | Ph | 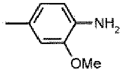 | H | / | / | 361 |
| **44** | COOH | Ph | 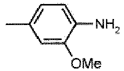 | Me | / | / | 375 |
| **45** | COOH | H | 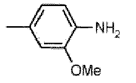 | Me | / | / | 299 |
| **46 Ex. 1** | CONH₂ | Ph | 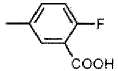 | H | TFA | / | 377 |
| **47** | CONH₂ | Ph | 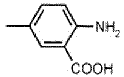 | H | / | / | 374 |
| **48** | CONH₂ | Ph | 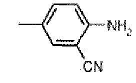 | H | TFA | / | 355 |
| **49** | CONH₂ | 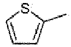 | 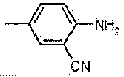 | H | / | / | 361 |
| **50** | COOH | cPr | 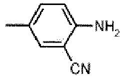 | H | / | / | 320 |
| **51** | CONH₂ | H | 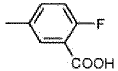 | H | / | / | 301 |
| **52** | CONH₂ | Ph | 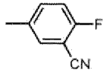 | H | / | / | 358 |
| **53 Ex. 3** | CHF₂ | Ph | 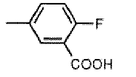 | H | / | / | 384 |
| **54** | CF₃ | Ph | 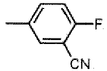 | H | / | / | 383 |
| **55** | CHF₂ | Ph | 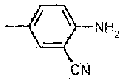 | H | HCl | 282 | 362 |
| **56 Ex. 4** | CF₃ | Ph | 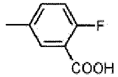 | H | / | / | 402 |
| **57 Ex. 6** | CF₃ | Ph | 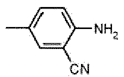 | Me | / | 269 | 394 |
| **58** | CF₃ | Ph | 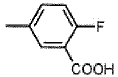 | Me | / | / | 416 |
| **59** | CF₃ | Ph | 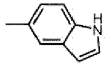 | H | / | / | 379 |
| **60** | CF₃ | Ph | 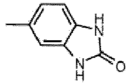 | H | / | 380 | 396 |
| **61 Ex. 5** | CF₃ | Ph | 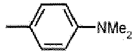 | H | / | 227 | 383 |
| **62** | CONHPh | H | 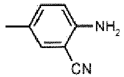 | H | HCl | / | 355 |
| **63** | 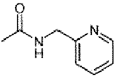 | H | 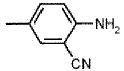 | H | HCl | / | 370 |
| **64** | 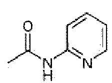 | H | 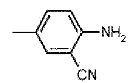 | H | HCl | / | 356 |
| **65** | 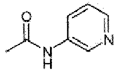 | H | 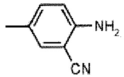 | H | HCl | / | 356 |
| **66** | 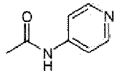 | H | 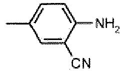 | H | HCl | / | 356 |
| **67** | CHF₂ | 4-Py | 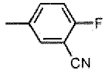 | Me | / | / | 380 |
| **68** | CONHPh | H | 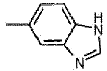 | H | HCl | / | 355 |
| **69** | 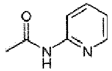 | H | 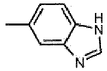 | H | HCl | / | 356 |
| **70** | 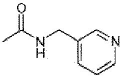 | H | 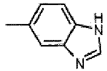 | H | HCl | / | 370 |
| **71** | CONHPh | H | 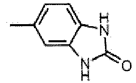 | H | / | / | 371 |
| **72 Ex. 10** | CHF₂ | H | 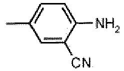 | Me | / | 251 | 300 |
| **73** | CHF₂ | H | 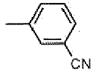 | Me | / | 162 | 285 |
| **74** | CHF₂ | H | 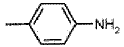 | Me | / | 149 | 275 |
| **75 Ex. 12** | CHF₂ | H | 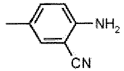 | H | / | 263 | 286 |
| **76** | CF₃ | Ph | 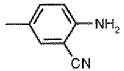 | 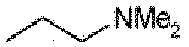 | / | 183 | 451 |
| **77** | CF₃ | Ph | 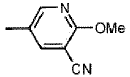 | Me | / | 250 | 410 |
| **78** | CHF₂ | Ph | 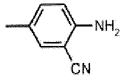 | Me | / | 246 | 376 |
| **79** | CHF₂ | Ph | 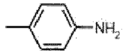 | Me | / | 176 | 351 |
| **80** | CHF₂ | Ph | 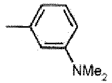 | Me | / | 154 | 379 |
| **81** | CF₃ | Ph | 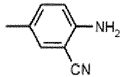 | 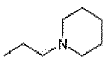 | / | 192 | 491 |
| **82** | CF₃ | Ph | 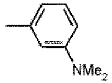 | 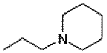 | HCl | 227 | 494 |
| **83 Ex. 13** | CHF₂ | Ph | 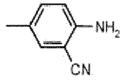 | 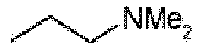 | / | 163 | 433 |
| **84** | CF₃ | H | 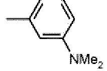 | 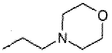 | / | 110 | 420 |
| **85** | CF₃ | H | 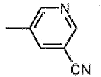 | 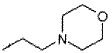 | / | / | 403 |
| **86** | CF₃ | H | 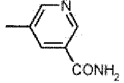 | 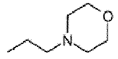 | / | 238 | 421 |
| **87** | CF₃ | H | 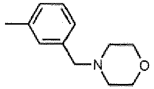 | Me | / | 105 | 377 |
| **88 Ex. 8** | CF₃ | H | 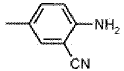 | Me | / | 276 | 318 |
| **89** | CF₃ | Ph | 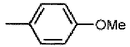 | Me | / | 181 | 384 |
| **90** | CF₃ | H | 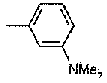 | Me | / | 91 | 321 |
| **91 Ex. 15** | CHF₂ | 3-Py | 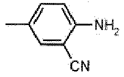 | H | / | 296 | 363 |
| **92** | CHF₂ | 4-Py | 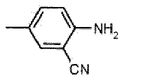 | H | / | 325 | 363 |
| **93 Ex. 14** | CF₃ | 3-Py | 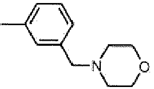 | Me | / | 155 | 454 |
| **94** | CHF₂ | 3MeO-Ph | 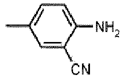 | Me | / | 233 | 392 |
| **95** | CF₃ | H | 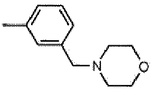 | Pr | HCl | 271 | 405 |
| **96** | CF₃ | H | 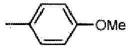 | Pr | / | 72 | 336 |
| **97** | CF₃ | 3MeO-Ph | 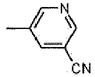 | Me | / | 194 | 410 |
| **98** | CF₃ | 3MeO-Ph | 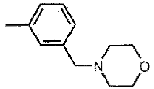 | Me | / | 114 | 483 |
| **99** | CF₃ | 3MeO-Ph | 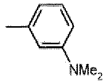 | Me | / | 138 | 427 |
| **100** | CF₃ | 3MeO-Ph | 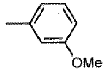 | Me | / | 133 | 414 |
| **101** | CONH₂ | 3-Py | 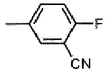 | H | / | / | 377 |

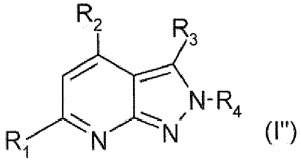
| **No.** | **R₂** | **R₃** | **R₁** | **R₄** | **Salt** | **m.p. (°C)** | **M+H⁺** |
|---|---|---|---|---|---|---|---|
| **102** | COOH | H | 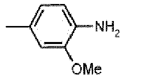 | Me | / | / | 299 |
| **103** | CONH₂ | H | 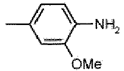 | Me | TFA | / | 412 |
| **104** | COOH | H | 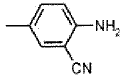 | Me | / | / | 294 |
| **105** | CHF₂ | H | 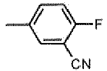 | Me | / | / | 303 |
| **106 Ex. 11** | CHF₂ | H | 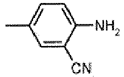 | Me | / | / | 300 |
| **107** | CHF₂ | H | 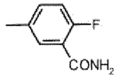 | Me | / | / | 321 |
| **108 Ex. 7** | CF₃ | Ph | 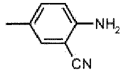 | Me | / | 295 | 394 |
| **109** | CF₃ | Ph | 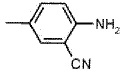 | 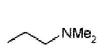 | / | 237 | 451 |
| **110** | CF₃ | Ph | 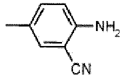 | 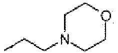 | / | 249 | 493 |
| **111** | CHF₂ | H | 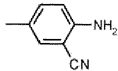 | 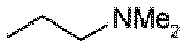 | / | 182 | 357 |
| **112** | CHF₂ | H | 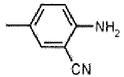 | 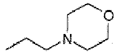 | / | 242 | 399 |
| **113** | CF₃ | H | 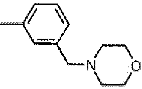 | 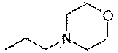 | / | 101 | 476 |
| **114 Ex. 9** | CF₃ | H | 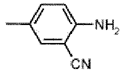 | Me | / | 249 | 318 |
| **115** | CF₃ | H | 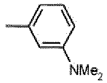 | Pr | HCl | 181 | 425 |
| **116** | CHF₂ | H | 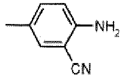 | 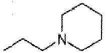 | / | 230 | 397 |
| **117** | CHF₂ | H | 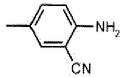 | Pr | / | 214 | 328 |
| **118** | CF₃ | H | 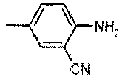 | Pr | / | 239 | 346 |
| **119** | CF₃ | H | 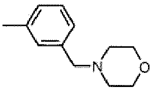 | Pr | / | 288 | 405 |
| **120** | CF₃ | H | 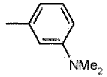 | Pr | / | 89 | 349 |
for use as a medicament.

11. Pharmaceutical composition, **characterized in that** it comprises a compound of formula (I) 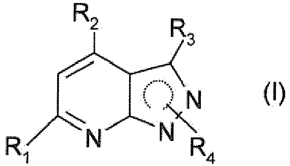 in which:
the representation of the pyrazole ring indicates that the substituent R₄ may be borne either by the nitrogen alpha to the pyridine ring (I') or by the nitrogen alpha to the carbon bearing a substituent R₃ (I") such that: 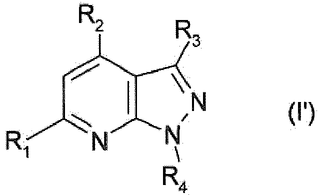 Either 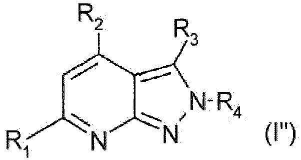
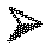 **R₁** represents an aryl, pyridyl or pyrazolyl group optionally substituted with one or more substituents chosen from:
∘ a halogen atom,
∘ a group -CF₃,
∘ a cyano group,
∘ a group -NR₆R₆' in which R₆ and R₆' are as defined below,
∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
∘ a group -COR₁₂ in which R₁₂ represents a hydroxyl group or a group -NR₆R₆', in which R₆ and R₆' are as defined below,
∘ a group -CONR₇R₇' such that R₇ and R₇' form, together with the nitrogen atom to which they are attached, a heterocycloalkyl comprising one or more heteroatoms chosen from a nitrogen atom and an oxygen atom,
∘ a group -(CH₂)ₚNHSO₂CH₃ in which p represents 0 or 1,
∘ a group -OR₁₃ in which R₁₃ represents a linear group (C₁-C₃)alkyl,
∘ a group (C₁-C₃)alkyl,
Or R₁ represents a bicyclic group of formula A below: 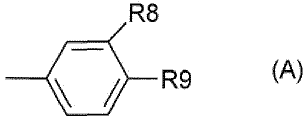 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
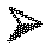 **R₂** represents a group:
∘ -CF₃,
∘ -CHF₂,
or
∘ -CONHR₅, in which R₅ is as defined below,
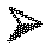 **R₃** represents:
∘ a hydrogen atom,
∘ an aryl group, optionally substituted with an alkoxymethyl group,
∘ a cycloalkyl group,
or
∘ a heteroaryl group chosen from thienyl and pyridyl groups,
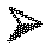 **R₄** represents:
∘ a hydrogen atom,
∘ a linear group (C₁-C₃)alkyl, optionally substituted with a group -NR₆R₆' in which R₆ and R'₆ are as defined below or a group -NR₇R_{7'}' such that R₇ and R_{7'} form, together with the nitrogen atom to which they are attached, a heterocycloalkyl comprising one or more heteroatoms chosen from a nitrogen atom and an oxygen atom,
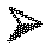 **R₅** represents:
∘ a hydrogen atom,
∘ a linear group (C₁-C₃)alkyl, optionally substituted with a pyridyl group,
or
∘ an aromatic group chosen from aryl and pyridyl,
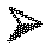 **R₆** and **R'₆,** which may be identical or different, represent a hydrogen atom or a linear alkyl group,
in the form of the base or of an acid-addition or base-addition salt
and also at least one pharmaceutically acceptable excipient.

12. Pharmaceutical composition according to the preceding claim, **characterized in that** it comprises a compound selected form the following compounds 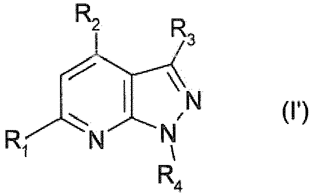
| **No.** | **R₂** | **R₃** | **R₁** | **R₄** | **Salt** | **m.p. (°C)** | **M+H⁺** |
|---|---|---|---|---|---|---|---|
| **1** | CF₃ | Ph | 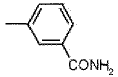 | Me | / | / | 397 |
| **2** | CF₃ | Ph | 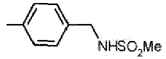 | Me | / | / | 461 |
| **3** | CF₃ | Ph | 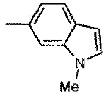 | Me | / | / | 407 |
| **4** | CF₃ | Ph | 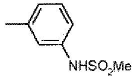 | Me | / | / | 447 |
| **5** | CF₃ | Ph | 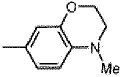 | Me | / | / | 426 |
| **6** | CF₃ | Ph | 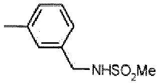 | Me | / | / | 461 |
| **7** | CF₃ | Ph | 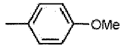 | Me | / | / | 384 |
| **8** | CF₃ | Ph | 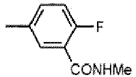 | Me | / | / | 429 |
| **9** | CF₃ | Ph | 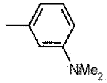 | Me | / | / | 397 |
| **10** | CF₃ | Ph | 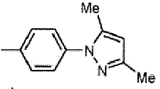 | Me | / | / | 448 |
| **11** | CF₃ | Ph | 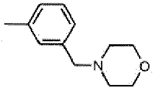 | Me | / | / | 453 |
| **12** | CF₃ | Ph | 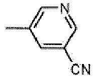 | Me | / | / | 380 |
| **13** | CF₃ | Ph | 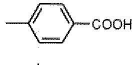 | Me | / | / | 398 |
| **14** | CF₃ | Ph | 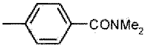 | Me | / | / | 425 |
| **15** | CF₃ | Ph | 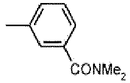 | Me | / | / | 425 |
| **16** | CF₃ | Ph | 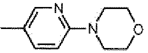 | Me | / | / | 440 |
| **17** | CF₃ | Ph | 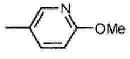 | Me | / | / | 385 |
| **18** | CF₃ | Ph | 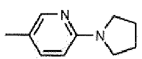 | Me | / | / | 424 |
| **19** | CF₃ | Ph | 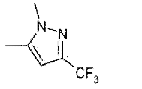 | Me | / | / | 426 |
| **20** | CF₃ | Ph | 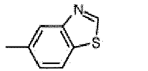 | Me | / | / | 411 |
| **21** | CF₃ | Ph | 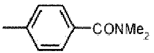 | H | HCl | / | 411 |
| **22** | CF₃ | Ph | 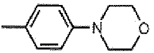 | H | HCl | / | 425 |
| **23** | CF₃ | Ph | 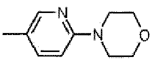 | H | HCl | / | 426 |
| **24** | CF₃ | Ph | 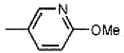 | H | HCl | / | 371 |
| **25** | CF₃ | Ph | 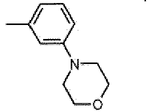 | H | HCl | / | 425 |
| **26** | CF₃ | Ph | 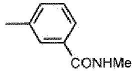 | H | HCl | / | 397 |
| **27** | CF₃ | Ph | 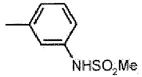 | H | HCl | / | 433 |
| **28** | CF₃ | Ph | 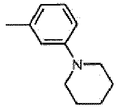 | H | HCl | / | 423 |
| **29** | CF₃ | Ph | 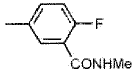 | H | HCl | / | 415 |
| **30** | CF₃ | Ph | 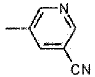 | H | HCl | / | 366 |
| **31** | CHF₂ | Ph | 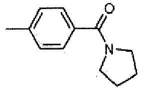 | H | HCl | 248 | 455 |
| **32** | CHF₂ | Ph | 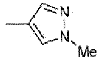 | H | HCl | / | 362 |
| **33** | CHF₂ | Ph | 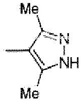 | H | HCl | / | 376 |
| **34** | CHF₂ | Ph | 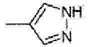 | H | HCl | / | 348 |
| **35** | CHF₂ | Ph | 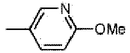 | H | HCl | / | 389 |
| **36** | CHF₂ | Ph | 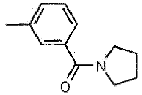 | H | HCl | / | 455 |
| **37** | CHF₂ | Ph | 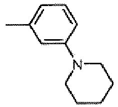 | H | HCl | 1 | 441 |
| **39** | CONHMe | Ph | 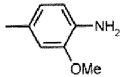 | H | TFA | / | 458 |
| **40** | CONH₂ | Ph | 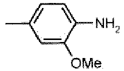 | H | TFA | / | 474 |
| **41** | CONHMe | H | 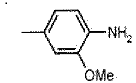 | H | TFA | / | 412 |
| **42** | CONH₂ | H | 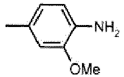 | H | TFA | / | 398 |
| **46 Ex. 1** | CONH₂ | Ph | 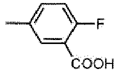 | H | TFA | / | 377 |
| **47** | CONH₂ | Ph | 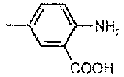 | H | / | / | 374 |
| **48** | CONH₂ | Ph | 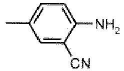 | H | TFA | / | 355 |
| **49** | CONH₂ | 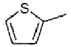 | 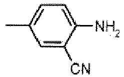 | H | / | / | 361 |
| **51** | CONH₂ | H | 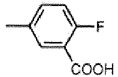 | H | / | / | 301 |
| **52** | CONH₂ | Ph | 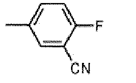 | H | / | / | 358 |
| **53 Ex. 3** | CHF₂ | Ph | 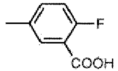 | H | / | / | 384 |
| **54** | CF₃ | Ph | 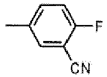 | H | / | / | 383 |
| **55** | CHF₂ | Ph | 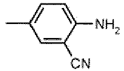 | H | HCl | 282 | 362 |
| **56 Ex. 4** | CF₃ | Ph | 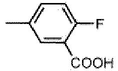 | H | / | / | 402 |
| **57 Ex. 6** | CF₃ | Ph | 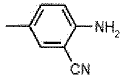 | Me | / | 269 | 394 |
| **58** | CF₃ | Ph | 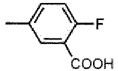 | Me | / | / | 416 |
| **59** | CF₃ | Ph | 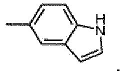 | H | / | / | 379 |
| **60** | CF₃ | Ph | 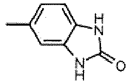 | H | / | 380 | 396 |
| **61 Ex. 5** | CF₃ | Ph | 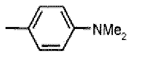 | H | / | 227 | 383 |
| **62** | CONHPh | H | 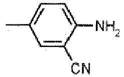 | H | HCl | / | 355 |
| **63** | 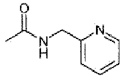 | H | 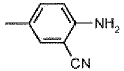 | H | HCl | / | 370 |
| **64** | 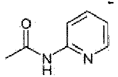 | H | 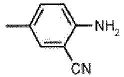 | H | HCl | / | 356 |
| **65** | 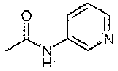 | H | 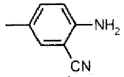 | H | HCl | / | 356 |
| **66** | 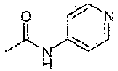 | H | 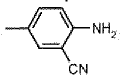 | H | HCl | / | 356 |
| **67** | CHF₂ | 4-Py | 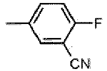 | Me | / | / | 380 |
| **68** | CONHPh | H | 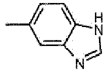 | H | HCl | / | 355 |
| **69** | 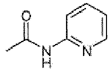 | H | 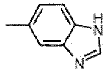 | H | HCl | 1 | 356 |
| **70** | 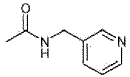 | H | 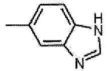 | H | HCl | / | 370 |
| **71** | CONHPh | H | 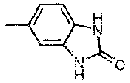 | H | / | / | 371 |
| **72 Ex. 10** | CHF₂ | H | 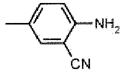 | Me | / | 251 | 300 |
| **73** | CHF₂ | H | 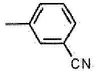 | Me | / | 162 | 285 |
| **74** | CHF₂ | H | 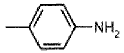 | Me | / | 149 | 275 |
| **75 Ex. 12** | CHF₂ | H | 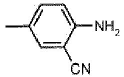 | H | / | 263 | 286 |
| **76** | CF₃ | Ph | 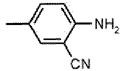 | 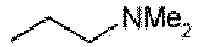 | / | 183 | 451 |
| **77** | CF₃ | Ph | 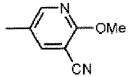 | Me | / | 250 | 410 |
| **78** | CHF₂ | Ph | 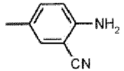 | Me | / | 246 | 376 |
| **79** | CHF₂ | Ph | 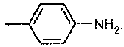 | Me | / | 176 | 351 |
| **80** | CHF₂ | Ph | 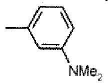 | Me | / | 154 | 379 |
| **81** | CF₃ | Ph | 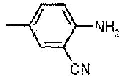 | 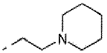 | / | 192 | 491 |
| **82** | CF₃ | Ph | 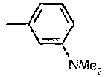 | 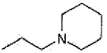 | HCl | 227 | 494 |
| **83 Ex. 13** | CHF₂ | Ph | 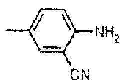 | 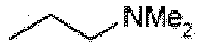 | / | 163 | 433 |
| **84** | CF₃ | H | 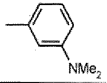 | 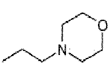 | / | 110 | 420 |
| **85** | CF₃ | H | 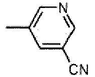 | 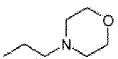 | / | / | 403 |
| **86** | CF₃ | H | 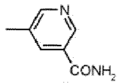 | 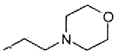 | / | 238 | 421 |
| **87** | CF₃ | H | 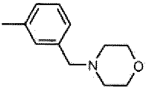 | Me | / | 105 | 377 |
| **88 Ex. 8** | CF₃ | H | 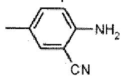 | Me | / | 276 | 318 |
| **89** | CF₃ | Ph | 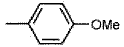 | Me | / | 181 | 384 |
| **90** | CF₃ | H | 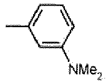 | Me | / | 91 | 321 |
| **91 Ex. 15** | CHF₂ | 3-Py | 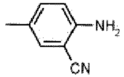 | H | / | 296 | 363 |
| **92** | CHF₂ | 4-Py | 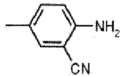 | H | / | 325 | 363 |
| **93 Ex. 14** | CF₃ | 3-Py | 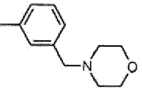 | Me | / | 155 | 454 |
| **94** | CHF₂ | 3MeO-Ph | 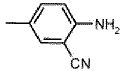 | Me | / | 233 | 392 |
| **95** | CF₃ | H | 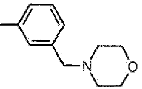 | Pr | HCl | 271 | 405 |
| **96** | CF₃ | H | 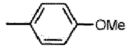 | Pr | / | 72 | 336 |
| **97** | CF₃ | 3MeO-Ph | 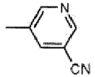 | Me | / | 194 | 410 |
| **98** | CF₃ | 3MeO-Ph | 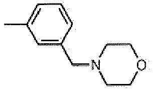 | Me | / | 114 | 483 |
| **99** | CF₃ | 3MeO-Ph | 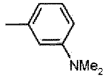 | Me | / | 138 | 427 |
| **100** | CF₃ | 3MeO-Ph | 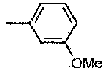 | Me | / | 133 | 414 |
| **101** | CONH₂ | 3-Py | 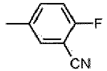 | H | / | / | 377 |

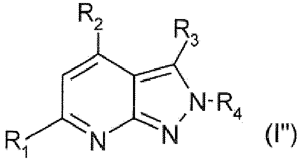
| **No.** | **R₂** | **R₃** | **R₁** | **R₄** | **Salt** | **m.p.(°C)** | **M+H⁺** |
|---|---|---|---|---|---|---|---|
| **103** | CONH₂ | H | 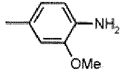 | Me | TFA | / | 412 |
| **105** | CHF₂ | H | 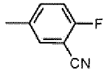 | Me | / | / | 303 |
| **106 Ex. 11** | CHF₂ | H | 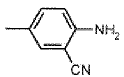 | Me | / | / | 300 |
| **107** | CHF₂ | H | 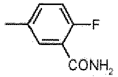 | Me | / | / | 321 |
| **108 Ex. 7** | CF₃ | Ph | 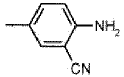 | Me | / | 295 | 394 |
| **109** | CF₃ | Ph | 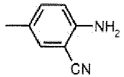 | 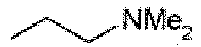 | / | 237 | 451 |
| **110** | CF₃ | Ph | 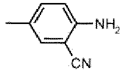 | 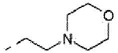 | / | 249 | 493 |
| **111** | CHF₂ | H | 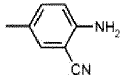 | 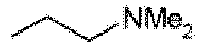 | / | 182 | 357 |
| **112** | CHF₂ | H | 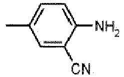 | 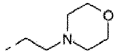 | / | 242 | 399 |
| **113** | CF₃ | H | 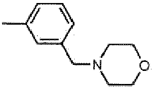 | 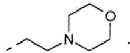 | / | 101 | 476 |
| **114 Ex.9** | CF₃ | H | 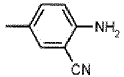 | Me | / | 249 | 318 |
| **115** | CF₃ | H | 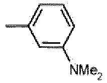 | Pr | HCl | 181 | 425 |
| **116** | CHF₂ | H | 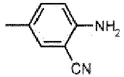 | 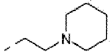 | / | 230 | 397 |
| **117** | CHF₂ | H | 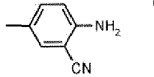 | Pr | / | 214 | 328 |
| **118** | CF₃ | H | 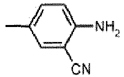 | Pr | / | 239 | 346 |
| **119** | CF₃ | H | 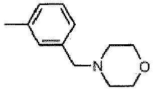 | Pr | / | 288 | 405 |
| **120** | CF₃ | H | 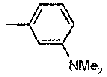 | Pr | / | 89 | 349 |
and also at least one pharmaceutically acceptable excipient.

13. Use of a compound of formula (I) 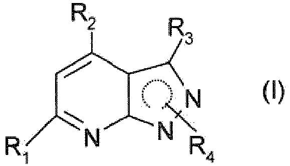 in which:
the representation of the pyrazole ring indicates that the substituent R₄ may be borne either by the nitrogen alpha to the pyridine ring (I') or by the nitrogen alpha to the carbon bearing a substituent R₃ (I") such that: 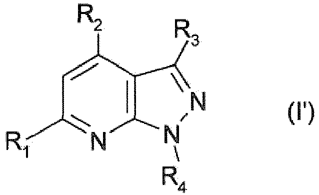 Either 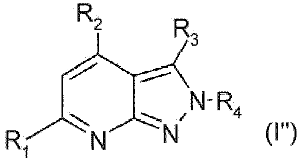
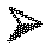 **R₁** represents an aryl, pyridyl or pyrazolyl group optionally substituted with one or more substituents chosen from:
∘ a halogen atom,
∘ a group -CF₃,
∘ a cyano group,
∘ a group -NR₆R₆' in which R₆ and R₆' are as defined below,
∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
∘ a group -COR₁₂ in which R₁₂ represents a hydroxyl group or a group -NR₆R₆', in which R₆ and R₆' are as defined below,
∘ a group -CONR₇R₇' such that R₇ and R₇' form, together with the nitrogen atom to which they are attached, a heterocycloalkyl comprising one or more heteroatoms chosen from a nitrogen atom and an oxygen atom,
∘ a group -(CH₂)ₚNHSO₂CH₃ in which p represents 0 or 1,
∘ a group -OR₁₃ in which R₁₃ represents a linear group (C₁-C₃)alkyl,
∘ a group (C₁-C₃)alkyl,
Or R₁ represents a bicyclic group of formula A below: 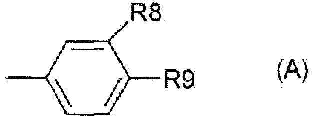 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
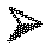 **R₂** represents a group:
∘ -CF₃,
∘ -CHF₂,
∘ -COOH,
or
∘ -CONHR₅, in which R₅ is as defined below,
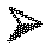 **R₃** represents:
∘ a hydrogen atom,
∘ an aryl group, optionally substituted with an alkoxymethyl group,
∘ a cycloalkyl group,
or
∘ a heteroaryl group chosen from thienyl and pyridyl groups,
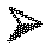 **R₄** represents:
∘ a hydrogen atom,
∘ a linear group (C₁-C₃)alkyl, optionally substituted with a group -NR₆R₆' in which R₆ and R'₆ are as defined below or a group -NR₇R₇' such that R₇ and R_{7'} form, together with the nitrogen atom to which they are attached, a heterocycloalkyl comprising one or more heteroatoms chosen from a nitrogen atom and an oxygen atom,
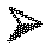 **R₅** represents:
∘ a hydrogen atom,
∘ a linear group (C₁-C₃)alkyl, optionally substituted with a pyridyl group, or
∘ an aromatic group chosen from aryl and pyridyl,
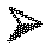 **R₆** and **R'₆**, which may be identical or different, represent a hydrogen atom or a linear alkyl group,
in the form of the base or of an acid-addition or base-addition salt
for the preparation of a medicament for treating and preventing diseases necessitating a modulation of the b-FGFs.

14. Combination of a compound 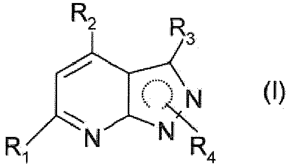 in which:
the representation of the pyrazole ring indicates that the substituent R₄ may be borne either by the nitrogen alpha to the pyridine ring (I') or by the nitrogen alpha to the carbon bearing a substituent R₃ (I") such that: 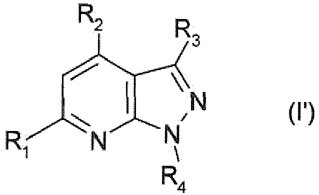 Either 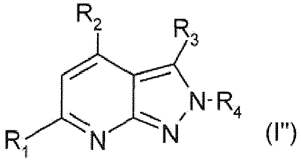
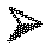 **R₁** represents an aryl, pyridyl or pyrazolyl group optionally substituted with one or more substituents chosen from:
∘ a halogen atom,
∘ a group -CF₃,
∘ a cyano group,
∘ a group -NR₆R₆' in which R₆ and R₆' are as defined below,
∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
∘ a group -COR₁₂ in which R₁₂ represents a hydroxyl group or a group -NR₆R₆', in which R₆ and R₆' are as defined below,
∘ a group -CONR₇R₇' such that R₇ and R₇' form, together with the nitrogen atom to which they are attached, a heterocycloalkyl comprising one or more heteroatoms chosen from a nitrogen atom and an oxygen atom,
∘ a group -(CH₂)ₚNHSO₂CH₃ in which p represents 0 or 1,
∘ a group -OR₁₃ in which R₁₃ represents a linear group (C₁-C₃)alkyl,
∘ a group (C₁-C₃)alkyl,
Or R₁ represents a bicyclic group of formula A below: 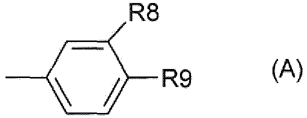 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
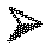 **R₂** represents a group:
∘ -CF₃,
∘ -CHF₂,
∘ -COOH,
or
∘ -CONHR₅, in which R₅ is as defined below,
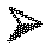 **R₃** represents:
∘ a hydrogen atom,
∘ an aryl group, optionally substituted with an alkoxymethyl group,
∘ a cycloalkyl group,
or
∘ a heteroaryl group chosen from thienyl and pyridyl groups,
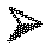 **R₄** represents:
∘ a hydrogen atom,
∘ a linear group (C₁-C₃)alkyl, optionally substituted with a group -NR₆R₆' in which R₆ and R'₆ are as defined below or a group -NR₇R_{7'}' such that R₇ and R_{7'} form, together with the nitrogen atom to which they are attached, a heterocycloalkyl comprising one or more heteroatoms chosen from a nitrogen atom and an oxygen atom,
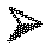 **R₅** represents:
∘ a hydrogen atom,
∘ a linear group (C₁-C₃)alkyl, optionally substituted with a pyridyl group,
or
∘ an aromatic group chosen from aryl and pyridyl,
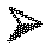 **R₆** and **R'₆**, which may be identical or different, represent a hydrogen atom or a linear alkyl group,
in the form of the base or of an acid-addition or base-addition salt
with one or more anticancer active principles and/or with any anti-VEGF.

15. Compound 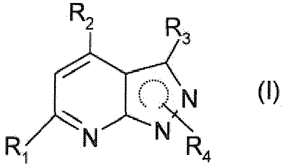 in which:
the representation of the pyrazole ring indicates that the substituent R₄ may be borne either by the nitrogen alpha to the pyridine ring (I') or by the nitrogen alpha to the carbon bearing a substituent R₃ (I") such that: 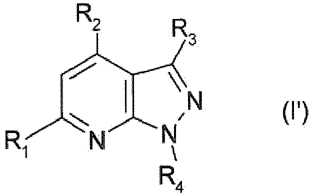 Either 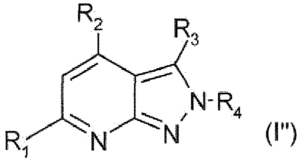
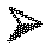 **R₁** represents an aryl, pyridyl or pyrazolyl group optionally substituted with one or more substituents chosen from:
∘ a halogen atom,
∘ a group -CF₃,
∘ a cyano group,
∘ a group -NR₆R₆' in which R₆ and R₆' are as defined below,
∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
∘ a group -COR₁₂ in which R₁₂ represents a hydroxyl group or a group -NR₆R₆', in which R₆ and R₆' are as defined below,
∘ a group -CONR₇R₇' such that R₇ and R₇' form, together with the nitrogen atom to which they are attached, a heterocycloalkyl comprising one or more heteroatoms chosen from a nitrogen atom and an oxygen atom,
∘ a group -(CH2)ₚNHSO₂CH₃ in which p represents 0 or 1,
∘ a group -OR₁₃ in which R₁₃ represents a linear group (C₁-C₃)alkyl,
∘ a group (C₁-C₃)alkyl,
Or R₁ represents a bicyclic group of formula A below: 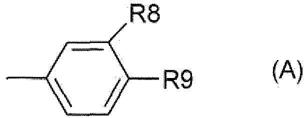 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
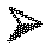 **R₂** represents a group:
∘ -CF₃,
∘ -CHF₂,
∘ -COOH,
or
∘ -CONHR₅, in which R₅ is as defined below,
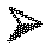 **R₃** represents:
∘ a hydrogen atom,
∘ an aryl group, optionally substituted with an alkoxymethyl group,
∘ a cycloalkyl group,
or
∘ a heteroaryl group chosen from thienyl and pyridyl groups,
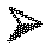 **R₄** represents:
∘ a hydrogen atom,
∘ a linear group (C₁-C₃)alkyl, optionally substituted with a group -NR₆R₆' in which R₆ and R'₆ are as defined below or a group -NR₇R_{7'}' such that R₇ and R_{7'} form, together with the nitrogen atom to which they are attached, a heterocycloalkyl comprising one or more heteroatoms chosen from a nitrogen atom and an oxygen atom,
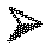 **R₅** represents:
∘ a hydrogen atom,
∘ a linear group (C₁-C₃)alkyl, optionally substituted with a pyridyl group, or
∘ an aromatic group chosen from aryl and pyridyl,
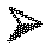 **R₆** and **R'₆**, which may be identical or different, represent a hydrogen atom or a linear alkyl group,
in the form of the base or of an acid-addition or base-addition salt,
for its use in the treatment and prevention of cancers, especially carcinomas with a substantial degree of vascularization such as lung, breast, prostate, pancreatic, bowel, kidney and oesophageal carcinomas, cancers that induce metastases, such as bowel cancer, liver cancer and stomach cancer, melanomas, gliomas, lymphomas and leukaemias, and also thrombopenias.

16. Compound 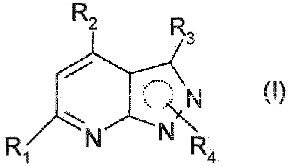 in which:
the representation of the pyrazole ring indicates that the substituent R₄ may be borne either by the nitrogen alpha to the pyridine ring (I') or by the nitrogen alpha to the carbon bearing a substituent R₃ (I") such that: 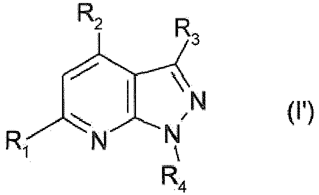 Either 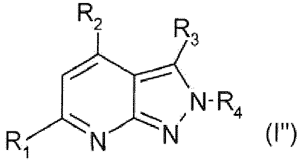
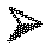 **R₁** represents an aryl, pyridyl or pyrazolyl group optionally substituted with one or more substituents chosen from:
∘ a halogen atom,
∘ a group -CF₃,
∘ a cyano group,
∘ a group -NR₆R₆' in which R₆ and R₆' are as defined below,
∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
∘ a group -COR₁₂ in which R₁₂ represents a hydroxyl group or a group -NR₆R₆', in which R₆ and R₆' are as defined below,
∘ a group -CONR₇R₇' such that R₇ and R₇' form, together with the nitrogen atom to which they are attached, a heterocycloalkyl comprising one or more heteroatoms chosen from a nitrogen atom and an oxygen atom,
∘ a group -(CH₂)ₚNHSO₂CH₃ in which p represents 0 or 1,
∘ a group -OR₁₃ in which R₁₃ represents a linear group (C₁-C₃)alkyl,
∘ a group (C₁-C₃)alkyl,
Or R₁ represents a bicyclic group of formula A below: 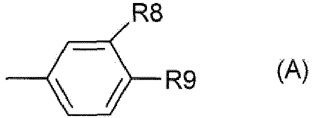 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
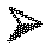 **R₂** represents a group:
∘ -CF₃,
∘ -CHF₂,
∘ -COOH,
or
∘ -CONHR₅, in which R₅ is as defined below,
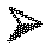 **R₃** represents:
∘ a hydrogen atom,
∘ an aryl group, optionally substituted with an alkoxymethyl group,
∘ a cycloalkyl group,
or
∘ a heteroaryl group chosen from thienyl and pyridyl groups,
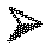 **R₄** represents:
∘ a hydrogen atom,
∘ a linear group (C₁-C₃)alkyl, optionally substituted with a group -NR₆R₆' in which R₆ and R'₆ are as defined below or a group -NR₇R_{7'}' such that R₇ and R_{7'} form, together with the nitrogen atom to which they are attached, a heterocycloalkyl comprising one or more heteroatoms chosen from a nitrogen atom and an oxygen atom,
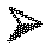 **R₅** represents:
∘ a hydrogen atom,
∘ a linear group (C₁-C₃)alkyl, optionally substituted with a pyridyl group, or
∘ an aromatic group chosen from aryl and pyridyl,
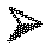 **R₆** and **R'₆**, which may be identical or different, represent a hydrogen atom or a linear alkyl group,
in the form of the base or of an acid-addition or base-addition salt
for its use in the treatment and prevention of cancers, especially carcinomas with a substantia! degree of vascularization such as lung, breast, prostate, pancreatic, bowel, kidney and oesophageal carcinomas, cancers that induce metastases, such as bowel cancer, liver cancer and stomach cancer, melanomas, gliomas, lymphomas and leukaemias, and also thrombopenias,
said compound of formula (I) being administered in combination with one or more anticancer active principles and/or with any anti-VEGF and/or being administered in combination with a radiotherapy.

17. Compound 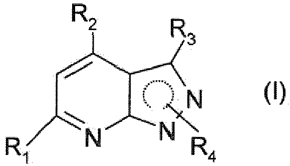 in which:
the representation of the pyrazole ring indicates that the substituent R₄ may be borne either by the nitrogen alpha to the pyridine ring (I') or by the nitrogen alpha to the carbon bearing a substituent R₃ (I") such that: 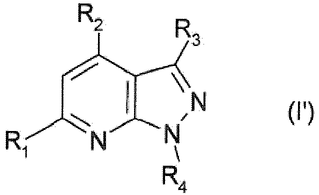 Either 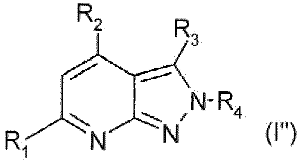
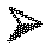 **R₁** represents an aryl, pyridyl or pyrazolyl group optionally substituted with one or more substituents chosen from:
∘ a halogen atom,
∘ a group -CF₃,
∘ a cyano group,
∘ a group -NR₆R₆' in which R₆ and R₆' are as defined below,
∘ a group -NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom, optionally substituted with one or more substituents chosen from a halogen atom and a linear or branched alkyl group,
∘ a group -CH₂NR₁₀R₁₁ such that R₁₀ and R₁₁ form, together with the nitrogen atom to which they are attached, a saturated or unsaturated heterocycle comprising one or more heteroatoms chosen from a nitrogen atom and an oxygen atom,
∘ a group -COR₁₂ in which R₁₂ represents a hydroxyl group or a group -NR₆R₆', in which R₆ and R₆' are as defined below,
∘ a group -CONR₇R₇' such that R₇ and R₇' form, together with the nitrogen atom to which they are attached, a heterocycloalkyl comprising one or more heteroatoms chosen from a nitrogen atom and an oxygen atom,
∘ a group -(CH₂)ₚNHSO₂CH₃ in which p represents 0 or 1,
∘ a group -OR₁₃ in which R₁₃ represents a linear group (C₁-C₃)alkyl,
∘ a group (C₁-C₃)alkyl,
Or R₁ represents a bicyclic group of formula A below: 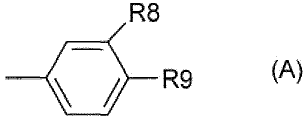 in which R₈ and R₉ form, together with the carbon atoms to which they are attached, a saturated or unsaturated heterocycle comprising one or more heteroatoms chosen from a nitrogen atom, an oxygen atom and a sulfur atom, optionally substituted with one or more linear alkyl groups,
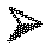 **R₂** represents a group:
∘ -CF₃,
∘ -CHF₂,
∘ -COOH,
or
∘ -CONHR₅, in which R₅ is as defined below,
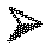 **R₃** represents:
∘ a hydrogen atom,
∘ an aryl group, optionally substituted with an alkoxymethyl group,
∘ a cycloalkyl group,
or
∘ a heteroaryl group chosen from thienyl and pyridyl groups,
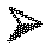 **R₄** represents:
∘ a hydrogen atom,
∘ a linear group (C₁-C₃)alkyl, optionally substituted with a group -NR₆R₆' in which R₆ and R'₆ are as defined below or a group -NR₇R_{7'}' such that R₇ and R_{7'} form, together with the nitrogen atom to which they are attached, a heterocycloalkyl comprising one or more heteroatoms chosen from a nitrogen atom and an oxygen atom,
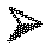 **R₅** represents:
∘ a hydrogen atom,
∘ a linear group (C₁-C₃)alkyl, optionally substituted with a pyridyl group, or
∘ an aromatic group chosen from aryl and pyridyl,
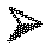 **R₆** and **R'₆,** which may be identical or different, represent a hydrogen atom or a linear alkyl group,
in the form of the base or of an acid-addition or base-addition salt
for its use in the treatment and prevention of cardiovascular diseases such as atherosclerosis, post-angioplasty restenosis, diseases associated with complications arising after the insertion of endovascular prostheses and/or aorto-coronary bypasses or other vascular grafts, cardiac hypotrophy, vascular complications of diabetes such as diabetic retinopathy, hepatic, renal and pulmonary fibroses, neuropathic pain, chronic inflammatory diseases such as rheumatoid arthritis or IBD, prostate hyperplasia, psoriasis, clear-cell acanthoma, osteoarthritis, achondroplasias (ACH), hypochondroplasias (HCH), TD (thanatophoric dysplasia), obesity, and macular degeneration, such as age-related macular degeneration (AMD).

18. Compound for its use according to anyone of claims 15 to 17 wherein said compound is selected form the following compounds : 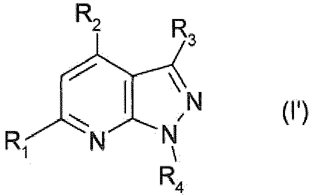
| **No.** | **R₂** | **R₃** | **R₁** | **R₄** | **Salt** | **m.p.(°C)** | **M+H⁺** |
|---|---|---|---|---|---|---|---|
| **1** | CF₃ | Ph | 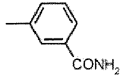 | Me | / | / | 397 |
| **2** | CF₃ | Ph | 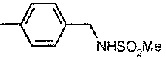 | Me | / | / | 461 |
| **3** | CF₃ | Ph | 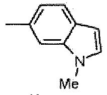 | Me | / | / | 407 |
| **4** | CF₃ | Ph | 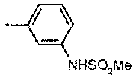 | Me | / | / | 447 |
| **5** | CF₃ | Ph | 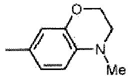 | Me | / | / | 426 |
| **6** | CF₃ | Ph | 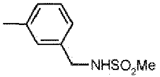 | Me | / | / | 461 |
| **7** | CF₃ | Ph | 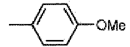 | Me | / | / | 384 |
| **8** | CF₃ | Ph | 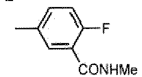 | Me | / | / | 429 |
| **9** | CF₃ | Ph | 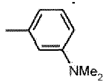 | Me | / | / | 397 |
| **10** | CF₃ | Ph | 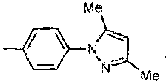 | Me | / | / | 448 |
| **11** | CF₃ | Ph | 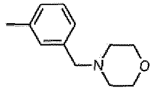 | Me | / | / | 453 |
| **12** | CF₃ | Ph | 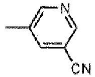 | Me | / | / | 380 |
| **13** | CF₃ | Ph | 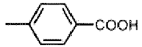 | Me | / | / | 398 |
| **14** | CF₃ | Ph | 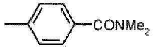 | Me | / | / | 425 |
| **15** | CF₃ | Ph | 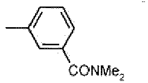 | Me | / | / | 425 |
| **16** | CF₃ | Ph | 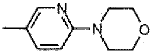 | Me | / | / | 440 |
| **17** | CF₃ | Ph | 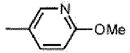 | Me | / | / | 385 |
| **18** | CF₃ | Ph | 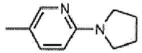 | Me | / | / | 424 |
| **19** | CF₃ | Ph | 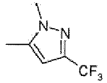 | Me | / | / | 426 |
| **20** | CF₃ | Ph | 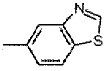 | Me | / | / | 411 |
| **21** | CF₃ | Ph | 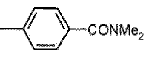 | H | HCl | / | 411 |
| **22** | CF₃ | Ph | 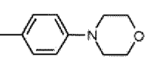 | H | HCl | / | 425 |
| **23** | CF₃ | Ph | 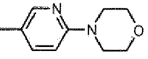 | H | HCl | / | 426 |
| **24** | CF₃ | Ph | 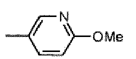 | H | HCl | / | 371 |
| **25** | CF₃ | Ph | 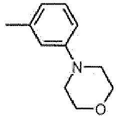 | H | HCl | / | 425 |
| **26** | CF₃ | Ph | 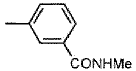 | H | HCl | / | 397 |
| **27** | CF₃ | Ph | 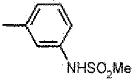 | H | HCl | / | 433 |
| **28** | CF₃ | Ph | 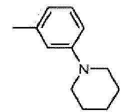 | H | HCl | / | 423 |
| **29** | CF₃ | Ph | 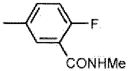 | H | HCl | / | 415 |
| **30** | CF₃ | Ph | 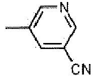 | H | HCl | / | 366 |
| **31** | CHF₂ | Ph | 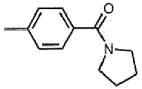 | H | HCl | 248 | 455 |
| **32** | CHF₂ | Ph | 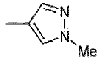 | H | HCl | / | 362 |
| **33** | CHF₂ | Ph | 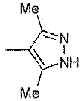 | H | HCl | / | 376 |
| **34** | CHF₂ | Ph | 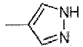 | H | HCl | / | 348 |
| **35** | CHF₂ | Ph | 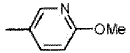 | H | HCl | / | 389 |
| **36** | CHF₂ | Ph | 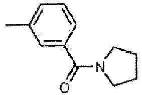 | H | HCl | / | 455 |
| **37** | CHF₂ | Ph | 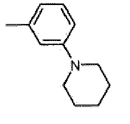 | H | HCl | / | 441 |
| **38 Ex. 2** | COOH | H | 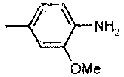 | H | / | / | 285 |
| **39** | CONHMe | Ph | 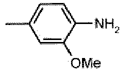 | H | TFA | / | 458 |
| **40** | CONH₂ | Ph | 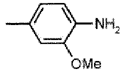 | H | TFA | / | 474 |
| **41** | CONHMe | H | 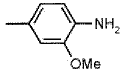 | H | TFA | / | 412 |
| **42** | CONH₂ | H | 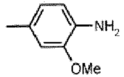 | H | TFA | / | 398 |
| **43** | COOH | Ph | 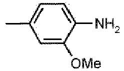 | H | / | / | 361 |
| **44** | COOH | Ph | 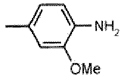 | Me | / | / | 375 |
| **45** | COOH | H | 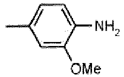 | Me | / | / | 299 |
| **46 Ex. 1** | CONH₂ | Ph | 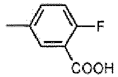 | H | TFA | / | 377 |
| **47** | CONH₂ | Ph | 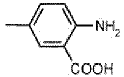 | H | / | / | 374 |
| **48** | CONH₂ | Ph | 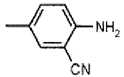 | H | TFA | / | 355 |
| **49** | CONH₂ | 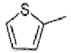 | 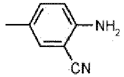 | H | / | / | 361 |
| **50** | COOH | cPr | 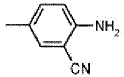 | H | / | / | 320 |
| **51** | CONH₂ | H | 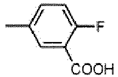 | H | / | / | 301 |
| **52** | CONH₂ | Ph | 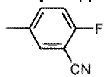 | H | / | / | 358 |
| **53 Ex. 3** | CHF₂ | Ph | 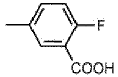 | H | / | / | 384 |
| **54** | CF₃ | Ph | 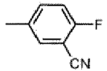 | H | / | / | 383 |
| **55** | CHF₂ | Ph | 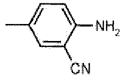 | H | HCl | 282 | 362 |
| **56 Ex. 4** | CF₃ | Ph | 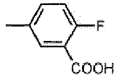 | H | / | / | 402 |
| **57 Ex. 6** | CF₃ | Ph | 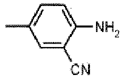 | Me | / | 269 | 394 |
| **58** | CF₃ | Ph | 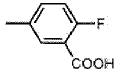 | Me | / | / | 416 |
| **59** | CF₃ | Ph | 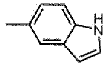 | H | / | / | 379 |
| **60** | CF₃ | Ph | 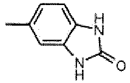 | H | / | 380 | 396 |
| **61 Ex. 5** | CF₃ | Ph | 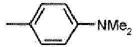 | H | / | 227 | 383 |
| **62** | CONHPh | H | 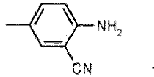 | H | HCl | / | 355 |
| **63** | 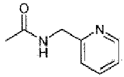 | H | 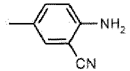 | H | HCl | / | 370 |
| **64** | 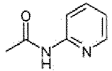 | H | 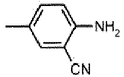 | H | HCl | / | 356 |
| **65** | 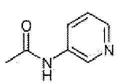 | H | 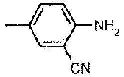 | H | HCl | / | 356 |
| **66** | 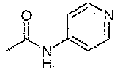 | H | 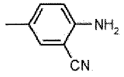 | H | HCl | / | 356 |
| **67** | CHF₂ | 4-Py | 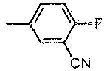 | Me | / | / | 380 |
| **68** | CONHPh | H | 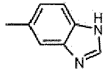 | H | HCl | / | 355 |
| **69** | 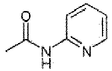 | H | 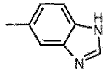 | H | HCl | / | 356 |
| **70** | 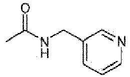 | H | 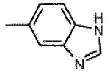 | H | HCl | / | 370 |
| **71** | CONHPh | H | 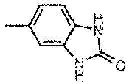 | H | / | / | 371 |
| **72 Ex. 10** | CHF₂ | H | 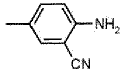 | Me | / | 251 | 300 |
| **73** | CHF₂ | H | 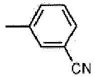 | Me | / | 162 | 285 |
| **74** | CHF₂ | H | 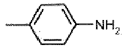 | Me | / | 149 | 275 |
| **75 Ex. 12** | CHF₂ | H | 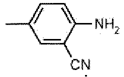 | H | / | 263 | 286 |
| **76** | CF₃ | Ph | 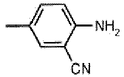 | 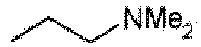 | / | 183 | 451 |
| **77** | CF₃ | Ph | 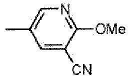 | Me | / | 250 | 410 |
| **78** | CHF₂ | Ph | 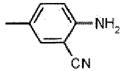 | Me | / | 246 | 376 |
| **79** | CHF₂ | Ph | 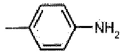 | Me | / | 176 | 351 |
| **80** | CHF₂ | Ph | 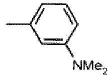 | Me | / | 154 | 379 |
| **81** | CF₃ | Ph | 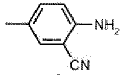 | 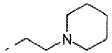 | / | 192 | 491 |
| **82** | CF₃ | Ph | 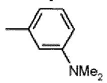 | 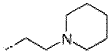 | HCl | 227 | 494 |
| **83 Ex. 13** | CHF₂ | Ph | 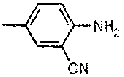 | 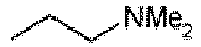 | / | 163 | 433 |
| **84** | CF₃ | H | 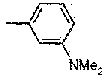 | 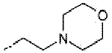 | / | 110 | 420 |
| **85** | CF₃ | H | 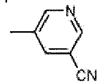 | 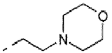 | / | / | 403 |
| **86** | CF₃ | H | 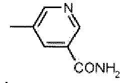 | 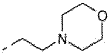 | / | 238 | 421 |
| **87** | CF₃ | H | 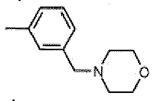 | Me | / | 105 | 377 |
| **88 Ex. 8** | CF₃ | H | 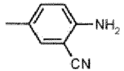 | Me | / | 276 | 318 |
| **89** | CF₃ | Ph | 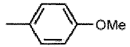 | Me | / | 181 | 384 |
| **90** | CF₃ | H | 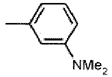 | Me | / | 91 | 321 |
| **91 Ex. 15** | CHF₂ | 3-Py | 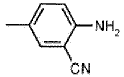 | H | / | 296 | 363 |
| **92** | CHF₂ | 4-Py | 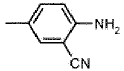 | H | / | 325 | 363 |
| **93 Ex. 14** | CF₃ | 3-Py | 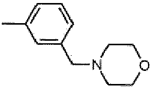 | Me | / | 155 | 454 |
| **94** | CHF₂ | 3MeO-Ph | 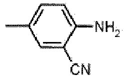 | Me | / | 233 | 392 |
| **95** | CF₃ | H | 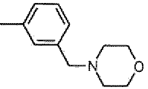 | Pr | HCl | 271 | 405 |
| **96** | CF₃ | H | 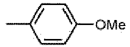 | Pr | / | 72 | 336 |
| **97** | CF₃ | 3MeO-Ph | 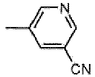 | Me | / | 194 | 410 |
| **98** | CF₃ | 3MeO-Ph | 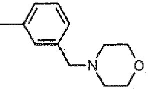 | Me | / | 114 | 483 |
| **99** | CF₃ | 3MeO-Ph | 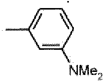 | Me | / | 138 | 427 |
| **100** | CF₃ | 3MeO-Ph | 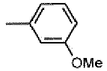 | Me | / | 133 | 414 |
| **101** | CONH₂ | 3-Py | 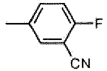 | H | / | / | 377 |

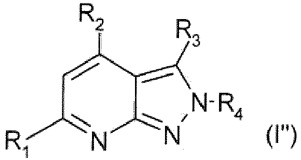
| **No.** | **R₂** | **R₃** | **R₁** | **R₄** | **Salt** | **m.p. (°C)** | **M+H⁺** |
|---|---|---|---|---|---|---|---|
| **102** | COOH | H | 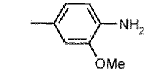 | Me | / | / | 299 |
| **103** | CONH₂ | H | 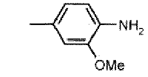 | Me | TFA | / | 412 |
| **104** | COOH | H | 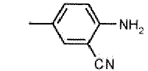 | Me | / | / | 294 |
| **105** | CHF₂ | H | 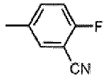 | Me | / | / | 303 |
| **106 Ex. 11** | CHF₂ | H | 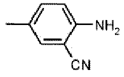 | Me | / | / | 300 |
| **107** | CHF₂ | H | 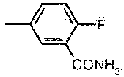 | Me | / | / | 321 |
| **108 Ex. 7** | CF₃ | Ph | 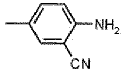 | Me | / | 295 | 394 |
| **109** | CF₃ | Ph | 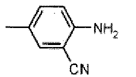 | 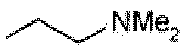 | / | 237 | 451 |
| **110** | CF₃ | Ph | 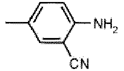 | 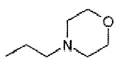 | / | 249 | 493 |
| **111** | CHF₂ | H | 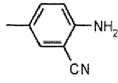 | 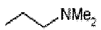 | / | 182 | 357 |
| **112** | CHF₂ | H | 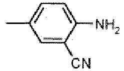 | 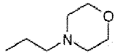 | / | 242 | 399 |
| **113** | CF₃ | H | 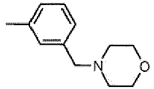 | 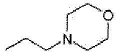 | / | 101 | 476 |
| **114 Ex. 9** | CF₃ | H | 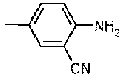 | Me | / | 249 | 318 |
| **115** | CF₃ | H | 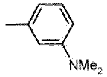 | Pr | HCl | 181 | 425 |
| **116** | CHF₂ | H | 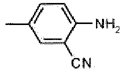 | 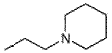 | / | 230 | 397 |
| **117** | CHF₂ | H | 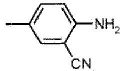 | Pr | / | 214 | 328 |
| **118** | CF₃ | H | 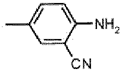 | Pr | / | 239 | 346 |
| **119** | CF₃ | H | 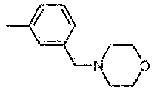 | Pr | / | 288 | 405 |
| **120** | CF₃ | H | 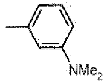 | Pr | / | 89 | 349 |

## Patentansprüche

1. Verbindung der Formel (I) 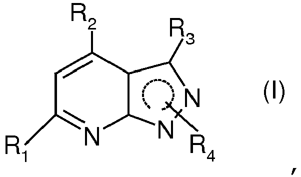 in welcher:
die Darstellung des Pyrazolrings zeigt, dass der Substituent R₄ sich entweder am Stickstoff alpha zum Pyridinring (I') oder am Stickstoff alpha zum einen Substituenten R₃ tragenden Kohlenstoff (I") befinden kann, also: 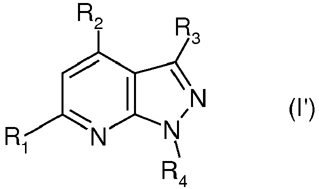 entweder 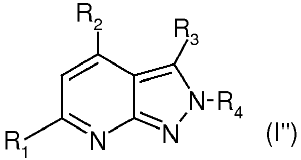
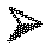 **R₁** für eine Aryl-, Pyridyl- oder Pyrazolylgruppe steht, gegebenenfalls substituiert durch einen oder mehrere Substituenten ausgewählt aus:
∘ einem Fluoratom,
∘ einer -CF₃-Gruppe
∘ einer Cyanogruppe,
∘ einer -NR₆R₆'-Gruppe, in welcher R₆ und R₆' wie unten definiert sind,
∘ einer -NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden, gegebenenfalls substituiert durch einen oder mehrere aus einem Halogenatom und einer geradkettigen oder verzweigten Alkylgruppe ausgewählte Substituenten,
∘ einer -CH₂NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden,
∘ einer -COR₁₂-Gruppe, in welcher R₁₂ für eine Hydroxylgruppe oder eine -NR₆R₆'-Gruppe, in welcher R₆ und R₆' wie unten definiert sind, steht,
∘ einer -CONR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden,
∘ einer - (CH₂) ₚNHSO₂CH₃-Gruppe, in welcher p für 0 oder 1 steht,
∘ einer -OR₁₃-Gruppe, in welcher R₁₃ für eine geradkettige (C₁-C₃)-Alkylgruppe steht,
∘ einer Ethyl-, Propyl- oder Isopropylgruppe,
oder R₁ für eine bicyclische Gruppe der Formel A unten steht: 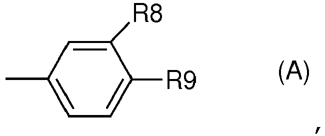 in welcher R₈ und R₉ zusammen mit den Kohlenstoffatomen, an die sie gebunden sind, einen gesättigten oder ungesättigten Heterocyclus mit einem oder mehreren aus einem Stickstoffatom, einem Sauerstoffatom und einem Schwefelatom ausgewählten Heteroatomen bilden, so dass die Gruppe (A) eine Dihydrobenzimidazolonyl-, Indolyl-, Dihydrobenzoxazinyl-, Benzothiazolyl- oder Benzimidazolylgruppe bildet, gegebenenfalls substituiert durch eine oder mehrere geradkettige Alkylgruppen,
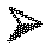 **R₂** für eine Gruppe:
∘ -CF₃,
∘ -CHF₂,
oder
∘ -CONHR₅, in welcher R₅ wie unten definiert ist, steht,
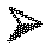 **R₃** für:
∘ ein Wasserstoffatom,
∘ eine Arylgruppe, gegebenenfalls substituiert durch eine Alkoxymethylgruppe,
oder
∘ eine Heteroarylgruppe ausgewählt aus Thienyl- und Pyridylgruppen steht,
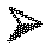 **R₄** für:
∘ ein Wasserstoffatom,
∘ (C₁)-Alkyl, geradkettiges (C₃) -Alkyl oder geradkettiges (C₁-C₃)-Alkyl, substituiert durch eine -NR₆R₆'-Gruppe, in welcher R₆ und R₆' wie unten definiert sind, oder eine -NR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden, steht,
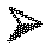 **R₅** für:
∘ ein Wasserstoffatom,
∘ eine geradkettige (C₁-C₃) -Alkylgruppe, gegebenenfalls substituiert durch eine Pyridylgruppe,
oder
∘ eine aromatische Gruppe ausgewählt aus Aryl und Pyridyl steht,
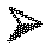 **R₆** und **R₆'**, die gleich oder verschieden sein können, für ein Wasserstoffatom oder eine geradkettige Alkylgruppe stehen,
in Form der Base oder eines Säureadditions- oder Basenadditionssalzes,
mit Ausnahme der folgenden Verbindungen:
2-Fluor-5-(3-phenyl-4-trifluormethyl-1H-pyrazolo[3,4-b]pyridin-6-yl)benzoesäure,
3-(1-Methyl-3-phenyl-4-trifluormethyl-1H-pyrazolo[3,4-b]pyridin-6-yl)benzamid,
N-[4-(1-Methyl-3-phenyl-4-trifluormethyl-1H-pyrazolo[3,4-b]pyridin-6-yl)benzyl]methansulfonamid,
1-Methyl-6-(1-methyl-1H-indol-6-yl)-3-phenyl-4-trifluormethyl-1H-pyrazolo[3,4-b]pyridin;
N-[3-(1-Methyl-3-phenyl-4-trifluormethyl-1H-pyrazolo[3,4-b]pyridin-6-yl)phenyl]methansulfonamid,
4-Methyl-7-(1-methyl-3-phenyl-4-trifluormethyl-1H-pyrazolo[3,4-b]pyridin-6-yl)-3,4-dihydro-2H-benzo[1,4]oxazin,
N-[3-(1-Methyl-3-phenyl-4-trifluormethyl-1H-pyrazolo[3,4-b]pyridin-6-yl)benzyl]methansulfonamid,
6-(4-Methoxyphenyl)-1-methyl-3-phenyl-4-trifluormethyl-1H-pyrazolo[3,4-b]pyridin,
2-Fluor-N-methyl-5-(1-methyl-3-phenyl-4-trifluormethyl-1H-pyrazolo [3,4-b]pyridin-6-yl)benzamid,
Dimethyl[3-(1-methyl-3-phenyl-4-trifluormethyl-1H-pyrazolo[3,4-b]pyridin-6-yl)phenyl)amin,
6-[4-(3,5-Dimethylpyrazol-1-yl)phenyl]-1-methyl-3-phenyl-4-trifluormethyl-1H-pyrazolo[3,4-b]pyridin,
1-Methyl-6-(3-morpholin-4-ylmethylphenyl)-3-phenyl-4-trifluormethyl-1H-pyrazolo[3,4-b]pyridin,
5-(1-Methyl-3-phenyl-4-trifluormethyl-1H-pyrazolo[3,4-b]pyridin-6-yl)nicotinonitril,
4-(1-Methyl-3-phenyl-4-trifluormethyl-1H-pyrazolo[3,4-b]pyridin-6-yl)benzoesäure,
N,N-Dimethyl-4-(1-methyl-3-phenyl-4-trifluormethyl-1H-pyrazolo[3,4-b]pyridin-6-yl)benzamid,
N,N-Dimethyl-3-(1-methyl-3-phenyl-4-trifluormethyl-1H-pyrazolo[3,4-b]pyridin-6-yl)benzamid,
1-Methyl-6-(6-morpholin-4-ylpyridin-3-yl)-3-phenyl-4-trifluormethyl-1H-pyrazolo[3,4-b]pyridin,
6-(6-Methoxypyridin-3-yl)-1-methyl-3-phenyl-4-trifluormethyl-1H-pyrazolo[3,4-b]pyridin,
1-Methyl-3-phenyl-6-(6-pyrrolidin-1-ylpyridin-3-yl)-4-trifluormethyl-1H-pyrazolo[3,4-b]pyridin,
1-Methyl-6-(2-methyl-5-trifluormethyl-2H-pyrazol-3-yl)-3-phenyl-4-trifluormethyl-1H-pyrazolo[3,4-b]pyridin,
6-Benzothiazol-5-yl-1-methyl-3-phenyl-4-trifluormethyl-1H-pyrazolo[3,4-b]pyridin,
N,N-Dimethyl-4-(3-phenyl-4-trifluormethyl-1H-pyrazolo[3,4-b]pyridin-6-yl)benzamid,
6-(4-Morpholin-4-ylphenyl)-3-phenyl-4-trifluormethyl-1H-pyrazolo[3,4-b]pyridin,
6-(6-Morpholin-4-ylpyridin-3-yl)-3-phenyl-4-trifluormethyl-1H-pyrazolo[3,4-b]pyridin,
6-(6-Methoxypyridin-3-yl)-3-phenyl-4-trifluormethyl-1H-pyrazolo[3,4-b]pyridin,
6-(3-Morpholin-4-ylphenyl)-3-phenyl-4-trifluormethyl-1H-pyrazolo[3,4-b]pyridin,
N-Methyl-3-(3-phenyl-4-trifluormethyl-1H-pyrazolo[3,4-b]pyridin-6-yl)benzamid,
N-[3-(3-Phenyl-4-trifluormethyl-1H-pyrazolo[3,4-b]pyridin-6-yl)phenyl]methansulfonamid,
3-Phenyl-6-(3-piperidin-1-ylphenyl)-4-trifluormethyl-1H-pyrazolo[3,4-b]pyridin,
2-Fluor-N-methyl-5-(3-phenyl-4-trifluormethyl-1H-pyrazolo[3,4-b]pyridin-6-yl)benzamid,
5-(3-Phenyl-4-trifluormethyl-1H-pyrazolo[3,4-b]pyridin-6-yl)nicotinnitril,
2-Fluor-5-(1-methyl-3-phenyl-4-trifluormethyl-1H-pyrazolo[3,4-b]pyridin-6-yl)benzoesäure,
2-Amino-5-(4-difluormethyl-2-methyl-2H-pyrazolo[3,4-b]pyridin-6-yl)benzonitril,
Dimethyl[4-(3-phenyl-4-trifluormethyl-1H-pyrazolo[3,4-b]pyridin-6-yl)phenyl]amin,
4-(3-Phenyl-4-trifluormethyl-1H-pyrazolo[3,4-b]pyridin-6-yl)phenylamin,
6-(4-Methoxyphenyl)-3-phenyl-4-trifluormethyl-1H-pyrazolo[3,4-b]pyridin,
2-Fluor-5-(3-phenyl-4-trifluormethyl-1H-pyrazolo[3,4-b]pyridin-6-yl)benzonitril,
6-(1-Ethyl-1H-pyrazol-4-yl)-1-methyl-4-(trifluormethyl)-1H-pyrazolo[3,4-b]pyridin,
4-(Difluormethyl)-6-(1-ethyl-1H-pyrazol-4-yl)-1-methyl-1H-pyrazolo[3,4-b]pyridin,
6-(4-Ethylphenyl)-1-methyl-4-(trifluormethyl)-1H-pyrazolo[3,4-b]pyridin,
6-(4-Methoxyphenyl)-1-methyl-4-(trifluormethyl)-1H-pyrazolo[3,4-b]pyridin,
6-(4-Fluorphenyl)-1-methyl-4-(trifluormethyl)-1H-pyrazolo[3,4-b]pyridin,
4-(Difluormethyl)-6-(2-methoxyphenyl)-1-methyl-1H-pyrazolo[3,4-b]pyridin,
6-(3-Methoxyphenyl)-1-methyl-4-(trifluormethyl)-1H-pyrazolo[3,4-b]pyridin,
6-(2,5-Dimethoxyphenyl)-1-methyl-4-(trifluormethyl)-1H-pyrazolo[3,4-b]pyridin,
4-(Difluormethyl)-6-(4-ethylphenyl)-1-methyl-1H-pyrazolo[3,4-b]pyridin,
4-(Difluormethyl)-6-(4-methoxyphenyl)-1-methyl-1H-pyrazolo[3,4-b]pyridin,
4-(Difluormethyl)-6-(3-methoxyphenyl)-1-methyl-1H-pyrazolo[3,4-b]pyridin,
4-(Difluormethyl)-6-(4-fluorphenyl)-1-methyl-1H-pyrazolo[3,4-b]pyridin,
4-(Difluormethyl)-6-(3,4-dimethoxyphenyl)-1-methyl-1H-pyrazolo[3,4-b]pyridin,
4-(Difluormethyl)-1-methyl-6-phenyl-1H-pyrazolo[3,4-b]pyridin,
1-Methyl-6-phenyl-4-(trifluormethyl)-1H-pyrazolo[3,4-b]pyridin.

2. Verbindungen der Formel (I) nach Anspruch 1, **dadurch gekennzeichnet, dass** R₂ für eine Gruppe:
∘ -CHF₂, ausgenommen, wenn der an dem Stickstoff alpha zu R₃ befindliche Rest R₄ für eine Methylgruppe steht und R₃ für ein Wasserstoffatom steht,
oder
∘ -CONHR₅, in welcher R₅ für ein Wasserstoffatom oder eine geradkettige (C₁-C₃)-Alkylgruppe, gegebenenfalls substituiert durch eine Pyridylgruppe oder eine aromatische Gruppe ausgewählt aus Aryl und Pyridyl, steht, steht,
in Form der Base oder eines Säureadditions- oder Basenadditionssalzes.

3. Verbindungen der Formel (I) nach einem der Ansprüche 1 oder 2, **dadurch gekennzeichnet, dass** R₁ für eine Aryl-, Pyridyl- oder Pyrazolylgruppe steht, gegebenenfalls substituiert durch einen oder mehrere Substituenten ausgewählt aus:
∘ einem Fluoratom,
und
∘ einer -COR₁₂-Gruppe, in welcher R₁₂ für eine Hydroxylgruppe steht,
in Form der Base oder eines Säureadditions- oder Basenadditionssalzes.

4. Verbindungen der Formel (I) nach Ansprüchen 1 bis 3, **dadurch gekennzeichnet, dass** R₁ für eine Arylgruppe steht, in Form der Base oder eines Säureadditions- oder Basenadditionssalzes.

5. Verbindungen der Formel (I) nach einem der Ansprüche 1, 3 und 4, **dadurch gekennzeichnet, dass** R₂ für eine Gruppe:
∘ -CF₃,
∘ -CHF₂,
oder
∘ -CONHR₅, in welcher R₅ für ein Wasserstoffatom oder eine geradkettige (C₁-C₃)-Alkylgruppe, gegebenenfalls substituiert durch eine Pyridylgruppe oder eine aromatische Gruppe ausgewählt aus Aryl und Pyridyl, steht, steht,
in Form der Base oder eines Säureadditions- oder Basenadditionssalzes.

6. Verbindung der Formel (I) nach einem der vorhergehenden Ansprüche, **dadurch gekennzeichnet, dass** sie aus den folgenden Verbindungen ausgewählt ist:
[4-(4-Difluormethyl-3-phenyl-1H-pyrazolo[3,4-b]pyridin-6-yl)phenyl]pyrrolidin-1-ylmethanon,
4-Difluormethyl-6-(1-methyl-1H-pyrazol-4-yl)-3-phenyl-1H-pyrazolo[3,4-b]pyridin,
4-Difluormethyl-6-(3,5-dimethyl-1H-pyrazol-4-yl)-3-phenyl-1H-pyrazolo[3,4-b]pyridin,
4-Difluormethyl-3-phenyl-6-(1H-pyrazol-4-yl)-1H-pyrazolo[3,4-b]pyridin,
4-Difluormethyl-6-(6-methoxypyridin-3-yl)-3-phenyl-1H-pyrazolo[3,4-b]pyridin,
[3-(4-Difluormethyl-3-phenyl-1H-pyrazolo[3,4-b]pyridin-6-yl)phenyl]pyrrolidin-1-ylmethanon,
4-Difluormethyl-3-phenyl-6-(3-piperidin-1-ylphenyl)-1H-pyrazolo[3,4-b]pyridin,
6-(4-Amino-3-methoxyphenyl)-3-phenyl-1H-pyrazolo[3,4-b]pyridin-4-carbonsäuremethylamid,
6-(4-Amino-3-methoxyphenyl)-3-phenyl-1H-pyrazolo[3,4-b]pyridin-4-carbonsäureamid,
6-(4-Amino-3-methoxyphenyl)-1H-pyrazolo[3,4-b]pyridin-4-carbonsäuremethylamid,
4-(4-Amino-3-methoxyphenyl)-1H-pyrazolo[3,4-b]pyridin-6-carbonsäuremethylamid,
6-(4-Amino-3-methoxyphenyl)-1H-pyrazolo[3,4-b]pyridin-4-carbonsäureamid,
6-(4-Amino-3-methoxyphenyl)-2-methyl-2H-pyrazolo[3,4-b]pyridin-4-carbonsäureamid,
5-(4-Carbamoyl-3-phenyl-1H-pyrazolo[3,4-b]pyridin-6-yl)-2-fluorbenzoesäure,
2-Amino-5-(4-carbamoyl-3-phenyl-1H-pyrazolo[3,4-b]pyridin-6-yl)benzoesäure,
6-(4-Amino-3-cyanophenyl)-3-phenyl-1H-pyrazolo[3,4-b]pyridin-4-carbonsäureamid,
6-(4-Amino-3-cyanophenyl)-3-thiophen-2-yl-1H-pyrazolo[3,4-b]pyridin-4-carbonsäureamid,
5-(4-Carbamoyl-1H-pyrazolo[3,4-b]pyridin-6-yl)-2-fluorbenzoesäure,
6-(3-Cyano-4-fluorphenyl)-3-phenyl-1H-pyrazolo[3,4-b]pyridin-4-carbonsäureamid,
5-(4-Difluormethyl-3-phenyl-1H-pyrazolo[3,4-b]pyridin-6-yl)-2-fluorbenzoesäure,
2-Amino-5-(4-difluormethyl-3-phenyl-1H-pyrazolo[3,4-b]pyridin-6-yl)benzonitril,
2-Amino-5-(1-methyl-3-phenyl-4-trifluormethyl-1H-pyrazolo[3,4-b]pyridin-6-yl)benzonitril,
6-(3-Carbamoyl-4-fluorphenyl)-3-pyridin-3-yl-1H-pyrazolo[3,4-b]pyridin-4-carbonsäureamid,
5-(4-Difluormethyl-2-methyl-2H-pyrazolo[3,4-b]pyridin-6-yl)-2-fluorbenzonitril,
6-(1H-Indol-6-yl)-3-phenyl-4-trifluormethyl-1H-pyrazolo[3,4-b]pyridin,
5-(3-Phenyl-4-trifluormethyl-1H-pyrazolo[3,4-b]pyridin-6-yl)-1,3-dihydrobenzimidazol-2-on,
6-(4-Amino-3-cyanophenyl)-1H-pyrazolo[3,4-b]pyridin-4-carbonsäurephenylamid,
6-(4-Amino-3-cyanophenyl)-1H-pyrazolo[3,4-b]pyridin-4-carbonsäure-(pyridin-2-ylmethyl)amid,
6-(4-Amino-3-cyanophenyl)-1H-pyrazolo[3,4-b]pyridin-4-carbonsäurepyridin-2-ylamid,
6-(4-Amino-3-cyanophenyl)-1H-pyrazolo[3,4-b]pyridin-4-carbonsäurepyridin-3-ylamid,
6-(4-Amino-3-cyanophenyl)-1H-pyrazolo[3,4-b]pyridin-4-carbonsäurepyridin-4-ylamid,
5-(4-Difluormethyl-2-methyl-2H-pyrazolo[3,4-b]pyridin-6-yl)-2-fluorbenzamid,
5-(4-Difluormethyl-1-methyl-3-pyridin-4-yl-1H-pyrazolo[3,4-b]pyridin-6-yl)-2-fluorbenzonitril,
2-Amino-5-(2-methyl-3-phenyl-4-trifluormethyl-2H-pyrazolo[3,4-b]pyridin-6-yl)benzonitril,
6-(1H-Benzimidazol-5-yl)-1H-pyrazolo[3,4-b]pyridin-4-carbonsäurephenylamid,
6-(1H-Benzimidazol-5-yl)-1H-pyrazolo[3,4-b]pyridin-4-carbonsäurepyridin-2-ylamid,
6-(1H-Benzimidazol-5-yl)-1H-pyrazolo[3,4-b]pyridin-4-carbonsäure-(pyridin-3-ylmethyl)amid,
6-(2-Oxo-2,3-dihydro-1H-benzimidazol-5-yl)-1H-pyrazolo[3,4-b]pyridin-4-carbonsäurephenylamid,
2-Amino-5-(4-difluormethyl-1-methyl-1H-pyrazolo[3,4-b]pyridin-6-yl)benzonitril,
3-(4-Difluormethyl-1-methyl-1H-pyrazolo[3,4-b]pyridin-6-yl)benzonitril,
4-(4-Difluormethyl-1-methyl-1H-pyrazolo[3,4-b]pyridin-6-yl)phenylamin,
2-Amino-5-[2-(2-dimethylaminoethyl)-3-phenyl-4-trifluormethyl-2H-pyrazolo[3,4-b]pyridin-6-yl]benzonitril,
2-Amino-5-(4-difluormethyl-1H-pyrazolo[3,4-b]pyridin-6-yl)benzonitril,
2-Amino-5-[1-(2-dimethylaminoethyl)-3-phenyl-4-trifluormethyl-1H-pyrazolo[3,4-b]pyridin-6-yl]benzonitril,
2-Amino-5-[2-(2-morpholin-4-ylethyl)-3-phenyl-4-trifluormethyl-2H-pyrazolo[3,4-b]pyridin-6-yl]benzonitril,
2-Methoxy-5-(1-methyl-3-phenyl-4-trifluormethyl-1H-pyrazolo[3,4-b]pyridin-6-yl)nicotinonitril,
2-Amino-5-(4-difluormethyl-1-methyl-3-phenyl-1H-pyrazolo[3,4-b]pyridin-6-yl)benzonitril,
4-(4-Difluormethyl-1-methyl-3-phenyl-1H-pyrazolo[3,4-b]pyridin-6-yl)phenylamin,
[3-(4-Difluormethyl-1-methyl-3-phenyl-1H-pyrazolo[3,4-b]pyridin-6-yl)phenyl]dimethylamin,
2-Amino-5-[3-phenyl-1-(2-piperidin-1-ylethyl)-4-trifluormethyl-1H-pyrazolo[3,4-b]pyridin-6-yl]benzonitril,
Dimethyl-{3-[3-phenyl-1-(2-piperidin-1-ylethyl)-4-trifluormethyl-1H-pyrazolo[3,4-b]pyridin-6-yl]phenyl}amin,
2-Amino-5-[4-difluormethyl-2-(2-dimethylaminoethyl)-2H-pyrazolo[3,4-b]pyridin-6-yl]benzonitril,
2-Amino-5-[4-difluormethyl-2-(2-morpholin-4-ylethyl)-2H-pyrazolo[3,4-b]pyridin-6-yl]benzonitril,
2-Amino-5-[4-difluormethyl-1-(2-dimethylaminoethyl)-3-phenyl-1H-pyrazolo[3,4-b]pyridin-6-yl]benzonitril,
2-(2-Morpholin-4-ylethyl)-6-(3-morpholin-4-ylmethylphenyl)-4-trifluormethyl-2H-pyrazolo[3,4-b]pyridin,
Dimethyl-{3-[1-(2-morpholin-4-ylethyl)-4-trifluormethyl-1H-pyrazolo[3,4-b]pyridin-6-yl]phenyl}amin,
5-[1-(2-Morpholin-4-ylethyl)-4-trifluormethyl-1H-pyrazolo[3,4-b]pyridin-6-yl]nicotinonitril,
5-[1-(2-Morpholin-4-ylethyl)-4-trifluormethyl-1H-pyrazolo[3,4-b]pyridin-6-yl]nicotinamid,
2-Amino-5-(2-methyl-4-trifluormethyl-2H-pyrazolo[3,4-b]pyridin-6-yl)benzonitril,
1-Methyl-6-(3-morpholin-4-ylmethylphenyl)-4-trifluormethyl-1H-pyrazolo[3,4-b]pyridin,
2-Amino-5-(1-methyl-4-trifluormethyl-1H-pyrazolo[3,4-b]pyridin-6-yl)benzonitril,
Dimethyl-[3-(1-methyl-4-trifluormethyl-1H-pyrazolo[3,4-b]pyridin-6-yl)phenyl]amin,
Dimethyl-[3-(3-phenyl-2-propyl-4-trifluormethyl-2H-pyrazolo[3,4-b]pyridin-6-yl)phenyl]amin,
2-Amino-5-[4-difluormethyl-2-(2-piperidin-1-ylethyl)-2H-pyrazolo[3,4-b]pyridin-6-yl]benzonitril,
2-Amino-5-(4-difluormethyl-3-pyridin-3-yl-1H-pyrazolo[3,4-b]pyridin-6-yl)benzonitril,
2-Amino-5-(4-difluormethyl-2-propyl-2H-pyrazolo[3,4-b]pyridin-6-yl)benzonitril,
2-Amino-5-(4-difluormethyl-3-pyridin-4-yl-1H-pyrazolo[3,4-b]pyridin-6-yl)benzonitril,
1-Methyl-6-(3-morpholin-4-ylmethylphenyl)-3-pyridin-3-yl-4-trifluormethyl-1H-pyrazolo[3,4-b]pyridin,
2-Amino-5-[4-difluormethyl-3-(3-methoxyphenyl)-1H-pyrazolo[3,4-b]pyridin-6-yl]benzonitril,
2-Amino-5-(2-propyl-4-trifluormethyl-2H-pyrazolo[3,4-b]pyridin-6-yl)benzonitril,
6-(3-Morpholin-4-ylmethylphenyl)-2-propyl-4-trifluormethyl-2H-pyrazolo[3,4-b]pyridin,
Dimethyl-[3-(2-propyl-4-trifluormethyl-2H-pyrazolo[3,4-b]pyridin-6-yl)phenyl] amin,
6-(3-Morpholin-4-ylmethylphenyl)-1-propyl-4-trifluormethyl-1H-pyrazolo[3,4-b]pyridin,
6-(4-Methoxyphenyl)-1-propyl-4-trifluormethyl-1H-pyrazolo[3,4-b]pyridin,
5-[3-(3-Methoxyphenyl)-1-methyl-4-trifluormethyl-1H-pyrazolo[3,4-b]pyridin-6-yl]nicotinonitril,
3-(3-Methoxyphenyl)-1-methyl-6-(3-morpholin-4-ylmethylphenyl)-4-trifluormethyl-1H-pyrazolo[3,4-b]pyridin,
{3-[3-(3-Methoxyphenyl)-1-methyl-4-trifluormethyl-1H-pyrazolo[3,4-b]pyridin-6-yl]phenyl}dimethylamin,
3-(3-Methoxyphenyl)-6-(4-methoxyphenyl)-1-methyl-4-trifluormethyl-1H-pyrazolo[3,4-b]pyridin.

7. Verfahren zur Herstellung der Verbindungen der Formel (I) nach Anspruch 1, gemäß Schema 5 wie folgt: 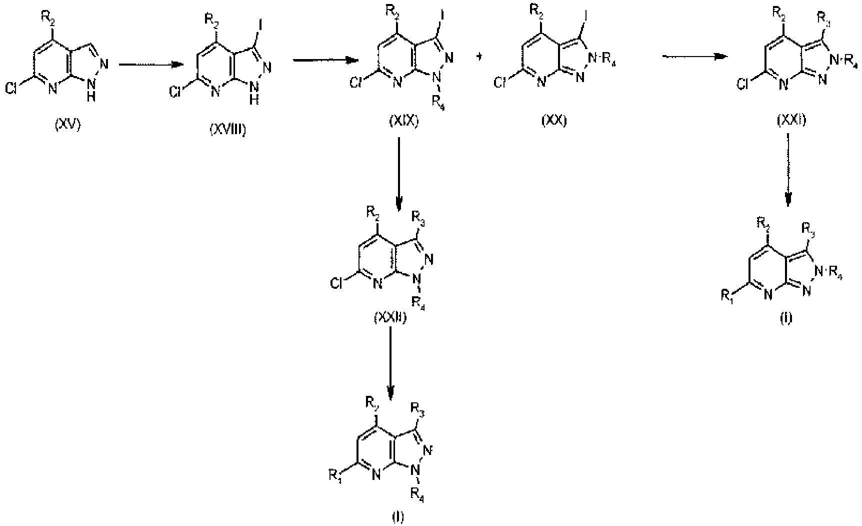 wobei R₂ für eine -CHF₂- oder -CF₃-Gruppe steht und R₁, R₃ und R₄ wie oben definiert sind, wobei allerdings R₃ und R₄ nicht für ein Wasserstoffatom stehen, **dadurch gekennzeichnet, dass**:
- die Verbindung der Formel (XV) einer Iodierungsreaktion in Gegenwart von N-Iodsuccinimid unterzogen wird, unter Erhalt der Verbindung der Formel (XVIII),
- die Verbindung der Formel (XVIII) dann einer Alkylierungsreaktion in Gegenwart eines halogenierten Derivats der Formel R₄-X unterzogen wird, unter Erhalt der Verbindungen der Formeln (XIX) und (XX),
- die Verbindungen der Formeln (XIX) und (XX) getrennt in Gegenwart eines Palladiumkatalysators, eines Liganden und einer Base einer Umsetzung mit Phenylboron- oder Heteroarylboronderivaten oder Phenylboronatestern oder Heteroarylboronatestern gemäß einer Suzuki-Kupplung unterzogen werden, unter Erhalt der Verbindungen der Formeln (XXI) und (XXII),
- die Verbindungen der Formeln (XXI) und (XXII) getrennt in Gegenwart eines Palladiumkatalysators, eines Liganden und einer Base einer Reaktion mit Phenylboron- oder Heteroarylboronderivaten oder Phenylboronatestern oder Heteroarylboronatestern gemäß einer Suzuki-Kupplung unterzogen werden, unter Erhalt der Verbindung der Formel (I), in welcher R₂ für eine -CHF₂- oder -CF₃-Gruppe steht und R₁, R₃ und R₄ wie oben definiert sind.

8. Verfahren zur Herstellung der Verbindungen der Formel (I) nach Anspruch 1, gemäß Schema 6 wie folgt: 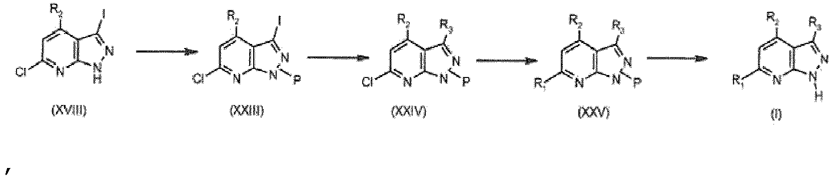 wobei R₂ für eine -CHF₂- oder -CF₃-Gruppe steht und R₁ und R₃ wie oben definiert sind, wobei allerdings R₃ für ein Wasserstoffatom steht, **dadurch gekennzeichnet, dass**:
- die Verbindung der Formel (XVIII) einer Alkylierungsreaktion in Gegenwart einer Schutzgruppe P unterzogen wird, unter Erhalt der Verbindung der Formel (XXIII),
- die Verbindung der Formel (XXIII) einer Umsetzung mit Phenylboron- oder Heteroarylboronderivaten oder Phenylboronatestern oder Heteroarylboronatestern gemäß einer Suzuki-Kupplung unterzogen wird, unter Erhalt der Verbindung der Formel (XXIV),
- die Verbindung der Formel (XXIV) einer Reaktion mit Phenylboron- oder Heteroarylboronderivaten oder Phenylboronatestern oder Heteroarylboronatestern gemäß einer Suzuki-Kupplung unterzogen wird, unter Erhalt der Verbindung der Formel (XXV),
- die Verbindung der Formel (XXV) einer Entschützungsreaktion unterzogen wird, unter Erhalt der Verbindung der Formel (I), in welcher R₂ für eine -CHF₂- oder -CF₃-Gruppe steht.

9. Verbindung der Formel (I) 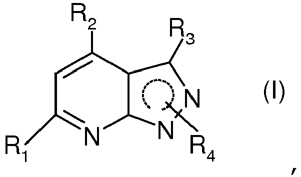 in welcher:
die Darstellung des Pyrazolrings zeigt, dass der Substituent R₄ sich entweder am Stickstoff alpha zum Pyridinring (I') oder am Stickstoff alpha zum einen Substituenten R₃ tragenden Kohlenstoff (I") befinden kann, also: 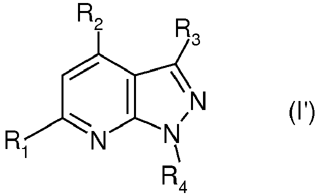 entweder 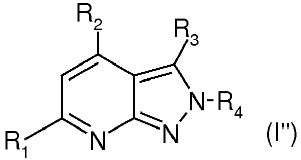
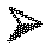 **R₁** für eine Aryl-, Pyridyl- oder Pyrazolylgruppe steht, gegebenenfalls substituiert durch einen oder mehrere Substituenten ausgewählt aus:
∘ einem Halogenatom,
∘ einer -CF₃-Gruppe
∘ einer Cyanogruppe,
∘ einer -NR₆R₆'-Gruppe, in welcher R₆ und R₆' wie unten definiert sind,
∘ einer -NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden, gegebenenfalls substituiert durch einen oder mehrere aus einem Halogenatom und einer geradkettigen oder verzweigten Alkylgruppe ausgewählte Substituenten,
∘ einer -CH₂NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden,
∘ einer -COR₁₂-Gruppe, in welcher R₁₂ für eine Hydroxylgruppe oder eine -NR₆R₆'-Gruppe, in welcher R₆ und R₆' wie unten definiert sind, steht,
∘ einer -CONR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden,
∘ einer - (CH₂)ₚNHSO₂CH₃-Gruppe, in welcher p für 0 oder 1 steht,
∘ einer -OR₁₃-Gruppe, in welcher R₁₃ für eine geradkettige (C₁-C₃)-Alkylgruppe steht,
∘ einer (C₁-C₃) -Alkylgruppe,
oder R₁ für eine bicyclische Gruppe der Formel A unten steht: 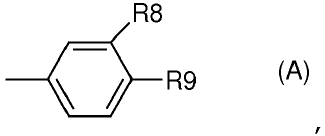 in welcher R₈ und R₉ zusammen mit den Kohlenstoffatomen, an die sie gebunden sind, einen gesättigten oder ungesättigten Heterocyclus mit einem oder mehreren aus einem Stickstoffatom, einem Sauerstoffatom und einem Schwefelatom ausgewählten Heteroatomen bilden, gegebenenfalls substituiert durch eine oder mehrere geradkettige Alkylgruppen,
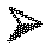 **R₂** für eine Gruppe:
∘ -CF₃,
∘ -CHF₂,
∘ -COOH
oder
∘ -CONHR₅, in welcher R₅ wie unten definiert ist, steht,
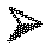 **R₃** für:
∘ ein Wasserstoffatom,
∘ eine Arylgruppe, gegebenenfalls substituiert durch eine Alkoxymethylgruppe,
∘ eine Cycloalkylgruppe
oder
∘ eine Heteroarylgruppe ausgewählt aus Thienyl- und Pyridylgruppen steht,
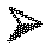 **R₄** für:
∘ ein Wasserstoffatom,
∘ eine geradkettige (C₁-C₃) -Alkylgruppe, gegebenenfalls substituiert durch eine -NR₆R₆'-Gruppe, in welcher R₆ und R₆' wie unten definiert sind, oder eine -NR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden, steht,
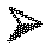 **R₅** für:
∘ ein Wasserstoffatom,
∘ eine geradkettige (C₁-C₃) -Alkylgruppe, gegebenenfalls substituiert durch eine Pyridylgruppe,
oder
∘ eine aromatische Gruppe ausgewählt aus Aryl und Pyridyl steht,
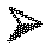 **R₆** und **R₆'**, die gleich oder verschieden sein können, für ein Wasserstoffatom oder eine geradkettige Alkylgruppe stehen,
in Form der Base oder eines Säureadditions- oder Basenadditionssalzes,
zur Verwendung als Medikament.

10. Verbindung nach dem vorhergehenden Anspruch, wobei die Verbindung aus den folgenden Verbindungen ausgewählt ist: 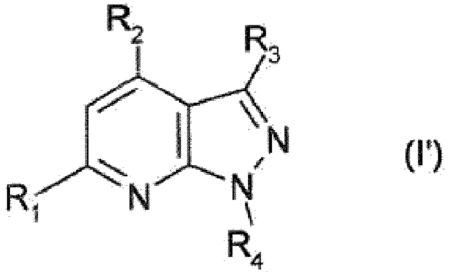
| **Nr.** | **R₂** | **R₃** | **R₁** | **R₄** | **Salz** | **Schmp.(°C)** | **M+H⁺** |
|---|---|---|---|---|---|---|---|
| **1** | CF₃ | Ph | 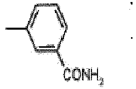 | Me | / | / | 397 |
| **2** | CF₃ | Ph | 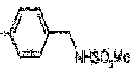 | Me | / | / | 461 |
| **3** | CF₃ | Ph | 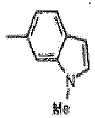 | Me | / | / | 407 |
| **4** | CF₃ | Ph | 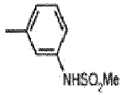 | Me | / | / | 447 |
| **5** | CF₃ | Ph | 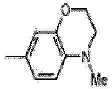 | Me | / | / | 426 |
| **6** | CF₃ | Ph | 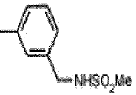 | Me | / | / | 461 |
| **7** | CF₃ | Ph | 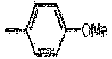 | Me | / | / | 384 |
| **8** | CF₃ | Ph | 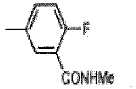 | Me | / | / | 429 |
| **9** | CF₃ | Ph | 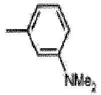 | Me | / | / | 397 |
| **10** | CF₃ | Ph | 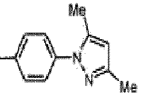 - | Me | / | / | 448 |
| **11** | CF₃ | Ph | 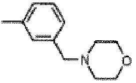 | Me | / | / | 453 |
| **12** | CF₃ | Ph | 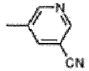 | Me | / | / | 380 |
| **13** | CF₃ | Ph | 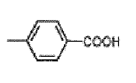 | Me | / | / | 398 |
| **14** | CF₃ | Ph | 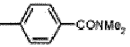 | Me | / | / | 425 |
| **15** | CF₃ | Ph | 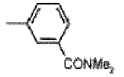 | Me | / | / | 425 |
| **16** | CF₃ | Ph | 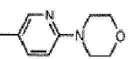 | Me | / | / | 440 |
| **17** | CF₃ | Ph | 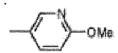 | Me | / | / | 385 |
| **18** | CF₃ | Ph | 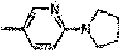 | Me | / | / | 424 |
| **19** | CF₃ | Ph | 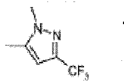 | Me | / | / | 426 |
| **20** | CF₃ | Ph | 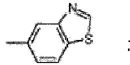 | Me | / | / | 411 |
| **21** | CF₃ | Ph | 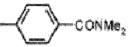 | H | HCl | / | 411 |
| **22** | CF₃ | Ph | 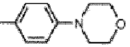 | H | HCl | / | 425 |
| **23** | CF₃ | Ph | 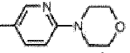 | H | HCl | / | 426 |
| **24** | CF₃ | Ph | 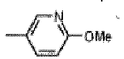 | H | HCl | / | 371 |
| **25** | CF₃ | Ph | 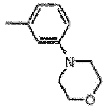 | H | HCl | / | 425 |
| **26** | CF₃ | Ph | 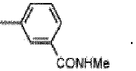 | H | HCl | / | 397 |
| **27** | CF₃ | Ph | 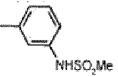 | H | HCl | / | 433 |
| **28** | CF₃ | Ph | 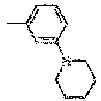 | H | HCl | / | 423 |
| **29** | CF₃ | Ph | 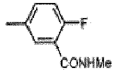 | H | HCl | / | 415 |
| **30** | CF₃ | Ph | 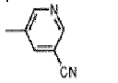 | H | HCl | / | 366 |
| **31** | CHF₂ | Ph | 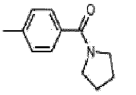 | H | HCl | 248 | 455 |
| **32** | CHF₂ | Ph | 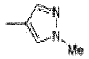 | H | HCl | / | 362 |
| **33** | CHF₂ | Ph | 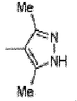 | H | HCl | / | 376 |
| **34** | CHF₂ | Ph | 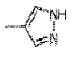 | H | HCl | / | 348 |
| **35** | CHF₂ | Ph | 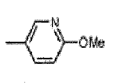 | H | HCl | / | 389 |
| **36** | CHF₂ | Ph | 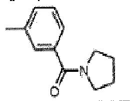 | H | HCl | / | 455 |
| **37** | CHF₂ | Ph | 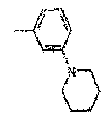 | H | HCl | / | 441 |
| **38 Bsp. 2** | COOH | H | 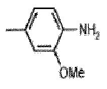 | H | / | / | 285 |
| **39** | CONHMe | Ph | 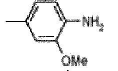 | H | TFA | / | 458 |
| **40** | CONH₂ | Ph | 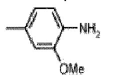 | H | TFA | / | 474 |
| **41** | CONHMe | H | 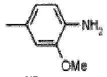 | H | TFA | / | 412 |
| **42** | CONH₂ | H | 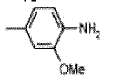 | H | TFA | / | 398 |
| **43** | COOH | Ph | 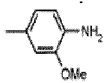 | H | / | / | 361 |
| **44** | COOH | Ph | 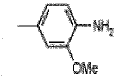 | Me | / | / | 375 |
| **45** | COOH | H | 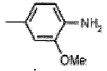 | Me | / | / | 299 |
| **46 Bsp. 1** | CONH₂ | Ph | 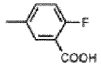 | H | TFA | / | 377 |
| **47** | CONH₂ | Ph | 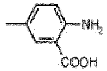 | H | / | / | 374 |
| **48** | CONH₂ | Ph | 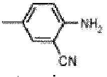 | H | TFA | / | 355 |
| **49** | CONH₂ | 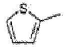 | 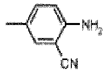 | H | / | / | 361 |
| **50** | COOH | cPr | 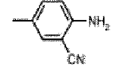 | H | / | / | 320 |
| **51** | CONH₂ | H | 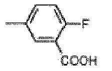 | H | / | / | 301 |
| **52** | CONH₂ | Ph | 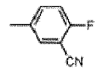 | H | / | / | 358 |
| **53 Bsp. 3** | CHF₂ | Ph | 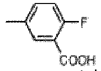 | H | / | / | 384 |
| **54** | CF₃ | Ph | 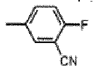 | H | / | / | 383 |
| **55** | CHF₂ | Ph | 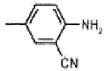 | H | HCl | 282 | 362 |
| **56 Bsp. 4** | CF₃ | Ph | 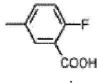 | H | / | / | 402 |
| **57 Bsp. 6** | CF₃ | Ph | 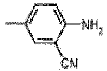 | Me | / | 269 | 394 |
| **58** | CF₃ | Ph | 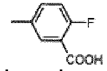 | Me | / | / | 416 |
| **59** | CF₃ | Ph | 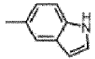 | H | / | / | 379 |
| **60** | CF₃ | Ph | 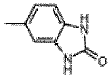 | H | / | 380 | 396 |
| **61 Bsp. 5** | CF₃ | Ph | 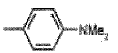 | H | / | 227 | 383 |
| **62** | CONHPh | H | 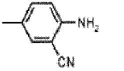 | H | HCl | / | 355 |
| **63** | 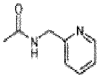 | H | 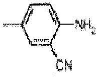 | H | HCl | / | 370 |
| **64** | 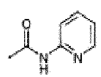 | H | 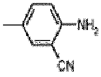 | H | HCl | / | 356 |
| **65** | 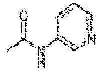 | H | 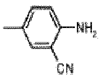 | H | HCl | / | 356 |
| **66** | 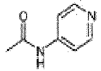 | H | 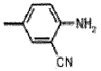 | H | HCl | / | 356 |
| **67** | CHF₂ | 4-Py | 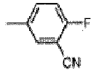 | Me | / | / | 380 |
| **68** | CONHPh | H | 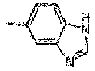 | H | HCl | / | 355 |
| **69** | 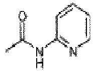 | H | 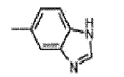 | H | HCl | / | 356 |
| **70** | 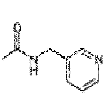 | H | 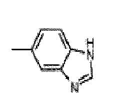 | H | HCl | / | 370 |
| **71** | CONHPh | H | 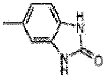 | H | / | / | 371 |
| **72 Bsp. 10** | CHF₂ | H | 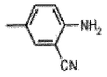 | Me | / | 251 | 300 |
| **73** | CHF₂ | H | 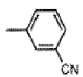 | Me | / | 162 | 285 |
| **74** | CHF₂ | H | 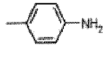 | Me | / | 149 | 275 |
| **75 Bsp. 12** | CHF₂ | H | 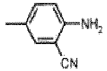 | H | / | 263 | 286 |
| **76** | CF₃ | Ph | 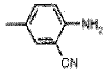 | 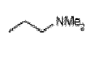 | / | 183 | 451 |
| **77** | CF₃ | Ph | 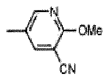 | Me | / | 250 | 410 |
| **78** | CHF₂ | Ph | 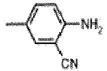 | Me | / | 246 | 376 |
| **79** | CHF₂ | Ph | 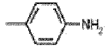 | Me | / | 176 | 351 |
| **80** | CHF₂ | Ph | 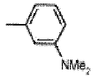 | Me | / | 154 | 379 |
| **81** | CF₃ | Ph | 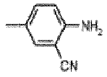 | 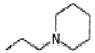 | / | 192 | 491 |
| **82** | CF₃ | Ph | 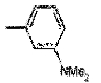 | 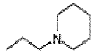 | HCl | 227 | 494 |
| **83 Bsp. 13** | CHF₂ | Ph | 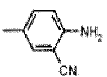 | 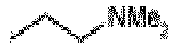 | / | 163 | 433 |
| **84** | CF₃ | H | 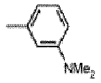 | 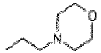 | / | **110** | 420 |
| **85** | CF₃ | H | 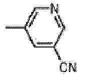 | 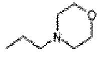 | / | / | 403 |
| **86** | CF₃ | H | 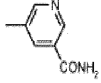 | 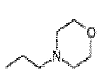 | / | 238 | 421 |
| **87** | CF₃ | H | 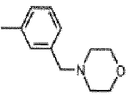 | Me | / | 105 | 377 |
| **88 Bsp. 8** | CF₃ | H | 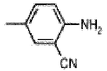 | Me | / | 276 | 318 |
| **89** | CF₃ | Ph | 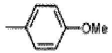 | Me | / | 181 | 384 |
| **90** | CF₃ | H | 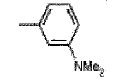 | Me | / | 91 | 321 |
| **91 Bsp. 15** | CHF₂ | 3-Py | 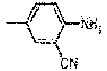 | H | / | 296 | 363 |
| **92** | CHF₂ | 4-Py | 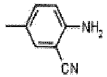 | H | / | 325 | 363 |
| **93 Bsp. 14** | CF₃ | 3-Py | 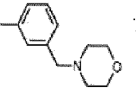 | Me | / | 155 | 454 |
| **94** | CHF₂ | 3MeO-Ph | 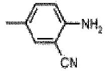 | Me | / | 233 | 392 |
| **95** | CF₃ | H | 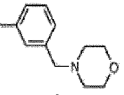 | Pr | HCl | 271 | 405 |
| **96** | CF₃ | H | 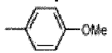 | Pr | / | 72 | 336 |
| **97** | CF₃ | 3MeO-Ph | 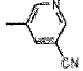 | Me | / | 194 | 410 |
| **98** | CF₃ | 3MeO-Ph | 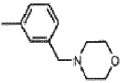 | Me | / | 114 | 483 |
| **99** | CF₃ | 3MeO-PH | 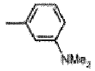 | Me | / | 138 | 427 |
| **100** | CF₃ | 3MeO-Ph | 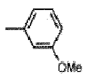 | Me | / | 133 | 414 |
| **101** | CONH₂ | 3-Py | 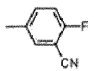 | H | / | / | 377 |

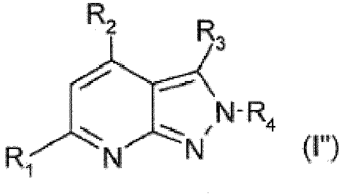
| **Nr.** | **R₂** | **R₃** | **R₁** | **R₄** | **Salz** | **Schmp. (°C)** | **M+H⁺** |
|---|---|---|---|---|---|---|---|
| **102** | COOH | H | 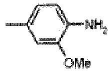 | Me | / | / | 299 |
| **103** | ^{.}CONH₂ | H | 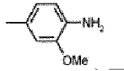 | Me | TFA | / | 412 |
| **104** | COOH | H | 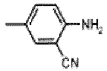 | Me | / | / | 294 |
| **105** | CHF₂ | H | 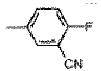 | Me | / | / | 303 |
| **106 Bsp. 11** | CHF₂ | H | 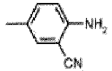 | Me | / | / | 300 |
| **107** | CHF₂ | H | 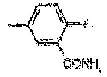 | Me | / | / | 321 |
| **108 Bsp. 7** | CF₃ | Ph | 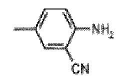 | Me | / | 295 | 394 |
| **109** | CF₃ | Ph | 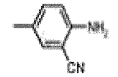 | 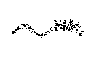 | / | 237 | 451 |
| **110** | CF₃ | **Ph** | 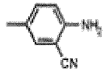 | 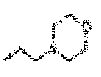 | / | 249 | 493 |
| **111** | CHF₂ | H | 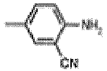 | 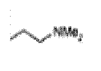 | / | 182 | 357 |
| **112** | CHF₂ | H | 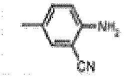 | 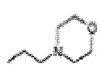 | / | 242 | 399 |
| **113** | CF₃ | H | 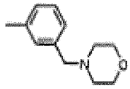 | 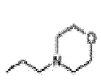 | / | 101 | 476 |
| **114 Bsp.9** | CF₃ | H | 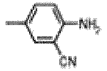 | Me | / | 249 | 318 |
| **115** | CF₃ | H | 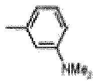 | Pr | HCl | 181 | 425 |
| **116** | CHF₂ | H | 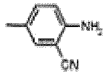 | 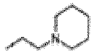 | / | 230 | 397 |
| **117** | CHF₂ | H | 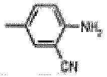 | Pr | / | 214 | 328 |
| **118** | CF₃ | H | 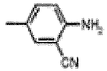 | Pr | / | 239 | 346 |
| **110** | CF₃ | H | 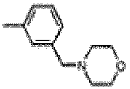 | Pr | / | 288 | 405 |
| **120** | CF₃ | H | 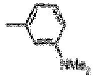 | Pr | / | 89 | 349 |
zur Verwendung als Medikament.

11. Pharmazeutische Zusammensetzung, **dadurch gekennzeichnet, dass** sie eine Verbindung der Formel (I) umfasst 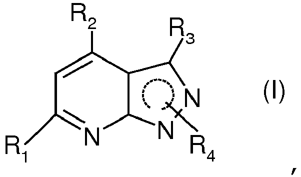 in welcher:
die Darstellung des Pyrazolrings zeigt, dass der Substituent R₄ sich entweder am Stickstoff alpha zum Pyridinring (I') oder am Stickstoff alpha zum einen Substituenten R₃ tragenden Kohlenstoff (I") befinden kann, also: 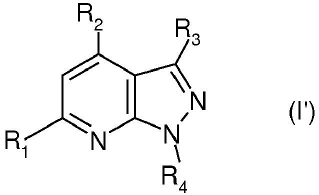 entweder 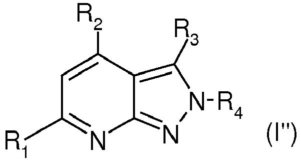
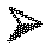 **R₁** für eine Aryl-, Pyridyl- oder Pyrazolylgruppe steht, gegebenenfalls substituiert durch einen oder mehrere Substituenten ausgewählt aus:
∘ einem Halogenatom,
∘ einer -CF₃-Gruppe
∘ einer Cyanogruppe,
∘ einer -NR₆R₆'-Gruppe, in welcher R₆ und R₆' wie unten definiert sind,
∘ einer -NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden, gegebenenfalls substituiert durch einen oder mehrere aus einem Halogenatom und einer geradkettigen oder verzweigten Alkylgruppe ausgewählte Substituenten,
∘ einer -CH₂NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden,
∘ einer -COR₁₂-Gruppe, in welcher R₁₂ für eine Hydroxylgruppe oder eine -NR₆R₆'-Gruppe, in welcher R₆ und R₆' wie unten definiert sind, steht,
∘ einer -CONR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden,
∘ einer -(CH₂)ₚNHSO₂CH₃-Gruppe, in welcher p für 0 oder 1 steht,
∘ einer -OR₁₃-Gruppe, in welcher R₁₃ für eine geradkettige (C₁-C₃)-Alkylgruppe steht,
∘ einer (C₁-C₃)-Alkylgruppe,
oder R₁ für eine bicyclische Gruppe der Formel A unten steht: 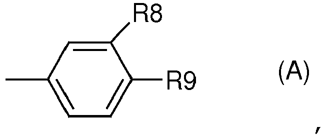 in welcher R₈ und R₉ zusammen mit den Kohlenstoffatomen, an die sie gebunden sind, einen gesättigten oder ungesättigten Heterocyclus mit einem oder mehreren aus einem Stickstoffatom, einem Sauerstoffatom und einem Schwefelatom ausgewählten Heteroatomen bilden, gegebenenfalls substituiert durch eine oder mehrere geradkettige Alkylgruppen,
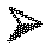 **R₂** für eine Gruppe:
∘ -CF₃,
∘ -CHF₂
oder
∘ -CONHR₅, in welcher R₅ wie unten definiert ist, steht,
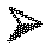 **R₃** für:
∘ ein Wasserstoffatom,
∘ eine Arylgruppe, gegebenenfalls substituiert durch eine Alkoxymethylgruppe,
∘ eine Cycloalkylgruppe
oder
∘ eine Heteroarylgruppe ausgewählt aus Thienyl- und Pyridylgruppen steht,
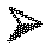 **R₄** für:
∘ ein Wasserstoffatom,
∘ eine geradkettige (C₁-C₃)-Alkylgruppe, gegebenenfalls substituiert durch eine -NR₆R₆'-Gruppe, in welcher R₆ und R₆' wie unten definiert sind, oder eine -NR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden, steht,
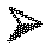 **R₅** für:
∘ ein Wasserstoffatom,
∘ eine geradkettige (C₁-C₃)-Alkylgruppe, gegebenenfalls substituiert durch eine Pyridylgruppe,
oder
∘ eine aromatische Gruppe ausgewählt aus Aryl und Pyridyl steht,
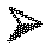 **R₆** und **R₆'**, die gleich oder verschieden sein können, für ein Wasserstoffatom oder eine geradkettige Alkylgruppe stehen,
in Form der Base oder eines Säureadditions- oder Basenadditionssalzes,
und außerdem mindestens einen pharmazeutisch unbedenklichen Exzipienten.

12. Pharmazeutische Zusammensetzung nach dem vorhergehenden Anspruch, **dadurch gekennzeichnet, dass** sie eine aus den folgenden Verbindungen ausgewählte Verbindung umfasst: 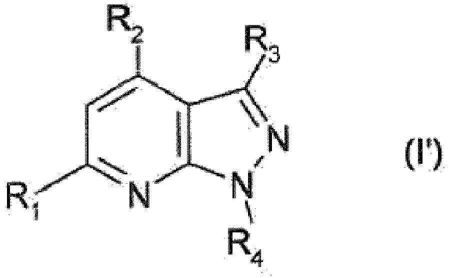
| Nr. | **R₂** | **R₃** | **R₁** | **R₄** | **Salz** | **Schmp.(°C)** | **M+H⁺** |
|---|---|---|---|---|---|---|---|
| **1** | CF₃ | Ph | 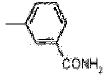 | Me | / | / | 397 |
| **2** | CF₃ | Ph | 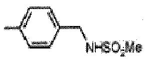 | Me | / | / | 461 |
| **3** | CF₃ | Ph | 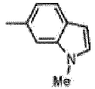 | Me | / | / | 407 |
| **4** | CF₃ | Ph | 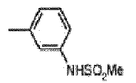 | Me | / | / | 447 |
| **5** | CF₃ | Ph | 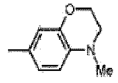 | Me | / | / | 426 |
| **6** | CF₃ | Ph | 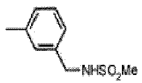 | Me | / | / | 461 |
| **7** | CF₃ | Ph | 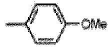 | Me | / | / | 384 |
| **8** | CF₃ | Ph | 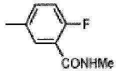 | Me | / | / | 429 |
| **9** | CF₃ | Ph | 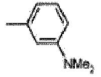 | Me | / | / | 397 |
| **10** | CF₃ | Ph | 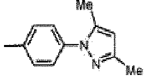 | Me | / | / | 448 |
| **11** | CF₃ | Ph | 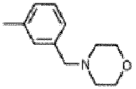 | Me | / | / | 453 |
| **12** | CF₃ | Ph | 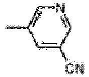 | Me | / | / | 380 |
| **13** | CF₃ | Ph | 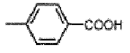 | Me | / | / | 398 |
| **14** | CF₃ | Ph | 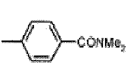 | Me | / | / | 425 |
| **15** | CF₃ | Ph | 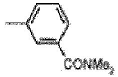 | Me | / | / | 425 |
| **16** | CF₃ | Ph | 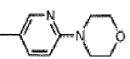 | Me | / | / | 440 |
| **17** | CF₃ | Ph | 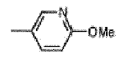 | Me | / | / | 385 |
| **18** | CF₃ | Ph | 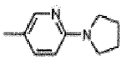 | Me | / | / | 424 |
| **19** | CF₃ | Ph | 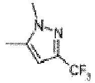 | Me | / | / | 426 |
| **20** | CF₃ | Ph | 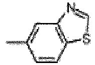 | Me | / | / | 411 |
| **21** | CF₃ | Ph | 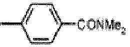 | H | HCl | / | 411 |
| **22** | CF₃ | Ph | 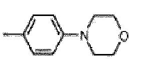 | H | HCl | / | 425 |
| **23** | CF₃ | Ph | 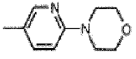 | H | HCl | / | 426 |
| **24** | CF₃ | Ph | 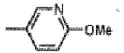 | H | HCl | / | 371 |
| **25** | CF₃ | Ph | 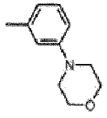 | H | HCl | / | 425 |
| **26** | CF₃ | Ph | 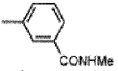 | H | HCl | / | 397 |
| **27** | CF₃ | Ph | 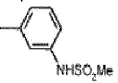 | H | HCl | / | 433 |
| **28** | CF₃ | Ph | 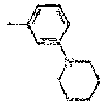 | H | HCl | / | 423 |
| **29** | CF₃ | Ph | 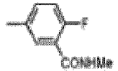 | H | HCl | / | 415 |
| **30** | CF₃ | Ph | 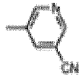 | H | HCl | / | 366 |
| **31** | CHF₂ | Ph | 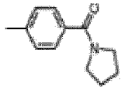 | H | HCl | 248 | 455 |
| **32** | CHF₂ | Ph | 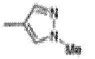 | H | HCl | / | 362 |
| **33** | CHF₂ | Ph | 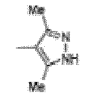 | H | HCl | / | 376 |
| **34** | CHF₂ | Ph | 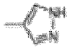 | H | HCl | / | 348 |
| **35** | CHF₂ | Ph | 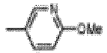 | H | HCl | / | 389 |
| **36** | CHF₂ | Ph | 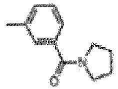 | H | HCl | / | 455 |
| **37** | CHF₂ | Ph | 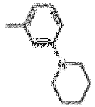 | H | HCl | / | 441 |
| **39** | CONHMe | Ph | 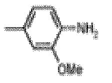 | H | TFA | / | 458 |
| **40** | CONH₂ | Ph | 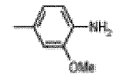 | H | TFA | / | 474 |
| **41** | CONHMe | H | 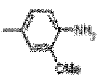 | H | TFA | / | 412 |
| **42** | CONH₂ | H | 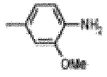 | H | TFA | / | 398 |
| **46 Bsp.1** | CONH₂ | Ph | 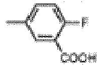 | H | TFA | / | 377 |
| **47** | CONH₂ | Ph | 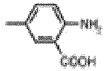 | H | / | / | 374 |
| **48** | CONH₂ | Ph | 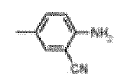 | H | TFA | / | 355 |
| **49** | CONH₂ | 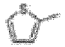 | 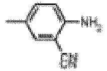 | H | / | / | 361 |
| **51** | CONH₂ | H | 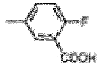 | H | / | / | 301 |
| **52** | CONH₂ | Ph | 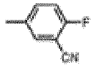 | H | / | / | 358 |
| **53 Bsp.3** | CHF₂ | Ph | 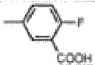 | H | | / | 384 |
| **54** | CF₃ | Ph | 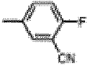 | H | / | / | 383 |
| **55** | CHF₂ | Ph | 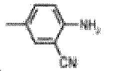 | H | HCl | 282 | 362 |
| **56 Bsp.4** | CF₃ | Ph | 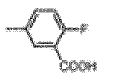 | H | / | / | 402 |
| **57 Bsp. 5** | CF₃ | Ph | 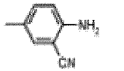 | Me | / | 269 | 394 |
| **58** | CF₃ | Ph | 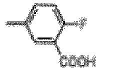 | Me | / | / | 416 |
| **59** | CF₃ | Ph | 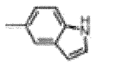 | H | / | / | 379 |
| **60** | CF₃ | Ph | 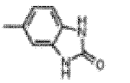 | H | / | / 380 | 396 |
| **61 Bsp.5** | CF₃ | Ph | 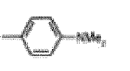 | H | / | 227 | 383 |
| **62** | CONHPh | H | 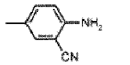 | H | HCl | / | 355 |
| **63** | 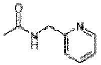 | H | 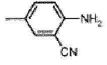 | H | HCl | / | 370 |
| **64** | 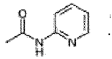 | H | 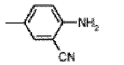 | H | HCl | / | 356 |
| **65** | 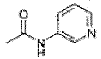 | H | 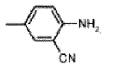 | H | HCl | / | 356 |
| **66** | 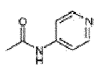 | H | 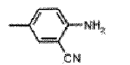 | H | HCl | / | 356 |
| **67** | CHF₂ | 4-Py | 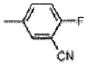 | Me | / | / | 380 |
| **68** | CONHPh | H | 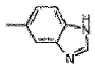 | H | HCl | / | 355 |
| **69** | 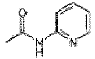 | H | 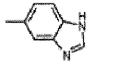 | H | HCl | / | 356 |
| **70** | 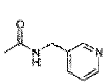 | H | 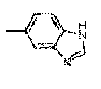 | H | HCl | / | 370 |
| **71** | CONHPh | H | 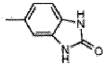 | H | / | / | 371 |
| **72 Bsp. 10** | CHF₂ | H | 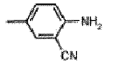 | Me | / | 251 | 300 |
| **73** | CHF₂ | H | 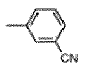 | Me | / | 162 | 285 |
| **74** | CHF₂ | H | 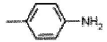 | Me | / | 149 | 275 |
| **75 Bsp. 12** | CHF₂ | H | 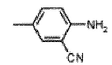 | H | / | 263 | 286 |
| **76** | CF₃ | Ph | 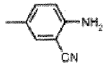 | 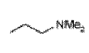 | / | 183 | 45 1 |
| **77** | CF₃ | Ph | 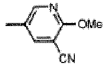 | Me | / | 250 | 410 |
| **78** | CHF₂ | Ph | 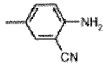 | Me | / | 246 | 376 |
| **79** | CHF₂ | Ph | 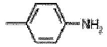 | Me | / | 176 | 351 |
| **80** | CHF₂ | Ph | 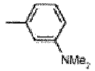 | Me | / | 154 | 379 |
| **81** | CF₃ | Ph | 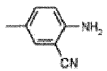 | 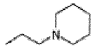 | / | 192 | 491 |
| **82** | CF₃ | Ph | 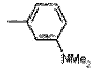 | 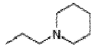 | HCl | 227 | 494 |
| **83 Bsp. 13** | CHF₂ | Ph | 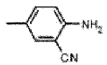 | 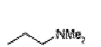 | / | 163 | 433 |
| **84** | CF₃ | H | 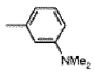 | 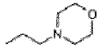 | / | 110 | 420 |
| **85** | CF₃ | H | 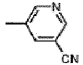 | 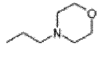 | / | / | 403 |
| **86** | CF₃ | H | 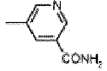 | 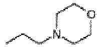 | / | 238 | 421 |
| **87** | CF₃ | H | 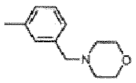 | Me | / | 105 | 377 |
| **88 Bsp. 8** | CF₃ | H | 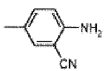 | Me | / | 276 | 318 |
| **89** | CF₃ | Ph | 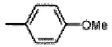 | Me | / | 181 | 384 |
| **90** | CF₃ | H | 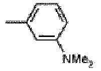 | Me | / | 91 | 321 |
| **91 Bsp. 15** | CHF₂ | 3-Py | 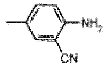 | H | / | 296 | 363 |
| **92** | CHF₂ | 4-Py | 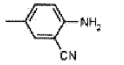 | H | / | 325 | 363 |
| **93 Bsp. 14** | CF₃ | 3-Py | 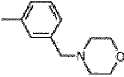 | Me | / | 155 | 454 |
| **94** | CHF₂ | 3MeO-Ph | 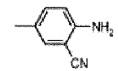 | Me | / | 233 | 392 |
| **95** | CF₃ | H | 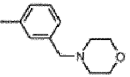 | Pr | HCl | 271 | 405 |
| **96** | CF₃ | H | 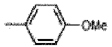 | Pr | / | 72 | 336 |
| **97** | CF₃ | 3MeO-Ph | 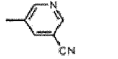 | Me | / | 194 | 410 |
| **98** | CF₃ | 3MeO-Ph | 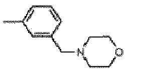 | Me | / | 114 | 483 |
| 99 | CF₃ | 3MeO-Ph | 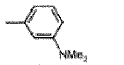 | Me | / | 138 | 427 |
| **100** | CF₃ | 3MeO-Ph | 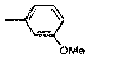 | Me | / | 133 | 414 |
| **101** | CONH₂ | 3-Py | 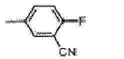 | H | / | / | 377 |

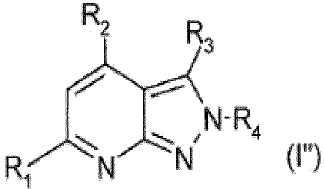
| **Nr.** | **R₂** | **R₃** | **R₁** | **R₄** | **Salz** | **Schmp. (°C)** | **M+H⁺** |
|---|---|---|---|---|---|---|---|
| **102** | COOH | H | 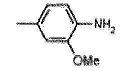 | Me | / | / | 299 |
| **103** | CONH₂ | H | 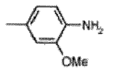 | Me | TFA | / | 412 |
| **104** | COOH | H | 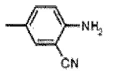 | Me | / | / | 294 |
| **105** | CHF₂ | H | 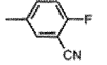 | Me | / | / | 303 |
| **106 Ex. 11** | CHF₂ | H | 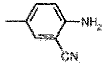 | Me | / | / | 300 |
| **107** | CHF₂ | H | 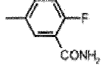 | Me | / | / | 321 |
| **108 Ex. 7** | CF₃ | Ph | 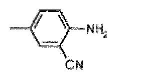 | Me | / | 295 | 394 |
| **109** | CF₃ | Ph | 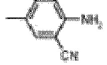 | 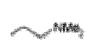 | / | 237 | 451 |
| **110** | CF₃ | Ph | 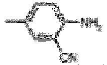 | 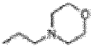 | / | 249 | 493 |
| **111** | CHF₂ | H | 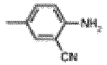 | 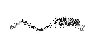 | / | 182 | 357 |
| **112** | CHF₂ | H | 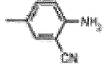 | 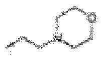 | / | 242 | 399 |
| **113** | CF₃ | H | 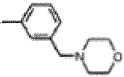 | 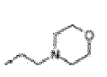 | / | 101 | 476 |
| **114 Bsp.9** | CF₃ | H | 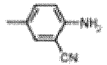 | Me | / | 249 | 318 |
| **115** | CF₃ | H | 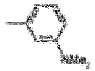 | Pr | HCl | 181 | 425 |
| **116** | CHF₂ | H | 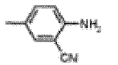 | 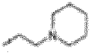 | / | 230 | 397 |
| **117** | CHF₂ | H | 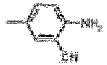 | Pr | / | 214 | 328 |
| **118** | CF₃ | H | 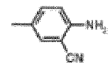 | Pr | / | 239 | 346 |
| **119** | CF₃ | H | 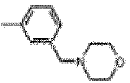 | Pr | / | 288 | 405 |
| **120** | CF₃ | H | 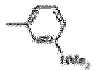 | Pr | / | 89 | 349 |
und außerdem mindestens einen pharmazeutisch unbedenklichen Exzipienten.

13. Verwendung einer Verbindung der Formel (I) 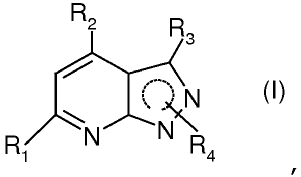 in welcher: die Darstellung des Pyrazolrings zeigt, dass der Substituent R₄ sich entweder am Stickstoff alpha zum Pyridinring (I') oder am Stickstoff alpha zum einen Substituenten R₃ tragenden Kohlenstoff (I") befinden kann, also: 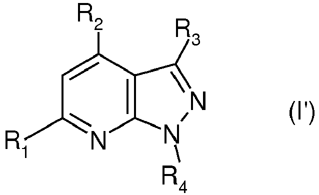 entweder 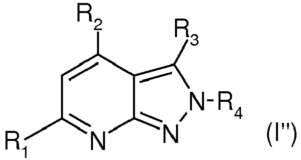
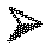 **R₁** für eine Aryl-, Pyridyl- oder Pyrazolylgruppe steht, gegebenenfalls substituiert durch einen oder mehrere Substituenten ausgewählt aus:
∘ einem Halogenatom,
∘ einer -CF₃-Gruppe
∘ einer Cyanogruppe,
∘ einer -NR₆R₆'-Gruppe, in welcher R₆ und R₆' wie unten definiert sind,
∘ einer -NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden, gegebenenfalls substituiert durch einen oder mehrere aus einem Halogenatom und einer geradkettigen oder verzweigten Alkylgruppe ausgewählte Substituenten,
∘ einer -CH₂NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden,
∘ einer -COR₁₂-Gruppe, in welcher R₁₂ für eine Hydroxylgruppe oder eine -NR₆R₆'-Gruppe, in welcher R₆ und R₆' wie unten definiert sind, steht,
∘ einer -CONR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden,
∘ einer -(CH₂)ₚNHSO₂CH₃-Gruppe, in welcher p für 0 oder 1 steht,
∘ einer -OR₁₃-Gruppe, in welcher R₁₃ für eine geradkettige (C₁-C₃)-Alkylgruppe steht,
∘ einer (C₁-C₃) -Alkylgruppe,
oder R₁ für eine bicyclische Gruppe der Formel A unten steht: 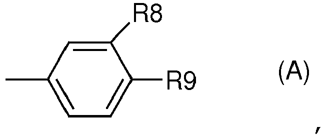 in welcher R₈ und R₉ zusammen mit den Kohlenstoffatomen, an die sie gebunden sind, einen gesättigten oder ungesättigten Heterocyclus mit einem oder mehreren aus einem Stickstoffatom, einem Sauerstoffatom und einem Schwefelatom ausgewählten Heteroatomen bilden, gegebenenfalls substituiert durch eine oder mehrere geradkettige Alkylgruppen,
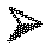 **R₂** für eine Gruppe:
∘ -CF₃,
∘ -CHF₂,
∘ -COOH
oder
∘ -CONHR₅, in welcher R₅ wie unten definiert ist, steht,
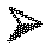 **R₃** für:
∘ ein Wasserstoffatom,
∘ eine Arylgruppe, gegebenenfalls substituiert durch eine Alkoxymethylgruppe,
∘ eine Cycloalkylgruppe
oder
∘ eine Heteroarylgruppe ausgewählt aus Thienyl- und Pyridylgruppen steht,
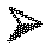 **R₄** für:
∘ ein Wasserstoffatom,
∘ eine geradkettige (C₁-C₃)-Alkylgruppe, gegebenenfalls substituiert durch eine -NR₆R₆'-Gruppe, in welcher R₆ und R₆' wie unten definiert sind, oder eine -NR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden, steht,
**R₅** für:
∘ ein Wasserstoffatom,
∘ eine geradkettige (C₁-C₃)-Alkylgruppe, gegebenenfalls substituiert durch eine Pyridylgruppe, oder
∘ eine aromatische Gruppe ausgewählt aus Aryl und Pyridyl steht,
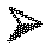 **R₆** und **R₆'**, die gleich oder verschieden sein können, für ein Wasserstoffatom oder eine geradkettige Alkylgruppe stehen,
in Form der Base oder eines Säureadditions- oder Basenadditionssalzes,
zur Herstellung eines Medikaments zur Behandlung und Prävention von Krankheiten, bei denen eine Modulation der b-FGFs erforderlich ist.

14. Kombination einer Verbindung 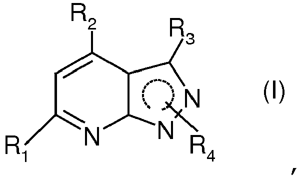 in welcher:
die Darstellung des Pyrazolrings zeigt, dass der Substituent R₄ sich entweder am Stickstoff alpha zum Pyridinring (I') oder am Stickstoff alpha zum einen Substituenten R₃ tragenden Kohlenstoff (I") befinden kann, also: 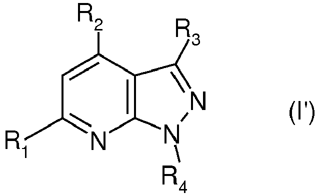 entweder 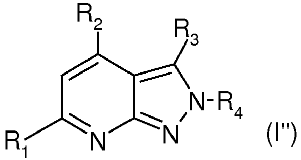
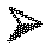 **R₁** für eine Aryl-, Pyridyl- oder Pyrazolylgruppe steht, gegebenenfalls substituiert durch einen oder mehrere Substituenten ausgewählt aus:
∘ einem Halogenatom,
∘ einer -CF₃-Gruppe
∘ einer Cyanogruppe,
∘ einer -NR₆R₆'-Gruppe, in welcher R₆ und R₆' wie unten definiert sind,
∘ einer -NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden, gegebenenfalls substituiert durch einen oder mehrere aus einem Halogenatom und einer geradkettigen oder verzweigten Alkylgruppe ausgewählte Substituenten,
∘ einer -CH₂NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden,
∘ einer -COR₁₂-Gruppe, in welcher R₁₂ für eine Hydroxylgruppe oder eine -NR₆R₆'-Gruppe, in welcher R₆ und R₆' wie unten definiert sind, steht,
∘ einer -CONR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden,
∘ einer -(CH₂)ₚNHSO₂CH₃-Gruppe, in welcher p für 0 oder 1 steht,
∘ einer -OR₁₃-Gruppe, in welcher R₁₃ für eine geradkettige (C₁-C₃)-Alkylgruppe steht,
∘ einer (C₁-C₃)-Alkylgruppe,
oder R₁ für eine bicyclische Gruppe der Formel A unten steht: 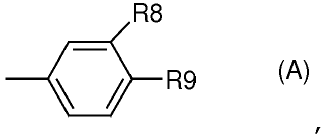 in welcher R₈ und R₉ zusammen mit den Kohlenstoffatomen, an die sie gebunden sind, einen gesättigten oder ungesättigten Heterocyclus mit einem oder mehreren aus einem Stickstoffatom, einem Sauerstoffatom und einem Schwefelatom ausgewählten Heteroatomen bilden, gegebenenfalls substituiert durch eine oder mehrere geradkettige Alkylgruppen,
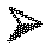 **R₂** für eine Gruppe:
∘ -CF₃,
∘ -CHF₂,
∘ -COOH
oder
∘ -CONHR₅, in welcher R₅ wie unten definiert ist, steht,
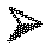 **R₃** für:
∘ ein Wasserstoffatom,
∘ eine Arylgruppe, gegebenenfalls substituiert durch eine Alkoxymethylgruppe,
∘ eine Cycloalkylgruppe
oder
∘ eine Heteroarylgruppe ausgewählt aus Thienyl- und Pyridylgruppen steht,
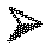 **R₄** für:
∘ ein Wasserstoffatom,
∘ eine geradkettige (C₁-C₃)-Alkylgruppe, gegebenenfalls substituiert durch eine -NR₆R₆'-Gruppe, in welcher R₆ und R₆' wie unten definiert sind, oder eine -NR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden, steht,
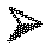 **R₅** für:
∘ ein Wasserstoffatom,
∘ eine geradkettige (C₁-C₃)-Alkylgruppe, gegebenenfalls substituiert durch eine Pyridylgruppe,
oder
∘ eine aromatische Gruppe ausgewählt aus Aryl und Pyridyl steht,
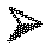 **R₆** und **R₆'**, die gleich oder verschieden sein können, für ein Wasserstoffatom oder eine geradkettige Alkylgruppe stehen,
in Form der Base oder eines Säureadditions- oder Basenadditionssalzes,
mit einem oder mehreren gegen Krebs wirkenden Prinzipien und/oder mit einem Anti-VEGF.

15. Verbindung 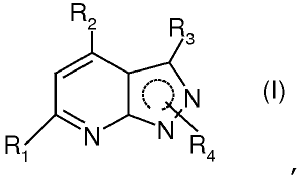 in welcher:
die Darstellung des Pyrazolrings zeigt, dass der Substituent R₄ sich entweder am Stickstoff alpha zum Pyridinring (I') oder am Stickstoff alpha zum einen Substituenten R₃ tragenden Kohlenstoff (I") befinden kann, also: 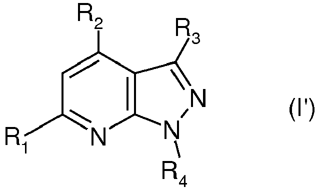 entweder 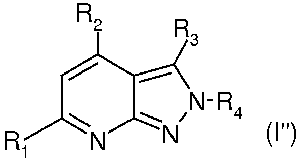
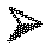 **R₁** für eine Aryl-, Pyridyl- oder Pyrazolylgruppe steht, gegebenenfalls substituiert durch einen oder mehrere Substituenten ausgewählt aus:
∘ einem Halogenatom,
∘ einer -CF₃-Gruppe
∘ einer Cyanogruppe,
∘ einer -NR₆R₆'-Gruppe, in welcher R₆ und R₆' wie unten definiert sind,
∘ einer -NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden, gegebenenfalls substituiert durch einen oder mehrere aus einem Halogenatom und einer geradkettigen oder verzweigten Alkylgruppe ausgewählte Substituenten,
∘ einer -CH₂NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden,
∘ einer -COR₁₂-Gruppe, in welcher R₁₂ für eine Hydroxylgruppe oder eine -NR₆R₆'-Gruppe, in welcher R₆ und R₆' wie unten definiert sind, steht,
∘ einer -CONR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden,
∘ einer -(CH₂)ₚNHSO₂CH₃-Gruppe, in welcher p für 0 oder 1 steht,
∘ einer -OR₁₃-Gruppe, in welcher R₁₃ für eine geradkettige (C₁-C₃)-Alkylgruppe steht,
∘ einer (C₁-C₃)-Alkylgruppe,
oder R₁ für eine bicyclische Gruppe der Formel A unten steht: 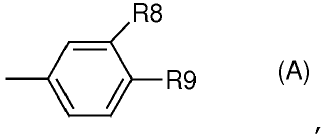 in welcher R₈ und R₉ zusammen mit den Kohlenstoffatomen, an die sie gebunden sind, einen gesättigten oder ungesättigten Heterocyclus mit einem oder mehreren aus einem Stickstoffatom, einem Sauerstoffatom und einem Schwefelatom ausgewählten Heteroatomen bilden, gegebenenfalls substituiert durch eine oder mehrere geradkettige Alkylgruppen,
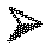 **R₂** für eine Gruppe:
∘ -CF₃,
∘ -CHF₂,
∘ -COOH
oder
∘ -CONHR₅, in welcher R₅ wie unten definiert ist, steht,
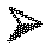 **R₃** für:
∘ ein Wasserstoffatom,
∘ eine Arylgruppe, gegebenenfalls substituiert durch eine Alkoxymethylgruppe,
∘ eine Cycloalkylgruppe
oder
∘ eine Heteroarylgruppe ausgewählt aus Thienyl- und Pyridylgruppen steht,
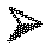 **R₄** für:
∘ ein Wasserstoffatom,
∘ eine geradkettige (C₁-C₃)-Alkylgruppe, gegebenenfalls substituiert durch eine -NR₆R₆'-Gruppe, in welcher R₆ und R₆' wie unten definiert sind, oder eine -NR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden, steht,
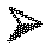 **R₅** für:
∘ ein Wasserstoffatom,
∘ eine geradkettige (C₁-C₃)-Alkylgruppe, gegebenenfalls substituiert durch eine Pyridylgruppe, oder
∘ eine aromatische Gruppe ausgewählt aus Aryl und Pyridyl steht,
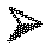 **R₆** und **R₆'**, die gleich oder verschieden sein können, für ein Wasserstoffatom oder eine geradkettige Alkylgruppe stehen,
in Form der Base oder eines Säureadditions- oder Basenadditionssalzes,
zur Verwendung bei der Behandlung und Prävention von Krebserkrankungen, insbesondere Karzinomen mit einem beträchtlichen Vaskularisierungsgrad wie Lungen-, Brust-, Prostata-, Bauchspeicheldrüsen-, Darm-, Nieren- und Speiseröhrenkarzinomen, metastaseninduzierenden Krebserkrankungen wie Darmkrebs, Leberkrebs und Magenkrebs, Melanomen, Gliomen, Lymphomen und Leukämien sowie Thrombopenien.

16. Verbindung 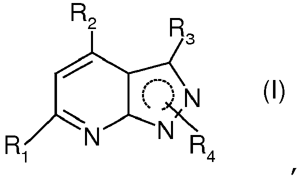 in welcher:
die Darstellung des Pyrazolrings zeigt, dass der Substituent R₄ sich entweder am Stickstoff alpha zum Pyridinring (I') oder am Stickstoff alpha zum einen Substituenten R₃ tragenden Kohlenstoff (I") befinden kann, also: 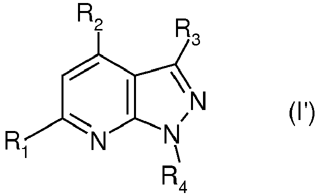 entweder 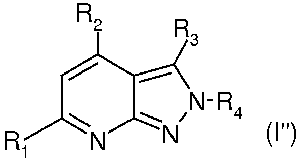
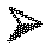 **R₁** für eine Aryl-, Pyridyl- oder Pyrazolylgruppe steht, gegebenenfalls substituiert durch einen oder mehrere Substituenten ausgewählt aus:
∘ einem Halogenatom,
∘ einer -CF₃-Gruppe
∘ einer Cyanogruppe,
∘ einer -NR₆R₆'-Gruppe, in welcher R₆ und R₆' wie unten definiert sind,
∘ einer -NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden, gegebenenfalls substituiert durch einen oder mehrere aus einem Halogenatom und einer geradkettigen oder verzweigten Alkylgruppe ausgewählte Substituenten,
∘ einer -CH₂NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden,
∘ einer -COR₁₂-Gruppe, in welcher R₁₂ für eine Hydroxylgruppe oder eine -NR₆R₆'-Gruppe, in welcher R₆ und R₆' wie unten definiert sind, steht,
∘ einer -CONR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden,
∘ einer -(CH₂)ₚNHSO₂CH₃-Gruppe, in welcher p für 0 oder 1 steht,
∘ einer -OR₁₃-Gruppe, in welcher R₁₃ für eine geradkettige (C₁-C₃)-Alkylgruppe steht,
∘ einer (C₁-C₃)-Alkylgruppe,
oder R₁ für eine bicyclische Gruppe der Formel A unten steht: 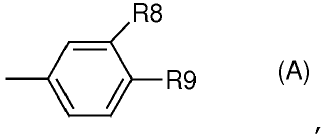 in welcher R₈ und R₉ zusammen mit den Kohlenstoffatomen, an die sie gebunden sind, einen gesättigten oder ungesättigten Heterocyclus mit einem oder mehreren aus einem Stickstoffatom, einem Sauerstoffatom und einem Schwefelatom ausgewählten Heteroatomen bilden, gegebenenfalls substituiert durch eine oder mehrere geradkettige Alkylgruppen,
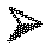 **R₂** für eine Gruppe:
∘ -CF₃,
∘ -CHF₂,
∘ -COOH
oder
∘ -CONHR₅, in welcher R₅ wie unten definiert ist, steht,
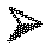 **R₃** für:
∘ ein Wasserstoffatom,
∘ eine Arylgruppe, gegebenenfalls substituiert durch eine Alkoxymethylgruppe,
∘ eine Cycloalkylgruppe
oder
∘ eine Heteroarylgruppe ausgewählt aus Thienyl- und Pyridylgruppen steht,
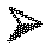 **R₄** für:
∘ ein Wasserstoffatom,
∘ eine geradkettige (C₁-C₃)-Alkylgruppe, gegebenenfalls substituiert durch eine -NR₆R₆'-Gruppe, in welcher R₆ und R₆' wie unten definiert sind, oder eine -NR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden, steht,
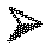 **R₅** für:
∘ ein Wasserstoffatom,
∘ eine geradkettige (C₁-C₃)-Alkylgruppe, gegebenenfalls substituiert durch eine Pyridylgruppe,
oder
∘ eine aromatische Gruppe ausgewählt aus Aryl und Pyridyl steht,
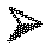 **R₆** und **R₆'**, die gleich oder verschieden sein können, für ein Wasserstoffatom oder eine geradkettige Alkylgruppe stehen,
in Form der Base oder eines Säureadditions- oder Basenadditionssalzes,
zur Verwendung bei der Behandlung und Prävention von Krebserkrankungen, insbesondere Karzinomen mit einem beträchtlichen Vaskularisierungsgrad wie Lungen-, Brust-, Prostata-, Bauchspeicheldrüsen-, Darm-, Nieren- und Speiseröhrenkarzinomen, metastaseninduzierenden Krebserkrankungen wie Darmkrebs, Leberkrebs und Magenkrebs, Melanomen, Gliomen, Lymphomen und Leukämien sowie Thrombopenien,
wobei die Verbindung der Formel (I) in Kombination mit einem oder mehreren gegen Krebs wirkenden Prinzipien und/oder mit einem Anti-VEGF verabreicht wird und/oder in Kombination mit Strahlentherapie verabreicht wird.

17. Verbindung 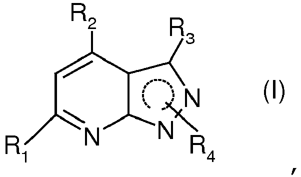 in welcher:
die Darstellung des Pyrazolrings zeigt, dass der Substituent R₄ sich entweder am Stickstoff alpha zum Pyridinring (I') oder am Stickstoff alpha zum einen Substituenten R₃ tragenden Kohlenstoff (I") befinden kann, also: 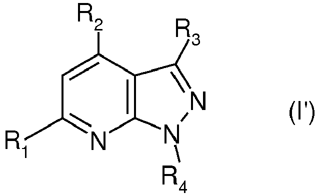 entweder 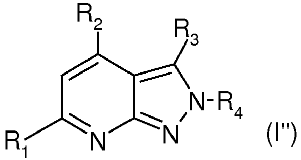
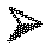 **R₁** für eine Aryl-, Pyridyl- oder Pyrazolylgruppe steht, gegebenenfalls substituiert durch einen oder mehrere Substituenten ausgewählt aus:
∘ einem Halogenatom,
∘ einer -CF₃-Gruppe
∘ einer Cyanogruppe,
∘ einer -NR₆R₆'-Gruppe, in welcher R₆ und R₆' wie unten definiert sind,
∘ einer -NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden, gegebenenfalls substituiert durch einen oder mehrere aus einem Halogenatom und einer geradkettigen oder verzweigten Alkylgruppe ausgewählte Substituenten,
∘ einer -CH₂NR₁₀R₁₁-Gruppe, in welcher R₁₀ und R₁₁ zusammen mit dem Stickstoffatom, an das sie gebunden sind, einen gesättigten oder ungesättigten Heterocyclus mit einem oder mehreren aus einem Stickstoffatom und einem Sauerstoffatom ausgewählten Heteroatomen bilden,
∘ einer -COR₁₂-Gruppe, in welcher R₁₂ für eine Hydroxylgruppe oder eine -NR₆R₆'-Gruppe, in welcher R₆ und R₆' wie unten definiert sind, steht,
∘ einer -CONR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden,
∘ einer -(CH₂)ₚNHSO₂CH₃-Gruppe, in welcher p für 0 oder 1 steht,
∘ einer -OR₁₃-Gruppe, in welcher R₁₃ für eine geradkettige (C₁-C₃)-Alkylgruppe steht,
∘ einer (C₁-C₃)-Alkylgruppe,
oder R₁ für eine bicyclische Gruppe der Formel A unten steht: 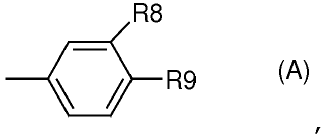 in welcher R₈ und R₉ zusammen mit den Kohlenstoffatomen, an die sie gebunden sind, einen gesättigten oder ungesättigten Heterocyclus mit einem oder mehreren aus einem Stickstoffatom, einem Sauerstoffatom und einem Schwefelatom ausgewählten Heteroatomen bilden, gegebenenfalls substituiert durch eine oder mehrere geradkettige Alkylgruppen,
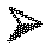 **R₂** für eine Gruppe:
∘ -CF₃,
∘ -CHF₂,
∘ -COOH
oder
∘ -CONHR₅, in welcher R₅ wie unten definiert ist, steht,
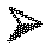 **R₃** für:
∘ ein Wasserstoffatom,
∘ eine Arylgruppe, gegebenenfalls substituiert durch eine Alkoxymethylgruppe,
∘ eine Cycloalkylgruppe
oder
∘ eine Heteroarylgruppe ausgewählt aus Thienyl- und Pyridylgruppen steht,
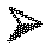 **R₄** für:
∘ ein Wasserstoffatom,
∘ eine geradkettige (C₁-C₃)-Alkylgruppe, gegebenenfalls substituiert durch eine -NR₆R₆'-Gruppe, in welcher R₆ und R₆' wie unten definiert sind, oder eine -NR₇R₇'-Gruppe, in welcher R₇ und R₇' zusammen mit dem Stickstoffatom, an das sie gebunden sind, ein Heterocycloalkyl mit einem oder mehreren aus einem Stickstoffatom und einem Sauerstoffatom ausgewählten Heteroatomen bilden, steht,
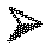 **R₅** für:
∘ ein Wasserstoffatom,
∘ eine geradkettige (C₁-C₃)-Alkylgruppe, gegebenenfalls substituiert durch eine Pyridylgruppe,
oder
∘ eine aromatische Gruppe ausgewählt aus Aryl und Pyridyl steht,
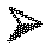 **R₆** und **R₆'**, die gleich oder verschieden sein können, für ein Wasserstoffatom oder eine geradkettige Alkylgruppe stehen,
in Form der Base oder eines Säureadditions- oder Basenadditionssalzes,
zur Verwendung bei der Behandlung und Prävention von Herz-Kreislauf-Krankheiten wie Atherosklerose, Post-Angioplastie-Restenose, mit Komplikationen nach dem Einführen von endovaskulären Prothesen und/oder aortokoronaren Bypässen oder anderen Gefäßtransplantaten assoziierten Krankheiten, kardialer Hypotrophie, vaskulären Komplikationen von Diabetes wie diabetischer Retinopathie, Leber-, Nieren- und Lungenfibrosen, neuropathischen Schmerzen, chronischen entzündlichen Krankheiten wie rheumatoider Arthritis oder IBD, Prostatahyperplasie, Psoriasis, Klarzellakanthom, Osteoarthritis, Achondroplasien (ACH), Hypochondroplasien (HCH), TD (thanatophorer Dysplasie), Obesitas und Makuladegeneration wie altersbedingter Makuladegeneration (AMD).

18. Verbindung zur Verwendung nach einem der Ansprüche 15 bis 17, wobei die Verbindung aus den folgenden Verbindungen ausgewählt ist: 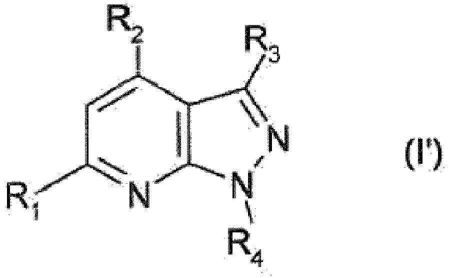
| Nr. | **R₂** | **R₃** | **R₁** | **R₄** | **Salz** | **Schmp.(°C)** | **M+H⁺** |
|---|---|---|---|---|---|---|---|
| **1** | CF₃ | Ph | 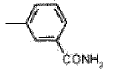 | Me | / | / | 397 |
| **2** | CF₃ | Ph | 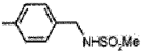 | Me | / | / | 461 |
| **3** | CF₃ | Ph | 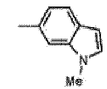 | Me | / | / | 407 |
| **4** | CF₃ | Ph | 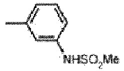 | Me | / | / | 447 |
| **5** | CF₃ | Ph | 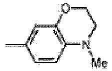 | Me | / | / | 426 |
| **6** | CF₃ | Ph | 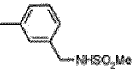 | Me | / | / | 461 |
| **7** | CF₃ | Ph | 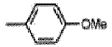 | Me | / | / | 384 |
| **8** | CF₃ | Ph | 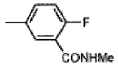 | Me | / | / | 429 |
| **9** | CF₃ | Ph | 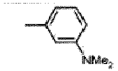 | Me | / | / | 397 |
| **10** | CF₃ | Ph | 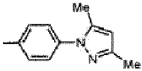 | Me | / | / | 448 |
| **11** | CF₃ | Ph | 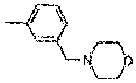 | Me | / | / | 453 |
| **12** | CF₃ | Ph | 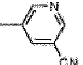 | Me | / | / | 380 |
| **13** | CF₃ | Ph | 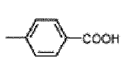 | Me | / | / | 398 |
| **14** | CF₃ | Ph | 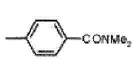 | Me | / | / | 425 |
| **15** | CF₃ | Ph | 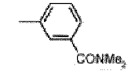 | Me | / | / | 425 |
| **16** | CF₃ | Ph | 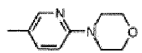 | Me | / | / | 440 |
| **17** | CF₃ | Ph | 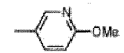 | Me | / | / | 385 |
| **18** | CF₃ | Ph | 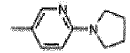 | Me | / | / | 424 |
| **19** | CF₃ | Ph | 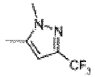 | Me | / | / | 426 |
| **20** | CF₃ | Ph | 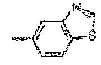 | Me | / | / | 411 |
| **21** | CF₃ | Ph | 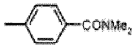 | H | HCl | / | 411 |
| **22** | CF₃ | Ph | 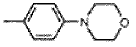 | H | HCl | / | 425 |
| **23** | CF₃ | Ph | 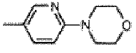 | H | HCl | / | 426 |
| **24** | CF₃ | Ph | 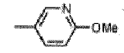 | H | HCl | / | 371 |
| **25** | CF₃ | Ph | 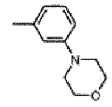 | H | HCl | / | 425 |
| **26** | CF₃ | Ph | 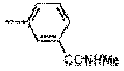 | H | HCl | / | 397 |
| **27** | CF₃ | Ph | 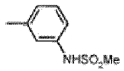 | H | HCl | / | 433 |
| **28** | CF₃ | Ph | 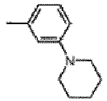 | H | HCl | / | 423 |
| **29** | CF₃ | Ph | 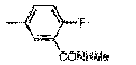 | H | HCl | / | 415 |
| **30** | CF₃ | Ph | 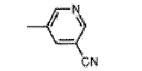 | H | HCl | / | 366 |
| **31** | CHF₂ | Ph | 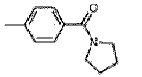 | H | HCl | 248 | 455 |
| **32** | CHF₂ | Ph | 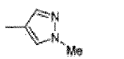 | H | HCl | / | 362 |
| **33** | CHF₂ | Ph | 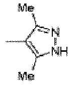 | H | HCl | / | 376 |
| **34** | CHF₂ | Ph | 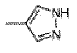 | H | HCl | / | 348 |
| **35** | CHF₂ | Ph | 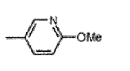 | H | HCl | / | 389 |
| **36** | CHF₂ | Ph | 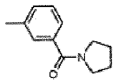 | H | HCl | / | 455 |
| **37** | CHF₂ | Ph | 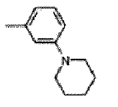 | H | HCl | / | 441 |
| **38 Bsp. 2** | COOH | H | 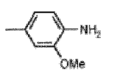 | H | / | / | 285 |
| **39** | CONHMe | Ph | 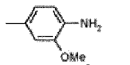 | H | TFA | / | 458 |
| **40** | CONH₂ | Ph | 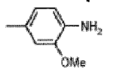 | H | TFA | / | 474 |
| **41** | CONHMe | H | 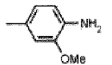 | H | TFA | / | 412 |
| **42** | CONH₂ | H | 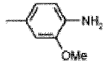 | H | TFA | / | 398 |
| **43** | COOH | Ph | 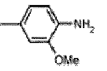 | H | / | / | 361 |
| **44** | COOH | Ph | 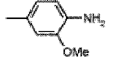 | Me | / | / | 375 |
| **45** | COOH | H | 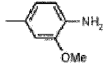 | Me | / | / | 299 |
| **46 Bsp. 1** | CONH₂ | Ph | 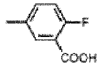 | H | TFA | / | 377 |
| **47** | CONH₂ | Ph | 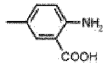 | H | / | / | 374 |
| **48** | CONH₂ | Ph | 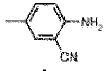 | H | TFA | / | 355 |
| **49** | CONH₂ | 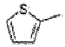 | 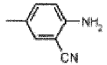 | H | / | / | 351 |
| **50** | COOH | cPr | 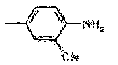 | H | / | / | 320 |
| **51** | CONH₂ | H | 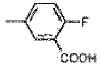 | H | / | / | 301 |
| **52** | CONH₂ | Ph | 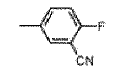 | H | / | / | 358 |
| **53 Bsp. 3** | CHF₂ | Ph | 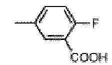 | H | / | / | 384 |
| **54** | CF₃ | Ph | 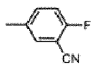 | H | / | / | 383 |
| **55** | CHF₂ | Ph | 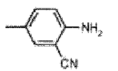 | H | HCl | 282 | 362 |
| **56 Bsp. 4** | CF₃ | Ph | 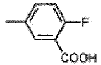 | H | / | / | 402 |
| **57 Bsp. 6** | CF₃ | Ph | 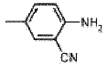 | Me | / | 269 | 394 |
| **58** | CF₃ | Ph | 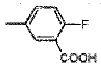 | Me | / | / | 416 |
| **59** | CF₃ | Ph | 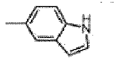 | H | / | / | 379 |
| **60** | CF₃ | Ph | 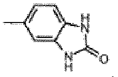 | H | / | 380 | 396 |
| **61 Bsp. 5** | CF₃ | Ph | 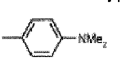 | H | / | 227 | 383 |
| **62** | CONHPh | H | 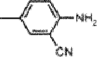 | H | HCl | / | 355 |
| **63** | 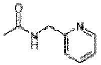 | H | 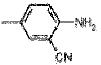 | H | HCl | / | 370 |
| **64** | 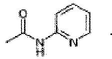 | H | 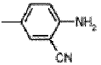 | H | HCl | / | 356 |
| **65** | 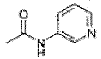 | H | 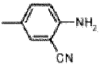 | H | HCl | / | 356 |
| **66** | 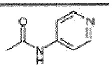 | H | 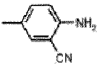 | H | HCl | / | 356 |
| **67** | CHF₂ | 4-Py | 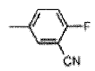 | Me | / | / | 380 |
| **68** | CONHPh | H | 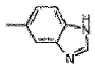 | H | HCl | / | 355 |
| **69** | 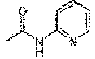 | H | 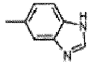 | H | HCl | / | 356 |
| **70** | 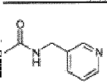 | H | 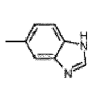 | H | HCl | / | 370 |
| **71** | CONHPh | H | 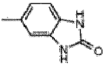 | H | / | / | 371 |
| **72 Bsp. 10** | CHF₂ | H | 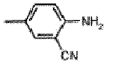 | Me | / | 251 | 300 |
| **73** | CHF₂ | H | 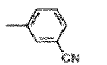 | Me | / | 162 | 285 |
| **74** | CHF₂ | H | 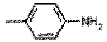 | Me | / | 149 | 275 |
| **75 Bsp. 12** | CHF₂ | H | 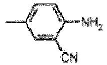 | H | / | 263 | 286 |
| **76** | CF₃ | Ph | 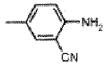 | 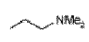 | / | 183 | 45 1 |
| **77** | CF₃ | Ph | 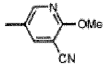 | Me | / | 250 | 410 |
| **78** | CHF₂ | Ph | 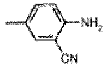 | Me | / | 246 | 376 |
| **79** | CHF₂ | Ph | 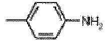 | Me | / | 176 | 351 |
| **80** | CHF₂ | Ph | 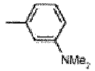 | Me | / | 154 | 379 |
| **81** | CF₃ | Ph | 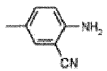 | 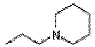 | / | 192 | 491 |
| **82** | CF₃ | Ph | 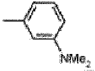 | 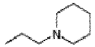 | HCl | 227 | 494 |
| **83 Bsp. 13** | CHF₂ | Ph | 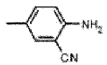 | 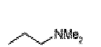 | / | 163 | 433 |
| **84** | CF₃ | H | 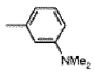 | 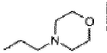 | / | 110 | 420 |
| **85** | CF₃ | H | 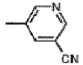 | 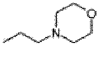 | / | / | 403 |
| **86** | CF₃ | H | 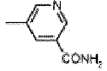 | 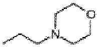 | / | 238 | 421 |
| **87** | CF₃ | H | 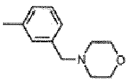 | Me | / | 105 | 377 |
| **88 Bsp. 8** | CF₃ | H | 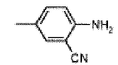 | Me | / | 276 | 318 |
| **89** | CF₃ | Ph | 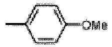 | Me | / | 181 | 384 |
| **90** | CF₃ | H | 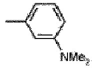 | Me | / | 91 | 321 |
| **91 Bsp. 15** | CHF₂ | 3-Py | 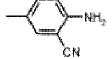 | H | / | 296 | 363 |
| **92** | CHF₂ | 4-Py | 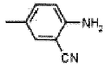 | H | / | 325 | 363 |
| **93 Bsp. 14** | CF₃ | 3-Py | 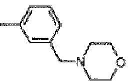 | Me | / | 155 | 454 |
| **94** | CHF₂ | 3MeO-Ph | 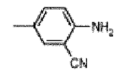 | Me | / | 233 | 392 |
| **95** | CF₃ | H | 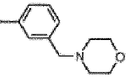 | Pr | HCl | 271 | 405 |
| **96** | CF₃ | H | 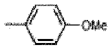 | Pr | / | 72 | 336 |
| **97** | CF₃ | 3MeO-Ph | 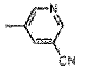 | Me | / | 194 | 410 |
| **98** | CF₃ | 3MeO-Ph | 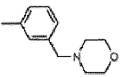 | Me | / | 114 | 483 |
| **99** | CF₃ | 3MeO-Ph | 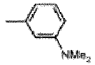 | Me | / | 138 | 427 |
| **100** | CF₃ | 3MeO-Ph | 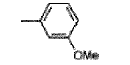 | Me | / | 133 | 414 |
| **101** | CONH₂ | 3-Py | 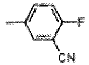 | H | / | / | 377 |

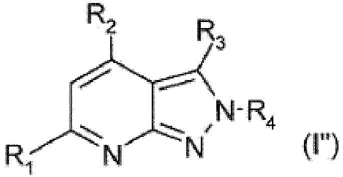
| **Nr.** | **R₂** | **R₃** | **R₁** | **R₄** | **Salz** | **Schmp. (°C)** | **M+H⁺** |
|---|---|---|---|---|---|---|---|
| **102** | COOH | H | 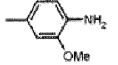 | Me | / | / | 299 |
| **103** | CONH₂ | H | 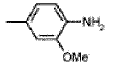 | Me | TFA | / | 412 |
| **104** | COOH | H | 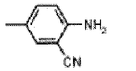 | Me | / | / | 294 |
| **105** | CHF₂ | H | 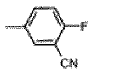 | Me | / | / | 303 |
| **106 Bsp. 11** | CH F₂ | H | 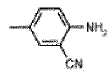 | Me | / | / | 300 |
| **107** | CHF₂ | H | 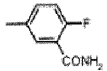 | Me | / | / | 321 |
| **108 Bsp. 7** | CF₃ | Ph | 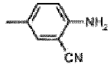 | Me | / | 295 | 394 |
| **109** | CF₃ | Ph | 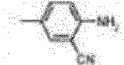 | 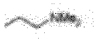 | / | 237 | 451 |
| **110** | CF₃ | Ph | 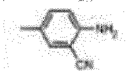 | 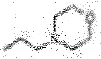 | / | 249 | 493 |
| **111** | CHF₂ | H | 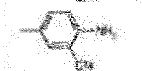 | 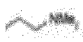 | | 182 | 357 |
| **112** | CHF₂ | H | 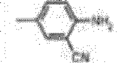 | 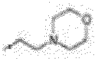 | / | 242 | 399 |
| **113** | CF₃ | H | 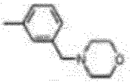 | 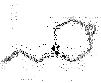 | / | 101 | 476 |
| **114 Bsp. 9** | CF₃ | H | 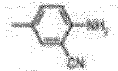 | Me | / | 249 | 318 |
| **115** | CF₃ | H | 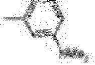 | Pr | HCl | 181 | 425 |
| **116** | CHF₂ | H | 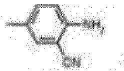 | 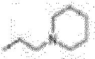 | / | 230 | 397 |
| **117** | CHF₂ | H | 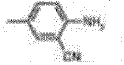 | Pr | / | 214 | 328 |
| **118** | CF₃ | H | 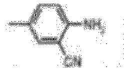 | Pr | / | 239 | 346 |
| **119** | CF₃ | H | 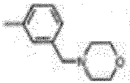 | Pr | / | 288 | 405 |
| **120** | CF₃ | H | 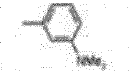 | Pr | / | 89 | 349 |

## Revendications

1. Composé correspondant à la formule (I) : 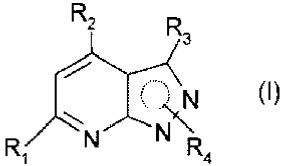 dans lequel :
la représentation du cycle pyrazole indique que le substituant R₄ peut être porté par l'azote alpha par rapport au cycle pyridine (I') ou par l'azote alpha par rapport au carbone portant un substituant R₃ (I") de sorte que : 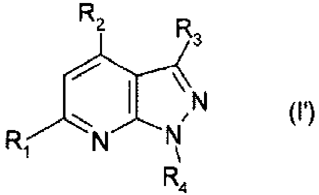 soit 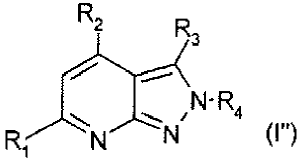
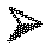 R₁ représente un groupe aryle, pyridyle ou pyrazolyle facultativement substitué par un ou plusieurs substituants choisis parmi :
∘ un atome de fluor,
∘ un groupe -CF₃,
∘ un groupe cyano,
∘ un groupe -NR₆R₆' dans lequel R₆ et R₆' sont tels que définis ci-dessous,
∘ un groupe -NR₁₀R₁₁ de sorte que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène, facultativement substitués par un ou plusieurs substituants choisis parmi un atome d'halogène et un groupe alkyle linéaire ou ramifié,
∘ un groupe -CH₂NR₁₀R₁₁ tel que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène,
∘ un groupe -COR₁₂ dans lequel R₁₂ représente un groupe hydroxyle ou un groupe -NR₆R₆', dans lequel R₆ et R₆' sont tels que définis ci-dessous,
∘ un groupe -CONR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
∘ un groupe -(CH₂)ₚNHSO₂CH₃ dans lequel p représente 0 ou 1,
∘ un groupe -OR₁₃ dans lequel R₁₃ représente un groupe alkyle en C₁-C₃ linéaire,
∘ un groupe éthyle, propyle ou isopropyle,
ou R₁ représente un groupe bicyclique de formule A ci-dessous : 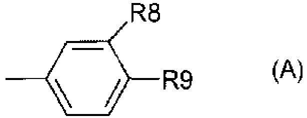 dans lequel R₈ et R₉ forment, conjointement avec les atomes de carbone auxquels ils sont liés, un hétérocycle saturé ou insaturé comprenant un ou plusieurs hétéroatomes choisis parmi un atome d'azote, un atome d'oxygène et un atome de soufre, de sorte que le groupe (A) forme un groupe dihydrobenzimidazolonyle, indolyle, dihydrobenzoxazinyle, benzothiazolyle ou benzimidazolyle, facultativement substitué par un ou plusieurs groupes alkyle linéaires,
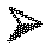 R₂ représente un groupe :
∘ -CF₃,
∘ -CHF₂,
ou
∘ -CONHR₅, dans lequel R₅ est tel que défini ci-dessous,
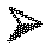 R₃ représente :
∘ un atome d'hydrogène,
∘ un groupe aryle, facultativement substitué par un groupe alcoxyméthyle,
ou
∘ un groupe hétéroaryle choisi parmi des groupes thiényle et pyridyle,
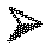 R₄ représente :
∘ un atome d'hydrogène,
∘ un alkyle en C₁, en C₃ linéaire, ou en C₁-C₃ linéaire substitué par un groupe -NR₆R₆' dans lequel R₆ et R₆' sont tels que définis ci-dessous ou un groupe -NR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
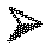 R₅ représente :
∘ un atome d'hydrogène,
∘ un groupe alkyle en C₁-C₃ linéaire, facultativement substitué par un groupe pyridyle, ou
∘ un groupe aromatique choisi parmi aryle et pyridyle,
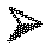 R₆ et R₆', qui peuvent être identiques ou différents, représentent un atome d'hydrogène ou un groupe alkyle linéaire,
sous la forme de la base ou d'un sel d'addition d'acide ou d'addition de base,
à l'exception des composés suivants :
acide 2-fluoro-5-(3-phényl-4-trifluorométhyl-1H-pyrazolo[3,4-b]pyridin-6-yl)benzoïque ;
3-(1-méthyl-3-phényl-4-trifluorométhyl-1H-pyrazolo[3,4-b]pyridin-6-yl)benzamide ;
N-[4-(1-méthyl-3-phényl-4-trifluorométhyl-1H-pyrazolo[3,4-b]pyridin-6-yl)benzyl]méthanesulfonamide ;
1-méthyl-6-(1-méthyl-1H-indol-6-yl)-3-phényl-4-trifluorométhyl-1H-pyrazolo[3,4-b]pyridine ;
N-[3-(1-méthyl-3-phényl-4-trifluorométhyl-1H-pyrazolo[3,4-b]pyridin-6-yl)phényl]méthanesulfonamide ;
4-méthyl-7-(1-méthyl-3-phényl-4-trifluorométhyl-1H-pyrazolo[3,4-b]pyridin-6-yl)-3,4-dihydro-2H-benzo[1,4]oxazine ;
N-[3-(1-méthyl-3-phényl-4-trifluorométhyl-1H-pyrazolo[3,4-b]pyridin-6-yl)benzyl]méthanesulfonamide ;
6-(4-méthoxyphényl)-1-méthyl-3-phényl-4-trifluorométhyl-1H-pyrazolo[3,4-b]pyridine ;
2-fluoro-N-méthyl-5-(1-méthyl-3-phényl-4-trifluorométhyl-1H-pyrazolo[3,4-b]pyridin-6-yl)benzamide ;
diméthyl[3-(1-méthyl-3-phényl-4-trifluorométhyl-1H-pyrazolo[3,4-b]pyridin-6-yl)phényl]amine ;
6-[4-(3,5-diméthylpyrazol-1-yl)phényl]-1-méthyl-3-phényl-4-trifluorométhyl-1H-pyrazolo[3,4-b]pyridine ;
1-méthyl-6-(3-morpholin-4-ylméthylphényl)-3-phényl-4-trifluorométhyl-1H-pyrazolo[3,4-b]pyridine ;
5-(1-méthyl-3-phényl-4-trifluorométhyl-1H-pyrazolo[3,4-b]pyridin-6-yl)nicotinonitrile ;
acide 4-(1-méthyl-3-phényl-4-trifluorométhyl-1H-pyrazolo[3,4-b]pyridin-6-yl)benzoïque ;
N,N-diméthyl-4-(1-méthyl-3-phényl-4-trifluorométhyl-1H-pyrazolo[3,4-b]pyridin-6-yl)benzamide ;
N,N-diméthyl-3-(1-méthyl-3-phényl-4-trifluorométhyl-1H-pyrazolo[3,4-b]pyridin-6-yl)benzamide ;
1-méthyl-6-(6-morpholin-4-ylpyridin-3-yl)-3-phényl-4-trifluorométhyl-1H-pyrazolo[3,4-b]pyridine ;
6-(6-méthoxypyridin-3-yl)-1-méthyl-3-phényl-4-trifluorométhyl-1H-pyrazolo[3,4-b]pyridine ;
1-méthyl-3-phényl-6-(6-pyrrolidin-1-ylpyridin-3-yl)-4-trifluorométhyl-1H-pyrazolo[3,4-b]pyridine ;
1-méthyl-6-(2-méthyl-5-trifluorométhyl-2H-pyrazol-3-yl)-3-phényl-4-trifluorométhyl-1H-pyrazolo[3,4-b]pyridine ;
6-benzothiazol-5-yl-1-méthyl-3-phényl-4-trifluorométhyl-1H-pyrazolo[3,4-b]pyridine ;
N,N-diméthyl-4-(3-phényl-4-trifluorométhyl-1H-pyrazolo[3,4-b]pyridin-6-yl)benzamide ;
6-(4-morpholin-4-ylphényl)-3-phényl-4-trifluorométhyl-1H-pyrazolo[3,4-b]pyridine ;
6-(6-morpholin-4-ylpyridin-3-yl)-3-phényl-4-trifluorométhyl-1H-pyrazolo[3,4-b]pyridine ;
6-(6-méthoxypyridin-3-yl)-3-phényl-4-trifluorométhyl-1H-pyrazolo[3,4-b]pyridine ;
6-(3-morpholin-4-ylphényl)-3-phényl-4-trifluorométhyl-1H-pyrazolo[3,4-b]pyridine ;
N-méthyl-3-(3-phényl-4-trifluorométhyl-1H-pyrazolo[3,4-b)pyridin-6-yl)benzamide ;
N-[3-(3-phényl-4-trifluorométhyl-1H-pyrazolo[3,4-b]pyridin-6-yl)phényl]méthanesulfonamide ;
3-phényl-6-(3-pipéridin-1-ylphényl)-4-trifluorométhyl-1H-pyrazolo[3,4-b]pyridine ;
2-fluoro-N-méthyl-5-(3-phényl-4-trifluorométhyl-1H-pyrazolo[3,4-b]pyridin-6-yl)benzamide ;
5-(3-phényl-4-trifluorométhyl-1H-pyrazolo[3,4-b]pyridin-6-yl)nicotinonitrile ;
acide 2-fluoro-5-(1-méthyl-3-phényl-4-trifluorométhyl-1H-pyrazolo[3,4-b]pyridin-6-yl)benzoïque ;
2-amino-5-(4-difluorométhyl-2-méthyl-2H-pyrazolo[3,4-b]pyridin-6-yl)benzonitrile ;
diméthyl[4-(3-phényl-4-trifluorométhyl-1H-pyrazolo[3,4-b]pyridin-6-yl)phényl]amine ;
4-(3-phényl-4-trifluorométhyl-1H-pyrazolo[3,4-b]pyridin-6-yl)phénylamine ;
6-(4-méthoxyphényl)-3-phényl-4-trifluorométhyl-1H-pyrazolo[3,4-b]pyridine ;
2-fluoro-5-(3-phényl-4-trifluorométhyl-1H-pyrazolo[3,4-b]pyridin-6-yl)benzonitrile ;
6-(1-éthyl-1H-pyrazol-4-yl)-1-méthyl-4(trifluorométhyl)-1H-pyrazolo[3,4-b]pyridine ;
4-(difluorométhyl)-6-(1-éthyl-1H-pyrazol-4-yl)-1-méthyl-1H-pyrazolo[3,4-b]pyridine ;
6-(4-éthylphényl)-1-méthyl-4-(trifluorométhyl)-1H-pyrazolo[3,4-b]pyridine ;
6-(4-méthoxyphényl)-1-méthyl-4-(trifluorométhyl)-1H-pyrazolo[3,4-b]pyridine ;
6-(4-fluorophényl)-1-méthyl-4-(trifluorométhyl)-1H-pyrazolo[3,4-b]pyridine ;
4-(difluorométhyl)-6-(2-méthoxyphényl)-1-méthyl-1H-pyrazolo[3,4-b]pyridine ;
6-(3-méthoxyphényl)-1-méthyl-4-(trifluorométhyl)-1H-pyrazolo[3,4-b]pyridine ;
6-(2,5-diméthoxyphényl)-1-méthyl-4-(trifluorométhyl)-1H-pyrazolo[3,4-b]pyridine ;
4-(difluorométhyl)-6-(4-éthylphényl)-1-méthyl-1H-pyrazolo[3,4-b]pyridine ;
4-(difluorométhyl)-6-(4-méthoxyphényl)-1-méthyl-1H-pyrazolo[3,4-b]pyridine ;
4-(difluorométhyl)-6-(3-méthoxyphényl)-1-méthyl-1H-pyrazolo[3,4-b]pyridine ;
4-(difluorométhyl)-6-(4-fluorophényl)-1-méthyl-1H-pyrazolo[3,4-b]pyridine ;
4-(difluorométhyl)-6-(3,4-diméthoxyphényl)-1-méthyl-1H-pyrazolo[3,4-b]pyridine ;
4-(difluorométhyl)-1-méthyl-6-phényl-1H-pyrazolo[3,4-b]pyridine ;
1-méthyl-6-phényl-4-(trifluorométhyl)-1H-pyrazolo[3,4-b]pyridine.

2. Composés de formule (I) selon la revendication 1, **caractérisés en ce que** R₂ représente un groupe :
∘ -CHF₂, sauf lorsque R₄ situé sur l'azote alpha par rapport à R₃ représente un groupe méthyle et R₃ représente un atome d'hydrogène,
ou
∘ -CONHR₅, dans lequel R₅ représente un atome d'hydrogène ou un groupe alkyle en C₁-C₃ linéaire, facultativement substitué par un groupe pyridyle ou un groupe aromatique choisi parmi aryle et pyridyle,
sous la forme de la base ou d'un sel d'addition d'acide ou d'addition de base.

3. Composés de formule (I) selon l'une quelconque des revendications 1 et 2, **caractérisés en ce que** R₁ représente un groupe aryle, pyridyle ou pyrazolyle, facultativement substitué par un ou plusieurs substituants choisis parmi :
∘ un atome de fluor,
et
∘ un groupe -COR₁₂, dans lequel R₁₂ représente un groupe hydroxyle, sous la forme de la base ou d'un sel d'addition d'acide ou d'addition de base.

4. Composés de formule (I) selon les revendications 1 à 3, **caractérisés en ce que** R₁ représente un groupe aryle, sous la forme de la base ou d'un sel d'addition d'acide ou d'addition de base.

5. Composés de formule (I) selon l'une quelconque des revendications 1, 3 et 4, **caractérisés en ce que** R₂ représente un groupe :
∘ -CF₃,
∘ -CHF₂,
ou
∘ -CONHR₅, dans lequel R₅ représente un atome d'hydrogène ou un groupe alkyle en C₁-C₃ linéaire, facultativement substitué par un groupe pyridyle ou un groupe aromatique choisi parmi aryle et pyridyle,
sous la forme de la base ou d'un sel d'addition d'acide ou d'addition de base.

6. Composé de formule (I) selon l'une des revendications précédentes, **caractérisé en ce qu'**il est choisi parmi les composés suivants :
[4-(4-difluorométhyl-3-phényl-1H-pyrazolo[3,4-b]pyridin-6-yl)phényl]pyrrolidin-1-ylméthanone ;
4-difluorométhyl-6-(1-méthyl-1H-pyrazol-4-yl)-3-phényl-1H-pyrazolo[3,4-b]pyridine ;
4-difluorométhyl-6-(3,5-diméthyl-1H-pyrazol-4-yl)-3-phényl-1H-pyrazolo[3,4-b]pyridine ;
4-difluorométhyl-3-phényl-6-(1H-pyrazol-4-yl)-1H-pyrazolo[3,4-b]pyridine ;
4-difluorométhyl-6-(6-méthoxypyridin-3-yl)-3-phényl-1H-pyrazolo[3,4-b]pyridine ;
[3-(4-difluorométhyl-3-phényl-1H-pyrazolo[3,4-b]pyridin-6-yl)phényl]pyrrolidin-1-ylméthanone ;
4-difluorométhyl-3-phényl-6-(3-pipéridin-1-ylphényl)-1H-pyrazolo[3,4-b]pyridine ;
méthylamide d'acide 6-(4-amino-3-méthoxyphényl)-3-phényl-1H-pyrazolo[3,4-b]pyridine-4-carboxylique ;
6-(4-amino-3-méthoxyphényl)-3-phényl-1H-pyrazolo[3,4-b]pyridine-4-carboxamide ;
méthylamide d'acide 6-(4-amino-3-méthoxyphényl)-1H-pyrazolo[3,4-b]pyridine-4-carboxylique ;
méthylamide d'acide 4-(4-amino-3-méthoxyphényl)-1H-pyrazolo[3,4-b]pyridine-6-carboxylique ;
6-(4-amino-3-méthoxyphényl)-1H-pyrazolo[3,4-b]pyridine-4-carboxamide ;
6-(4-amino-3-méthoxyphényl)-2-méthyl-2H-pyrazolo[3,4-b]pyridine-4-carboxamide ;
acide 5-(4-carbamoyl-3-phényl-1H-pyrazolo[3,4-b]pyridin-6-yl)-2-fluorobenzoïque ;
acide 2-amino-5-(4-carbamoyl-3-phényl-1H-pyrazolo[3,4-b]pyridin-6-yl)benzoïque ;
6-(4-amino-3-cyanophényl)-3-phényl-1H-pyrazolo[3,4-b]pyridine-4-carboxamide ;
6-(4-amino-3-cyanophényl)-3-thiophén-2-yl-1H-pyrazolo[3,4-b]pyridine-4-carboxamide ;
acide 5-(4-carbamoyl-1H-pyrazolo[3,4-b]pyridin-6-yl)-2-fluorobenzoïque ;
6-(3-cyano-4-fluorophényl)-3-phényl-1H-pyrazolo[3,4-b]pyridine-4-carboxamide ;
acide 5-(4-difluorométhyl-3-phényl-1H-pyrazolo[3,4-b]pyridin-6-yl)-2-fluorobenzoïque ;
2-amino-5-(4-difluorométhyl-3-phényl-1H-pyrazolo[3,4-b]pyridin-6-yl)benzonitrile ;
2-amino-5-(1-méthyl-3-phényl-4-trifluorométhyl-1H-pyrazolo[3,4-b]pyridin-6-yl)benzonitrile ;
6-(3-carbamoyl-4-fluorophényl)-3-pyridin-3-yl-1H-pyrazolo[3,4-b]pyridine-4-carboxamide ;
5-(4-difluorométhyl-2-méthyl-2H-pyrazolo[3,4-b]pyridin-6-yl)-2-fluorobenzonitrile ;
6-(1H-indol-6-yl)-3-phényl-4-trifluorométhyl-1H-pyrazolo[3,4-b]pyridine ;
5-(3-phényl-4-trifluorométhyl-1H-pyrazolo[3,4-b]pyridin-6-yl)-1,3-dihydrobenzimidazol-2-one ;
phénylamide d'acide 6-(4-amino-3-cyanophényl)-1H-pyrazolo[3,4-b]pyridine-4-carboxylique ;
(pyridin-2-ylméthyl)amide d'acide 6-(4-amino-3-cyanophényl)-1H-pyrazolo[3,4-b]pyridine-4-carboxylique ;
pyridin-2-ylamide d'acide 6-(4-amino-3-cyanophényl)-1H-pyrazolo[3,4-b]pyridine-4-carboxylique ;
pyridin-3-ylamide d'acide 6-(4-amino-3-cyanophényl)-1H-pyrazolo[3,4-b]pyridine-4-carboxylique ;
pyridin-4-ylamide d'acide 6-(4-amino-3-cyanophényl)-1H-pyrazolo[3,4-b]pyridine-4-carboxylique ;
5-(4-difluorométhyl-2-méthyl-2H-pyrazolo[3,4-b]pyridin-6-yl)-2-fluorobenzamide ;
5-(4-difluorométhyl-1-méthyl-3-pyridin-4-yl-1H-pyrazolo[3,4-b]pyridin-6-yl)-2-fluorobenzonitrile ;
2-amino-5-(2-méthyl-3-phényl-4-trifluorométhyl-2H-pyrazolo[3,4-b]pyridin-6-yl)benzonitrile ;
phénylamide d'acide 6-(1H-benzimidazol-5-yl)-1H-pyrazolo[3,4-b]pyridine-4-carboxylique ;
pyridin-2-ylamide d'acide 6-(1H-benzimidazol-5-yl)-1H-pyrazolo[3,4-b]pyridine-4-carboxylique ;
(pyridin-3-ylméthyl)amide d'acide 6-(1H-benzimidazol-5-yl)-1H-pyrazolo[3,4-b]pyridine-4-carboxylique ;
phénylamide d'acide 6-(2-oxo-2,3-dihydro-1H-benzimidazol-5-yl)-1H-pyrazolo[3,4-b]pyridine-4-carboxylique ;
2-amino-5-(4-difluorométhyl-1-méthyl-1H-pyrazolo[3,4-b]pyridin-6-yl)benzonitrile ;
3-(4-difluorométhyl-1-méthyl-1H-pyrazolo[3,4-b]pyridin-6-yl)benzonitrile ;
4-(4-difluorométhyl-1-méthyl-1H-pyrazolo[3,4-b]pyridin-6-yl)phénylamine ;
2-amino-5-[2-(2-diméthylaminoéthyl)-3-phényl-4-trifluorométhyl-2H-pyrazolo[3,4-b]pyridin-6-yl]benzonitrile ;
2-amino-5-(4-difluorométhyl-1H-pyrazolo[3,4-b]pyridin-6-yl)benzonitrile ;
2-amino-5-[1-(2-diméthylaminoéthyl)-3-phényl-4-trifluorométhyl-1H-pyrazolo[3,4-b]pyridin-6-yl]benzonitrile ;
2-amino-5-[2-(2-morpholin-4-yléthyl)-3-phényl-4-trifluorométhyl-2H-pyrazolo[3,4-b]pyridin-6-yl]benzonitrile ;
2-méthoxy-5-(1-méthyl-3-phényl-4-trifluorométhyl-1H-pyrazolo[3,4-b]pyridin-6-yl)nicotinonitrile ;
2-amino-5-(4-difluorométhyl-1-méthyl-3-phényl-1H-pyrazolo[3,4-b]pyridin-6-yl)benzonitrile ;
4-(4-difluorométhyl-1-méthyl-3-phényl-1H-pyrazolo[3,4-b]pyridin-6-yl)phénylamine ;
[3-(4-difluorométhyl-1-méthyl-3-phényl-1H-pyrazolo[3,4-b]pyridin-6-yl)phényl]diméthylamine ;
2-amino-5-[3-phényl-1-(2-pipéridin-1-yléthyl)-4-trifluorométhyl-1H-pyrazolo[3,4-b]pyridin-6-yl]benzonitrile ;
diméthyl{3-[3-phényl-1-(2-pipéridin-1-yléthyl)-4-trifluorométhyl-1H-pyrazolo[3,4-b]pyridin-6-yl]phényl}amine ;
2-amino-5-[4-difluorométhyl-2-(2-diméthylaminoéthyl)-2H-pyrazolo[3,4-b]pyridin-6-yl]benzonitrile ;
2-amino-5-[4-difluorométhyl-2-(2-morpholin-4-yléthyl)-2H-pyrazolo[3,4-b]pyridin-6-yl]benzonitrile ;
2-amino-5-[4-difluorométhyl-1-(2-diméthylaminoéthyl)-3-phényl-1H-pyrazolo[3,4-b]pyridin-6-yl]benzonitrile ;
2-(2-morpholin-4-yléthyl)-6-(3-morpholin-4-ylméthylphényl)-4-trifluorométhyl-2H-pyrazolo[3,4-b]pyridine ;
diméthyl{3-[1-(2-morpholin-4-yléthyl)-4-trifluorométhyl-1H-pyrazolo[3,4-b]pyridin-6-yl]phényl}amine ;
5-[1-(2-morpholin-4-yléthyl)-4-trifluorométhyl-1H-pyrazolo[3,4-b]pyridin-6-yl]nicotinonitrile ;
5-[1-(2-morpholin-4-yléthyl)-4-trifluorométhyl-1H-pyrazolo[3,4-b]pyridin-6-yl]nicotinamide ;
2-amino-5-(2-méthyl-4-trifluorométhyl-2H-pyrazolo[3,4-b]pyridin-6-yl)benzonitrile ;
1-méthyl-6-(3-morpholin-4-ylméthylphényl)-4-trifluorométhyl-1H-pyrazolo[3,4-b]pyridine ;
2-amino-5-(1-méthyl-4-trifluorométhyl-1H-pyrazolo[3,4-b]pyridin-6-yl)benzonitrile ;
diméthyl[3-(1-méthyl-4-trifluorométhyl-1H-pyrazolo[3,4-b]pyridin-6-yl)phényl]amine ;
diméthyl[3-(3-phényl-2-propyl-4-trifluorométhyl-2H-pyrazolo[3,4-b]pyridin-6-yl)phényl]amine ;
2-amino-5-[4-difluorométhyl-2-(2-pipéridin-1-yléthyl)-2H-pyrazolo[3,4-b]pyridin-6-yl]benzonitrile ;
2-amino-5-(4-difluorométhyl-3-pyridin-3-yl-1H-pyrazolo[3,4-b]pyridin-6-yl)benzonitrile ;
2-amino-5-(4-difluorométhyl-2-propyl-2H-pyrazolo[3,4-b]pyridin-6-yl)benzonitrile ;
2-amino-5-(4-difluorométhyl-3-pyridin-4-yl-1H-pyrazolo[3,4-b]pyridin-6-yl)benzonitrile ;
1-méthyl-6-(3-morpholin-4-ylméthylphényl)-3-pyridin-3-yl-4-trifluorométhyl-1H-pyrazolo[3,4-b]pyridine ;
2-amino-5-[4-difluorométhyl-3-(3-méthoxyphényl)-1H-pyrazolo[3,4-b]pyridin-6-yl]benzonitrile ;
2-amino-5-(2-propyl-4-trifluorométhyl-2H-pyrazolo[3,4-b]pyridin-6-yl)benzonitrile ;
6-(3-morpholin-4-ylméthylphényl)-2-propyl-4-trifluorométhyl-2H-pyrazolo[3,4-b]pyridine ;
diméthyl[3-(2-propyl-4-trifluorométhyl-2H-pyrazolo[3,4-b]pyridin-6-yl)phényl]amine ;
6-(3-morpholin-4-ylméthylphényl)-1-propyl-4-trifluorométhyl-1H-pyrazolo[3,4-b]pyridine ;
6-(4-méthoxyphényl)-1-propyl-4-trifluorométhyl-1H-pyrazolo[3,4-b]pyridine ;
5-[3-(3-méthoxyphényl)-1-méthyl-4-trifluorométhyl-1H-pyrazolo[3,4-b]pyridin-6-yl]nicotinonitrile ;
3-(3-méthoxyphényl)-1-méthyl-6-(3-morpholin-4-ylméthylphényl)-4-trifluorométhyl-1H-pyrazolo[3,4-b]pyridine ;
{3-[3-(3-méthoxyphényl)-1-méthyl-4-trifluorométhyl-1H-pyrazolo[3,4-b]pyridin-6-yl]phényl}diméthylamine ;
3-(3-méthoxyphényl)-6-(4-méthoxyphényl)-1-méthyl-4-trifluorométhyl-1H-pyrazolo[3,4-b]pyridine.

7. Procédé de préparation des composés de formule (I) selon la revendication 1, selon le schéma 5 comme suit 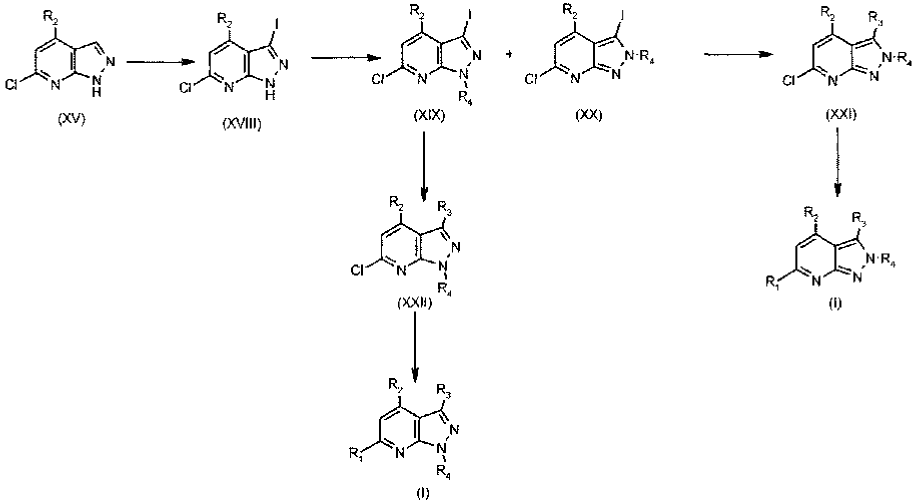 dans lequel R₂ représente un groupe -CHF₂ ou -CF₃, et R₁, R₃ et R₄ sont tels que définis précédemment, à l'exception de R₃ et R₄ représentant un atome d'hydrogène, **caractérisé en ce que** :
- le composé de formule (XV) est soumis à une réaction d'iodation en présence de N-iodosuccinimide afin d'obtenir le composé de formule (XVIII),
- le composé de formule (XVIII) est ensuite soumis à une réaction d'alkylation en présence d'un dérivé halogéné de formule R₄-X afin d'obtenir les composés de formules (XIX) et (XX),
- les composés de formules (XIX) et (XX) sont séparément soumis, en présence d'un catalyseur au palladium, d'un ligand et d'une base à une réaction avec des dérivés phénylboriques ou hétéroarylboriques ou des esters de phénylborate ou des esters d'hétéroarylborate selon un couplage de Suzuki, afin d'obtenir les composés de formules (XXI) et (XXII),
- les composés de formules (XXI) et (XXII) sont séparément soumis, en présence d'un catalyseur au palladium, d'un ligand et d'une base, à une réaction avec des dérivés phénylboriques ou hétéroarylboriques ou des esters de phénylborate ou des esters d'hétéroarylborate selon un couplage de Suzuki, afin d'obtenir le composé de formule (I) dans lequel R₂ représente un groupe -CHF₂ ou -CF₃ et R₁, R₃ et R₄ sont tels que définis précédemment.

8. Procédé de préparation des composés de formule (I) selon la revendication 1, selon le schéma 6 comme suit 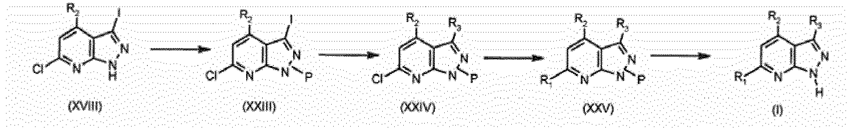 dans lequel R₂ représente un groupe -CHF₂ ou -CF₃, et R₁ et R₃ sont tels que définis précédemment, à l'exception de R₃ représentant un atome d'hydrogène, **caractérisé en ce que** :
- le composé de formule (XVIII) est soumis à une réaction d'alkylation en présence d'un groupe protecteur P afin d'obtenir le composé de formule (XXIII),
- le composé de formule (XXIII) est soumis à une réaction avec des dérivés phénylboriques ou hétéroarylboriques ou des esters de phénylborate ou des esters d'hétéroarylborate selon un couplage de Suzuki, afin d'obtenir le composé de formule (XXIV),
- le composé de formule (XXIV) est soumis à une réaction avec des dérivés phénylboriques ou hétéroarylboriques ou des esters de phénylborate ou des esters d'hétéroarylborate selon un couplage de Suzuki, afin d'obtenir le composé de formule (XXV),
- le composé de formule (XXV) est soumis à une réaction de déprotection afin d'obtenir le composé de formule (I) dans lequel R₂ représente un groupe -CHF₂ ou -CF₃.

9. Composé de formule (I) 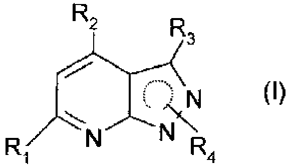 dans lequel :
la représentation du cycle pyrazole indique que le substituant R₄ peut être porté par l'azote alpha par rapport au cycle pyridine (I') ou par l'azote alpha par rapport au carbone portant un substituant R₃ (I") de sorte que : 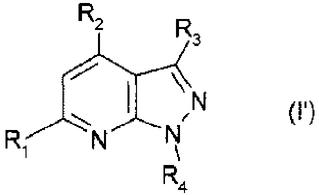 soit 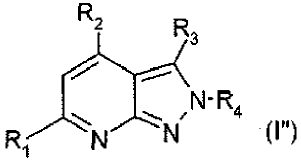
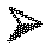 R₁ représente un groupe aryle, pyridyle ou pyrazolyle facultativement substitué par un ou plusieurs substituants choisis parmi :
∘ un atome d'halogène,
∘ un groupe -CF₃,
∘ un groupe cyano,
∘ un groupe -NR₆R₆' dans lequel R₆ et R₆' sont tels que définis ci-dessous,
∘ un groupe -NR₁₀R₁₁ tel que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène, facultativement substitués par un ou plusieurs substituants choisis parmi un atome d'halogène et un groupe alkyle linéaire ou ramifié,
∘ un groupe -CH₂NR₁₀R₁₁ tel que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène,
∘ un groupe -COR₁₂ dans lequel R₁₂ représente un groupe hydroxyle ou un groupe -NR₆R₆', dans lequel R₆ et R₆' sont tels que définis ci-dessous,
∘ un groupe -CONR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
∘ un groupe -(CH₂)ₚNHSO₂CH₃ dans lequel p représente 0 ou 1,
∘ un groupe -OR₁₃ dans lequel R₁₃ représente un groupe alkyle en C₁-C₃ linéaire,
∘ un groupe alkyle en C₁-C₃,
ou R₁ représente un groupe bicyclique de formule A ci-dessous : 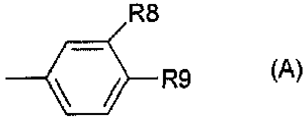 dans lequel R₈ et R₉ forment, conjointement avec les atomes de carbone auxquels ils sont liés, un hétérocycle saturé ou insaturé comprenant un ou plusieurs hétéroatomes choisis parmi un atome d'azote, un atome d'oxygène et un atome de soufre, facultativement substitués par un ou plusieurs groupes alkyle linéaires,
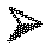 R₂ représente un groupe :
∘ -CF₃,
∘ -CHF₂,
∘ -COOH, ou
∘ -CONHR₅, dans lequel R₅ est tel que défini ci-dessous,
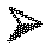 R₃ représente :
∘ un atome d'hydrogène,
∘ un groupe aryle, facultativement substitué par un groupe alcoxyméthyle,
∘ un groupe cycloalkyle,
ou
∘ un groupe hétéroaryle choisi parmi des groupes thiényle et pyridyle,
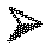 R₄ représente :
∘ un atome d'hydrogène,
∘ un groupe alkyle en C₁-C₃ linéaire, facultativement substitué par un groupe -NR₆R₆' dans lequel R₆ et R₆' sont tels que définis ci-dessous ou un groupe -NR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
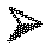 R₅ représente :
∘ un atome d'hydrogène,
∘ un groupe alkyle en C₁-C₃ linéaire, facultativement substitué par un groupe pyridyle,
ou
∘ un groupe aromatique choisi parmi aryle et pyridyle,
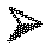 R₆ et R₆', qui peuvent être identiques ou différents, représentent un atome d'hydrogène ou un groupe alkyle linéaire,
sous la forme de la base ou d'un sel d'addition d'acide ou d'addition de base, pour utilisation en tant que médicament.

10. Composé selon la revendication précédente, ledit composé étant choisi parmi les composés suivants : 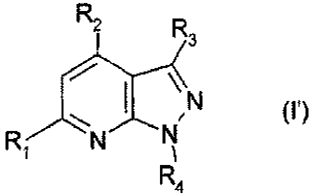
| **N°** | **R₂** | **R₃** | **R₁** | **R₄** | **Sel** | **p.f. (°C)** | **M+H⁺** |
|---|---|---|---|---|---|---|---|
| **1** | CF₃ | Ph | 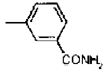 | Me | / | / | 397 |
| **2** | CF₃ | Ph | 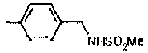 | Me | / | / | 461 |
| **3** | CF₃ | Ph | 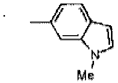 | Me | / | / | 407 |
| **4** | CF₃ | Ph | 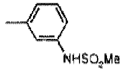 | Me | / | / | 447 |
| **5** | CF₃ | Ph | 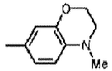 | Me | / | / | 426 |
| **6** | CF₃ | Ph | 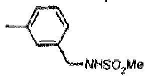 | Me | / | / | 461 |
| **7** | CF₃ | Ph | 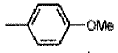 | Me | / | / | 384 |
| **8** | CF₃ | Ph | 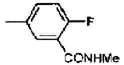 | Me | / | / | 429 |
| **9** | CF₃ | Ph | 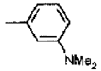 | Me | / | / | 397 |
| **10** | CF₃ | Ph | 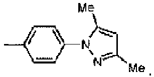 | Me | / | / | 448 |
| **11** | CF₃ | Ph | 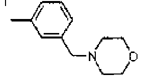 | Me | / | / | 453 |
| **12** | CF₃ | Ph | 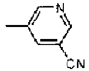 | Me | / | / | 380 |
| **13** | CF₃ | Ph | 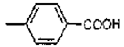 | Me | / | / | 398 |
| **14** | CF₃ | Ph | 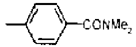 | Me | / | / | 425 |
| **15** | CF₃ | Ph | 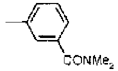 | Me | / | / | 425 |
| **16** | CF₃ | Ph | 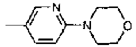 | Me | / | / | 440 |
| **17** | CF₃ | Ph | 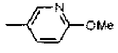 | Me | / | / | 385 |
| **18** | CF₃ | Ph | 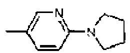 | Me | / | / | 424 |
| **19** | CF₃ | Ph | 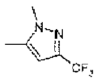 | Me | / | / | 426 |
| **20** | CF₃ | Ph | 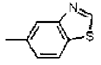 | Me | / | / | 411 |
| **21** | CF₃ | Ph | 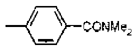 | H | HCl | / | 411 |
| **22** | CF₃ | Ph | 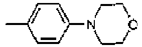 | H | HCl | / | 425 |
| **23** | CF₃ | Ph | 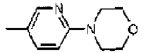 | H | HCl | / | 426 |
| **24** | CF₃ | Ph | 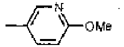 | H | HCl | / | 371 |
| **25** | CF₃ | Ph | 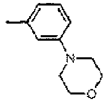 | H | HCl | / | 425 |
| **26** | CF₃ | Ph | 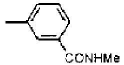 | H | HCl | / | 397 |
| **27** | CF₃ | Ph | 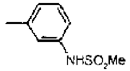 | H | HCl | / | 433 |
| **28** | CF₃ | Ph | 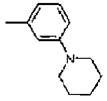 | H | HCl | / | 423 |
| **29** | CF₃ | Ph | 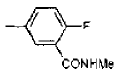 | H | HCl | / | 415 |
| **30** | CF₃ | Ph | 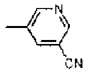 | H | HCl | / | 366 |
| **31** | CHF₂ | Ph | 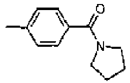 | H | HCl | 248 | 455 |
| **32** | CHF₂ | Ph | 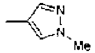 | H | HCl | / | 362 |
| **33** | CHF₂ | Ph | 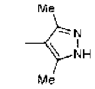 | H | HCl | / | 376 |
| **34** | CHF₂ | Ph | 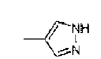 | H | HCl | / | 348 |
| **35** | CHF₂ | Ph | 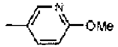 | H | HCl | / | 389 |
| **36** | CHF₂ | Ph | 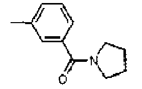 | H | HCl | / | 455 |
| **37** | CHF₂ | Ph | 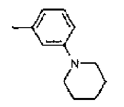 | H | HCl | / | 441 |
| **38 Ex. 2** | COOH | H | 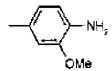 | H | / | / | 285 |
| **39** | CONHMe | Ph | 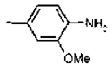 | H | TFA | / | 458 |
| **40** | CONH₂ | Ph | 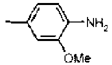 | H | TFA | / | 474 |
| **41** | CONHMe | H | 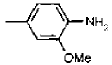 | H | TFA | / | 412 |
| **42** | CONH₂ | H | 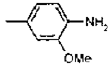 | H | TFA | / | 398 |
| **43** | COOH | Ph | 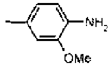 | H | / | / | 361 |
| **44** | COOH | Ph | 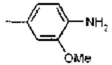 | Me | / | / | 375 |
| **45** | COOH | H | 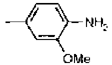 | Me | / | / | 299 |
| **46 Ex. 1** | CONH₂ | Ph | 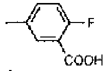 | H | TFA | / | 377 |
| **47** | CONH₂ | Ph | 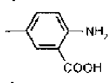 | H | / | / | 374 |
| **48** | CONH₂ | Ph | 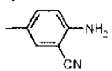 | H | TFA | / | 355 |
| **49** | CONH₂ | 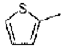 | 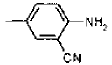 | H | / | / | 361 |
| **50** | COOH | cPr | 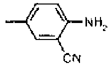 | H | / | / | 320 |
| **51** | CONH₂ | H | 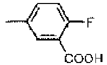 | H | / | / | 301 |
| **52** | CONH₂ | Ph | 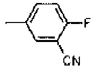 | H | / | / | 358 |
| **53 Ex. 3** | CHF₂ | Ph | 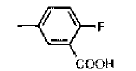 | H | / | / | 384 |
| **54** | CF₃ | Ph | 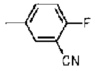 | H | / | / | 383 |
| **55** | CHF₂ | Ph | 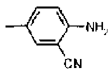 | H | HCl | 282 | 362 |
| **56 Ex. 4** | CF₃ | Ph | 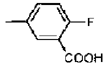 | H | / | / | 402 |
| **57 Ex. 6** | CF₃ | Ph | 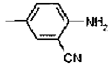 | Me | / | 269 | 394 |
| **58** | CF₃ | Ph | 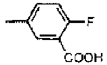 | Me | / | / | 416 |
| **59** | CF₃ | Ph | 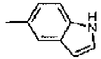 | H | / | / | 379 |
| **60** | CF₃ | Ph | 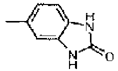 | H | / | 380 | 396 |
| **61 Ex. 5** | CF₃ | Ph | 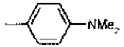 | H | / | 227 | 383 |
| **62** | CONHPh | H | 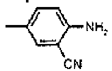 | H | HCl | / | 355 |
| **63** | 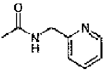 | H | 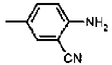 | H | HCl | / | 370 |
| **64** | 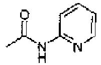 | H | 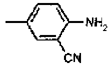 | H | HCl | / | 356 |
| **65** | 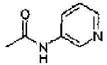 | H | 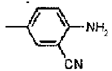 | H | HCl | / | 356 |
| **66** | 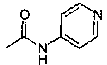 | H | 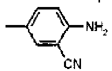 | H | HCl | / | 356 |
| **67** | CHF₂ | 4-Py | 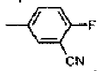 | Me | / | / | 380 |
| **68** | CONHPh | H | 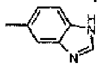 | H | HCl | / | 355 |
| **69** | 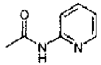 | H | 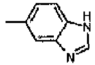 | H | HCl | / | 356 |
| **70** | 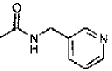 | H | 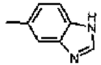 | H | HCl | / | 370 |
| **71** | CONHPh | H | 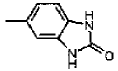 | H | / | / | 371 |
| **72 Ex. 10** | CHF₂ | H | 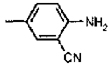 | Me | / | 251 | 300 |
| **73** | CHF₂ | H | 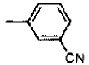 | Me | / | 162 | 285 |
| **74** | CHF₂ | H | 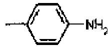 | Me | / | 149 | 275 |
| **75 Ex. 12** | CHF₂ | H | 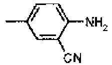 | H | / | 263 | 286 |
| **76** | CF₃ | Ph | 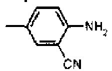 | 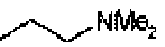 | / | 183 | 451 |
| **77** | CF₃ | Ph | 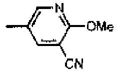 | Me | / | 250 | 410 |
| **78** | CHF₂ | Ph | 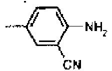 | Me | / | 246 | 376 |
| **79** | CHF₂ | Ph | 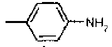 | Me | / | 176 | 351 |
| **80** | CHF₂ | Ph | 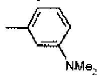 | Me | / | 154 | 379 |
| **81** | CF₃ | Ph | 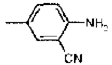 | 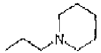 | / | 192 | 491 |
| **82** | CF₃ | Ph | 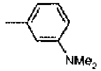 | 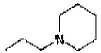 | HCl | 227 | 494 |
| **83 Ex. 13** | CHF₂ | Ph | 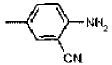 | 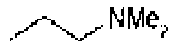 | / | 163 | 433 |
| **84** | CF₃ | H | 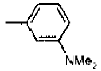 | 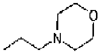 | / | 110 | 420 |
| **85** | CF₃ | H | 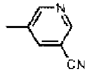 | 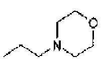 | / | / | 403 |
| **86** | CF₃ | H | 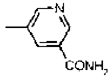 | 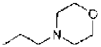 | / | 238 | 421 |
| **87** | CF₃ | H | 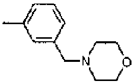 | Me | / | 105 | 377 |
| **88 Ex. 8** | CF₃ | H | 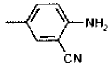 | Me | / | 276 | 318 |
| **89** | CF₃ | Ph | 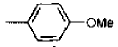 | Me | / | 181 | 384 |
| **90** | CF₃ | H | 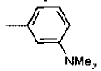 | Me | / | 91 | 321 |
| **91 Ex. 15** | CHF₂ | 3-Py | 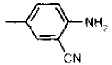 | H | / | 296 | 363 |
| **92** | CHF₂ | 4-Py | 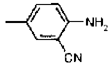 | H | / | 325 | 363 |
| **93 Ex. 14** | CF₃ | 3-Py | 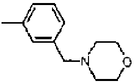 | Me | / | 155 | 454 |
| **94** | CHF₂ | 3MeO-Ph | 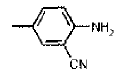 | Me | / | 233 | 392 |
| **95** | CF₃ | H | 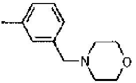 | Pr | HCl | 271 | 405 |
| **96** | CF₃ | H | 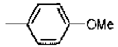 | Pr | / | 72 | 336 |
| **97** | CF₃ | 3MeO-Ph | 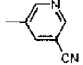 | Me | / | 194 | 410 |
| **98** | CF₃ | 3MeO-Ph | 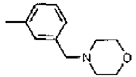 | Me | / | 114 | 483 |
| **99** | CF₃ | 3MeO-Ph | 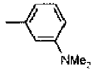 | Me | / | 138 | 427 |
| **100** | CF₃ | 3MeO-Ph | 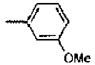 | Me | / | 133 | 414 |
| **101** | CONH₂ | 3-Py | 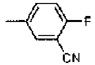 | H | / | / | 377 |

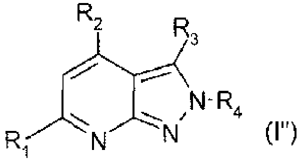
| **N°** | **R₂** | **R₃** | **R₁** | **R₄** | **Sel** | **p.f. (°C)** | **M+H⁺** |
|---|---|---|---|---|---|---|---|
| **102** | COOH | H | 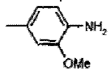 | Me | / | / | 299 |
| **103** | CONH₂ | H | 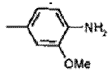 | Me | TFA | / | 412 |
| **104** | COOH | H | 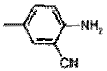 | Me | / | / | 294 |
| **105** | CHF₂ | H | 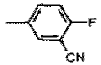 | Me | / | / | 303 |
| **106 Ex. 11** | CHF₂ | H | 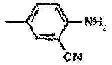 | Me | / | / | 300 |
| **107** | CHF₂ | H | 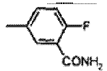 | Me | / | / | 321 |
| **108 Ex. 7** | CF₃ | Ph | 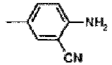 | **Me** | / | 295 | 394 |
| **109** | CF₃ | Ph | 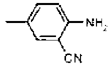 | 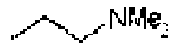 | / | 237 | 451 |
| **110** | CF₃ | Ph | 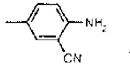 | 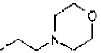 | / | 249 | 493 |
| **111** | CHF₂ | H | 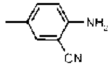 | 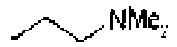 | / | 182 | 357 |
| **112** | CHF₂ | H | 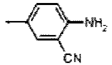 | 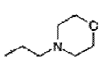 | / | 242 | 399 |
| **113** | CF₃ | H | 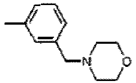 | 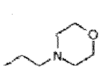 | / | 101 | 476 |
| **114 Ex. 9** | CF₃ | H | 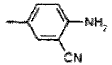 | Me | / | 249 | 318 |
| **115** | CF₃ | H | 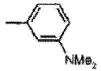 | Pr | HCl | 181 | 425 |
| **116** | CHF₂ | H | 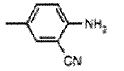 | 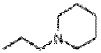 | / | 230 | 397 |
| **117** | CHF₂ | H | 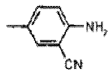 | Pr | / | 214 | 328 |
| **118** | CF₃ | H | 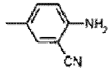 | Pr | / | 239 | 346 |
| **119** | CF₃ | H | 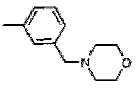 | Pr | / | 288 | 405 |
| **120** | CF₃ | H | 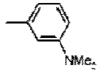 | Pr | / | 89 | 349 |
pour utilisation en tant que médicament.

11. Composition pharmaceutique, **caractérisée en ce qu'**elle comprend un composé de formule (I) 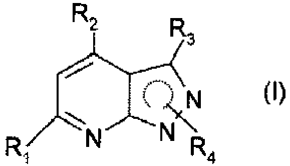 dans lequel :
la représentation du cycle pyrazole indique que le substituant R₄ peut être porté par l'azote alpha par rapport au cycle pyridine (I') ou par l'azote alpha par rapport au carbone portant un substituant R₃ (I") de sorte que : 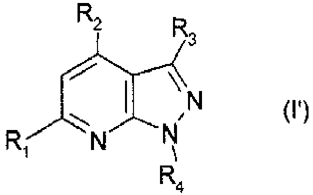 soit 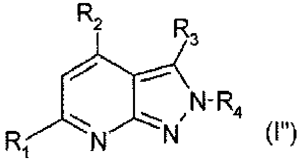
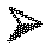 R₁ représente un groupe aryle, pyridyle ou pyrazolyle facultativement substitué par un ou plusieurs substituants choisis parmi :
∘ un atome d'halogène,
∘ un groupe -CF₃,
∘ un groupe cyano,
∘ un groupe -NR₆R₆' dans lequel R₆ et R₆' sont tels que définis ci-dessous,
∘ un groupe -NR₁₀R₁₁ tel que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène, facultativement substitués par un ou plusieurs substituants choisis parmi un atome d'halogène et un groupe alkyle linéaire ou ramifié,
∘ un groupe -CH₂NR₁₀R₁₁ tel que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène,
∘ un groupe -COR₁₂ dans lequel R₁₂ représente un groupe hydroxyle ou un groupe -NR₆R₆', dans lequel R₆ et R₆' sont tels que définis ci-dessous,
∘ un groupe -CONR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
∘ un groupe -(CH₂)ₚNHSO₂CH₃ dans lequel p représente 0 ou 1,
∘ un groupe -OR₁₃ dans lequel R₁₃ représente un groupe alkyle en C₁-C₃ linéaire,
∘ un groupe alkyle en C₁-C₃,
ou R₁ représente un groupe bicyclique de formule A ci-dessous ; 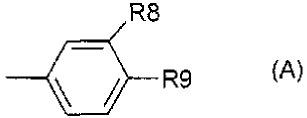 dans lequel R₈ et R₉ forment, conjointement avec les atomes de carbone auxquels ils sont liés, un hétérocycle saturé ou insaturé comprenant un ou plusieurs hétéroatomes choisis parmi un atome d'azote, un atome d'oxygène et un atome de soufre, facultativement substitués par un ou plusieurs groupes alkyle linéaires,
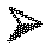 R₂ représente un groupe :
∘ -CF₃,
∘ -CHF₂,
ou
∘ -CONHR₅, dans lequel R₅ est tel que défini ci-dessous,
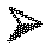 R₃ représente :
∘ un atome d'hydrogène,
∘ un groupe aryle, facultativement substitué par un groupe alcoxyméthyle,
∘ un groupe cycloalkyle,
ou
∘ un groupe hétéroaryle choisi parmi des groupes thiényle et pyridyle,
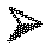 R₄ représente :
∘ un atome d'hydrogène,
∘ un groupe alkyle en C₁-C₃ linéaire, facultativement substitué par un groupe -NR₆R₆' dans lequel R₆ et R₆' sont tels que définis ci-dessous ou un groupe -NR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
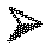 R₅ représente :
∘ un atome d'hydrogène,
∘ un groupe alkyle en C₁-C₃ linéaire, facultativement substitué par un groupe pyridyle,
ou
∘ un groupe aromatique choisi parmi aryle et pyridyle,
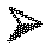 R₆ et R₆', qui peuvent être identiques ou différents, représentent un atome d'hydrogène ou un groupe alkyle linéaire,
sous la forme de la base ou d'un sel d'addition d'acide ou d'addition de base
et en outre, au moins un excipient pharmaceutiquement acceptable.

12. Composition pharmaceutique selon la revendication précédente, **caractérisée en ce qu'**elle comprend un composé choisi parmi les composés suivants 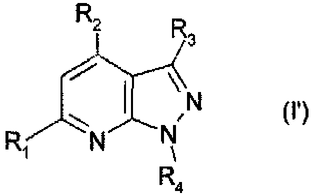
| **N°** | **R₂** | **R₃** | **R₁** | **R₄** | **Sel** | **p.f. (°C)** | **M+H⁺** |
|---|---|---|---|---|---|---|---|
| **1** | CF₃ | Ph | 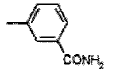 | **Me** | / | / | 397 |
| **2** | CF₃ | Ph | 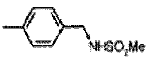 | **Me** | / | / | 461 |
| **3** | CF₃ | Ph | 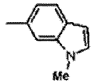 | **Me** | / | / | 407 |
| **4** | CF₃ | Ph | 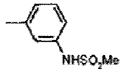 | **Me** | / | / | 447 |
| **5** | CF₃ | Ph | 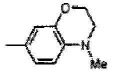 | **Me** | / | / | 426 |
| **6** | CF₃ | Ph | 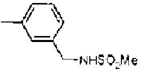 | Me | / | / | 461 |
| **7** | CF₃ | Ph | 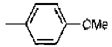 | Me | / | / | 384 |
| **8** | CF₃ | Ph | 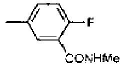 | Me | / | / | 429 |
| **9** | CF₃ | Ph | 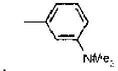 | Me | / | / | 397 |
| **10** | CF₃ | Ph | 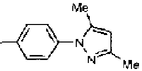 | Me | / | / | 448 |
| **11** | CF₃ | Ph | 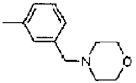 | Me | / | / | 453 |
| **12** | CF₃ | Ph | 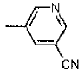 | Me | / | / | 380 |
| **13** | CF₃ | Ph | 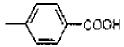 | Me | / | / | 398 |
| **14** | CF₃ | Ph | 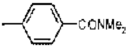 | Me | / | / | 425 |
| **15** | CF₃ | Ph | 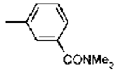 | Me | / | / | 425 |
| **16** | CF₃ | Ph | 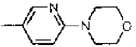 | Me | / | / | 440 |
| **17** | CF₃ | Ph | 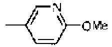 | Me | / | / | 385 |
| **18** | CF₃ | Ph | 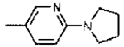 | Me | / | / | 424 |
| **19** | CF₃ | Ph | 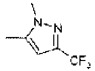 | Me | / | / | 426 |
| **20** | CF₃ | Ph | 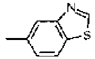 | Me | / | / | 411 |
| **21** | CF₃ | Ph | 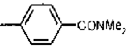 | H | HCl | / | 411 |
| **22** | CF₃ | Ph | 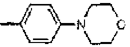 | H | HCl | / | 425 |
| **23** | CF₃ | Ph | 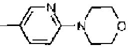 | H | HCl | / | 426 |
| **24** | CF₃ | Ph | 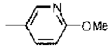 | H | HCl | / | 371 |
| **25** | CF₃ | Ph | 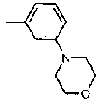 | H | HCl | / | 425 |
| **26** | CF₃ | Ph | 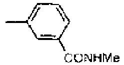 | H | HCl | / | 397 |
| **27** | CF₃ | Ph | 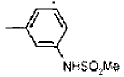 | H | HCl | / | 433 |
| **28** | CF₃ | Ph | 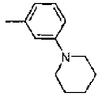 | H | HCl | / | 423 |
| **29** | CF₃ | Ph | 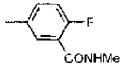 | H | HCl | / | 415 |
| **30** | CF₃ | Ph | 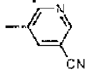 | H | HCl | / | 366 |
| **31** | CHF₂ | Ph | 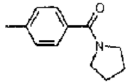 | H | HCl | 248 | 455 |
| **32** | CHF₂ | Ph | 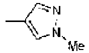 | H | HCl | / | 362 |
| **33** | CHF₂ | Ph | 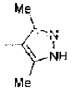 | H | HCl | / | 376 |
| **34** | CHF₂ | Ph | 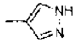 | H | HCl | / | 348 |
| **35** | CHF₂ | Ph | 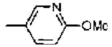 | H | HCl | / | 389 |
| **36** | CHF₂ | Ph | 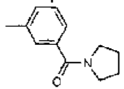 | H | HCl | / | 455 |
| **37** | CHF₂ | Ph | 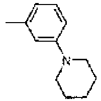 | H | HCl | / | 441 |
| **39** | CONHMe | Ph | 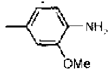 | H | TFA | / | 458 |
| **40** | CONH₂ | Ph | 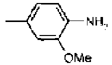 | H | TFA | / | 474 |
| **41** | CONHMe | H | 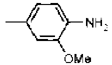 | H | TFA | / | 412 |
| **42** | CONH₂ | H | 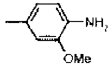 | H | TFA | / | 398 |
| **46 Ex. 1** | CONH₂ | Ph | 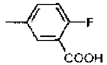 | H | TFA | / | 377 |
| **47** | CONH₂ | Ph | 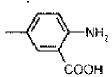 | H | / | / | 374 |
| **48** | CONH₂ | Ph | 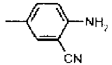 | H | TFA | / | 355 |
| **49** | CONH₂ | 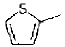 | 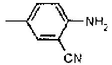 | H | / | / | 361 |
| **51** | CONH₂ | H | 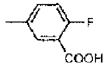 | H | / | / | 301 |
| **52** | CONH₂ | Ph | 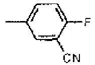 | H | / | / | 358 |
| **53 Ex. 3** | CHF₂ | Ph | 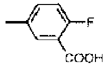 | H | / | / | 384 |
| **54** | CF₃ | Ph | 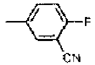 | H | / | / | 383 |
| **55** | CHF₂ | Ph | 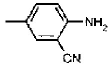 | H | HCl | 282 | 362 |
| **56 Ex. 4** | CF₃ | Ph | 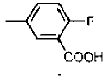 | H | / | / | 402 |
| **57 Ex. 6** | CF₃ | Ph | 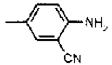 | Me | / | 269 | 394 |
| **58** | CF₃ | Ph | 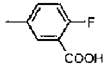 | Me | / | / | 416 |
| **59** | CF₃ | Ph | 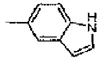 | H | / | / | 379 |
| **60** | CF₃ | Ph | 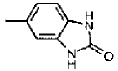 | H | / | 380 | 396 |
| **61 Ex. 5** | CF₃ | Ph | 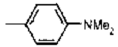 | H | / | 227 | 383 |
| **62** | CONHPh | H | 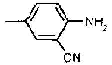 | H | HCl | / | 355 |
| **63** | 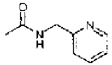 | H | 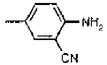 | H | HCl | / | 370 |
| **64** | 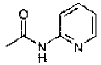 | H | 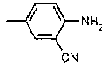 | H | HCl | / | 356 |
| **65** | 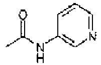 | H | 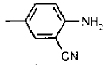 | H | HCl | / | 356 |
| **66** | 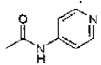 | H | 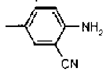 | H | HCl | / | 356 |
| **67** | CHF₂ | 4-Py | 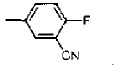 | Me | / | / | 380 |
| **68** | CONHPh | H | 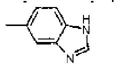 | H | HCl | / | 355 |
| **69** | 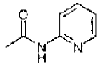 | H | 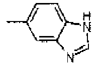 | H | HCl | / | 356 |
| **70** | 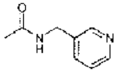 | H | 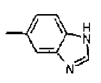 | H | HCl | / | 370 |
| **71** | CONHPh | H | 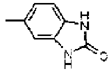 | H | / | / | 371 |
| **72 Ex. 10** | CHF₂ | H | 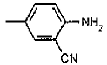 | Me | / | 251 | 300 |
| **73** | CHF₂ | H | 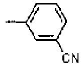 | Me | / | 162 | 285 |
| **74** | CHF₂ | H | 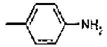 | Me | / | 149 | 275 |
| **75 Ex. 12** | CHF₂ | H | 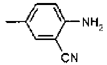 | H | / | 263 | 286 |
| **76** | CF₃ | Ph | 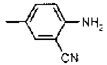 | 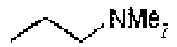 | / | 183 | 451 |
| **77** | CF₃ | Ph | 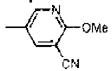 | Me | / | 250 | 410 |
| **78** | CHF₂ | Ph | 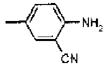 | Me | / | 246 | 376 |
| **79** | CHF₂ | Ph | 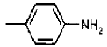 | Me | / | 176 | 351 |
| **80** | CHF₂ | Ph | 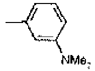 | Me | / | 154 | 379 |
| **81** | CF₃ | Ph | 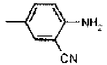 | 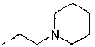 | / | 192 | 491 |
| **82** | CF₃ | Ph | 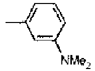 | 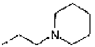 | HCl | 227 | 494 |
| **83 Ex. 13** | CHF₂ | Ph | 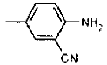 | 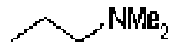 | / | 163 | 433 |
| **84** | CF₃ | H | 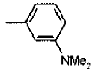 | 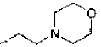 | / | 110 | 420 |
| **85** | CF₃ | H | 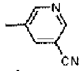 | 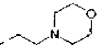 | / | / | 403 |
| **86** | CF₃ | H | 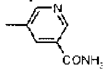 | 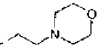 | / | 238 | 421 |
| **87** | CF₃ | H | 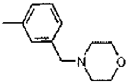 | Me | / | 105 | 377 |
| **88 Ex. 8** | CF₃ | H | 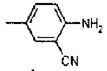 | Me | / | 276 | 318 |
| **89** | CF₃ | Ph | 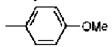 | Me | / | 181 | 384 |
| **90** | CF₃ | H | 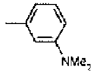 | Me | / | 91 | 321 |
| **91 Ex. 15** | CHF₂ | 3-Py | 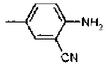 | H | / | 296 | 363 |
| **92** | CHF₂ | 4-Py | 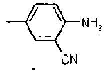 | H | / | 325 | 363 |
| **93 Ex. 14** | CF₃ | 3-Py | 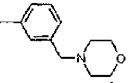 | Me | / | 155 | 454 |
| **94** | CHF₂ | 3MeO-Ph | 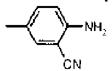 | Me | / | 233 | 392 |
| **95** | CF₃ | H | 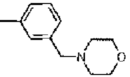 | Pr | HCl | 271 | 405 |
| **96** | CF₃ | H | 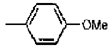 | Pr | / | 72 | 336 |
| **97** | CF₃ | 3MeO-Ph | 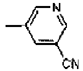 | Me | / | 194 | 410 |
| **98** | CF₃ | 3MeO-Ph | 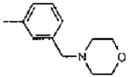 | Me | / | 114 | 483 |
| **99** | CF₃ | 3MeO-Ph | 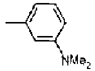 | Me | / | 138 | 427 |
| **100** | CF₃ | 3MeO-Ph | 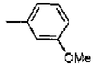 | Me | / | 133 | 414 |
| **101** | CONH₂ | 3-Py | 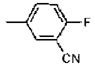 | H | / | / | 377 |

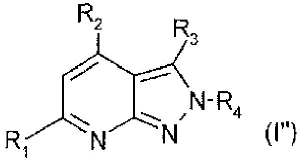
| **N°** | **R₂** | **R₃** | **R₁** | **R₄** | **Sel** | **p.f. (°C)** | **M+H⁺** |
|---|---|---|---|---|---|---|---|
| **103** | CONH₂ | H | 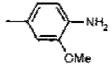 | Me | TFA | / | 412 |
| **105** | CHF₂ | H | 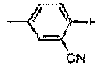 | Me | / | / | 303 |
| **106 Ex. 11** | CHF₂ | H | 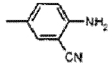 | Me | / | / | 300 |
| **107** | CHF₂ | H | 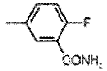 | Me | / | / | 321 |
| **108 Ex. 7** | CF₃ | Ph | 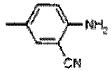 | Me | / | 295 | 394 |
| **109** | CF₃ | Ph | 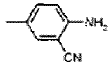 | 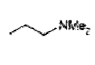 | / | 237 | 451 |
| **110** | CF₃ | Ph | 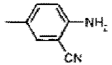 | 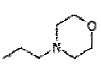 | / | 249 | 493 |
| **111** | CHF₂ | H | 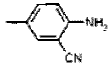 | 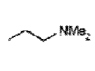 | / | 182 | 357 |
| **112** | CHF₂ | H | 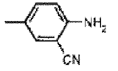 | 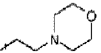 | / | 242 | 399 |
| **113** | CF₃ | H | 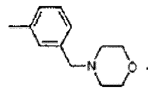 | 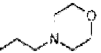 | / | 101 | 476 |
| **114 Ex.9** | CF₃ | H | 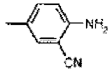 | Me | / | 249 | 318 |
| **115** | CF₃ | H | 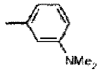 | Pr | HCl | 181 | 425 |
| **116** | CHF₂ | H | 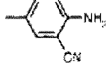 | 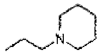 | / | 230 | 397 |
| **117** | CHF₂ | H | 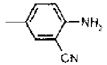 | Pr | / | 214 | 328 |
| **118** | CF₃ | H | 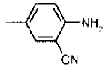 | Pr | / | 239 | 346 |
| **119** | CF₃ | H | 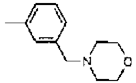 | Pr | / | 288 | 405 |
| **120** | CF₃ | H | 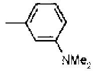 | Pr | / | 89 | 349 |
et en outre, au moins un excipient pharmaceutiquement acceptable.

13. Utilisation d'un composé de formule (I) 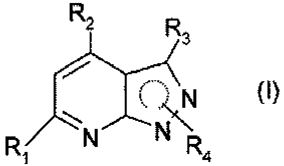 dans lequel :
la représentation du cycle pyrazole indique que le substituant R₄ peut être porté par l'azote alpha par rapport au cycle pyridine (I') ou par l'azote alpha par rapport au carbone portant un substituant R₃ (I") de sorte que : 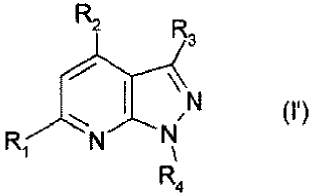 soit 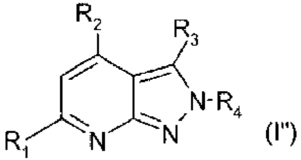
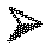 R₁ représente un groupe aryle, pyridyle ou pyrazolyle facultativement substitué par un ou plusieurs substituants choisis parmi :
∘ un atome d'halogène,
∘ un groupe -CF₃,
∘ un groupe cyano,
∘ un groupe -NR₆R₆' dans lequel R₆ et R₆' sont tels que définis ci-dessous,
∘ un groupe -NR₁₀R₁₁ tel que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène, facultativement substitués par un ou plusieurs substituants choisis parmi un atome d'halogène et un groupe alkyle linéaire ou ramifié,
∘ un groupe -CH₂NR₁₀R₁₁ tel que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène,
∘ un groupe -COR₁₂ dans lequel R₁₂ représente un groupe hydroxyle ou un groupe -NR₆R₆', dans lequel R₆ et R₆' sont tels que définis ci-dessous,
∘ un groupe -CONR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
∘ un groupe -(CH₂)ₚNHSO₂CH₃ dans lequel p représente 0 ou 1,
∘ un groupe -OR₁₃ dans lequel R₁₃ représente un groupe alkyle en C₁-C₃ linéaire,
∘ un groupe alkyle en C₁-C₃,
ou R₁ représente un groupe bicyclique de formule A ci-dessous : 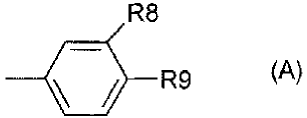 dans lequel R₈ et R₉ forment, conjointement avec les atomes de carbone auxquels ils sont liés, un hétérocycle saturé ou insaturé comprenant un ou plusieurs hétéroatomes choisis parmi un atome d'azote, un atome d'oxygène et un atome de soufre, facultativement substitués par un ou plusieurs groupes alkyle linéaires,
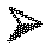 R₂ représente un groupe :
∘ -CF₃,
∘ -CHF₂,
∘ -COOH,
ou
∘ -CONHR₅, dans lequel R₅ est tel que défini ci-dessous,
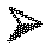 R₃ représente :
∘ un atome d'hydrogène,
∘ un groupe aryle, facultativement substitué par un groupe alcoxyméthyle,
∘ un groupe cycloalkyle,
ou
∘ un groupe hétéroaryle choisi parmi des groupes thiényle et pyridyle,
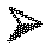 R₄ représente :
∘ un atome d'hydrogène,
∘ un groupe alkyle en C₁-C₃ linéaire, facultativement substitué par un groupe -NR₆R₆', dans lequel R₆ et R₆' sont tels que définis ci-dessous ou un groupe -NR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
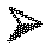 R₅ représente :
∘ un atome d'hydrogène,
∘ un groupe alkyle en C₁-C₃ linéaire, facultativement substitué par un groupe pyridyle,
ou
∘ un groupe aromatique choisi parmi aryle et pyridyle,
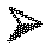 R₆ et R₆', qui peuvent être identiques ou différents, représentent un atome d'hydrogène ou un groupe alkyle linéaire,
sous la forme de la base ou d'un sel d'addition d'acide ou d'addition de base
pour la préparation d'un médicament pour traiter et prévenir des maladies nécessitant une modulation des b-FGF.

14. Combinaison d'un composé 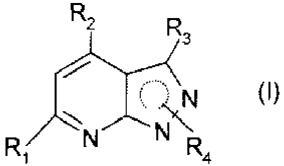 dans lequel :
la représentation du cycle pyrazole indique que le substituant R₄ peut être porté par l'azote alpha par rapport au cycle pyridine (I') ou par l'azote alpha par rapport au carbone portant un substituant R₃ (I") de sorte que : 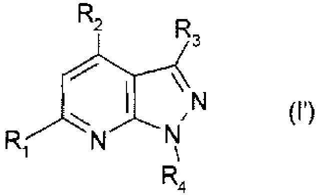 soit 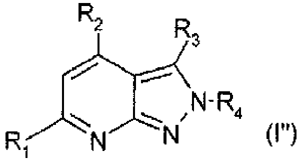
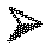 R₁ représente un groupe aryle, pyridyle ou pyrazolyle facultativement substitué par un ou plusieurs substituants choisis parmi :
∘ un atome d'halogène,
∘ un groupe -CF₃,
∘ un groupe cyano,
∘ un groupe -NR₆R₆' dans lequel R₆ et R₆' sont tels que définis ci-dessous,
o un groupe -NR₁₀R₁₁ tel que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène, facultativement substitués par un ou plusieurs substituants choisis parmi un atome d'halogène et un groupe alkyle linéaire ou ramifié,
o un groupe -CH₂NR₁₀R₁₁ tel que R₁₀ et R₁₁, forment, conjointement avec l'atome d'azote auquel ils sont liés, un hétérocycle saturé ou insaturé comprenant un ou plusieurs hétéroatomes choisis parmi un atome d'azote et un atome d'oxygène,
∘ un groupe -COR₁₂ dans lequel R₁₂ représente un groupe hydroxyle ou un groupe -NR₆R₆' , dans lequel R₆ et R₆' sont tels que définis ci-dessous,
∘ un groupe -CONR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
∘ un groupe -(CH₂)ₚNHSO₂CH₃ dans lequel p représente 0 ou 1,
∘ un groupe -OR₁₃ dans lequel R₁₃ représente un groupe alkyle en C₁-C₃ linéaire,
∘ un groupe alkyle en C₁-C₃,
ou R₁ représente un groupe bicyclique de formule A ci-dessous : 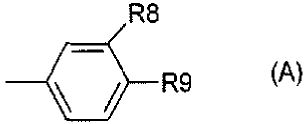 dans lequel R₈ et R₉ forment, conjointement avec les atomes de carbone auxquels ils sont liés, un hétérocycle saturé ou insaturé comprenant un ou plusieurs hétéroatomes choisis parmi un atome d'azote, un atome d'oxygène et un atome de soufre, facultativement substitués par un ou plusieurs groupes alkyle linéaires,
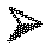 R₂ représente un groupe :
∘ -CF₃,
∘ -CHF₂,
∘ -COOH,
ou
∘ -CONHR₅, dans lequel R₅ est tel que défini ci-dessous,
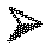 R₃ représente :
∘ un atome d'hydrogène,
∘ un groupe aryle, facultativement substitué par un groupe alcoxyméthyle,
∘ un groupe cycloalkyle,
ou
∘ un groupe hétéroaryle choisi parmi des groupes thiényle et pyridyle,
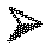 R₄ représente :
∘ un atome d'hydrogène,
∘ un groupe alkyle en C₁-C₃ linéaire, facultativement substitué par un groupe -NR₆R₆' dans lequel R₆ et R₆' sont tels que définis ci-dessous ou un groupe -NR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
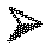 R₅ représente :
∘ un atome d'hydrogène,
∘ un groupe alkyle en C₁-C₃ linéaire, facultativement substitué par un groupe pyridyle,
ou
∘ un groupe aromatique choisi parmi aryle et pyridyle,
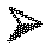 R₆ et R₆', qui peuvent être identiques ou différents, représentent un atome d'hydrogène ou un groupe alkyle linéaire,
sous la forme de la base ou d'un sel d'addition d'acide ou d'addition de base
avec une ou plusieurs substances actives anticancéreuses et/ou avec un anti-VEGF quelconque.

15. Composé 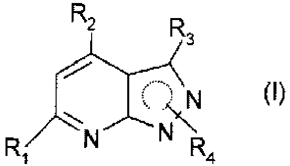 dans lequel :
la représentation du cycle pyrazole indique que le substituant R₄ peut être porté par l'azote alpha par rapport au cycle pyridine (I') ou par l'azote alpha par rapport au carbone portant un substituant R₃ (I") de sorte que : 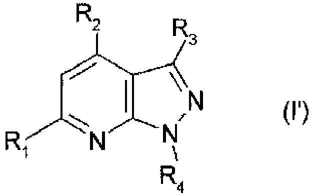 soit 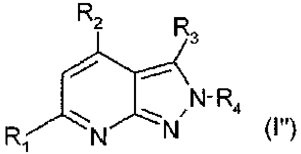
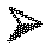 R₁ représente un groupe aryle, pyridyle ou pyrazolyle facultativement substitué par un ou plusieurs substituants choisis parmi :
∘ un atome d'halogène,
∘ un groupe -CF₃,
∘ un groupe cyano,
∘ un groupe -NR₆R₆' dans lequel R₆ et R₆' sont tels que définis ci-dessous,
∘ un groupe -NR₁₀R₁₁ tel que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène, facultativement substitués par un ou plusieurs substituants choisis parmi un atome d'halogène et un groupe alkyle linéaire ou ramifié,
o un groupe -CH₂NR₁₀R₁₁ tel que R₁₀ et R₁₁, forment, conjointement avec l'atome d'azote auquel ils sont liés, un hétérocycle saturé ou insaturé comprenant un ou plusieurs hétéroatomes choisis parmi un atome d'azote et un atome d'oxygène,
∘ un groupe -COR₁₂ dans lequel R₁₂ représente un groupe hydroxyle ou un groupe -NR₆R₆' , dans lequel R₆ et R₆' sont tels que définis ci-dessous,
∘ un groupe -CONR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
∘ un groupe -(CH₂)ₚNHSO₂CH₃ dans lequel p représente 0 ou 1,
∘ un groupe -OR₁₃ dans lequel R₁₃ représente un groupe alkyle en C₁-C₃ linéaire,
∘ un groupe alkyle en C₁-C₃,
ou R₁ représente un groupe bicyclique de formule A ci-dessous : 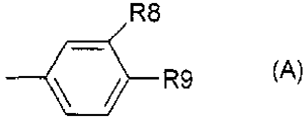 dans lequel R₈ et R₉ forment, conjointement avec les atomes de carbone auxquels ils sont liés, un hétérocycle saturé ou insaturé comprenant un ou plusieurs hétéroatomes choisis parmi un atome d'azote, un atome d'oxygène et un atome de soufre, facultativement substitués par un ou plusieurs groupes alkyle linéaires,
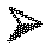 R₂ représente un groupe :
∘ -CF₃,
∘ -CHF₂,
∘ -COOH,
ou
∘ -CONHR₅, dans lequel R₅ est tel que défini ci-dessous,
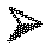 R₃ représente :
∘ un atome d'hydrogène,
∘ un groupe aryle, facultativement substitué par un groupe alcoxyméthyle,
∘ un groupe cycloalkyle,
ou
∘ un groupe hétéroaryle choisi parmi des groupes thiényle et pyridyle,
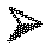 R₄ représente :
∘ un atome d'hydrogène,
∘ un groupe alkyle en C₁-C₃ linéaire, facultativement substitué par un groupe -NR₆R₆' dans lequel R₆ et R₆' sont tels que définis ci-dessous ou un groupe -NR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
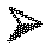 R₅ représente :
∘ un atome d'hydrogène,
∘ un groupe alkyle en C₁-C₃ linéaire, facultativement substitué par un groupe pyridyle,
ou
∘ un groupe aromatique choisi parmi aryle et pyridyle,
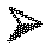 R₆ et R₆', qui peuvent être identiques ou différents, représentent un atome d'hydrogène ou un groupe alkyle linéaire,
sous la forme de la base ou d'un sel d'addition d'acide ou d'addition de base,
pour son utilisation dans le traitement et la prévention de cancers, en particulier de carcinomes ayant un degré élevé de vascularisation tels que des carcinomes du poumon, du sein, de la prostate, du pancréas, de l'intestin, du rein et de l'oesophage, des cancers qui induisent des métastases, tels que le cancer de l'intestin, le cancer du foie et le cancer de l'estomac, des mélanomes, des gliomes, des lymphomes et des leucémies, ainsi que des thrombopénies.

16. Composé 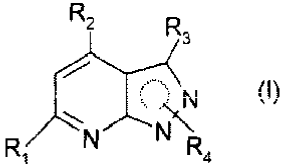 dans lequel :
la représentation du cycle pyrazole indique que le substituant R₄ peut être porté par l'azote alpha par rapport au cycle pyridine (I') ou par l'azote alpha par rapport au carbone portant un substituant R₃ (I") de sorte que : 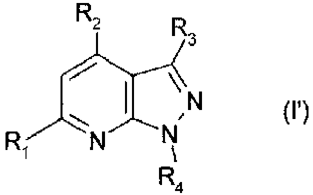 soit 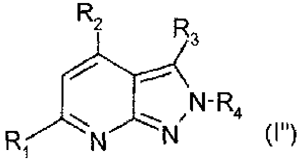
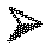 R₁ représente un groupe aryle, pyridyle ou pyrazolyle facultativement substitué par un ou plusieurs substituants choisis parmi :
∘ un atome d'halogène,
∘ un groupe -CF₃,
∘ un groupe cyano,
∘ un groupe -NR₆R₆' dans lequel R₆ et R₆' sont tels que définis ci-dessous,
∘ un groupe -NR₁₀R₁₁ tel que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène, facultativement substitués par un ou plusieurs substituants choisis parmi un atome d'halogène et un groupe alkyle linéaire ou ramifié,
∘ un groupe -CH₂NR₁₀R₁₁ tel que R₁₀ et R₁₁, forment, conjointement avec l'atome d'azote auquel ils sont liés, un hétérocycle saturé ou insaturé comprenant un ou plusieurs hétéroatomes choisis parmi un atome d'azote et un atome d'oxygène,
∘ un groupe -COR₁₂ dans lequel R₁₂ représente un groupe hydroxyle ou un groupe -NR₆R₆', dans lequel R₆ et R₆' sont tels que définis ci-dessous,
∘ un groupe -CONR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
∘ un groupe -(CH₂)ₚNHSO₂CH₃ dans lequel p représente 0 ou 1,
∘ un groupe -OR₁₃ dans lequel R₁₃ représente un groupe alkyle en C₁-C₃ linéaire,
∘ un groupe alkyle en C₁-C₃,
ou R₁ représente un groupe bicyclique de formule A ci-dessous : 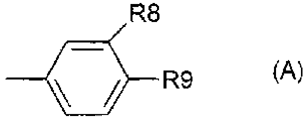 dans lequel R₈ et R₉ forment, conjointement avec les atomes de carbone auxquels ils sont liés, un hétérocycle saturé ou insaturé comprenant un ou plusieurs hétéroatomes choisis parmi un atome d'azote, un atome d'oxygène et un atome de soufre, facultativement substitués par un ou plusieurs groupes alkyle linéaires,
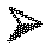 R₂ représente un groupe :
∘ -CF₃,
∘ -CHF₂,
∘ -COOH,
ou
∘ -CONHR₅, dans lequel R₅ est tel que défini ci-dessous,
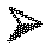 R₃ représente :
∘ un atome d'hydrogène,
∘ un groupe aryle, facultativement substitué par un groupe alcoxyméthyle,
∘ un groupe cycloalkyle,
ou
∘ un groupe hétéroaryle choisi parmi des groupes thiényle et pyridyle,
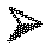 R₄ représente :
∘ un atome d'hydrogène,
∘ un groupe alkyle en C₁-C₃ linéaire, facultativement substitué par un groupe -NR₆R₆' dans lequel R₆ et R₆' sont tels que définis ci-dessous ou un groupe -NR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
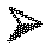 R₅ représente :
∘ un atome d'hydrogène,
∘ un groupe alkyle en C₁-C₃ linéaire, facultativement substitué par un groupe pyridyle,
ou
∘ un groupe aromatique choisi parmi aryle et pyridyle,
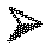 R₆ et R₆', qui peuvent être identiques ou différents, représentent un atome d'hydrogène ou un groupe alkyle linéaire,
sous la forme de la base ou d'un sel d'addition d'acide ou d'addition de base
pour son utilisation dans le traitement et la prévention de cancers, en particulier de carcinomes ayant un degré élevé de vascularisation tels que des carcinomes du poumon, du sein, de la prostate, du pancréas, de l'intestin, du rein et de l'oesophage, des cancers qui induisent des métastases, tels que le cancer de l'intestin, le cancer du foie et le cancer de l'estomac, des mélanomes, des gliomes, des lymphomes et des leucémies, ainsi que des thrombopénies,
ledit composé de formule (I) étant administré en combinaison avec une ou plusieurs substances actives anticancéreuses et/ou avec un anti-VEGF quelconque et/ou étant administré en combinaison avec une radiothérapie.

17. Composé 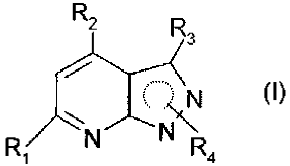 dans lequel :
la représentation du cycle pyrazole indique que le substituant R₄ peut être porté par l'azote alpha par rapport au cycle pyridine (I') ou par l'azote alpha par rapport au carbone portant un substituant R₃ (I") de sorte que : 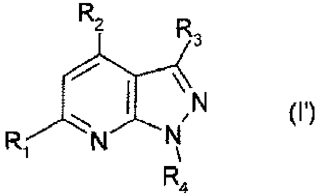 soit 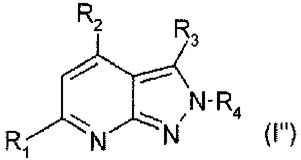
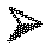 R₁ représente un groupe aryle, pyridyle ou pyrazolyle facultativement substitué par un ou plusieurs substituants choisis parmi :
∘ un atome d'halogène,
∘ un groupe -CF₃,
∘ un groupe cyano,
∘ un groupe -NR₆R₆' dans lequel R₆ et R₆' sont tels que définis ci-dessous,
o un groupe -NR₁₀R₁₁ tel que R₁₀ et R₁₁ forment, conjointement avec l'atome d'azote auquel ils sont liés, un hétérocycle saturé ou insaturé comprenant un ou plusieurs hétéroatomes choisis parmi un atome d'azote et un atome d'oxygène, facultativement substitués par un ou plusieurs substituants choisis parmi un atome d'halogène et un groupe alkyle linéaire ou ramifié,
o un groupe -CH₂NR₁₀R₁₁ tel que R₁₀ et R₁₁, forment, conjointement avec l'atome d'azote auquel ils sont liés, un hétérocycle saturé ou insaturé comprenant un ou plusieurs hétéroatomes choisis parmi un atome d'azote et un atome d'oxygène,
∘ un groupe -COR₁₂ dans lequel R₁₂ représente un groupe hydroxyle ou un groupe -NR₆R₆', dans lequel R₆ et R₆' sont tels que définis ci-dessous,
∘ un groupe -CONR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
∘ un groupe -(CH₂)ₚNHSO₂CH₃ dans lequel p représente 0 ou 1,
∘ un groupe -OR₁₃ dans lequel R₁₃ représente un groupe alkyle en C₁-C₃ linéaire,
∘ un groupe alkyle en C₁-C₃,
ou R₁ représente un groupe bicyclique de formule A ci-dessous : 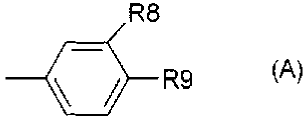 dans lequel R₈ et R₉ forment, conjointement avec les atomes de carbone auxquels ils sont liés, un hétérocycle saturé ou insaturé comprenant un ou plusieurs hétéroatomes choisis parmi un atome d'azote, un atome d'oxygène et un atome de soufre, facultativement substitués par un ou plusieurs groupes alkyle linéaires,
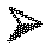 R₂ représente un groupe :
∘ -CF₃,
∘ -CHF₂,
∘ -COOH,
ou
∘ -CONHR₅, dans lequel R₅ est tel que défini ci-dessous,
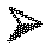 R₃ représente :
∘ un atome d'hydrogène,
∘ un groupe aryle, facultativement substitué par un groupe alcoxyméthyle,
∘ un groupe cycloalkyle,
ou
∘ un groupe hétéroaryle choisi parmi des groupes thiényle et pyridyle,
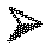 R₄ représente :
∘ un atome d'hydrogène,
∘ un groupe alkyle en C₁-C₃ linéaire, facultativement substitué par un groupe -NR₆R₆' dans lequel R₆ et R₆' sont tels que définis ci-dessous ou un groupe -NR₇R₇' tel que R₇ et R₇' forment, conjointement avec l'atome d'azote auquel ils sont liés, un hétérocycloalkyle comprenant un ou plusieurs hétéroatomes choisis parmi un atome d'azote et un atome d'oxygène,
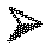 R₅ représente :
∘ un atome d'hydrogène,
∘ un groupe alkyle en C₁-C₃ linéaire, facultativement substitué par un groupe pyridyle,
ou
∘ un groupe aromatique choisi parmi aryle et pyridyle,
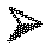 R₆ et R₆' qui peuvent être identiques ou différents, représentent un atome d'hydrogène ou un groupe alkyle linéaire,
sous la forme de la base ou d'un sel d'addition d'acide ou d'addition de base
pour son utilisation dans le traitement et la prévention de maladies cardiovasculaires telles que l'athérosclérose, une resténose post-angioplastie, des maladies associées à des complications survenant après l'insertion de prothèses endovasculaires et/ou pontages aorto-coronariens ou d'autres greffes vasculaires, l'hypotrophie cardiaque, des complications vasculaires du diabète telles que la rétinopathie diabétique, les fibroses hépatique, rénale et pulmonaire, une douleur neuropathique, des maladies inflammatoires chroniques telles que la polyarthrite rhumatoïde ou une maladie inflammatoire intestinale, une hyperplasie de la prostate, le psoriasis, un acanthome à cellules claires, l'arthrose, les achondroplasies (ACH), les hypochondroplasies (HCH), la dysplasie thanatophore (DT), l'obésité et la dégénérescence maculaire, telle que la dégénérescence maculaire liée à l'âge (DMLA).

18. Composé pour son utilisation selon l'une quelconque des revendications 15 à 17, ledit composé étant choisi parmi les composés suivants : 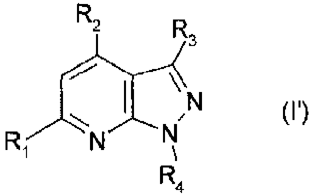
| **N°** | **R₂** | **R₃** | **R₁** | **R₄** | **Sel** | **p.f. (°C)** | **M+H⁺** |
|---|---|---|---|---|---|---|---|
| **1** | CF₃ | Ph | 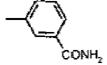 | Me | / | / | 397 |
| **2** | CF₃ | Ph | 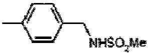 | Me | / | / | 461 |
| **3** | CF₃ | Ph | 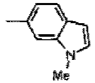 | Me | / | / | 407 |
| **4** | CF₃ | Ph | 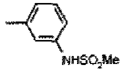 | Me | / | / | 447 |
| **5** | CF₃ | Ph | 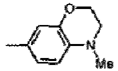 | Me | / | / | 426 |
| **6** | CF₃ | Ph | 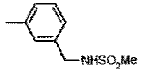 | Me | / | / | 461 |
| **7** | CF₃ | Ph | 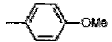 | Me | / | / | 384 |
| **8** | CF₃ | Ph | 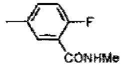 | Me | / | / | 429 |
| **9** | CF₃ | Ph | 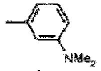 | Me | / | / | 397 |
| **10** | CF₃ | Ph | 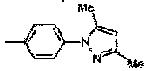 | Me | / | / | 448 |
| **11** | CF₃ | Ph | 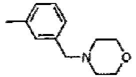 | Me | / | / | 453 |
| **12** | CF₃ | Ph | 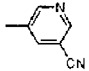 | Me | / | / | 380 |
| **13** | CF₃ | Ph | 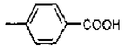 | Me | / | / | 398 |
| **14** | CF₃ | Ph | 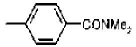 | Me | / | / | 425 |
| **15** | CF₃ | Ph | 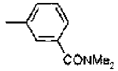 | Me | / | / | 425 |
| **16** | CF₃ | Ph | 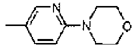 | Me | / | / | 440 |
| **17** | CF₃ | Ph | 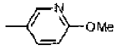 | Me | / | / | 385 |
| **18** | CF₃ | Ph | 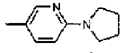 | Me | / | / | 424 |
| **19** | CF₃ | Ph | 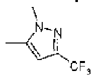 | Me | / | / | 426 |
| **20** | CF₃ | Ph | 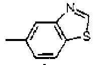 | Me | / | / | 411 |
| **21** | CF₃ | Ph | 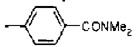 | H | HCl | / | 41 1 |
| **22** | CF₃ | Ph | 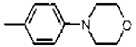 | H | HCl | / | 425 |
| **23** | CF₃ | Ph | 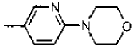 | H | HCl | / | 426 |
| **24** | CF₃ | Ph | 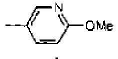 | H | HCl | / | 371 |
| **25** | CF₃ | Ph | 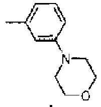 | H | HCl | / | 425 |
| **26** | CF₃ | Ph | 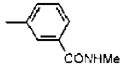 | H | HCl | / | 397 |
| **27** | CF₃ | Ph | 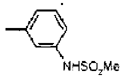 | H | HCl | / | 433 |
| **28** | CF₃ | Ph | 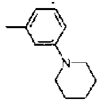 | H | HCl | / | 423 |
| **29** | CF₃ | Ph | 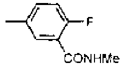 | H | HCl | / | 415 |
| **30** | CF₃ | Ph | 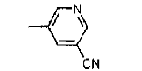 | H | HCl | / | 366 |
| **31** | CHF₂ | Ph | 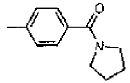 | H | HCl | 248 | 455 |
| **32** | CHF₂ | Ph | 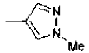 | H | HCl | / | 362 |
| **33** | CHF₂ | Ph | 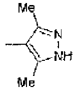 | H | HCl | / | 376 |
| **34** | CHF₂ | Ph | 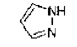 | H | HCl | / | 348 |
| **35** | CHF₂ | Ph | 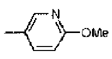 | H | HCl | / | 389 |
| **36** | CHF₂ | Ph | 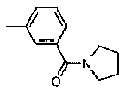 | H | HCl | / | 455 |
| **37** | CHF₂ | Ph | 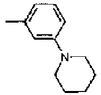 | H | HCl | / | 441 |
| **38 Ex. 2** | COOH | H | 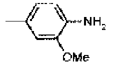 | H | / | / | 285 |
| **39** | CONHMe | Ph | 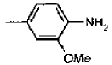 | H | TFA | / | 458 |
| **40** | CONH₂ | Ph | 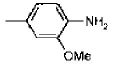 | H | TFA | / | 474 |
| **41** | CONHMe | H | 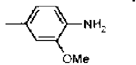 | H | TFA | / | 412 |
| **42** | CONH₂ | H | 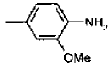 | H | TFA | / | 398 |
| **43** | COOH | Ph | 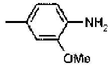 | H | / | / | 361 |
| **44** | COOH | Ph | 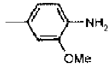 | Me | / | / | 375 |
| **45** | COOH | H | 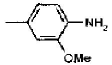 | Me | / | / | 299 |
| **46 Ex. 1** | CONH₂ | Ph | 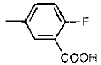 | H | TFA | / | 377 |
| **47** | CONH₂ | Ph | 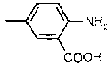 | H | / | / | 374 |
| **48** | CONH₂ | Ph | 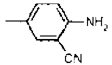 | H | TFA | / | 355 |
| **49** | CONH₂ | 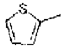 | 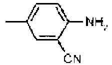 | H | / | / | 361 |
| **50** | COOH | cPr | 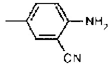 | H | / | / | 320 |
| **51** | CONH₂ | H | 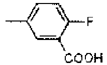 | H | / | / | 301 |
| **52** | CONH₂ | Ph | 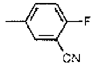 | H | / | / | 358 |
| **53 Ex. 3** | CHF₂ | Ph | 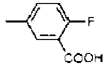 | H | / | / | 384 |
| **54** | CF₃ | Ph | 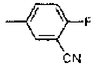 | H | / | / | 383 |
| **55** | CHF₂ | Ph | 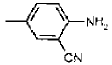 | H | HCl | 282 | 362 |
| **56 Ex. 4** | CF₃ | Ph | 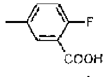 | H | / | / | 402 |
| **57 Ex. 6** | CF₃ | Ph | 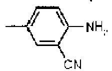 | Me | / | 269 | 394 |
| **58** | CF₃ | Ph | 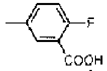 | Me | / | / | 416 |
| **59** | CF₃ | Ph | 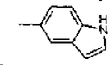 | H | / | / | 379 |
| **60** | CF₃ | Ph | 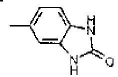 | H | / | 380 | 396 |
| **61 Ex. 5** | CF₃ | Ph | 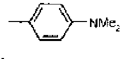 | H | / | 227 | 383 |
| **62** | CONHPh | H | 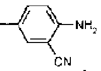 | H | HCl | / | 355 |
| **63** | 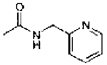 | H | 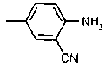 | H | HCl | / | 370 |
| **64** | 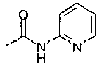 | H | 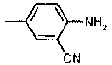 | H | HCl | / | 356 |
| **65** | 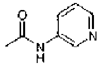 | H | 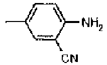 | H | HCl | / | 356 |
| **66** | 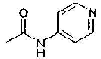 | H | 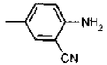 | H | HCl | / | 356 |
| **67** | CHF₂ | 4-Py | 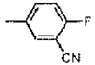 | Me | / | / | 380 |
| **68** | CONHPh | H | 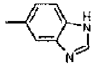 | H | HCl | / | 355 |
| **69** | 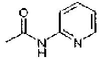 | H | 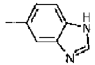 | H | HCl | / | 356 |
| **70** | 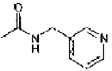 | H | 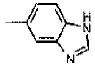 | H | HCl | / | 370 |
| **71** | CONHPh | H | 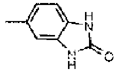 | H | / | / | 371 |
| **72 Ex. 10** | CHF₂ | H | 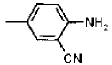 | Me | / | 251 | 300 |
| **73** | CHF₂ | H | 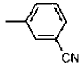 | Me | / | 162 | 285 |
| **74** | CHF₂ | H | 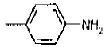 | Me | / | 149 | 275 |
| **75 Ex. 12** | CHF₂ | H | 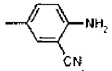 | H | / | 263 | 286 |
| **76** | CF₃ | Ph | 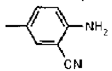 | 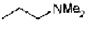 | / | 183 | 451 |
| **77** | CF₃ | Ph | 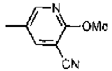 | Me | / | 250 | 410 |
| **78** | CHF₂ | Ph | 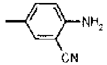 | Me | / | 246 | 376 |
| **79** | CHF₂ | Ph | 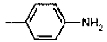 | Me | / | 176 | 351 |
| **80** | CHF₂ | Ph | 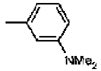 | Me | / | 154 | 379 |
| **81** | CF₃ | Ph | 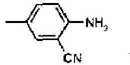 | 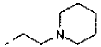 | / | 192 | 491 |
| **82** | CF₃ | Ph | 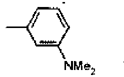 | 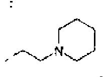 | HCl | 227 | 494 |
| **83 Ex. 13** | CHF₂ | Ph | 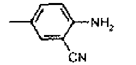 | 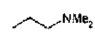 | / | 163 | 433 |
| **84** | CF₃ | H | 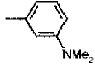 | 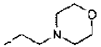 | / | 110 | 420 |
| **85** | CF₃ | H | 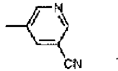 | 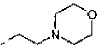 | / | / | 403 |
| **86** | CF₃ | H | 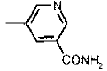 | 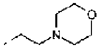 | / | 238 | 421 |
| **87** | CF₃ | H | 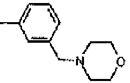 | Me | / | 105 | 377 |
| **88 Ex. 8** | CF₃ | H | 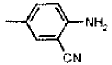 | Me | / | 276 | 31 8 |
| **89** | CF₃ | Ph | 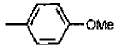 | Me | / | 181 | 384 |
| **90** | CF₃ | H | 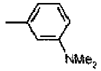 | Me | / | 91 | 321 |
| **91 Ex. 15** | CHF₂ | 3-Py | 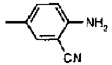 | H | / | 296 | 363 |
| **92** | CHF₂ | 4-Py | 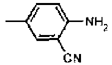 | H | / | 325 | 363 |
| **93 Ex. 14** | CF₃ | 3-Py | 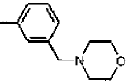 | Me | / | 155 | 454 |
| **94** | CHF₂ | 3MeO-Ph | 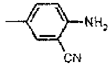 | Me | / | 233 | 392 |
| **95** | CF₃ | H | 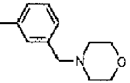 | Pr | HCl | 271 | 405 |
| **96** | CF₃ | H | 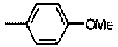 | Pr | / | 72 | 336 |
| **97** | CF₃ | 3MeO-Ph | 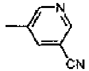 | Me | / | 194 | 410 |
| **98** | CF₃ | 3MeO-Ph | 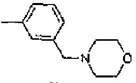 | Me | / | 114 | 483 |
| **99** | CF₃ | 3MeO-Ph | 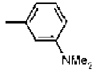 | Me | / | 138 | 427 |
| **100** | CF₃ | 3MeO-Ph | 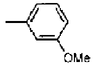 | Me | / | 133 | 414 |
| **101** | CONH₂ | 3-Py | 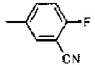 | H | / | / | 377 |

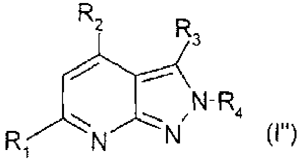
| **N°** | **R₂** | **R₃** | **R₁** | **R₄** | **Sel** | **p.f.(°C)** | **M+H⁺** |
|---|---|---|---|---|---|---|---|
| **102** | COOH | H | 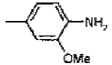 | Me | / | / | 299 |
| **103** | CONH₂ | H | 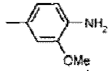 | Me | TFA | / | 412 |
| **104** | COOH | H | 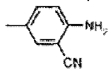 | Me | / | / | 294 |
| **105** | CHF₂ | H | 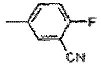 | Me | / | / | 303 |
| **106 Ex. 11** | CHF₂ | H | 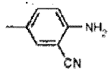 | Me | / | / | 300 |
| **107** | CHF₂ | H | 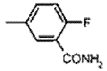 | Me | / | / | 321 |
| **108 Ex. 7** | CF₃ | Ph | 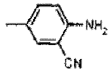 | Me | / | 295 | 394 |
| **109** | CF₃ | Ph | 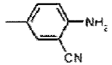 | 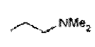 | / | 237 | 451 |
| **110** | CF₃ | Ph | 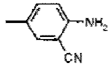 | 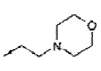 | / | 249 | 493 |
| **111** | CHF₂ | H | 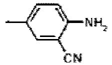 | 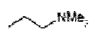 | / | 182 | 357 |
| **112** | CHF₂ | H | 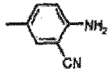 | 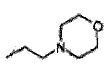 | | 242 | 399 |
| **113** | CF₃ | H | 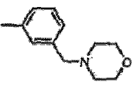 | 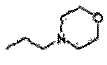 | / | 101 | 476 |
| **114 Ex. 9** | CF₃ | H | 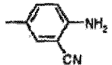 | Me | / | 249 | 318 |
| | | | | | | | |
| **115** | CF₃ | H | 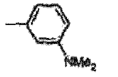 | Pr | HCl | 181 | 425 |
| **116** | CHF₂ | H | 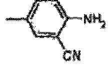 | 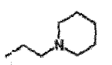 | / | 230 | 397 |
| **117** | CHF₂ | H | 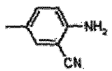 | Pr | / | 214 | 328 |
| **118** | CF₃ | H | 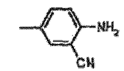 | Pr | / | 239 | 346 |
| **119** | CF₃ | H | 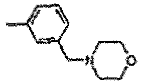 | Pr | / | 288 | 405 |
| **120** | CF₃ | H | 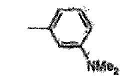 1 | Pr | / | 89 | 349 |
